# EUROPEAN PATENT APPLICATION

(11) **EP 4 843 990 A1**
(43) Date of publication of application: **25.11.2026**
(21) Application number: 25741469.8
(22) Date of filing: 13.01.2025
(51) Int. Cl.: C07D 498/22, C07D 471/22, A61K 31/529, A61P 35/00

(54) **AMINO HETEROARYL MACROCYCLIC COMPOUNDS AS WELL AS PREPARATION METHOD THEREFOR AND USE THEREOF**

(30) Priority: 19.01.2024 CN 202410082850; 01.04.2024 CN 202410390203; 10.08.2024 CN 202411094474
(71) Applicant: Beijing Avistone Biotechnology Co., Ltd., Beijing 100102 (CN)
(72) Inventor: LI, Gong, Beijing 100102 (CN); SHI, Hepeng, Beijing 100102 (CN); ZHANG, Peilong, Beijing 100102 (CN); LI, Xiangqiu, Beijing 100102 (CN)
(74) Representative: IK-IP LTD
(86) International application number: PCT/CN2025/072126
(87) International publication number: WO 2025/152908

(57) **Abstract**

Amino heteroaryl macrocyclic compounds, a preparation method therefor and the use thereof. The structure of the amino heteroaryl macrocyclic compounds is shown as formula I: (I), wherein the definitions of A, M, E, X1, X2, L2, R1, R2, R3, R4, m, n and t are as described in the description. The amino heteroaryl macrocyclic compounds have efficient and highly selective inhibitory effects on epidermal growth factor receptors (EGFRs), and thus can be used for treating related diseases induced by EGFR anomalies, especially in the treatment of cancer diseases.

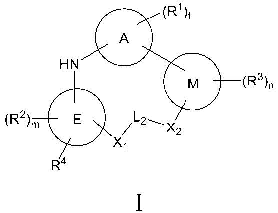

## Description

### FIELD OF THE INVENTION

The present invention belongs to the technical field of chemical drug preparation, specifically relates to amino heteroaryl macrocyclic compounds and pharmaceutical compositions thereof, as well as to the use of such compounds and pharmaceutical compositions thereof in the treatment of protein kinase-mediated diseases such as cancer.

### BACKGROUND

Protein kinases are enzymatic components of signal transduction pathways that catalyze the transfer of the terminal phosphate from ATP to the hydroxyl of tyrosine, serine and/or threonine residues of proteins. Therefore, compounds that inhibit the function of protein kinases (i.e., protein kinase inhibitors) are valuable tools for assessing the physiological consequences of protein kinase activity. Although protein kinase inhibitors have achieved great success in targeted cancer therapy, secondary mutations in the kinase domain occur during treatment, leading to drug resistance. Consequently, there is an unmet clinical need for protein kinase inhibitors that are active against both primary driver gene mutations and secondary drug-resistant mutations.

EGFR is a member of the receptor tyrosine kinase ErbB receptor family. When EGFR binds to its ligand extracellularly, the receptor undergoes homodimerization or heterodimerization and autophosphorylation, activating downstream cascade signaling pathways, ultimately promoting cell growth, proliferation, and division. Overexpression (upregulation) or overactivity (amplification or mutation) of EGFR has been confirmed to be associated with many cancers, including head and neck cancer, ovarian cancer, cervical cancer, bladder cancer, esophageal cancer, gastric cancer, breast cancer, endometrial cancer, colorectal cancer, non-small cell lung cancer, and glioblastoma.

As an oncogene, EGFR has given rise to targeted therapy with small molecule inhibitors directed against EGFR. In non-small cell lung cancer (NSCLC), 50%-80% of patients are caused by EGFR mutations (mainly exon 19 in-frame deletion mutation Ex19De1 and exon 21 L858R mutation where lysine at position 858 is replaced by arginine, both also referred to as EGFR sensitive mutations). Currently, three generations of EGFR small molecule inhibitors have been approved for the treatment of NSCLC patients with EGFR mutations. First-generation and second-generation EGFR inhibitors such as Iressa, Tarceva, Afatinib, Dacomitinib, etc., have achieved great success in the treatment of NSCLC. However, they exhibit clinically significant side effects such as rash and diarrhea, and patients develop drug resistance after about one year of treatment. Clinical data show that approximately 50% of patients with acquired resistance result from the emergence of the T790M (exon 20 T790M mutation where threonine at position 790 is replaced by methionine) mutant.

Third-generation EGFR inhibitors such as Osimertinib are irreversible inhibitors that can covalently bind to C797, significantly enhancing activity. They can be used as second-line treatment for patients with EGFR T790M mutation after progression on first- and second-generation EGFR inhibitors, and also as first-line treatment for patients with EGFR sensitizing mutations. However, when Osimertinib is used as second-line treatment for patients with EGFR T790M mutation, C797X triple resistance mutations (exon 20 C797 mutation to serine or glycine, etc.) inevitably emerges after 9.9-10.1 months of treatment.

Thress KS first reported that 40% of patients treated with Osimertinib for T790M mutation developed C797S triple resistance mutation. Furthermore, Lee JY et al. reported that 24% of patients with T790M mutation developed C797S triple resistance mutation after Osimertinib treatment. Furthermore, Papadimitrakopoulou V.A. analyzed 73 patients who progressed on Osimertinib in the AURA3 clinical trial and found that 15% of patients developed C797S triple resistance mutation. Osimertinib has demonstrated excellent efficacy as first-line treatment compared to standard therapy with first-generation EGFR inhibitors (Gefitinib or Erlotinib), with PFS of 18.9 months vs. 10.2 months. Analysis of 91 patients who progressed on Osimertinib in the FLAURA trial found that 7% of patients had acquired the C797S double resistance mutation.

For patients with EGFR sensitive mutations, the current best recommended first-line therapy is the use of third-generation EGFR inhibitors such as Osimertinib. Consequently, C797X double resistance mutations inevitably emerge after a period of Osimertinib treatment. Currently, no drug has been approved for the treatment of patients harboring C797X mutations, representing an unmet clinical need.

In addition to the common sensitizing mutations Ex19Del and L858R, approximately 4-10% of lung cancer patients harbor EGFR exon 20 insertion mutations, with most occurring at amino acid positions 766-775 located after the C-helix of the EGFR kinase domain, and a minority occurring at positions 762-764. Mobocertinib is the only EGFR small molecule inhibitor that received FDA accelerated approval based on a single-arm trial on September 15, 2021, with an ORR of 28% and a DOR of 17.5 months for patients with EGFR exon 20 insertion mutations. However, on October 2, 2023, Takeda Pharmaceutical announced the voluntary global withdrawal of Mobocertinib from the market because the phase III randomized controlled trial EXCLAIM-2 comparing Mobocertinib with platinum-based chemotherapy did not meet the prespecified clinical endpoints. Furthermore, other approved EGFR inhibitors are ineffective against EGFR exon 20 insertion mutations, showing poor efficacy with ORRs ranging from 0% to 28%. Amivantamab, an EGFR/c-Met bispecific antibody, was approved by the FDA on May 21, 2021, with an ORR of 40% and a DOR of 11.1 months. However, 64% of patients experience infusion-related adverse reactions.

Therefore, the develop a new generation of EGFR inhibitors that are active against both EGFR sensitizing mutations and EGFR drug-resistant mutations, while maintaining selectivity over wild-type EGFR is of great significance.

### SUMMARY OF THE INVENTION

To address the deficiencies of the prior art, the present invention aims to provide an amino heteroaryl macrocyclic compound as well as preparation method and use thereof. The amino heteroaryl macrocyclic compound exhibits good EGFR inhibitory activity and high selectivity against wild-type EGFR.

The present invention is achieved through the following technical solutions.

In a first aspect, the present invention provides an amino heteroaryl macrocyclic compound represented by Formula I, or a pharmaceutically acceptable salt, stereoisomer, racemate, tautomer, isotope-labeled derivative, deuterated derivative, N-oxide, prodrug, hydrate, or solvate thereof 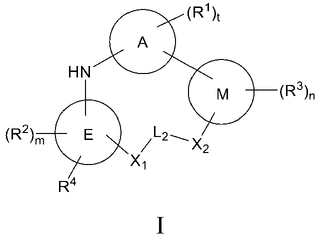 wherein in Formula I,
ring A is a 5-12 membered heteroaryl, 6-12 membered aryl, or 3-12 membered heterocyclyl, the 5-12 membered heteroaryl, 6-12 membered aryl, or 3-12 membered heterocyclyl is connected to the N atom of NH and to ring M via two different atoms, respectively;
ring E is a 5-12 membered heteroaryl, 6-12 membered aryl, or 3-12 membered heterocyclyl, the 5-12 membered heteroaryl, 6-12 membered aryl, or 3-12 membered heterocyclyl is connected to the N atom of NH and to X₁ via two different atoms, respectively;
ring M is a 5-12 membered heteroaryl, 6-12 membered aryl, 3-12 membered heterocyclyl, 3-12 membered cycloalkyl, or 3-12 membered cycloalkenyl, the 5-12 membered heteroaryl, 6-12 membered aryl, 3-12 membered heterocyclyl, 3-12 membered cycloalkyl, or 3-12 membered cycloalkenyl is connected to ring A and to X₂ via two different atoms;
L₂ is C₁₋₆ alkylene, C₂₋₆ alkenylene, C₂₋₆ alkynylene, C₁₋₆ heteroalkylene containing 1-3 atoms selected from N, O, S, C(=O), S(=O)(=W¹), or C₂₋₆ heteroalkenylene containing 1-3 atoms selected from N, O, S, C(=O), S(=O)(=W¹), the C₁₋₆ alkylene, C₂₋₆ alkenylene, C₂₋₆ alkynylene, C₁₋₆ heteroalkylene containing 1-3 atoms selected from N, O, S, C(=O), S(=O)(=W¹), and C₂₋₆ heteroalkenylene containing 1-3 atoms selected from N, O, S, C(=O), S(=O)(=W¹) are optionally substituted with one or more identical or different R⁵;
X₁ and X₂ are each independently absent or are each independently NR^{1a}, - C(R^{2a})(R^{3a})-, O, S, C(=O), or S(=O)(=W¹);
each R¹ is independently hydrogen, deuterium, halogen, CN, NO₂, C₁₋₆ alkyl, halo C₁₋₆ alkyl, C₁₋₆ alkoxy, C₂₋₆ alkenyl, C₂₋₆ alkynyl, 3-12 membered cycloalkyl, 3-12 membered heterocyclyl, 6-10 membered aryl, or 5-10 membered heteroaryl, the C₁₋₆ alkyl, halo C₁₋₆ alkyl, C₁₋₆ alkoxy, C₂₋₆ alkenyl, C₂₋₆ alkynyl, 3-12 membered cycloalkyl, 3-12 membered heterocyclyl, 6-10 membered aryl, or 5-10 membered heteroaryl is optionally substituted with one or more R';
R² and R³ are each independently hydrogen, deuterium, halogen, CN, NO₂, C₁₋₆ alkyl, C₂₋₆ alkenyl, C₂₋₆ alkynyl, C₀₋₆ alkyl-3-12 membered cycloalkyl, C₀₋₆ alkyl-3-12 membered heterocyclyl, C₀₋₆ alkyl-6-10 membered aryl, C₀₋₆ alkyl-5-10 membered heteroaryl, 3-12 membered cycloalkyl-C₁₋₆ alkyl, 3-12 membered heterocyclyl-C₁₋₆ alkyl, 6-10 membered aryl-C₁₋₆ alkyl, 5-10 membered heteroaryl-C₁₋₆ alkyl, OR^{e1}, OC(=O)R^{e1}, OC(=O)NR^{e1}R^{e2}, OS(=O)(=W¹)R^{e1}, OS(=O)(=W¹)NR^{e1}R^{e2}, SR^{e1}, S(=O)(=W¹)R^{e1}, S(=O)(=W¹)NR^{e1}R^{e2}, NR^{e1}R^{e2}, NR^{e1}C(=O)R^{e2}, NR^{e1}S(=O)(=W¹)R^{e2}, NR^{e1}C(=O)OR^{e2}, NR^{e1}C(=O)NR^{e2}, C(=O)R^{e1}, C(=O)NR^{e1}, C(=O)OR^{e1}, PR^{e1}R^{e2}, P(=O)R^{e1}R^{e2}, P(=O)₂R^{e1}R^{e2}, P(=O)OR^{e2}, or oxo (=O), the C₁₋₆ alkyl, C₂₋₆ alkenyl, C₂₋₆ alkynyl, C₀₋₆ alkyl-3-12 membered cycloalkyl, C₀₋₆ alkyl-3-12 membered heterocyclyl, C₀₋₆ alkyl-6-10 membered aryl, C₀₋₆ alkyl-5-10 membered heteroaryl, 3-12 membered cycloalkyl-C₁₋₆ alkyl, 3-12 membered heterocyclyl-C₁₋₆ alkyl, 6-10 membered aryl-C₁₋₆ alkyl, and 5-10 membered heteroaryl-C₁₋₆ alkyl are optionally substituted with one or more R';
or, two R²together with the atoms to which they are respectively attached to form a 3-12 membered cycloalkyl, 3-12 membered cycloalkenyl, 3-12 membered heterocyclyl, 5-14 membered heteroaryl, or 6-14 membered aryl, the 3-12 membered cycloalkyl, 3-12 membered cycloalkenyl, 3-12 membered heterocyclyl, 5-14 membered heteroaryl, or 6-14 membered aryl is optionally substituted with one or more R';
R⁴ is 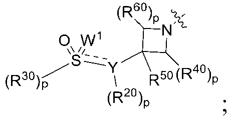
each R⁵ is independently deuterium, halogen, CN, NO₂, C₁₋₆ alkyl, C₂₋₆ alkenyl, C₂₋₆ alkynyl, C₀₋₆ alkyl-3-12 membered cycloalkyl, C₀₋₆ alkyl-3-12 membered heterocyclyl, C₀₋₆ alkyl-6-10 membered aryl, C₀₋₆ alkyl-5-10 membered heteroaryl, 3-12 membered cycloalkyl-C₁₋₆ alkyl, 3-12 membered heterocyclyl-C₁₋₆ alkyl, 6-10 membered aryl-C₁₋₆ alkyl, 5-10 membered heteroaryl-C₁₋₆ alkyl, OR^{e1}, OC(=O)R^{e1}, OC(=O)NR^{e1}R^{e2}, OS(=O)(=W¹)R^{e1}, OS(=O)(=W¹)NR^{e1}R^{e2}, SR^{e1}, S(=O)(=W¹)R^{e1}, S(=O)(=W¹)NR^{e1}R^{e2}, NR^{e1}R^{e2}, NR^{e1}C(=O)R^{e2}, NR^{e1}S(=O)(=W¹)R^{e2}, NR^{e1}C(=O)OR^{e2}, NR^{e1}C(=O)NR^{e2}, C(=O)R^{e1}, C(=O)NR^{e1}, C(=O)OR^{e1}, PR^{e1}R^{e2}, P(=O)R^{e1}R^{e2}, P(=O)₂R^{e1}R^{e2}, P(=O)OR^{e2}, or oxo (=O), the C₁₋₆ alkyl, C2-6 alkenyl, C2-₆ alkynyl, C₀₋₆ alkyl-3-12 membered cycloalkyl, C₀₋₆ alkyl-3-12 membered heterocyclyl, C₀₋₆ alkyl-6-10 membered aryl, C₀₋₆ alkyl-5-10 membered heteroaryl, 3-12 membered cycloalkyl-C₁₋₆ alkyl, 3-12 membered heterocyclyl-C₁₋₆ alkyl, 6-10 membered aryl-C₁₋₆ alkyl, and 5-10 membered heteroaryl-C₁₋₆ alkyl are optionally substituted with one or more R';
or, two R⁵together with the atoms to which they are respectively attached to form a 3-12 membered cycloalkyl, 6-12 membered aryl, 5-10 membered heteroaryl, 3-12 membered cycloalkenyl, or 3-12 membered heterocyclyl, the 3-12 membered cycloalkyl, 6-12 membered aryl, 5-10 membered heteroaryl, 3-12 membered cycloalkenyl, or 3-12 membered heterocyclyl is optionally substituted with one or more R';
R^{1a} and R^{2a} are each independently absent or are each independently hydrogen, deuterium, halogen, C₁₋₆ alkyl, halo C₁₋₆ alkyl, C₂₋₆ alkenyl, C₂₋₆ alkynyl, 3-12 membered cycloalkyl, 3-12 membered heterocyclyl, 6-10 membered aryl, 5-10 membered heteroaryl, S(=O)(=W¹)R^{e1}, S(=O)(=W¹)NR^{e1}R^{e2}, C(=O)R^{e1}, C(=O)NR^{e1}, C(=O)OR^{e1}, S(=O)(=W¹)NR^{e1}, P(=O)R^{e1}R^{e2}, P(=O)₂R^{e1}R^{e2}, or P(=O)OR^{e2}, the C₁₋₆ alkyl, halo C₁₋₆ alkyl, C2-6 alkenyl, C2-6 alkynyl, 3-12 membered cycloalkyl, 3-12 membered heterocyclyl, 6-10 membered aryl, or 5-10 membered heteroaryl is optionally substituted with one or more R';
or, R² and R^{1a}together with the atoms to which they are respectively attached to form a 3-12 membered heterocyclyl or 5-12 membered heteroaryl, the 3-12 membered heterocyclyl or 5-12 membered heteroaryl is optionally substituted with one or more R';
or, R³ and R^{1a}together with the atoms to which they are respectively attached to form a 3-12 membered heterocyclyl or 5-12 membered heteroaryl, the 3-12 membered heterocyclyl or 5-12 membered heteroaryl is optionally substituted with one or more R';
R^{3a} is hydrogen, deuterium, halogen, CN, NO₂, C₁₋₆ alkyl, C₂₋₆ alkenyl, C₂₋₆ alkynyl, 3-12 membered cycloalkyl, 3-12 membered heterocyclyl, 6-10 membered aryl, 5-10 membered heteroaryl, OR^{e1}, OC(=O)R^{e1}, OC(=O)NR^{e1}R^{e2}, OS(=O)(=W¹)R^{e1}, OS(=O)(=W¹)NR^{e1}R^{e2}, SR^{e1}, S(=O)(=W¹)R^{e1}, S(=O)(=W¹)NR^{e1}R^{e2}, NR^{e1}R^{e2}, NR^{e1}C(=O)R^{e2}, NR^{e1}C(=O)OR^{e2}, NR^{e1}C(=O)NR^{e2}, NR^{e1}S(=O)(=W¹)R^{e2}, C(=O)R^{e1}, C(=O)NR^{e1}, C(=O)OR^{e1}, PR^{e1}R^{e2}, P(=O)R^{e1}R^{e2}, P(=O)₂R^{e1}R^{e2}, P(-O)OR^{e2}, or oxo (=O), the C₁₋₆ alkyl, C2-6 alkenyl, C2-6 alkynyl, 3-12 membered cycloalkyl, 3-12 membered heterocyclyl, 6-10 membered aryl, or 5-10 membered heteroaryl is optionally substituted with one or more R';
or, R^{2a} and R^{3a}together with the carbon atom to which they are attached to form a 3-12 membered cycloalkyl or 3-12 membered heterocyclyl, the 3-12 membered cycloalkyl or 3-12 membered heterocyclyl is optionally substituted with one or more R';
or, R² and R^{3a}together with the atoms to which they are respectively attached to form a 3-12 membered cycloalkyl, 3-12 membered heterocyclyl, 6-12 membered aryl, or 5-12 membered heteroaryl, the 3-12 membered cycloalkyl, 3-12 membered heterocyclyl, 6-12 membered aryl, or 5-12 membered heteroaryl is optionally substituted with one or more R';
or, R³ and R^{3a}together with the atoms to which they are respectively attached to form a 3-12 membered cycloalkyl, 3-12 membered heterocyclyl, 6-12 membered aryl, or 5-12 membered heteroaryl, the 3-12 membered cycloalkyl, 3-12 membered heterocyclyl, 6-12 membered aryl, or 5-12 membered heteroaryl is optionally substituted with one or more R';
R' is deuterium, halogen, CN, NO₂, C₁₋₆ alkyl, halo C₁₋₆ alkyl, C₂₋₆ alkenyl, C₂₋₆ alkynyl, 3-12 membered cycloalkyl, 3-12 membered heterocyclyl, 6-10 membered aryl, 5-10 membered heteroaryl, OR^{f1}, OC(=O)R^{f1}, OC(=O)NR^{f1}R^{f2}, OS(=O)(=W¹)R^{f1}, OS(=O)(=W¹)NR^{f1}R^{f2}, SR^{f1}, S(=O)(=W¹)R^{f1}, S(=O)(=W¹)NR^{f1}R^{f2}, NR^{f1}R^{f2}, NR^{f1}C(=O)R^{f2}, NR^{f1}S(=O)(=W¹)R^{f2}, NR^{f1}C(=O)OR^{f2}, NR^{f1}C(=O)NR^{f2}, C(=O)R^{f1}, C(=O)NR^{f1}, C(=O)OR^{f1}, - (O)(OR^{f1})₂, PR^{f1}R^{f2}, P(=O)R^{f1}R^{f2}, P(=O)₂R^{f1}R^{f2}, P(=O)OR^{f2}, or oxo (=O);
R^{e1}, R^{e2}, R^{f1}, and R^{f2} are each independently hydrogen, deuterium, C₁₋₆ alkyl, halo C₁₋₆ alkyl, C₂₋₆ alkenyl, C₂₋₆ alkynyl, 3-12 membered cycloalkyl, 3-12 membered heterocyclyl, 6-10 membered aryl, or 5-10 membered heteroaryl, the C₁₋₆ alkyl, C₂₋₆ alkenyl, C₂₋₆ alkynyl, 3-12 membered cycloalkyl, 3-12 membered heterocyclyl, 6-10 membered aryl, or 5-10 membered heteroaryl is optionally substituted with one or more R";
or, R^{e1} and R^{e2}together with the atoms to which they are attached to form a 3-12 membered heterocyclyl, the 3-12 membered heterocyclyl is optionally substituted with one or more groups selected from: deuterium, halogen, CN, NO₂, C₁₋₆ alkyl, halo C₁₋₆ alkyl, C₂₋₆ alkenyl, C₂₋₆ alkynyl, 3-12 membered cycloalkyl, 3-12 membered heterocyclyl, 6-10 membered aryl, 5-10 membered heteroaryl, and OR^{g1};
or, R^{f1} and R^{f2}together with the atoms to which they are attached to form a 3-12 membered heterocyclyl, the 3-12 membered heterocyclyl is optionally substituted with one or more groups selected from: deuterium, halogen, CN, NO₂, C₁₋₆ alkyl, halo C₁₋₆ alkyl, C₂₋₆ alkenyl, C₂₋₆ alkynyl, 3-12 membered cycloalkyl, 3-12 membered heterocyclyl, 6-10 membered aryl, 5-10 membered heteroaryl, and OR^{g1};
R" is deuterium, halogen, CN, NO₂, C₁₋₆ alkyl, halo C₁₋₆ alkyl, C₂₋₆ alkenyl, C₂₋₆ alkynyl, 3-12 membered cycloalkyl, 3-12 membered heterocyclyl, 6-10 membered aryl, 5-10 membered heteroaryl, OR^{g1}, OC(=O)R^{g1}, OC(=O)NR^{g1}R^{g2}, OS(=O)(=W¹)R^{g1}, OS(=O)(=W¹)NR^{g1}R^{g2}, SR^{g1}, S(=O)(=W¹)R^{g1}, S(=O)(=W¹)NR^{g1}R^{g2}, NR^{g1}R^{g2}, NR^{g1}C(=O)R^{g2}, NR^{g1}S(=O)(=W¹)R^{g2}, NR^{g1}C(=O)OR^{g2}, NR^{g1}C(=O)NR^{g2}, C(=O)R^{g1}, C(=O)NR^{g1}, C(=O)OR^{g1}, -(O)(OR^{g1})₂, PR^{g1}R^{g2}, P(=O)R^{g1}R^{g2}, P(=O)₂R^{g1}R^{g2}, P(=O)OR^{g2}, or oxo (=O);
R^{g1} and R^{g2} are each independently hydrogen, deuterium, C₁₋₆ alkyl, halo C₁₋₆ alkyl, C₂₋₆ alkenyl, or 3-12 membered cycloalkyl;
W¹ is absent, O, or NR^{g1};
Y is N or C;
- - - represents a single bond or a double bond;
R³⁰ is hydrogen, deuterium, C₁₋₆ alkyl, halo C₁₋₆ alkyl, amino, C₁₋₆ alkylamino, di(C₁₋₆ alkyl)amino, C₃₋₆ cycloalkylamino, C₂₋₆ alkenyl, C₂₋₆ alkynyl, C₀₋₆ alkyl-3-12 membered cycloalkyl, or C₀₋₆ alkyl-3-12 membered heterocyclyl, the C₁₋₆ alkyl, halo C₁₋₆ alkyl, C₂₋₆ alkenyl, C₂₋₆ alkynyl, C₀₋₆ alkyl-3-12 membered cycloalkyl, and C₀₋₆ alkyl-3-12 membered heterocyclyl are optionally substituted with one or more R'; or, two R³⁰together with the atoms to which they are respectively attached to form a 3-6 membered cycloalkyl or 3-7 membered heterocyclyl, the 3-6 membered cycloalkyl or 3-7 membered heterocyclyl is optionally substituted with one or more R';
R²⁰, R⁴⁰, R⁵⁰, and R⁶⁰ are each independently hydrogen, deuterium, halogen, CN, amino, hydroxyl, C₁₋₆ alkyl, halo C₁₋₆ alkyl, C₂₋₆ alkenyl, C₂₋₆ alkynyl, C₁₋₆ alkoxy, C₁₋₆ alkylamino, di(C₁₋₆ alkyl)amino, C₀₋₆ alkyl-3-12 membered cycloalkyl, C₀₋₆ alkyl-3-12 membered heterocyclyl, or oxo (=O), the C₁₋₆ alkyl, halo C₁₋₆ alkyl, C₂₋₆ alkenyl, C₂₋₆ alkynyl, C₁₋₆ alkoxy, C₁₋₆ alkylamino, di(C₁₋₆ alkyl)amino, C₀₋₆ alkyl-3-12 membered cycloalkyl, and C₀₋₆ alkyl-3-12 membered heterocyclyl are optionally substituted with one or more R';
or, two R²⁰together with the atoms to which they are respectively attached to form a 3-6 membered cycloalkyl or 3-7 membered heterocyclyl, the 3-6 membered cycloalkyl or 3-7 membered heterocyclyl is optionally substituted with one or more R';
or, R²⁰ and R⁵⁰together with the atoms to which they are respectively attached to form a 3-6 membered cycloalkyl or 3-7 membered heterocyclyl, the 3-6 membered cycloalkyl or 3-7 membered heterocyclyl is optionally substituted with one or more R';
or, R⁴⁰ and R⁶⁰together with the atoms to which they are respectively attached to form a 3-6 membered cycloalkyl or 3-7 membered heterocyclyl, the 3-6 membered cycloalkyl or 3-7 membered heterocyclyl is optionally substituted with one or more R';
m, n, and t are each independently 0, 1, 2, 3, or 4;
p is each independently 1 or 2.

In another aspect, the present invention also provides an amino heteroaryl macrocyclic compound represented by Formula I, or a pharmaceutically acceptable salt, stereoisomer, racemate, tautomer, isotope-labeled derivative, deuterated derivative, N-oxide, prodrug, hydrate, or solvate thereof,
wherein, in Formula I,
ring A is a 5-12 membered heteroaryl, 6-12 membered aryl, or 3-12 membered heterocyclyl, the 5-12 membered heteroaryl, 6-12 membered aryl, or 3-12 membered heterocyclyl is connected to the N atom of NH and to ring M via two different atoms, respectively;
ring E is a 5-12 membered heteroaryl, 6-12 membered aryl, or 3-12 membered heterocyclyl, the 5-12 membered heteroaryl, 6-12 membered aryl, or 3-12 membered heterocyclyl is connected to the N atom of NH and to X₁ via two different atoms, respectively;
ring M is a 5-12 membered heteroaryl, 6-12 membered aryl, 3-12 membered heterocyclyl, 3-12 membered cycloalkyl, or 3-12 membered cycloalkenyl, the 5-12 membered heteroaryl, 6-12 membered aryl, 3-12 membered heterocyclyl, 3-12 membered cycloalkyl, or 3-12 membered cycloalkenyl is connected to ring A and to X₂ via two different atoms;
L₂ is C₁₋₆ alkylene, C₂₋₆ alkenylene, C₂₋₆ alkynylene, C₁₋₆ heteroalkylene containing 1-3 atoms selected from N, O, S, C(=O), S(=O)(=W¹), or C₂₋₆ heteroalkenylene containing 1-3 atoms selected from N, O, S, C(=O), S(=O)(=W¹), the C₁₋₆ alkylene, C₂₋₆ alkenylene, C₂₋₆ alkynylene, C₁₋₆ heteroalkylene containing 1-3 atoms selected from N, O, S, C(=O), S(=O)(=W¹), and C₂₋₆ heteroalkenylene containing 1-3 atoms selected from N, O, S, C(=O), S(=O)(=W¹) are optionally substituted with one or more identical or different R⁵;
X₁ and X₂ are each independently absent or are each independently NR^{1a}, - C(R^{2a})(R^{3a})-, O, S, C(=O), or S(=O)(=W¹);
each R¹ is independently hydrogen, deuterium, halogen, CN, NO₂, C₁₋₆ alkyl, halo C₁₋₆ alkyl, C₁₋₆ alkoxy, C₂₋₆ alkenyl, C₂₋₆ alkynyl, 3-12 membered cycloalkyl, 3-12 membered heterocyclyl, 6-10 membered aryl, or 5-10 membered heteroaryl, the C₁₋₆ alkyl, halo C₁₋₆ alkyl, C₁₋₆ alkoxy, C₂₋₆ alkenyl, C₂₋₆ alkynyl, 3-12 membered cycloalkyl, 3-12 membered heterocyclyl, 6-10 membered aryl, or 5-10 membered heteroaryl is optionally substituted with one or more R';
R² and R³ are each independently hydrogen, deuterium, halogen, CN, NO₂, C₁₋₆ alkyl, C₂₋₆ alkenyl, C₂₋₆ alkynyl, C₀₋₆ alkyl-3-12 membered cycloalkyl, C₀₋₆ alkyl-3-12 membered heterocyclyl, C₀₋₆ alkyl-6-10 membered aryl, C₀₋₆ alkyl-5-10 membered heteroaryl, 3-12 membered cycloalkyl-C₁₋₆ alkyl, 3-12 membered heterocyclyl-C₁₋₆ alkyl, 6-10 membered aryl-C₁₋₆ alkyl, 5-10 membered heteroaryl-C₁₋₆ alkyl, OR^{e1}, OC(=O)R^{e1}, OC(=O)NR^{e1}R^{e2}, OS(=O)(=W¹)R^{e1}, OS(=O)(=W¹)NR^{e1}R^{e2}, SR^{e1}, S(=O)(=W¹)R^{e1}, S(=O)(=W¹)NR^{e1}R^{e2}, NR^{e1}R^{e2}, NR^{e1}C(=O)R^{e2}, NR^{e1}S(=O)(=W¹)R^{e2}, NR^{e1}C(=O)OR^{e2}, NR^{e1}C(=O)NR^{e2}, C(=O)R^{e1}, C(=O)NR^{e1}, C(=O)OR^{e1}, PR^{e1}R^{e2}, P(=O)R^{e1}R^{e2}, P(=O)₂R^{e1}R^{e2}, P(=O)OR^{e2}, or oxo (=O), the C₁₋₆ alkyl, C₂₋₆ alkenyl, C₂₋₆ alkynyl, C₀₋₆ alkyl-3-12 membered cycloalkyl, C₀₋₆ alkyl-3-12 membered heterocyclyl, C₀₋₆ alkyl-6-10 membered aryl, C₀₋₆ alkyl-5-10 membered heteroaryl, 3-12 membered cycloalkyl-C₁₋₆ alkyl, 3-12 membered heterocyclyl-C₁₋₆ alkyl, 6-10 membered aryl-C₁₋₆ alkyl, and 5-10 membered heteroaryl-C₁₋₆ alkyl are optionally substituted with one or more R';
or, two R²together with the atoms to which they are respectively attached to form a 3-12 membered cycloalkyl, 3-12 membered cycloalkenyl, 3-12 membered heterocyclyl, 5-14 membered heteroaryl, or 6-14 membered aryl, the 3-12 membered cycloalkyl, 3-12 membered cycloalkenyl, 3-12 membered heterocyclyl, 5-14 membered heteroaryl, or 6-14 membered aryl is optionally substituted with one or more R';
R⁴ is R³⁰-S(=O)(W¹)-Y(R²⁰)ₚ-C₀₋₄ alkyl-4-14 membered heterocyclyl, (R³⁰)ₚN-Y(R²⁰)ₚ-C₀₋₄ alkyl-4-14 membered heterocyclyl, R³⁰-S(=O)(W¹)-Y(R²⁰)ₚ-C₀₋₄ alkyl-5-6 membered heteroaryl, or R³⁰-S(=O)(W¹)-Y(R²⁰)ₚ-C₀₋₄ alkyl-4-12 membered cycloalkyl, the R³⁰-S(=O)(W¹)-Y(R²⁰)ₚ-C₀₋₄ alkyl-4-14 membered heterocyclyl, (R³⁰)ₚN-Y(R²⁰)ₚ-C₀₋₄ alkyl-4-14 membered heterocyclyl, R³⁰-S(=O)(W¹)-Y(R²⁰)ₚ-C₀₋₄ alkyl-5-6 membered heteroaryl, or R³⁰-S(=O)(W¹)-Y(R²⁰)ₚ-C₀₋₄ alkyl-4-12 membered cycloalkyl is optionally substituted with one or more R';
each R⁵ is independently deuterium, halogen, CN, NO₂, C₁₋₆ alkyl, C₂₋₆ alkenyl, C₂₋₆ alkynyl, C₀₋₆ alkyl-3-12 membered cycloalkyl, C₀₋₆ alkyl-3-12 membered heterocyclyl, C₀₋₆ alkyl-6-10 membered aryl, C₀₋₆ alkyl-5-10 membered heteroaryl, 3-12 membered cycloalkyl-C₁₋₆ alkyl, 3-12 membered heterocyclyl-C₁₋₆ alkyl, 6-10 membered aryl-C₁₋₆ alkyl, 5-10 membered heteroaryl-C₁₋₆ alkyl, OR^{e1}, OC(=O)R^{e1}, OC(=O)NR^{e1}R^{e2}, OS(=O)(=W¹)R^{e1}, OS(=O)(=W¹)NR^{e1}R^{e2}, SR^{e1}, S(=O)(=W¹)R^{e1}, S(=O)(=W¹)NR^{e1}R^{e2}, NR^{e1}R^{e2}, NR^{e1}C(=O)R^{e2}, NR^{e1}S(=O)(=W¹)R^{e2}, NR^{e1}C(=O)OR^{e2}, NR^{e1}C(=O)NR^{e2}, C(=O)R^{e1}, C(=O)NR^{e1}, C(=O)OR^{e1}, PR^{e1}R^{e2}, P(=O)R^{e1}R^{e2}, P(=O)2R^{e1}R^{e2}, P(=O)OR^{e2}, or oxo (=O), the C₁₋₆ alkyl, C2-6 alkenyl, C2-₆ alkynyl, C₀₋₆ alkyl-3-12 membered cycloalkyl, C₀₋₆ alkyl-3-12 membered heterocyclyl, C₀₋₆ alkyl-6-10 membered aryl, C₀₋₆ alkyl-5-10 membered heteroaryl, 3-12 membered cycloalkyl-C₁₋₆ alkyl, 3-12 membered heterocyclyl-C₁₋₆ alkyl, 6-10 membered aryl-C₁₋₆ alkyl, and 5-10 membered heteroaryl-C₁₋₆ alkyl are optionally substituted with one or more R';
or, two R⁵together with the atoms to which they are respectively attached to form a 3-12 membered cycloalkyl, 6-12 membered aryl, 5-10 membered heteroaryl, 3-12 membered cycloalkenyl, or 3-12 membered heterocyclyl, the 3-12 membered cycloalkyl, 6-12 membered aryl, 5-10 membered heteroaryl, 3-12 membered cycloalkenyl, or 3-12 membered heterocyclyl is optionally substituted with one or more R';
R^{1a} and R^{2a} are each independently absent or are each independently hydrogen, deuterium, halogen, C₁₋₆ alkyl, halo C₁₋₆ alkyl, C₂₋₆ alkenyl, C₂₋₆ alkynyl, 3-12 membered cycloalkyl, 3-12 membered heterocyclyl, 6-10 membered aryl, 5-10 membered heteroaryl, S(=O)(=W¹)R^{e1}, S(=O)(=W¹)NR^{e1}R^{e2}, C(=O)R^{e1}, C(=O)NR^{e1}, C(=O)OR^{e1}, S(=O)(=W¹)NR^{e1}, P(=O)R^{e1}R^{e2}, P(=O)₂R^{e1}R^{e2}, or P(=O)OR^{e2}, the C₁₋₆ alkyl, halo C₁₋₆ alkyl, C2-6 alkenyl, C2-6 alkynyl, 3-12 membered cycloalkyl, 3-12 membered heterocyclyl, 6-10 membered aryl, or 5-10 membered heteroaryl is optionally substituted with one or more R';
or, R² and R^{1a}together with the atoms to which they are respectively attached to form a 3-12 membered heterocyclyl or 5-12 membered heteroaryl, the 3-12 membered heterocyclyl or 5-12 membered heteroaryl is optionally substituted with one or more R';
or, R³ and R^{1a}together with the atoms to which they are respectively attached to form a 3-12 membered heterocyclyl or 5-12 membered heteroaryl, the 3-12 membered heterocyclyl or 5-12 membered heteroaryl is optionally substituted with one or more R';
R^{3a} is hydrogen, deuterium, halogen, CN, NO₂, C₁₋₆ alkyl, C₂₋₆ alkenyl, C₂₋₆ alkynyl, 3-12 membered cycloalkyl, 3-12 membered heterocyclyl, 6-10 membered aryl, 5-10 membered heteroaryl, OR^{e1}, OC(=O)R^{e1}, OC(=O)NR^{e1}R^{e2}, OS(=O)(=W¹)R^{e1}, OS(=O)(=W¹)NR^{e1}R^{e2}, SR^{e1}, S(=O)(=W¹)R^{e1}, S(=O)(=W¹)NR^{e1}R^{e2}, NR^{e1}R^{e2}, NR^{e1}C(=O)R^{e2}, NR^{e1}C(=O)OR^{e2}, NR^{e1}C(=O)NR^{e2}, NR^{e1}S(=O)(=W¹)R^{e2}, C(=O)R^{e1}, C(=O)NR^{e1}, C(=O)OR^{e1}, PR^{e1}R^{e2}, P(=O)R^{e1}R^{e2}, P(=O)₂R^{e1}R^{e2}, P(-O)OR^{e2}, or oxo (=O), the C₁₋₆ alkyl, C2-6 alkenyl, C2-6 alkynyl, 3-12 membered cycloalkyl, 3-12 membered heterocyclyl, 6-10 membered aryl, or 5-10 membered heteroaryl is optionally substituted with one or more R';
or, R^{2a} and R^{3a}together with the carbon atom to which they are attached to form a 3-12 membered cycloalkyl or 3-12 membered heterocyclyl, the 3-12 membered cycloalkyl or 3-12 membered heterocyclyl is optionally substituted with one or more R';
or, R² and R^{3a}together with the atoms to which they are respectively attached to form a 3-12 membered cycloalkyl, 3-12 membered heterocyclyl, 6-12 membered aryl, or 5-12 membered heteroaryl, the 3-12 membered cycloalkyl, 3-12 membered heterocyclyl, 6-12 membered aryl, or 5-12 membered heteroaryl is optionally substituted with one or more R';
or, R³ and R^{3a}together with the atoms to which they are respectively attached to form a 3-12 membered cycloalkyl, 3-12 membered heterocyclyl, 6-12 membered aryl, or 5-12 membered heteroaryl, the 3-12 membered cycloalkyl, 3-12 membered heterocyclyl, 6-12 membered aryl, or 5-12 membered heteroaryl is optionally substituted with one or more R';
R' is deuterium, halogen, CN, NO₂, C₁₋₆ alkyl, halo C₁₋₆ alkyl, C₂₋₆ alkenyl, C₂₋₆ alkynyl, 3-12 membered cycloalkyl, 3-12 membered heterocyclyl, 6-10 membered aryl, 5-10 membered heteroaryl, OR^{f1}, OC(=O)R^{f1}, OC(=O)NR^{f1}R^{f2}, OS(=O)(=W¹)R^{f1}, OS(=O)(=W¹)NR^{f1}R^{f2}, SR^{f1}, S(=O)(=W¹)R^{f1}, S(=O)(=W¹)NR^{f1}R^{f2}, NR^{f1}R^{f2}, NR^{f1}C(=O)R^{f2}, NR^{f1}S(=O)(=W¹)R^{f2}, NR^{f1}C(=O)OR^{f2}, NR^{f1}C(=O)NR^{f2}, C(=O)R^{f1}, C(=O)NR^{f1}, C(=O)OR^{f1}, - (O)(OR^{f1})₂, PR^{f1}R^{f2}, P(=O)R^{f1}R^{f2}, P(=O)₂R^{f1}R^{f2}, P(=O)OR^{f2}, or oxo (=O);
R^{e1}, R^{e2}, R^{f1}, and R^{f2} are each independently hydrogen, deuterium, C₁₋₆ alkyl, halo Ci-₆ alkyl, C₂₋₆ alkenyl, C₂₋₆ alkynyl, 3-12 membered cycloalkyl, 3-12 membered heterocyclyl, 6-10 membered aryl, or 5-10 membered heteroaryl, the C₁₋₆ alkyl, C₂₋₆ alkenyl, C₂₋₆ alkynyl, 3-12 membered cycloalkyl, 3-12 membered heterocyclyl, 6-10 membered aryl, or 5-10 membered heteroaryl is optionally substituted with one or more R";
or, R^{e1} and R^{e2}together with the atoms to which they are attached to form a 3-12 membered heterocyclyl, the 3-12 membered heterocyclyl is optionally substituted with one or more groups selected from: deuterium, halogen, CN, NO₂, C₁₋₆ alkyl, halo C₁₋₆ alkyl, C₂₋₆ alkenyl, C₂₋₆ alkynyl, 3-12 membered cycloalkyl, 3-12 membered heterocyclyl, 6-10 membered aryl, 5-10 membered heteroaryl, and OR^{g1};
or, R^{f1} and R^{f2}together with the atoms to which they are attached to form a 3-12 membered heterocyclyl, the 3-12 membered heterocyclyl is optionally substituted with one or more groups selected from: deuterium, halogen, CN, NO₂, C₁₋₆ alkyl, halo C₁₋₆ alkyl, C₂₋₆ alkenyl, C₂₋₆ alkynyl, 3-12 membered cycloalkyl, 3-12 membered heterocyclyl, 6-10 membered aryl, 5-10 membered heteroaryl, and OR^{g1};
R" is deuterium, halogen, CN, NO₂, C₁₋₆ alkyl, halo C₁₋₆ alkyl, C₂₋₆ alkenyl, C₂₋₆ alkynyl, 3-12 membered cycloalkyl, 3-12 membered heterocyclyl, 6-10 membered aryl, 5-10 membered heteroaryl, OR^{g1}, OC(=O)R^{g1}, OC(=O)NR^{g1}R^{g2}, OS(=O)(=W¹)R^{g1}, OS(=O)(=W¹)NR^{g1}R^{g2}, SR^{g1}, S(=O)(=W¹)R^{g1}, S(=O)(=W¹)NR^{g1}R^{g2}, NR^{g1}R^{g2}, NR^{g1}C(=O)R^{g2}, NR^{g1}S(=O)(=W¹)R^{g2}, NR^{g1}C(=O)OR^{g2}, NR^{g1}C(=O)NR^{g2}, C(=O)R^{g1}, C(=O)NR^{g1}, C(=O)OR^{g1}, -(O)(OR^{g1})₂, PR^{g1}R^{g2}, P(=O)R^{g1}R^{g2}, P(=O)₂R^{g1}R^{g2}, P(=O)OR^{g2}, or oxo (=O);
R^{g1} and R^{g2} are each independently hydrogen, deuterium, C₁₋₆ alkyl, halo C₁₋₆ alkyl, C₂₋₆ alkenyl, or 3-12 membered cycloalkyl;
W¹ is absent, O, or NR^{g1};
Y is absent or is N or C;
each R²⁰ is independently hydrogen, deuterium, halogen, CN, amino, hydroxyl, C₁₋₆ alkyl, halo C₁₋₆ alkyl, C₂₋₆ alkenyl, C₂₋₆ alkynyl, C₁₋₆ alkoxy, C₁₋₆ alkylamino, di(C₁₋₆ alkyl)amino, C₀₋₆ alkyl-3-12 membered cycloalkyl, C₀₋₆ alkyl-3-12 membered heterocyclyl, or oxo (=O), the C₁₋₆ alkyl, halo C₁₋₆ alkyl, C₂₋₆ alkenyl, C₂₋₆ alkynyl, C₁₋₆ alkoxy, C₁₋₆ alkylamino, di(C₁₋₆ alkyl)amino, C₀₋₆ alkyl-3-12 membered cycloalkyl, and C₀₋₆ alkyl-3-12 membered heterocyclyl are optionally substituted with one or more R'; or, two R²⁰together with the atoms to which they are respectively attached to form a 3-6 membered cycloalkyl or 3-7 membered heterocyclyl, the 3-6 membered cycloalkyl or 3-7 membered heterocyclyl is optionally substituted with one or more R';
R³⁰ is hydrogen, deuterium, C₁₋₆ alkyl, halo C₁₋₆ alkyl, amino, C₁₋₆ alkylamino, di(C₁₋₆ alkyl)amino, C₃₋₆ cycloalkylamino, C₂₋₆ alkenyl, C₂₋₆ alkynyl, C₀₋₆ alkyl-3-12 membered cycloalkyl, or C₀₋₆ alkyl-3-12 membered heterocyclyl, the C₁₋₆ alkyl, halo C₁₋₆ alkyl, C₂₋₆ alkenyl, C₂₋₆ alkynyl, C₀₋₆ alkyl-3-12 membered cycloalkyl, and C₀₋₆ alkyl-3-12 membered heterocyclyl is optionally substituted with one or more R'; or, two R³⁰together with the atoms to which they are respectively attached to form a 3-6 membered cycloalkyl or 3-7 membered heterocyclyl, the 3-6 membered cycloalkyl or 3-7 membered heterocyclyl is optionally substituted with one or more R';
or, R²⁰ and R⁵⁰together with the atoms to which they are respectively attached to form a 3-6 membered cycloalkyl or 3-7 membered heterocyclyl, the 3-6 membered cycloalkyl or 3-7 membered heterocyclyl is optionally substituted with one or more R';
or, R⁴⁰ and R⁶⁰together with the atoms to which they are respectively attached to form a 3-6 membered cycloalkyl or 3-7 membered heterocyclyl, the 3-6 membered cycloalkyl or 3-7 membered heterocyclyl is optionally substituted with one or more R';
m, n, and t are each independently 0, 1, 2, 3, or 4;
p is each independently 1 or 2.

In certain embodiments, in Formula I,
ring A is a 5-12 membered heteroaryl, 6-12 membered aryl, or 3-12 membered heterocyclyl, the 5-12 membered heteroaryl, 6-12 membered aryl, or 3-12 membered heterocyclyl is connected to the N atom of NH and to ring M via two different atoms, respectively;
ring E is a 5-12 membered heteroaryl, 6-12 membered aryl, or 3-12 membered heterocyclyl, the 5-12 membered heteroaryl, 6-12 membered aryl, or 3-12 membered heterocyclyl is connected to the N atom of NH and to X₁ via two different atoms, respectively;
ring M is a 5-12 membered heteroaryl, 6-12 membered aryl, 3-12 membered heterocyclyl, 3-12 membered cycloalkyl, or 3-12 membered cycloalkenyl, the 5-12 membered heteroaryl, 6-12 membered aryl, 3-12 membered heterocyclyl, 3-12 membered cycloalkyl, or 3-12 membered cycloalkenyl is connected to ring A and to X₂ via two different atoms;
L₂ is C₁₋₆ alkylene, C₂₋₆ alkenylene, C₂₋₆ alkynylene, C₁₋₆ heteroalkylene containing 1-3 atoms selected from N, O, S, C(=O), S(=O)(=W¹) , or C₂₋₆ heteroalkenylene containing 1-3 atoms selected from N, O, S, C(=O), S(=O)(=W¹), the C₁₋₆ alkylene, C₂₋₆ alkenylene, C₂₋₆ alkynylene, C₁₋₆ heteroalkylene containing 1-3 atoms selected from N, O, S, C(=O), S(=O)(=W¹) , and C₂₋₆ heteroalkenylene containing 1-3 atoms selected from N, O, S, C(=O), S(=O)(=W¹)is optionally substituted with one or more identical or different R⁵;
X₁ and X₂ are each independently absent or are each independently NR^{1a}, - C(R^{2a})(R^{3a})-, O, S, C(=O), or S(=O)(=^{W1});
each R¹ is independently hydrogen, deuterium, halogen, CN, NO₂, C₁₋₆ alkyl, halo C₁₋₆ alkyl, C₁₋₆ alkoxy, C₂₋₆ alkenyl, C₂₋₆ alkynyl, 3-12 membered cycloalkyl, 3-12 membered heterocyclyl, 6-10 membered aryl, or 5-10 membered heteroaryl, the C₁₋₆ alkyl, halo C₁₋₆ alkyl, C₁₋₆ alkoxy, C₂₋₆ alkenyl, C₂₋₆ alkynyl, 3-12 membered cycloalkyl, 3-12 membered heterocyclyl, 6-10 membered aryl, or 5-10 membered heteroaryl is optionally substituted with one or more R';
R² and R³ are each independently hydrogen, deuterium, halogen, CN, NO₂, C₁₋₆ alkyl, C₂₋₆ alkenyl, C₂₋₆ alkynyl, C₀₋₆ alkyl-3-12 membered cycloalkyl, C₀₋₆ alkyl-3-12 membered heterocyclyl, C₀₋₆ alkyl-6-10 membered aryl, C₀₋₆ alkyl-5-10 membered heteroaryl, 3-12 membered cycloalkyl-C₁₋₆ alkyl, 3-12 membered heterocyclyl-C₁₋₆ alkyl, 6-10 membered aryl-C₁₋₆ alkyl, 5-10 membered heteroaryl-C₁₋₆ alkyl, OR^{e1}, OC(=O)R^{e1}, OC(=O)NR^{e1}R^{e2}, OS(=O)(=W¹)R^{e1}, OS(=O)(=W¹)NR^{e1}R^{e2}, SR^{e1}, S(=O)(=W¹)R^{e1}, S(=O)(=W¹)NR^{e1}R^{e2}, NR^{e1}R^{e2}, NR^{e1}C(=O)R^{e2}, NR^{e1}S(=O)(=W¹)R^{e2}, NR^{e1}C(=O)OR^{e2}, NR^{e1}C(=O)NR^{e2}, C(=O)R^{e1}, C(=O)NR^{e1}, C(=O)OR^{e1}, PR^{e1}R^{e2}, P(=O)R^{e1}R^{e2}, P(=O)₂R^{e1}R^{e2}, P(=O)OR^{e2}, or oxo (=O), the C₁₋₆ alkyl, C₂₋₆ alkenyl, C₂₋₆ alkynyl, C₀₋₆ alkyl-3-12 membered cycloalkyl, C₀₋₆ alkyl-3-12 membered heterocyclyl, C₀₋₆ alkyl-6-10 membered aryl, C₀₋₆ alkyl-5-10 membered heteroaryl, 3-12 membered cycloalkyl-C₁₋₆ alkyl, 3-12 membered heterocyclyl-C₁₋₆ alkyl, 6-10 membered aryl-C₁₋₆ alkyl, and 5-10 membered heteroaryl-C₁₋₆ alkyl are optionally substituted with one or more R';
or, two R²together with the atoms to which they are respectively attached to form a 3-12 membered cycloalkyl, 3-12 membered cycloalkenyl, 3-12 membered heterocyclyl, 5-14 membered heteroaryl, or 6-14 membered aryl, the 3-12 membered cycloalkyl, 3-12 membered cycloalkenyl, 3-12 membered heterocyclyl, 5-14 membered heteroaryl, or 6-14 membered aryl is optionally substituted with one or more R';
R⁴ is 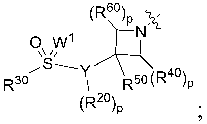
each R⁵ is independently deuterium, halogen, CN, NO2, C₁₋₆ alkyl, C₂₋₆ alkenyl, C₂₋₆ alkynyl, C₀₋₆ alkyl-3-12 membered cycloalkyl, C₀₋₆ alkyl-3-12 membered heterocyclyl, C₀₋₆ alkyl-6-10 membered aryl, C₀₋₆ alkyl-5-10 membered heteroaryl, 3-12 membered cycloalkyl-C₁₋₆ alkyl, 3-12 membered heterocyclyl-C₁₋₆ alkyl, 6-10 membered aryl-C₁₋₆ alkyl, 5-10 membered heteroaryl-C₁₋₆ alkyl, OR^{e1}, OC(=O)R^{e1}, OC(=O)NR^{e1}R^{e2}, OS(=O)(=W¹)R^{e1}, OS(=O)(=W¹)NR^{e1}R^{e2}, SR^{e1}, S(=O)(=W¹)R^{e1}, S(=O)(=W¹)NR^{e1}R^{e2}, NR^{e1}R^{e2}, NR^{e1}C(=O)R^{e2}, NR^{e1}S(=O)(=W¹)R^{e2}, NR^{e1}C(=O)OR^{e2}, NR^{e1}C(=O)NR^{e2}, C(=O)R^{e1}, C(=O)NR^{e1}, C(=O)OR^{e1}, PR^{e1}R^{e2}, P(=O)R^{e1}R^{e2}, P(=O)₂R^{e1}R^{e2}, P(=O)OR^{e2}, or oxo (=O), the C₁₋₆ alkyl, C2-6 alkenyl, C2-₆ alkynyl, C₀₋₆ alkyl-3-12 membered cycloalkyl, C₀₋₆ alkyl-3-12 membered heterocyclyl, C₀₋₆ alkyl-6-10 membered aryl, C₀₋₆ alkyl-5-10 membered heteroaryl, 3-12 membered cycloalkyl-C₁₋₆ alkyl, 3-12 membered heterocyclyl-C₁₋₆ alkyl, 6-10 membered aryl-C₁₋₆ alkyl, and 5-10 membered heteroaryl-C₁₋₆ alkyl are optionally substituted with one or more R';
or, two R⁵together with the atoms to which they are respectively attached to form a 3-12 membered cycloalkyl, 6-12 membered aryl, 5-10 membered heteroaryl, 3-12 membered cycloalkenyl, or 3-12 membered heterocyclyl, the 3-12 membered cycloalkyl, 6-12 membered aryl, 5-10 membered heteroaryl, 3-12 membered cycloalkenyl, or 3-12 membered heterocyclyl is optionally substituted with one or more R';
R^{1a} and R^{2a} are each independently absent or are each independently hydrogen, deuterium, halogen, C₁₋₆ alkyl, halo C₁₋₆ alkyl, C₂₋₆ alkenyl, C₂₋₆ alkynyl, 3-12 membered cycloalkyl, 3-12 membered heterocyclyl, 6-10 membered aryl, 5-10 membered heteroaryl, S(=O)(=W¹)R^{e1}, S(=O)(=W¹)NR^{e1}R^{e2}, C(=O)R^{e1}, C(=O)NR^{e1}, C(=O)OR^{e1}, S(=O)(=W¹)NR^{e1}, P(=O)R^{e1}R^{e2}, P(=O)₂R^{e1}R^{e2}, or P(=O)OR^{e2}, the C₁₋₆ alkyl, halo C₁₋₆ alkyl, C2-6 alkenyl, C2-6 alkynyl, 3-12 membered cycloalkyl, 3-12 membered heterocyclyl, 6-10 membered aryl, or 5-10 membered heteroaryl is optionally substituted with one or more R';
or, R² and R^{1a}together with the atoms to which they are respectively attached to form a 3-12 membered heterocyclyl or 5-12 membered heteroaryl, the 3-12 membered heterocyclyl or 5-12 membered heteroaryl is optionally substituted with one or more R';
or, R³ and R^{1a}together with the atoms to which they are respectively attached to form a 3-12 membered heterocyclyl or 5-12 membered heteroaryl, the 3-12 membered heterocyclyl or 5-12 membered heteroaryl is optionally substituted with one or more R';
R^{3a} is hydrogen, deuterium, halogen, CN, NO₂, C₁₋₆ alkyl, C₂₋₆ alkenyl, C₂₋₆ alkynyl, 3-12 membered cycloalkyl, 3-12 membered heterocyclyl, 6-10 membered aryl, 5-10 membered heteroaryl, OR^{e1}, OC(=O)R^{e1}, OC(=O)NR^{e1}R^{e2}, OS(=O)(=W¹)R^{e1}, OS(=O)(=W¹)NR^{e1}R^{e2}, SR^{e1}, S(=O)(=W¹)R^{e1}, S(=O)(=W¹)NR^{e1}R^{e2}, NR^{e1}R^{e2}, NR^{e1}C(=O)R^{e2}, NR^{e1}C(=O)OR^{e2}, NR^{e1}C(=O)NR^{e2}, NR^{e1}S(=O)(=W¹)R^{e2}, C(=O)R^{e1}, C(=O)NR^{e1}, C(=O)OR^{e1}, PR^{e1}R^{e2}, P(=O)R^{e1}R^{e2}, P(=O)₂R^{e1}R^{e2}, P(=O)OR^{e2}, or oxo (=O), the C₁₋₆ alkyl, C2-6 alkenyl, C2-6 alkynyl, 3-12 membered cycloalkyl, 3-12 membered heterocyclyl, 6-10 membered aryl, or 5-10 membered heteroaryl is optionally substituted with one or more R';
or, R^{2a} and R^{3a}together with the carbon atom to which they are attached to form a 3-12 membered cycloalkyl or 3-12 membered heterocyclyl, the 3-12 membered cycloalkyl or 3-12 membered heterocyclyl is optionally substituted with one or more R';
or, R² and R^{3a}together with the atoms to which they are respectively attached to form a 3-12 membered cycloalkyl, 3-12 membered heterocyclyl, 6-12 membered aryl, or 5-12 membered heteroaryl, the 3-12 membered cycloalkyl, 3-12 membered heterocyclyl, 6-12 membered aryl, or 5-12 membered heteroaryl is optionally substituted with one or more R';
or, R³ and R^{3a}together with the atoms to which they are respectively attached to form a 3-12 membered cycloalkyl, 3-12 membered heterocyclyl, 6-12 membered aryl, or 5-12 membered heteroaryl, the 3-12 membered cycloalkyl, 3-12 membered heterocyclyl, 6-12 membered aryl, or 5-12 membered heteroaryl is optionally substituted with one or more R';
R' is deuterium, halogen, CN, NO₂, C₁₋₆ alkyl, halo C₁₋₆ alkyl, C₂₋₆ alkenyl, C₂₋₆ alkynyl, 3-12 membered cycloalkyl, 3-12 membered heterocyclyl, 6-10 membered aryl, 5-10 membered heteroaryl, OR^{f1}, OC(=O)R^{f1}, OC(=O)NR^{f1}R^{f2}, OS(=O)(=W¹)R^{f1}, OS(=O)(=W¹)NR^{f1}R^{f2}, SR^{f1}, S(=O)(=W¹)R^{f1}, S(=O)(=W¹)NR^{f1}R^{f2}, NR^{f1}R^{f2}, NR^{f1}C(=O)R^{f2}, NR^{f1}S(=O)(=W¹)R^{f2}, NR^{f1}C(=O)OR^{f2}, NR^{f1}C(=O)NR^{f2}, C(=O)R^{f1}, C(=O)NR^{f1}, C(=O)OR^{f1}, - (O)(OR^{f1})₂, PR^{f1}R^{f2}, P(=O)R^{f1}R^{f2}, P(=O)₂R^{f1}R^{f2}, P(=O)OR^{f2}, or oxo (=O);
R^{e1}, R^{e2}, R^{f1}, and R^{f2} are each independently hydrogen, deuterium, C₁₋₆ alkyl, halo Ci-₆ alkyl, C₂₋₆ alkenyl, C₂₋₆ alkynyl, 3-12 membered cycloalkyl, 3-12 membered heterocyclyl, 6-10 membered aryl, or 5-10 membered heteroaryl, the C₁₋₆ alkyl, C₂₋₆ alkenyl, C₂₋₆ alkynyl, 3-12 membered cycloalkyl, 3-12 membered heterocyclyl, 6-10 membered aryl, or 5-10 membered heteroaryl is optionally substituted with one or more R";
or, R^{e1} and R^{e2}together with the atoms to which they are attached to form a 3-12 membered heterocyclyl, the 3-12 membered heterocyclyl is optionally substituted with one or more groups selected from: deuterium, halogen, CN, NO₂, C₁₋₆ alkyl, halo C₁₋₆ alkyl, C₂₋₆ alkenyl, C₂₋₆ alkynyl, 3-12 membered cycloalkyl, 3-12 membered heterocyclyl, 6-10 membered aryl, 5-10 membered heteroaryl, and OR^{g1};
or, R^{f1} and R^{f2}together with the atoms to which they are attached to form a 3-12 membered heterocyclyl, the 3-12 membered heterocyclyl is optionally substituted with one or more groups selected from: deuterium, halogen, CN, NO₂, C₁₋₆ alkyl, halo C₁₋₆ alkyl, C₂₋₆ alkenyl, C₂₋₆ alkynyl, 3-12 membered cycloalkyl, 3-12 membered heterocyclyl, 6-10 membered aryl, 5-10 membered heteroaryl, and OR^{g1};
R" is deuterium, halogen, CN, NO₂, C₁₋₆ alkyl, halo C₁₋₆ alkyl, C₂₋₆ alkenyl, C₂₋₆ alkynyl, 3-12 membered cycloalkyl, 3-12 membered heterocyclyl, 6-10 membered aryl, 5-10 membered heteroaryl, OR^{g1}, OC(=O)R^{g1}, OC(=O)NR^{g1}R^{g2}, OS(=O)(=W¹)R^{g1}, OS(=O)(=W¹)NR^{g1}R^{g2}, SR^{g1}, S(=O)(=W¹)R^{g1}, S(=O)(=W¹)NR^{g1}R^{g2}, NR^{g1}R^{g2}, NR^{g1}C(=O)R^{g2}, NR^{g1}S(=O)(=W¹)R^{g2}, NR^{g1}C(=O)OR^{g2}, NR^{g1}C(=O)NR^{g2}, C(=O)R^{g1}, C(=O)NR^{g1}, C(=O)OR^{g1}, -(O)(OR^{g1})₂, PR^{g1}R^{g2}, P(=O)R^{g1}R^{g2}, P(=O)₂R^{g1}R^{g2}, P(=O)OR^{g2}, or oxo (=O);
R^{g1} and R^{g2} are each independently hydrogen, deuterium, C₁₋₆ alkyl, halo C₁₋₆ alkyl, C₂₋₆ alkenyl, or 3-12 membered cycloalkyl;
W¹ is absent, O, or NR^{g1};
Y is N or C; provided that when Y is C, ring M is pyridine, pyrimidine, pyridone, pyridazine, oxazole, thiazole, imidazole, pyrrole, triazole, or pyridine N-oxide;
R³⁰ is hydrogen, deuterium, C₁₋₆ alkyl, halo C₁₋₆ alkyl, C₂₋₆ alkenyl, C₂₋₆ alkynyl, C₀₋₆ alkyl-3-12 membered cycloalkyl, or C₀₋₆ alkyl-3-12 membered heterocyclyl, the C₁₋₆ alkyl, halo C₁₋₆ alkyl, C₂₋₆ alkenyl, C₂₋₆ alkynyl, C₀₋₆ alkyl-3-12 membered cycloalkyl, and C₀₋₆ alkyl-3-12 membered heterocyclyl are optionally substituted with one or more R';
R²⁰, R⁴⁰, R⁵⁰, and R⁶⁰ are each independently hydrogen, deuterium, halogen, CN, amino, hydroxyl, C₁₋₆ alkyl, halo C₁₋₆ alkyl, C₂₋₆ alkenyl, C₂₋₆ alkynyl, C₁₋₆ alkoxy, C₁₋₆ alkylamino, di(C₁₋₆ alkyl)amino, C₀₋₆ alkyl-3-12 membered cycloalkyl, C₀₋₆ alkyl-3-12 membered heterocyclyl, or oxo (=O), the C₁₋₆ alkyl, halo C₁₋₆ alkyl, C₂₋₆ alkenyl, C₂₋₆ alkynyl, C₁₋₆ alkoxy, C₁₋₆ alkylamino, di(C₁₋₆ alkyl)amino, C₀₋₆ alkyl-3-12 membered cycloalkyl, and C₀₋₆ alkyl-3-12 membered heterocyclyl are optionally substituted with one or more R';
m, n, and t are each independently 0, 1, 2, 3, or 4;
p is each independently 1 or 2.

In certain embodiments, the compound of Formula I is further represented by Formula II: 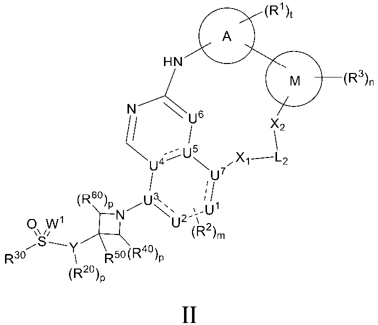
wherein A, M, X₁, X₂, L₂, R¹, R², R³, R²⁰, R³⁰, R⁴⁰, R⁵⁰, R⁶⁰, W¹, Y, m, n, t, and p are as defined above;
--- indicates absent or a single bond;
U¹ is absent or is N or CH;
U² is absent or is N or CH;
U³ is N, C, NR¹⁰⁰, O, S, or C(=O);
U⁴ and U⁵ are each independently N or C, and U⁴ and U⁵ are not both N simultaneously;
U⁶ is N or CH;
U⁷ is C, N, NR²⁰⁰, O, S, C(=O), C(=O)O, C(=O)NR³⁰⁰, OC(=O), or NR³⁰⁰C(=O);
R¹⁰⁰, R²⁰⁰, or R³⁰⁰ are each independently hydrogen, deuterium, C₁₋₆ alkyl, halo C₁₋₆ alkyl, C₀₋₆ alkyl-3-12 membered cycloalkyl, or C₀₋₆ alkyl-3-12 membered heterocyclyl;
When U¹ is absent, U² and U⁷ are connected by a single bond or a double bond; when U² is absent, U¹ and U³ are connected by a single bond or a double bond;
When U¹ and U² are N or CH, at most three among U¹, U², U³, U⁴, U⁵, and U⁷ are N simultaneously;
When Y is C, ring M is pyridine, pyrimidine, pyridone, pyridazine, oxazole, thiazole, imidazole, pyrrole, triazole, or pyridine N-oxide.

In certain embodiments, the compound of Formula I is further represented by Formula III: 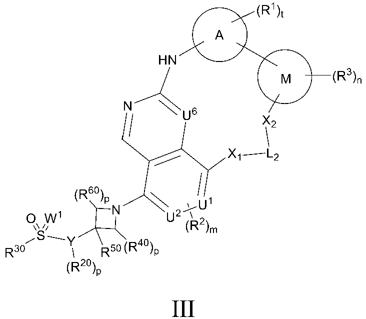
wherein A, M, X₁, X₂, L₂, R¹, R², R³, R²⁰, R³⁰, R⁴⁰, R⁵⁰, R⁶⁰, W¹, Y, m, n, t, and p are as defined above;
U¹, U², or U⁶ are each independently N or CH.

In certain embodiments, the compound of Formula I is further represented by Formula IV: 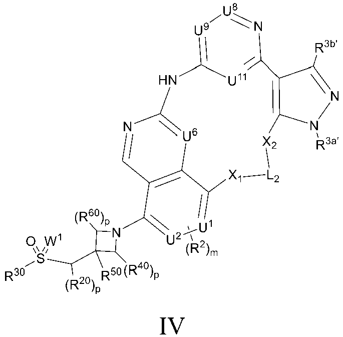
wherein X₁, X₂, R¹, R², R²⁰, R³⁰, R⁴⁰, R⁵⁰, R⁶⁰, W¹, m, and p are as defined above;
L₂ is C₁₋₆ alkylene, C₂₋₆ alkenylene, C₂₋₆ alkynylene, C₁₋₆ heteroalkylene containing 1-3 atoms selected from N, O, S, C(=O), S(=O)(=W¹), C₂₋₆ heteroalkenylene containing 1-3 atoms selected from N, O, S, C(=O), S(=O)(=W¹), the C₁₋₆ alkylene, C₂₋₆ alkenylene, C₂₋₆ alkynylene, C₁₋₆ heteroalkylene containing 1-3 atoms selected from N, O, S, C(=O), S(=O)(=W¹), C₂₋₆ heteroalkenylene containing 1-3 atoms selected from N, O, S, C(=O), S(=O)(=W¹) is optionally substituted with two or more identical or different R⁵;
At least two independent R⁵ together with the atoms to which they are attached to form a 3-12 membered cycloalkyl, 6-12 membered aryl, 5-10 membered heteroaryl, 3-12 membered cycloalkenyl, or 3-12 membered heterocyclyl, the 3-12 membered cycloalkyl, 6-12 membered aryl, 5-10 membered heteroaryl, 3-12 membered cycloalkenyl, or 3-12 membered heterocyclyl is optionally substituted with one or more R';
U¹, U², or U⁶ are each independently N or CH;
U⁸ is N, CR¹, O, S, or C(=O);
U⁹ is absent or is N, CR¹, O, S, or C(=O);
U¹¹ is N, CR¹, O, S, or C(=O);
R^{3a}' is hydrogen, deuterium, C₁₋₆ alkyl, C₂₋₆ alkenyl, C₂₋₆ alkynyl, C₀₋₆ alkyl-3-12 membered cycloalkyl, C₀₋₆ alkyl-3-12 membered heterocyclyl, OR^{e1}, the C₁₋₆ alkyl, C₂₋₆ alkenyl, C₂₋₆ alkynyl, C₀₋₆ alkyl-3-12 membered cycloalkyl, C₀₋₆ alkyl-3-12 membered heterocyclyl is optionally substituted with one or more R';
R^{3b}' is hydrogen, deuterium, halogen, CN, NO₂, C₁₋₆ alkyl, C₂₋₆ alkenyl, C₂₋₆ alkynyl, C₀₋₆ alkyl-3-12 membered cycloalkyl, C₀₋₆ alkyl-3-12 membered heterocyclyl, the C₁₋₆ alkyl, C₂₋₆ alkenyl, C₂₋₆ alkynyl, C₀₋₆ alkyl-3-12 membered cycloalkyl, C₀₋₆ alkyl-3-12 membered heterocyclyl is optionally substituted with one or more R';
R⁵, R', and R^{e1} are as defined above.

In certain embodiments, the compound of Formula I is further represented by Formula V: 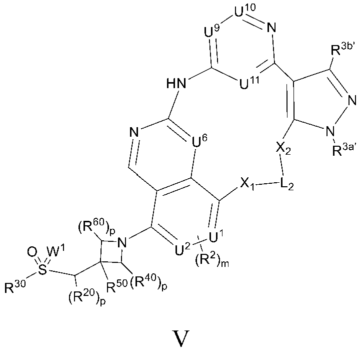
wherein X₁, X₂, R¹, R², R²⁰, R³⁰, R⁴⁰, R⁵⁰, R⁶⁰, W¹, m, and p are as defined above;
L₂ is C₁₋₆ alkylene, C₂₋₆ alkenylene, C₂₋₆ alkynylene, C₁₋₆ heteroalkylene containing 1-3 atoms selected from N, O, S, C(=O), S(=O)(=W¹), or C₂₋₆ heteroalkenylene containing 1-3 atoms selected from N, O, S, C(=O), S(=O)(=W¹), the C₁₋₆ alkylene, C₂₋₆ alkenylene, C₂₋₆ alkynylene, C₁₋₆ heteroalkylene containing 1-3 atoms selected from N, O, S, C(=O), S(=O)(=W¹), or C₂₋₆ heteroalkenylene containing 1-3 atoms selected from N, O, S, C(=O), S(=O)(=W¹) is optionally substituted with one or more identical or different R⁵;
U¹, U², or U⁶ are each independently N or CH;
U⁹ is absent or is N, CR¹, O, S, or C(=O);
U¹⁰ is CR¹';
U¹¹ is N, CR¹, O, S, or C(=O);
R¹' is hydrogen, deuterium, halogen, CN, NO₂, C₁₋₆ alkyl, halo C₁₋₆ alkyl, C₁₋₆ alkoxy, C₂₋₆ alkenyl, C₂₋₆ alkynyl, 3-12 membered cycloalkyl, 3-12 membered heterocyclyl, 6-10 membered aryl, or 5-10 membered heteroaryl, the C₁₋₆ alkyl, halo C₁₋₆ alkyl, C₁₋₆ alkoxy, C₂₋₆ alkenyl, C₂₋₆ alkynyl, 3-12 membered cycloalkyl, 3-12 membered heterocyclyl, 6-10 membered aryl, or 5-10 membered heteroaryl is optionally substituted with one or more R';
R^{3a}' is hydrogen, deuterium, C₁₋₆ alkyl, C₂₋₆ alkenyl, C₂₋₆ alkynyl, C₀₋₆ alkyl-3-12 membered cycloalkyl, C₀₋₆ alkyl-3-12 membered heterocyclyl, OR^{e1}, the C₁₋₆ alkyl, C₂₋₆ alkenyl, C₂₋₆ alkynyl, C₀₋₆ alkyl-3-12 membered cycloalkyl, C₀₋₆ alkyl-3-12 membered heterocyclyl is optionally substituted with one or more R';
R^{3b}' is hydrogen, deuterium, halogen, CN, NO₂, C₁₋₆ alkyl, C₂₋₆ alkenyl, C₂₋₆ alkynyl, C₀₋₆ alkyl-3-12 membered cycloalkyl, C₀₋₆ alkyl-3-12 membered heterocyclyl, the C₁₋₆ alkyl, C₂₋₆ alkenyl, C₂₋₆ alkynyl, C₀₋₆ alkyl-3-12 membered cycloalkyl, C₀₋₆ alkyl-3-12 membered heterocyclyl is optionally substituted with one or more R';
R⁵, R', and R^{e1} are as defined above.

In certain embodiments, in Formula I,
ring E is a 9-10 membered heteroaryl, the 9-10 membered heteroaryl is connected to NH and X₁ via two different atoms, respectively;
preferably, ring E is 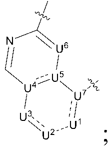
more preferably, ring E is selected from 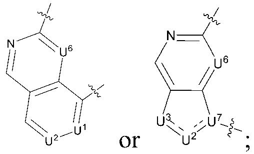
most preferably, ring E is selected from 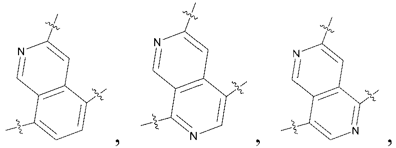 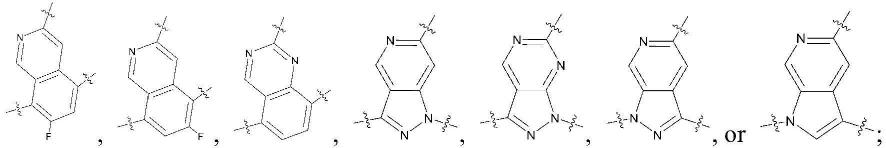
wherein, --- or -- indicates absence or a single bond; U¹, U², U³, U⁴, U⁵, U⁶, or U⁷ are as defined above.

In certain embodiments, ring A is a 5-6 membered heteroaryl;
preferably, ring A is pyridine, pyrimidine, pyridazine, pyrazine, triazine, or thiazole;
more preferably, ring A is selected from the following groups: 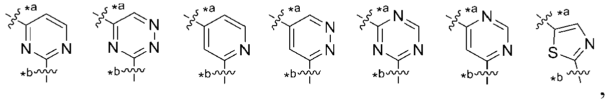 wherein *^{a} indicates the connection site to the N atom of NH, and *^{b} indicates the connection site to ring M.

In certain embodiments, ring M is a 5-6 membered heteroaryl or benzene;
preferably, ring M is pyridine, pyrimidine, pyridazine, imidazole, pyrazole, thiazole, oxazole, triazole, benzene, or pyridine N-oxide;
more preferably, ring M is selected from the following groups: 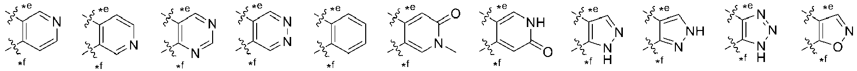 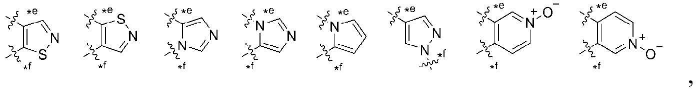 wherein *^{e} indicates the connection site to ring A, and *^{f} indicates the connection site to X₂.

In certain embodiments, L₂ is C₁₋₆ alkylene, C₁₋₆ alkenylene, or C₁₋₆ heteroalkylene containing 1-3 atoms selected from N, O, S, C(=O), S(=O)(=W¹), the C₁₋₆ alkylene, C₁₋₆ alkenylene, or C₁₋₆ heteroalkylene containing 1-3 atoms selected from N, O, S, C(=O), S(=O)(=W¹)is optionally substituted with one or more identical or different R⁵;
preferably, L₂ is methylene, ethylene, propylene, butylene, pentylene, vinylene, propenylene, butenylene, or ethyleneoxy, the methylene, ethylene, propylene, butylene, pentylene, vinylene, propenylene, butenylene, or ethyleneoxy is optionally substituted with one or more identical or different R⁵;

In certain embodiments, X₁ and X₂ are each independently absent or are each independently NR^{1a}, -C(R^{2a})(R^{3a})-, O, C(=O), S(=O), or S(=O)₂;
R^{1a} and R^{2a} are each independently hydrogen, deuterium, halogen, C₁₋₆ alkyl, halo C₁₋₆ alkyl, or 3-12 membered cycloalkyl, the C₁₋₆ alkyl, halo C₁₋₆ alkyl, or 3-12 membered cycloalkyl is optionally substituted with one or more R';
R^{3a} is hydrogen, deuterium, halogen, CN, C₁₋₆ alkyl, hydroxyl, hydroxy C₁₋₆ alkyl, C₁₋₆ alkoxy, amino, C₁₋₆ alkylamino, or di(C₁₋₆ alkyl)amino, the C₁₋₆ alkyl, hydroxy C₁₋₆ alkyl, C₁₋₆ alkoxy, C₁₋₆ alkylamino, or di(C₁₋₆ alkyl)amino is optionally substituted with one or more R';
or, R^{2a} and R^{3a} together with the connecting carbon atom to form a 3-6 membered cycloalkyl or a 3-6 membered heterocyclyl, the 3-6 membered cycloalkyl or 3-6 membered heterocyclyl is optionally substituted with one or more R';
preferably, X₁ is absent or is NR^{1a}, -C(R^{2a})(R^{3a})-, or O;
X₂ is -C(R^{2a})(R^{3a})-, O, C(=O), S(=O), or S(=O)₂;
R^{1a} and R^{2a} are each independently hydrogen, deuterium, F, Cl, methyl, ethyl, isopropyl, cyclopropyl, cyclobutyl, cyclopentyl, cyclohexyl, difluoromethyl, trifluoromethyl, difluoroethyl, trifluoroethyl, or hydroxyethyl;
R^{3a} is hydrogen, F, Cl, Br, methyl, ethyl, isopropyl, cyclopropyl, cyclobutyl, cyclopentyl, cyclohexyl, difluoromethyl, trifluoromethyl, difluoroethyl, trifluoroethyl, hydroxyethyl, amino, methylamino, dimethylamino, methoxy, ethoxy, or isopropoxy;
or, R^{2a} and R^{3a} together with the connecting carbon atom to form cyclopropyl, cyclobutyl, cyclopentyl, cyclohexyl, oxiranyl, or oxetanyl, the cyclopropyl, cyclobutyl, cyclopentyl, cyclohexyl, oxiranyl, or oxetanyl is optionally substituted with one or more R';

In certain embodiments, each R¹ is independently hydrogen, deuterium, halogen, CN, C₁₋₆ alkyl, halo C₁₋₆ alkyl, C₁₋₆ alkoxy, C₂₋₄ alkenyl, C₂₋₄ alkynyl, 3-6 membered cycloalkyl, or oxo, the C₁₋₆ alkyl, halo C₁₋₆ alkyl, C₁₋₆ alkoxy, C₂₋₄ alkenyl, C₂₋₄ alkynyl, or 3-6 membered cycloalkyl is optionally substituted with one or more R';
preferably, each R¹ is independently hydrogen, deuterium, halogen, CN, hydroxyl, hydroxyethyl, amino, methylamino, dimethylamino, ethylamino, diethylamino, cyclopropylamino, methyl, ethyl, isopropyl, vinyl, ethynyl, difluoromethyl, trifluoromethyl, difluoroethyl, trifluoroethyl, cyclopropyl, methoxy, ethoxy, cyclopropoxy, or oxo;
more preferably, each R¹ is independently hydrogen, deuterium, halogen, methyl, ethyl, isopropyl, or cyclopropyl;

In certain embodiments, each R² is independently hydrogen, deuterium, halogen, CN, C₁₋₆ alkyl, halo C₁₋₆ alkyl, C₂₋₆ alkenyl, C₂₋₆ alkynyl, C₁₋₆ alkoxy, C₁₋₆ alkoxy-C₁₋₆ alkyl, amino, C₁₋₄ alkylamino, di(C₁₋₄ alkyl)amino, C₀₋₆ alkyl-3-12 membered cycloalkyl, C₀₋₆ alkyl-3-12 membered heterocyclyl, 3-12 membered cycloalkyl-C₁₋₆ alkyl, 3-12 membered heterocyclyl-C₁₋₆ alkyl, or oxo (=O), the C₁₋₆ alkyl, halo C₁₋₆ alkyl, C₂₋₆ alkenyl, C₂₋₆ alkynyl, C₁₋₆ alkoxy, C₁₋₆ alkoxy-C₁₋₆ alkyl, amino, C₁₋₄ alkylamino, di(C₁₋₄ alkyl)amino, C₀₋₆ alkyl-3-12 membered cycloalkyl, C₀₋₆ alkyl-3-12 membered heterocyclyl, 3-12 membered cycloalkyl-C₁₋₆ alkyl, and 3-12 membered heterocyclyl-C₁₋₆ alkyl are optionally substituted with one or more deuterium, halogen, hydroxyl, methyl, ethyl, methoxy, ethoxy, or cyclopropyl;
preferably, each R² is independently hydrogen, deuterium, halogen, CN, methyl, ethyl, isopropyl, vinyl, ethynyl, difluoromethyl, trifluoromethyl, methoxy, ethoxy, isopropoxy, amino, methylamino, dimethylamino, ethylamino, or diethylamino, the methyl, ethyl, isopropyl, vinyl, ethynyl, difluoromethyl, methoxy, ethoxy, isopropoxy, amino, methylamino, dimethylamino, ethylamino, or diethylamino is optionally substituted with one or more deuterium, halogen, hydroxyl, methoxy, ethoxy, or cyclopropyl.

In certain embodiments, each R³ is independently hydrogen, deuterium, halogen, CN, C₁₋₆ alkyl, halo C₁₋₆ alkyl, C₂₋₄ alkenyl, C₂₋₄ alkynyl, C₁₋₆ alkoxy, C₁₋₆ alkoxy-C₁₋₆ alkyl, amino, C₁₋₄ alkylamino, di(C₁₋₄ alkyl)amino, C₀₋₆ alkyl-3-6 membered cycloalkyl, C₀₋₆ alkyl-3-6 membered heterocyclyl, 3-6 membered cycloalkyl-C₁₋₆ alkyl, 3-6 membered heterocyclyl-C₁₋₆ alkyl, oxo (=O), C(=O)R^{e1}, C(=O)NR^{e1}, or S(=O)₂R^{e1}, the C₁₋₆ alkyl, halo C₁₋₆ alkyl, C₂₋₄ alkenyl, C₂₋₄ alkynyl, C₁₋₆ alkoxy, C₁₋₆ alkoxy-C₁₋₆ alkyl, amino, C₁₋₄ alkylamino, di(C₁₋₄ alkyl)amino, C₀₋₆ alkyl-3-6 membered cycloalkyl, C₀₋₆ alkyl-3-6 membered heterocyclyl, 3-6 membered cycloalkyl-C₁₋₆ alkyl, and 3-6 membered heterocyclyl-C₁₋₆ alkyl is optionally substituted with one or more deuterium, F, Cl, hydroxyl, methyl, ethyl, methoxy, ethoxy, or cyclopropyl;
preferably, each R³ is independently hydrogen, deuterium, halogen, CN, hydroxyl, hydroxyethyl, amino, methylamino, ethylamino, dimethylamino, diethylamino, cyclopropylamino, methyl, ethyl, isopropyl, difluoromethyl, trifluoromethyl, difluoroethyl, trifluoroethyl, cyclopropyl, methoxy, ethoxy, cyclopropoxy, oxo, C(=O)R^{e1}, C(=O)NR^{e1}, or S(=O)₂R^{e1};
or, R³ and R^{1a}together with the atoms to which they are attached to form pyrrole, imidazole, thiazole, oxazole, isothiazole, isoxazole, or pyrazole;
R^{e1} is hydrogen, methyl, ethyl, amino, methylamino, ethylamino, dimethylamino, diethylamino, cyclopropylamino, cyclopropyl, cyclobutyl, cyclopentyl, or cyclohexyl;
more preferably, R³ is hydrogen, deuterium, halogen, methyl, ethyl, cyclopropyl, dimethylamino, C(=O)CH₃, C(=O)-cyclopropyl, C(=O)N(CH₃)₂, or S(=O)₂-cyclopropyl.

In certain embodiments, R⁴ is

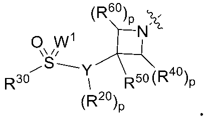

In certain embodiments, R⁴ is R³⁰-S(=O)(W¹)-Y(R²⁰)ₚ-C₀₋₄ alkyl-4-7 membered heterocyclyl, (R³⁰)ₚN-Y(R²⁰)ₚ-C₀₋₄ alkyl-4-7 membered heterocyclyl, R³⁰-S(=O)(W¹)-Y(R²⁰)ₚ-C₀₋₄ alkyl-5-6 membered heteroaryl, or R³⁰-S(=O)(W¹)-Y(R²⁰)ₚ-C₀₋₄ alkyl-4-7 membered cycloalkyl, the R³⁰-S(=O)(W¹)-Y(R²⁰)ₚ-C₀₋₄ alkyl-4-7 membered heterocyclyl, (R³⁰)ₚN-Y(R²⁰)ₚ-C₀₋₄ alkyl-4-7 membered heterocyclyl, R³⁰-S(=O)(W¹)-Y(R²⁰)ₚ-C₀₋₄ alkyl-5-6 membered heteroaryl, or R³⁰-S(=O)(W¹)-Y(R²⁰)ₚ-C₀₋₄ alkyl-4-7 membered cycloalkyl is optionally substituted with one or more R';
preferably, R⁴ is selected from 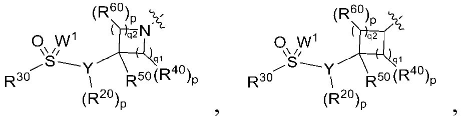 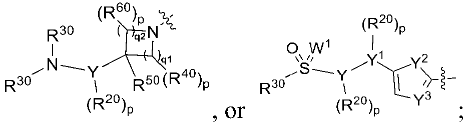
wherein Y is absent or is N or C;
Y¹ is absent or is N or C;
Y² and Y³ are each independently NR⁷⁰, N, CR⁷⁰, O, or S;
W¹ is absent, O, or NR^{g1};
R²⁰, R³⁰, R⁴⁰, R⁵⁰, and R⁶⁰ are as defined above;
each R⁷⁰ is independently hydrogen, deuterium, amino, C₁₋₆ alkyl, halo C₁₋₆ alkyl, C₀₋₂ alkyl-3-12 membered cycloalkyl, or C₀₋₂ alkyl-3-12 membered heterocyclyl, the C₁₋₆ alkyl, halo C₁₋₆ alkyl, C₀₋₂ alkyl-3-12 membered cycloalkyl, or C₀₋₂ alkyl-3-12 membered heterocyclyl is optionally substituted with one or more deuterium, CN, hydroxyl, or halogen;
q1 or q2 are each independently 1, 2, or 3;
or, two R²⁰together with the atoms to which they are respectively attached to form a cyclopropyl, cyclobutyl, cyclopentyl, or cyclohexyl, the cyclopropyl, cyclobutyl, cyclopentyl, or cyclohexyl is optionally substituted with one or more methyl, ethyl, isopropyl, cyclopropyl, difluoromethyl, difluoroethyl, trifluoromethyl, trifluoroethyl, or halogen;
or, two R³⁰together with the atoms to which they are attached to form an azetidinyl, azepanyl, or azocanyl, the azetidinyl, azepanyl, or azocanyl is optionally substituted with one or more methyl, ethyl, isopropyl, cyclopropyl, difluoromethyl, difluoroethyl, trifluoromethyl, trifluoroethyl, or halogen;
or, R²⁰ and R⁵⁰together with the atoms to which they are respectively attached to form an azetidinyl, azepanyl, azocanyl, cyclobutyl, cyclopentyl, or cyclohexyl, the azetidinyl, azepanyl, azocanyl, cyclobutyl, cyclopentyl, or cyclohexyl is optionally substituted with one or more methyl, ethyl, isopropyl, cyclopropyl, difluoromethyl, difluoroethyl, trifluoromethyl, trifluoroethyl, halogen, or cyano;
or, R⁴⁰ and R⁶⁰together with the atoms to which they are respectively attached to form an azetidinyl, azepanyl, or azocanyl, the azetidinyl, azepanyl, or azocanyl is optionally substituted with one or more methyl, ethyl, isopropyl, cyclopropyl, difluoromethyl, difluoroethyl, trifluoromethyl, trifluoroethyl, halogen, or cyano;
more preferably, R⁴ is selected from

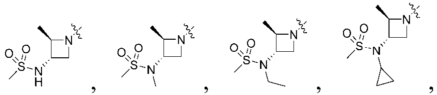

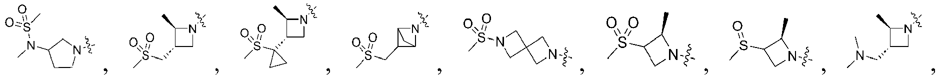

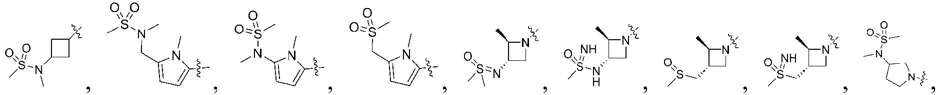

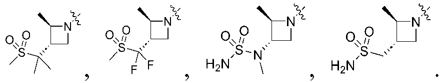

In certain embodiments, R⁵ is hydrogen, deuterium, halogen, CN, C₁₋₆ alkyl, C₀₋₂ alkyl-3-12 membered cycloalkyl, C₀₋₂ alkyl-3-12 membered heterocyclyl, C₁₋₆ alkoxy, or oxo, the C₁₋₆ alkyl, C₀₋₂ alkyl-3-12 membered cycloalkyl, C₀₋₂ alkyl-3-12 membered heterocyclyl, or C₁₋₆ alkoxy is optionally substituted with one or more R';
Alternatively, R⁵ is hydrogen, deuterium, halogen, CN, hydroxyl, C₁₋₆ alkyl, C₂₋₄ alkenyl, C₂₋₄ alkynyl, C₁₋₆ alkylamino, di(C₁₋₆ alkyl)amino, 3-6 membered cycloalkylamino, C₀₋₂ alkyl-3-6 membered cycloalkyl, C₀₋₂ alkyl-3-7 membered heterocyclyl, C₁₋₆ alkoxy, or oxo, the C₁₋₆ alkyl, C₂₋₄ alkenyl, C₂₋₄ alkynyl, C₁₋₆ alkylamino, di(C₁₋₆ alkyl)amino, 3-6 membered cycloalkylamino, C₀₋₂ alkyl-3-6 membered cycloalkyl, C₀₋₂ alkyl-3-7 membered heterocyclyl, C₁₋₆ alkoxy is optionally substituted with one or more hydroxyl, amino, cyano, halogen, C₁₋₄ alkyl, 3-6 membered cycloalkyl, or oxo;
or, two R⁵together with the atoms to which they are respectively attached to form a 3-6 membered cycloalkyl, phenyl, 4-7 membered heterocyclyl, 5-6 membered heterocyclyl , or 3-6 membered cycloalkenyl, the 3-6 membered cycloalkyl, phenyl, 4-7 membered heterocyclyl, 5-6 membered heterocyclyl , or 3-6 membered cycloalkenyl is optionally substituted with one or more R';
preferably, R⁵ is hydrogen, deuterium, halogen, CN, hydroxyl, hydroxyethyl, amino, methylamino, dimethylamino, cyclopropylamino, methyl, ethyl, difluoromethyl, trifluoromethyl, difluoroethyl, trifluoroethyl, isopropyl, cyclopropyl, methoxy, ethoxy, difluoromethoxy, trifluoromethoxy, difluoroethoxy, trifluoroethoxy, cyclopropoxy, or oxo;
or, two R⁵together with the atoms to which they are respectively attached to form a cyclopropyl, cyclobutyl, cyclopentyl, cyclohexyl, oxiranyl, oxetanyl, tetrahydrofuranyl, pyranyl, azetidinyl, or piperidinyl, the cyclopropyl, cyclobutyl, cyclopentyl, cyclohexyl, oxiranyl, oxetanyl, tetrahydrofuranyl, pyranyl, azetidinyl, or piperidinyl is optionally substituted with one or more R'.

In certain embodiments, R' is deuterium, halogen, CN, hydroxyl, hydroxyethyl, amino, methylamino, dimethylamino, cyclopropylamino, methyl, ethyl, isopropyl, difluoromethyl, trifluoromethyl, difluoroethyl, trifluoroethyl, isopropyl, cyclopropyl, methoxy, ethoxy, difluoromethoxy, trifluoromethoxy, difluoroethoxy, trifluoroethoxy, cyclopropoxy, or oxo.

In certain embodiments, R²⁰, R⁴⁰, R⁵⁰, and R⁶⁰ are each independently hydrogen, deuterium, halogen, CN, amino, hydroxyl, C₁₋₆ alkyl, halo C₁₋₆ alkyl, C₁₋₆ alkoxy, C₁₋₆ alkylamino, di(C₁₋₆ alkyl)amino, C₀₋₂ alkyl-3-12 membered cycloalkyl, C₀₋₂ alkyl-3-12 membered heterocyclyl, or oxo (=O), the C₁₋₆ alkyl, halo C₁₋₆ alkyl, C₁₋₆ alkoxy, C₁₋₆ alkylamino, di(C₁₋₆ alkyl)amino, C₀₋₂ alkyl-3-12 membered cycloalkyl, or C₀₋₂ alkyl-3-12 membered heterocyclyl is optionally substituted with one or more deuterium, hydroxyl, C₁₋₃ alkyl, or halogen;
preferably, R²⁰, R⁴⁰, R⁵⁰, and R⁶⁰ are each independently hydrogen, deuterium, F, Cl, Br, CN, hydroxyl, amino, methyl, ethyl, isopropyl, cyclopropyl, cyclobutyl, cyclopentyl, cyclohexyl, difluoromethyl, trifluoromethyl, difluoroethyl, trifluoroethyl, hydroxyethyl, methylamino, dimethylamino, methoxy, ethylamino, diethylamino, ethoxy, or oxo, the amino, methyl, ethyl, isopropyl, cyclopropyl, cyclobutyl, cyclopentyl, cyclohexyl, difluoroethyl, trifluoroethyl, hydroxyethyl, methylamino, dimethylamino, ethylamino, diethylamino, methoxy, or ethoxy is optionally substituted with one or more halogen, methyl, or deuterium.

In certain embodiments, each R³⁰ is independently hydrogen, deuterium, amino, C₁₋₆ alkyl, halo C₁₋₆ alkyl, C₀₋₂ alkyl-3-12 membered cycloalkyl, or C₀₋₂ alkyl-3-12 membered heterocyclyl, the amino, C₁₋₆ alkyl, halo C₁₋₆ alkyl, C₀₋₂ alkyl-3-12 membered cycloalkyl, or C₀₋₂ alkyl-3-12 membered heterocyclyl is optionally substituted with one or more deuterium, CN, hydroxyl, or halogen;
preferably, each R³⁰ is independently hydrogen, amino, methyl, ethyl, isopropyl, cyclopropyl, cyclobutyl, cyclopentyl, cyclohexyl, difluoromethyl, trifluoromethyl, difluoroethyl, trifluoroethyl, or hydroxyethyl, the amino, methyl, ethyl, isopropyl, cyclopropyl, cyclobutyl, cyclopentyl, cyclohexyl, difluoromethyl, difluoroethyl, trifluoroethyl, or hydroxyethyl is optionally substituted with one or more halogen or deuterium.

In certain embodiments, W¹ is absent, O, or NR^{g1}; R^{g1} is hydrogen, methyl, ethyl, or cyclopropyl;
preferably, W¹ is O.

In certain embodiments, Y is N or C; with the proviso that when Y is C, ring M is pyridine, pyrimidine, pyridone, pyridazine, oxazole, thiazole, imidazole, pyrrole, triazole, or pyridine N-oxide;
preferably, Y is N.

In certain embodiments,
R^{3a}' is hydrogen, deuterium, methyl, ethyl, propyl, trideuteriomethyl, difluoromethyl, trifluoromethyl, difluoroethyl, trifluoroethyl, vinyl, ethynyl, cyclopropyl, cyclobutyl, oxiranyl, oxetanyl, aziridinyl, or azetidinyl, the methyl, ethyl, propyl, vinyl, ethynyl, cyclopropyl, cyclobutyl, oxiranyl, oxetanyl, aziridinyl, or azetidinyl is optionally substituted with one or more groups selected from: deuterium, fluorine, chlorine, bromine, CN, methyl, ethyl, isopropyl, cyclopropyl, cyclobutyl, and oxo;
R^{3b}' is hydrogen, deuterium, fluorine, chlorine, bromine, CN, NO₂, methyl, ethyl, propyl, trideuteriomethyl, difluoromethyl, trifluoromethyl, difluoroethyl, trifluoroethyl, vinyl, ethynyl, cyclopropyl, cyclobutyl, oxiranyl, oxetanyl, aziridinyl, or azetidinyl, the methyl, ethyl, propyl, vinyl, ethynyl, cyclopropyl, cyclobutyl, oxiranyl, oxetanyl, aziridinyl, or azetidinyl is optionally substituted with one or more groups selected from: deuterium, fluorine, chlorine, bromine, CN, methyl, ethyl, isopropyl, cyclopropyl, cyclobutyl, and oxo;
R¹' is hydrogen, deuterium, fluorine, chlorine, bromine, CN, NO₂, hydroxyl, hydroxyethyl, amino, methylamino, dimethylamino, ethylamino, diethylamino, cyclopropylamino, methyl, ethyl, isopropyl, vinyl, ethynyl, trideuteriomethyl, difluoromethyl, trifluoromethyl, difluoroethyl, trifluoroethyl, cyclopropyl, methoxy, ethoxy, cyclopropoxy, or oxo.

In certain embodiments, the compound further has a structure represented by Formula VI: 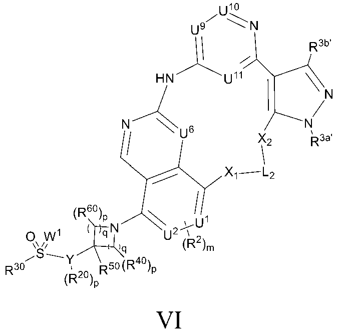 wherein,
L₂ is C₁₋₆ alkylene, C₂₋₆ alkenylene, C₂₋₆ alkynylene, C₁₋₆ heteroalkylene containing 1-3 atoms selected from N, O, S, C(=O), S(=O)(=W¹), or C₂₋₆ heteroalkenylene containing 1-3 atoms selected from N, O, S, C(=O), S(=O)(=W¹), the C₁₋₆ alkylene, C₂₋₆ alkenylene, C₂₋₆ alkynylene, C₁₋₆ heteroalkylene containing 1-3 atoms selected from N, O, S, C(=O), S(=O)(=W¹), or C₂₋₆ heteroalkenylene containing 1-3 atoms selected from N, O, S, C(=O), S(=O)(=W¹) is optionally substituted with one or more identical or different R⁵;
each R⁵ is independently deuterium, halogen, CN, NO₂, C₁₋₆ alkyl, C₂₋₆ alkenyl, C₂₋₆ alkynyl, C₀₋₆ alkyl-3-12 membered cycloalkyl, C₀₋₆ alkyl-3-12 membered heterocyclyl, C₀₋₆ alkyl-6-10 membered aryl, C₀₋₆ alkyl-5-10 membered heteroaryl, 3-12 membered cycloalkyl-C₁₋₆ alkyl, 3-12 membered heterocyclyl-C₁₋₆ alkyl, 6-10 membered aryl-C₁₋₆ alkyl, 5-10 membered heteroaryl-C₁₋₆ alkyl, OR^{e1}, OC(=O)R^{e1}, OC(=O)NR^{e1}R^{e2}, OS(=O)(=W¹)R^{e1}, OS(=O)(=W¹)NR^{e1}R^{e2}, SR^{e1}, S(=O)(=W¹)R^{e1}, S(=O)(=W¹)NR^{e1}R^{e2}, NR^{e1}R^{e2}, NR^{e1}C(=O)R^{e2}, NR^{e1}S(=O)(=W¹)R^{e2}, NR^{e1}C(=O)OR^{e2}, NR^{e1}C(=O)NR^{e2}, C(=O)R^{e1}, C(=O)NR^{e1}, C(=O)OR^{e1}, PR^{e1}R^{e2}, P(=O)R^{e1}R^{e2}, P(=O)₂R^{e1}R^{e2}, P(=O)OR^{e2}, or oxo (=O), the C₁₋₆ alkyl, C₂₋₆ alkenyl, C₂₋₆ alkynyl, C₀₋₆ alkyl-3-12 membered cycloalkyl, C₀₋₆ alkyl-3-12 membered heterocyclyl, C₀₋₆ alkyl-6-10 membered aryl, C₀₋₆ alkyl-5-10 membered heteroaryl, 3-12 membered cycloalkyl-C₁₋₆ alkyl, 3-12 membered heterocyclyl-C₁₋₆ alkyl, 6-10 membered aryl-C₁₋₆ alkyl, and 5-10 membered heteroaryl-C₁₋₆ alkyl are optionally substituted with one or more R';
or, two R⁵together with the atoms to which they are respectively attached to form a 3-12 membered cycloalkyl, 6-12 membered aryl, 5-10 membered heteroaryl, 3-12 membered cycloalkenyl, or 3-12 membered heterocyclyl, the 3-12 membered cycloalkyl, 6-12 membered aryl, 5-10 membered heteroaryl, 3-12 membered cycloalkenyl, or 3-12 membered heterocyclyl is optionally substituted with one or more R';
q is 1, 2, or 3;
Y, X₁, X₂, R¹, R², R²⁰, R³⁰, R⁴⁰, R⁵⁰, R⁶⁰, W¹, U¹, U², U⁶, U⁹, U¹⁰, U¹¹, R^{3a}', R^{3b}', m, and p are as defined above;
preferably, L₂ is methylene, ethylene, propylene, butylene, pentylene, vinylene, propenylene, butenylene, or ethyleneoxy, the methylene, ethylene, propylene, butylene, pentylene, vinylene, propenylene, butenylene, or ethyleneoxy is optionally substituted with one or more identical or different R⁵;
R⁵ is hydrogen, deuterium, halogen, CN, C₁₋₆ alkyl, C₀₋₂ alkyl-3-12 membered cycloalkyl, C₀₋₂ alkyl-3-12 membered heterocyclyl, C₁₋₆ alkoxy, or oxo, the C₁₋₆ alkyl, C₀₋₂ alkyl-3-12 membered cycloalkyl, C₀₋₂ alkyl-3-12 membered heterocyclyl, or C₁₋₆ alkoxy is optionally substituted with one or more R';
or, two R⁵together with the atoms to which they are respectively attached to form a 3-6 membered cycloalkyl, phenyl, 4-7 membered heterocyclyl, 5-6 membered heterocyclyl or 3-6 membered cycloalkenyl, the 3-6 membered cycloalkyl, phenyl, 4-7 membered heterocyclyl, 5-6 membered heterocyclyl, or 3-6 membered cycloalkenyl is optionally substituted with one or more R';
more preferably, L₂ is 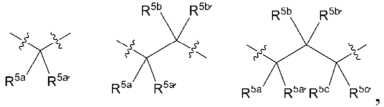 wherein R^{5a}, R^{5b}, R^{5c}, R^{5a'}, R^{5b'}, and R^{5c'} are each independently R⁵; R⁵ is as defined above; preferably, R^{5a}, R^{5b}, R^{5c}, R^{5a'}, R^{5b'}, and R^{5c'} are each independently hydrogen, deuterium, halogen, CN, hydroxyl, C₁₋₆ alkyl, C₂₋₄ alkenyl, C₂₋₄ alkynyl, C₁₋₆ alkylamino, di(C₁₋₆ alkyl)amino, 3-6 membered cycloalkylamino, C₀₋₂ alkyl-3-6 membered cycloalkyl, C₀₋₂ alkyl-3-7 membered heterocyclyl, C₁₋₆ alkoxy, or oxo, the C₁₋₆ alkyl, C₂₋₄ alkenyl, C₂₋₄ alkynyl, C₁₋₆ alkylamino, di(C₁₋₆ alkyl)amino, 3-6 membered cycloalkylamino, C₀₋₂ alkyl-3-6 membered cycloalkyl, C₀₋₂ alkyl-3-7 membered heterocyclyl, or C₁₋₆ alkoxy is optionally substituted with one or more hydroxyl, amino, cyano, halogen, C₁₋₄ alkyl, 3-6 membered cycloalkyl, or oxo;
or, R^{5a} and R^{5a'}together with the atoms to which they are attached to form a 3-6 membered cycloalkyl, 3-7 membered heterocyclyl, or 3-6 membered cycloalkenyl, the 3-6 membered cycloalkyl, 3-7 membered heterocyclyl, or 3-6 membered cycloalkenyl is optionally substituted with one or more R';
or, R^{5b} and R^{5b'}together with the atoms to which they are attached to form a 3-6 membered cycloalkyl, 3-7 membered heterocyclyl, or 3-6 membered cycloalkenyl, the 3-6 membered cycloalkyl, 3-7 membered heterocyclyl, or 3-6 membered cycloalkenyl is optionally substituted with one or more R';
or, R^{5c} and R^{5c'}together with the atoms to which they are attached to form a 3-6 membered cycloalkyl, 3-7 membered heterocyclyl, or 3-6 membered cycloalkenyl, the 3-6 membered cycloalkyl, 3-7 membered heterocyclyl, or 3-6 membered cycloalkenyl is optionally substituted with one or more R';
or, R^{5a} and R^{5b}together with the atoms to which they are attached to form a 3-6 membered cycloalkyl, 3-7 membered heterocyclyl, or 3-6 membered cycloalkenyl, the 3-6 membered cycloalkyl, 3-7 membered heterocyclyl, or 3-6 membered cycloalkenyl is optionally substituted with one or more R';
or, R^{5a} and R^{5c}together with the atoms to which they are attached to form a 3-6 membered cycloalkyl, 3-7 membered heterocyclyl, or 3-6 membered cycloalkenyl, the 3-6 membered cycloalkyl, 3-7 membered heterocyclyl, or 3-6 membered cycloalkenyl is optionally substituted with one or more R';
or, R^{5b} and R^{5c}together with the atoms to which they are attached to form a 3-6 membered cycloalkyl, 3-7 membered heterocyclyl, or 3-6 membered cycloalkenyl, the 3-6 membered cycloalkyl, 3-7 membered heterocyclyl, or 3-6 membered cycloalkenyl is optionally substituted with one or more R'.

In certain embodiments, the compound further has a structure represented by Formula VII: 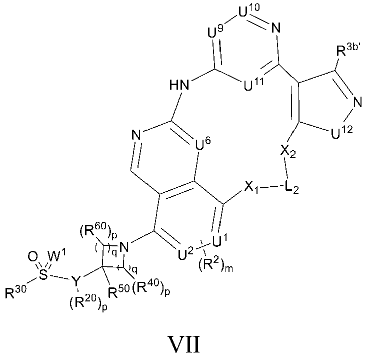
wherein, U¹² is -N(R^{3a}')-, -C(R^{3a}')=C(R^{3a}')-;
Y, X₁, X₂, L₂, R¹, R², R²⁰, R³⁰, R⁴⁰, R⁵⁰, R⁶⁰, W¹, p, q, U¹, U², U⁶, U⁹, U¹⁰, U¹¹, R^{3a}', and R^{3b}' are as defined above;
In certain embodiments, in Formula I:
   ring A is a 5-6 membered heteroaryl;
   ring M is a 5-6 membered heteroaryl or benzene;
   ring E is 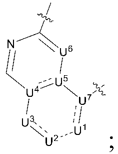
   L₂ is C₁₋₆ alkylene, C₁₋₆ alkenylene, or C₁₋₆ heteroalkylene containing 1-3 atoms selected from N, O, S, C(=O), S(=O)(=W¹), the C₁₋₆ alkylene, C₁₋₆ alkenylene, or C₁₋₆ heteroalkylene containing 1-3 atoms selected from N, O, S, C(=O), S(=O)(=W¹)is optionally substituted with one or more identical or different R⁵;
   X₁ and X₂ are each independently absent or are each independently NR^{1a}, - C(R^{2a})(R^{3a})-, O, C(=O), S(=O), or S(=O)₂;
   each R¹ is independently hydrogen, deuterium, halogen, CN, hydroxyl, hydroxyethyl, amino, methylamino, dimethylamino, ethylamino, diethylamino, cyclopropylamino, methyl, ethyl, isopropyl, vinyl, ethynyl, difluoromethyl, trifluoromethyl, difluoroethyl, trifluoroethyl, cyclopropyl, methoxy, ethoxy, cyclopropoxy, or oxo;
   each R² is independently hydrogen, deuterium, halogen, CN, C₁₋₆ alkyl, halo C₁₋₆ alkyl, C₂₋₆ alkenyl, C₂₋₆ alkynyl, C₁₋₆ alkoxy, C₁₋₆ alkoxy-C₁₋₆ alkyl, amino, C₁₋₄ alkylamino, di(C₁₋₄ alkyl)amino, C₀₋₆ alkyl-3-12 membered cycloalkyl, C₀₋₆ alkyl-3-12 membered heterocyclyl, 3-12 membered cycloalkyl-C₁₋₆ alkyl, 3-12 membered heterocyclyl-C₁₋₆ alkyl, or oxo (=O), the C₁₋₆ alkyl, halo C₁₋₆ alkyl, C₂₋₆ alkenyl, C₂₋₆ alkynyl, C₁₋₆ alkoxy, C₁₋₆ alkoxy-C₁₋₆ alkyl, amino, C₁₋₄ alkylamino, di(C₁₋₄ alkyl)amino, C₀₋₆ alkyl-3-12 membered cycloalkyl, C₀₋₆ alkyl-3-12 membered heterocyclyl, 3-12 membered cycloalkyl-C₁₋₆ alkyl, and 3-12 membered heterocyclyl-C₁₋₆ alkyl are optionally substituted with one or more deuterium, halogen, hydroxyl, methyl, ethyl, methoxy, ethoxy, or cyclopropyl;
   each R³ is independently hydrogen, deuterium, halogen, CN, C₁₋₆ alkyl, halo C₁₋₆ alkyl, C₂₋₄ alkenyl, C₂₋₄ alkynyl, C₁₋₆ alkoxy, C₁₋₆ alkoxy-C₁₋₆ alkyl, amino, C₁₋₄ alkylamino, di(C₁₋₄ alkyl)amino, C₀₋₆ alkyl-3-6 membered cycloalkyl, C₀₋₆ alkyl-3-6 membered heterocyclyl, 3-6 membered cycloalkyl-C₁₋₆ alkyl, 3-6 membered heterocyclyl-C₁₋₆ alkyl, oxo (=O), C(=O)R^{e1}, C(=O)NR^{e1}, or S(=O)₂R^{e1}, the C₁₋₆ alkyl, halo C₁₋₆ alkyl, C₂₋₄ alkenyl, C₂₋₄ alkynyl, C₁₋₆ alkoxy, C₁₋₆ alkoxy-C₁₋₆ alkyl, amino, C₁₋₄ alkylamino, di(C₁₋₄ alkyl)amino, C₀₋₆ alkyl-3-6 membered cycloalkyl, C₀₋₆ alkyl-3-6 membered heterocyclyl, 3-6 membered cycloalkyl-C₁₋₆ alkyl, and 3-6 membered heterocyclyl-C₁₋₆ alkyl is optionally substituted with one or more deuterium, F, Cl, hydroxyl, methyl, ethyl, methoxy, ethoxy, or cyclopropyl;
   R⁴ is 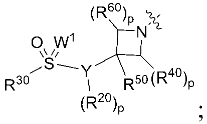
   R⁵ is hydrogen, deuterium, halogen, CN, C₁₋₆ alkyl, C₀₋₂ alkyl-3-12 membered cycloalkyl, C₀₋₂ alkyl-3-12 membered heterocyclyl, C₁₋₆ alkoxy, or oxo, the C₁₋₆ alkyl, C₀₋₂ alkyl-3-12 membered cycloalkyl, C₀₋₂ alkyl-3-12 membered heterocyclyl, or C₁₋₆ alkoxy is optionally substituted with one or more R';
   or, two R⁵together with the atoms to which they are respectively attached to form a 3-6 membered cycloalkyl, phenyl, 4-7 membered heterocyclyl, 5-6 membered heterocyclyl, or 3-6 membered cycloalkenyl, the 3-6 membered cycloalkyl, phenyl, 4-7 membered heterocyclyl, 5-6 membered heterocyclyl, or 3-6 membered cycloalkenyl is optionally substituted with one or more R';
   R^{1a} and R^{2a} are each independently hydrogen, deuterium, halogen, C₁₋₆ alkyl, halo C₁₋₆ alkyl, or 3-12 membered cycloalkyl, the C₁₋₆ alkyl, halo C₁₋₆ alkyl, or 3-12 membered cycloalkyl is optionally substituted with one or more R';
   R^{3a} is hydrogen, deuterium, halogen, CN, C₁₋₆ alkyl, hydroxyl, hydroxy C₁₋₆ alkyl, C₁₋₆ alkoxy, amino, C₁₋₆ alkylamino, or di(C₁₋₆ alkyl)amino, the C₁₋₆ alkyl, hydroxy C₁₋₆ alkyl, C₁₋₆ alkoxy, C₁₋₆ alkylamino, or di(C₁₋₆ alkyl)amino is optionally substituted with one or more R';
   or, R³ and R^{1a}together with the atoms to which they are attached to form pyrrole, imidazole, thiazole, oxazole, isothiazole, isoxazole, or pyrazole;
   or, R^{2a} and R^{3a}together with the carbon atom to which they are attached to form a 3-6 membered cycloalkyl or a 3-6 membered heterocyclyl, the 3-6 membered cycloalkyl or 3-6 membered heterocyclyl is optionally substituted with one or more R';
   R^{e1} is hydrogen, methyl, ethyl, amino, methylamino, ethylamino, dimethylamino, diethylamino, cyclopropylamino, cyclopropyl, cyclobutyl, cyclopentyl, or cyclohexyl;
   W¹ is absent, O, or NH;
   Y is N or C; provided that when Y is C, ring M is pyridine, pyrimidine, pyridone, pyridazine, oxazole, thiazole, imidazole, pyrrole, triazole, or pyridine N-oxide;
   --- indicates absent or a single bond;
   U¹ is absent or is N or C;
   U² is absent or is N or C;
   U³ is C or C(=O);
   U⁴ and U⁵ are each independently C;
   U⁶ is N or CH;
   U⁷ is C, N, N-methyl, N-ethyl, or N-cyclopropyl;
   R²⁰, R⁴⁰, R⁵⁰, and R⁶⁰ are each independently hydrogen, deuterium, halogen, CN, amino, hydroxyl, C₁₋₆ alkyl, halo C₁₋₆ alkyl, C₁₋₆ alkoxy, C₁₋₆ alkylamino, di(C₁₋₆ alkyl)amino, C₀₋₂ alkyl-3-12 membered cycloalkyl, C₀₋₂ alkyl-3-12 membered heterocyclyl, or oxo (=O), the C₁₋₆ alkyl, halo C₁₋₆ alkyl, C₁₋₆ alkoxy, C₁₋₆ alkylamino, di(C₁₋₆ alkyl)amino, C₀₋₂ alkyl-3-12 membered cycloalkyl, or C₀₋₂ alkyl-3-12 membered heterocyclyl is optionally substituted with one or more deuterium, hydroxyl, C₁₋₃ alkyl, or halogen;
   each R³⁰ is independently hydrogen, deuterium, amino, C₁₋₆ alkyl, halo C₁₋₆ alkyl, C₀₋₂ alkyl-3-12 membered cycloalkyl, or C₀₋₂ alkyl-3-12 membered heterocyclyl, the amino, C₁₋₆ alkyl, halo C₁₋₆ alkyl, C₀₋₂ alkyl-3-12 membered cycloalkyl, or C₀₋₂ alkyl-3-12 membered heterocyclyl is optionally substituted with one or more deuterium, CN, hydroxyl, or halogen;
   R' is deuterium, halogen, CN, hydroxyl, hydroxyethyl, amino, methylamino, dimethylamino, cyclopropylamino, methyl, ethyl, isopropyl, difluoromethyl, trifluoromethyl, difluoroethyl, trifluoroethyl, cyclopropyl, methoxy, ethoxy, difluoromethoxy, trifluoromethoxy, difluoroethoxy, trifluoroethoxy, cyclopropoxy, or oxo;
   m, n, and t are each independently 0, 1, 2, 3, or 4;
   p is each independently 1 or 2.

In certain specific embodiments, in Formula I:
ring A is a 5-6 membered heteroaryl;
ring M is a 5-6 membered heteroaryl or benzene;
ring E is 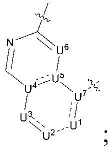
L₂ is C₁₋₆ alkylene, C₁₋₆ alkenylene, or C₁₋₆ heteroalkylene containing 1-3 atoms selected from N, O, S, C(=O), S(=O)(=W¹) , the C₁₋₆ alkylene, C₁₋₆ alkenylene, or C₁₋₆ heteroalkylene containing 1-3 atoms selected from N, O, S, C(=O), S(=O)(=W¹) is optionally substituted with one or more identical or different R⁵;
X₁ and X₂ are each independently absent or are each independently NR^{1a}, - C(R^{2a})(R^{3a})-, O, C(=O), S(=O), or S(=O)₂;
each R¹ is independently hydrogen, deuterium, halogen, CN, hydroxyl, hydroxyethyl, amino, methylamino, dimethylamino, ethylamino, diethylamino, cyclopropylamino, methyl, ethyl, isopropyl, vinyl, ethynyl, difluoromethyl, trifluoromethyl, difluoroethyl, trifluoroethyl, cyclopropyl, methoxy, ethoxy, cyclopropoxy, or oxo;
each R² is independently hydrogen, deuterium, halogen, CN, C₁₋₆ alkyl, halo C₁₋₆ alkyl, C₂₋₆ alkenyl, C₂₋₆ alkynyl, C₁₋₆ alkoxy, C₁₋₆ alkoxy-C₁₋₆ alkyl, amino, C₁₋₄ alkylamino, di(C₁₋₄ alkyl)amino, C₀₋₆ alkyl-3-12 membered cycloalkyl, C₀₋₆ alkyl-3-12 membered heterocyclyl, 3-12 membered cycloalkyl-C₁₋₆ alkyl, 3-12 membered heterocyclyl-C₁₋₆ alkyl, or oxo (=O), the C₁₋₆ alkyl, halo C₁₋₆ alkyl, C₂₋₆ alkenyl, C₂₋₆ alkynyl, C₁₋₆ alkoxy, C₁₋₆ alkoxy-C₁₋₆ alkyl, amino, C₁₋₄ alkylamino, di(C₁₋₄ alkyl)amino, C₀₋₆ alkyl-3-12 membered cycloalkyl, C₀₋₆ alkyl-3-12 membered heterocyclyl, 3-12 membered cycloalkyl-C₁₋₆ alkyl, and 3-12 membered heterocyclyl-C₁₋₆ alkyl are optionally substituted with one or more deuterium, halogen, hydroxyl, methyl, ethyl, methoxy, ethoxy, or cyclopropyl;
each R³ is independently hydrogen, deuterium, halogen, CN, C₁₋₆ alkyl, halo C₁₋₆ alkyl, C₂₋₄ alkenyl, C₂₋₄ alkynyl, C₁₋₆ alkoxy, C₁₋₆ alkoxy-C₁₋₆ alkyl, amino, C₁₋₄ alkylamino, di(C₁₋₄ alkyl)amino, C₀₋₆ alkyl-3-6 membered cycloalkyl, C₀₋₆ alkyl-3-6 membered heterocyclyl, 3-6 membered cycloalkyl-C₁₋₆ alkyl, 3-6 membered heterocyclyl-C₁₋₆ alkyl, oxo (=O), C(=O)R^{e1}, C(=O)NR^{e1}, or S(=O)₂R^{e1}, the C₁₋₆ alkyl, halo C₁₋₆ alkyl, C₂₋₄ alkenyl, C₂₋₄ alkynyl, C₁₋₆ alkoxy, C₁₋₆ alkoxy-C₁₋₆ alkyl, amino, C₁₋₄ alkylamino, di(C₁₋₄ alkyl)amino, C₀₋₆ alkyl-3-6 membered cycloalkyl, C₀₋₆ alkyl-3-6 membered heterocyclyl, 3-6 membered cycloalkyl-C₁₋₆ alkyl, and 3-6 membered heterocyclyl-C₁₋₆ alkyl are optionally substituted with one or more deuterium, F, Cl, hydroxyl, methyl, ethyl, methoxy, ethoxy, or cyclopropyl;
R⁴ is 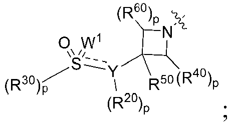
R⁵ is hydrogen, deuterium, halogen, CN, C₁₋₆ alkyl, C₀₋₂ alkyl-3-12 membered cycloalkyl, C₀₋₂ alkyl-3-12 membered heterocyclyl, C₁₋₆ alkoxy, or oxo, the C₁₋₆ alkyl, C₀₋₂ alkyl-3-12 membered cycloalkyl, C₀₋₂ alkyl-3-12 membered heterocyclyl, or C₁₋₆ alkoxy is optionally substituted with one or more R';
or, two R⁵together with the atoms to which they are respectively attached to form a 3-6 membered cycloalkyl, phenyl, 4-7 membered heterocyclyl, 5-6 membered heterocyclyl, or 3-6 membered cycloalkenyl, the 3-6 membered cycloalkyl, phenyl, 4-7 membered heterocyclyl, 5-6 membered heterocyclyl , or 3-6 membered cycloalkenyl is optionally substituted with one or more R';
R^{1a} and R^{2a} are each independently hydrogen, deuterium, halogen, C₁₋₆ alkyl, halo C₁₋₆ alkyl, or 3-12 membered cycloalkyl, the C₁₋₆ alkyl, halo C₁₋₆ alkyl, or 3-12 membered cycloalkyl is optionally substituted with one or more R';
R^{3a} is hydrogen, deuterium, halogen, CN, C₁₋₆ alkyl, hydroxyl, hydroxy C₁₋₆ alkyl, C₁₋₆ alkoxy, amino, C₁₋₆ alkylamino, or di(C₁₋₆ alkyl)amino, the C₁₋₆ alkyl, hydroxy C₁₋₆ alkyl, C₁₋₆ alkoxy, C₁₋₆ alkylamino, or di(C₁₋₆ alkyl)amino is optionally substituted with one or more R';
or, R³ and R^{1a}together with the atoms to which they are attached to form pyrrole, imidazole, thiazole, oxazole, isothiazole, isoxazole, or pyrazole;
or, R^{2a} and R^{3a}together with the carbon atom to which they are attached to form a 3-6 membered cycloalkyl or a 3-6 membered heterocyclyl, the 3-6 membered cycloalkyl or 3-6 membered heterocyclyl is optionally substituted with one or more R';
R^{e1} is hydrogen, methyl, ethyl, amino, methylamino, ethylamino, dimethylamino, diethylamino, cyclopropylamino, cyclopropyl, cyclobutyl, cyclopentyl, or cyclohexyl;
W¹ is absent, O, or NH;
Y is N or C;
--- indicates absent or a single bond;
U¹ is absent or is N or C;
U² is absent or is N or C;
U³ is C or C(=O);
U⁴ and U⁵ are each independently C;
U⁶ is N or CH;
U⁷ is C, N, N-methyl, N-ethyl, or N-cyclopropyl;
R²⁰, R⁴⁰, R⁵⁰, and R⁶⁰ are each independently hydrogen, deuterium, halogen, CN, amino, hydroxyl, C₁₋₆ alkyl, halo C₁₋₆ alkyl, C₁₋₆ alkoxy, C₁₋₆ alkylamino, di(C₁₋₆ alkyl)amino, C₀₋₂ alkyl-3-12 membered cycloalkyl, C₀₋₂ alkyl-3-12 membered heterocyclyl, or oxo (=O), the C₁₋₆ alkyl, halo C₁₋₆ alkyl, C₁₋₆ alkoxy, C₁₋₆ alkylamino, di(C₁₋₆ alkyl)amino, C₀₋₂ alkyl-3-12 membered cycloalkyl, or C₀₋₂ alkyl-3-12 membered heterocyclyl is optionally substituted with one or more deuterium, hydroxyl, C₁₋₃ alkyl, or halogen;
each R³⁰ is independently hydrogen, deuterium, amino, C₁₋₆ alkyl, halo C₁₋₆ alkyl, C₀₋₂ alkyl-3-12 membered cycloalkyl, or C₀₋₂ alkyl-3-12 membered heterocyclyl, the amino, C₁₋₆ alkyl, halo C₁₋₆ alkyl, C₀₋₂ alkyl-3-12 membered cycloalkyl, or C₀₋₂ alkyl-3-12 membered heterocyclyl is optionally substituted with one or more deuterium, CN, hydroxyl, or halogen;
R' is deuterium, halogen, CN, hydroxyl, hydroxyethyl, amino, methylamino, dimethylamino, cyclopropylamino, methyl, ethyl, isopropyl, difluoromethyl, trifluoromethyl, difluoroethyl, trifluoroethyl, cyclopropyl, methoxy, ethoxy, difluoromethoxy, trifluoromethoxy, difluoroethoxy, trifluoroethoxy, cyclopropoxy, or oxo;
m, n, and t are each independently 0, 1, 2, 3, or 4;
p is each independently 1 or 2.

In certain specific embodiments, in Formula VI or Formula VII,
L₂ is C₁₋₆ alkylene, C₁₋₆ alkenylene, or C₁₋₆ heteroalkylene containing 1-3 atoms selected from N, O, S, C(=O), S(=O)(=W¹), the C₁₋₆ alkylene, C₁₋₆ alkenylene, or C₁₋₆ heteroalkylene containing 1-3 atoms selected from N, O, S, C(=O), S(=O)(=W¹) is optionally substituted with one or more identical or different R⁵;
X₁ is absent or is NR^{1a}, -C(R^{2a})(R^{3a})-, or O;
X₂ is -C(R^{2a})(R^{3a})-, O, C(=O), S(=O), or S(=O)₂;
each R² is independently hydrogen, deuterium, halogen, CN, C₁₋₆ alkyl, halo C₁₋₆ alkyl, C₂₋₆ alkenyl, C₂₋₆ alkynyl, C₁₋₆ alkoxy, C₁₋₆ alkoxy-C₁₋₆ alkyl, amino, C₁₋₄ alkylamino, di(C₁₋₄ alkyl)amino, C₀₋₆ alkyl-3-12 membered cycloalkyl, C₀₋₆ alkyl-3-12 membered heterocyclyl, 3-12 membered cycloalkyl-C₁₋₆ alkyl, 3-12 membered heterocyclyl-C₁₋₆ alkyl, or oxo (=O), the C₁₋₆ alkyl, halo C₁₋₆ alkyl, C₂₋₆ alkenyl, C₂₋₆ alkynyl, C₁₋₆ alkoxy, C₁₋₆ alkoxy-C₁₋₆ alkyl, amino, C₁₋₄ alkylamino, di(C₁₋₄ alkyl)amino, C₀₋₆ alkyl-3-12 membered cycloalkyl, C₀₋₆ alkyl-3-12 membered heterocyclyl, 3-12 membered cycloalkyl-C₁₋₆ alkyl, and 3-12 membered heterocyclyl-C₁₋₆ alkyl are optionally substituted with one or more deuterium, halogen, hydroxyl, methyl, ethyl, methoxy, ethoxy, or cyclopropyl;
each R⁵ is independently hydrogen, deuterium, halogen, CN, C₁₋₆ alkyl, C₀₋₂ alkyl-3-12 membered cycloalkyl, C₀₋₂ alkyl-3-12 membered heterocyclyl, C₁₋₆ alkoxy, or oxo, the C₁₋₆ alkyl, C₀₋₂ alkyl-3-12 membered cycloalkyl, C₀₋₂ alkyl-3-12 membered heterocyclyl, or C₁₋₆ alkoxy is optionally substituted with one or more R';
or, two R⁵together with the atoms to which they are respectively attached to form a 3-6 membered cycloalkyl, phenyl, 4-7 membered heterocyclyl, 5-6 membered heterocyclyl, or 3-6 membered cycloalkenyl, the 3-6 membered cycloalkyl, phenyl, 4-7 membered heterocyclyl, 5-6 membered heterocyclyl, or 3-6 membered cycloalkenyl is optionally substituted with one or more R';
R^{1a} and R^{2a} are each independently hydrogen, deuterium, halogen, C₁₋₆ alkyl, halo C₁₋₆ alkyl, or 3-12 membered cycloalkyl, the C₁₋₆ alkyl, halo C₁₋₆ alkyl, or 3-12 membered cycloalkyl is optionally substituted with one or more R';
R^{3a} is hydrogen, deuterium, halogen, CN, C₁₋₆ alkyl, hydroxyl, hydroxy C₁₋₆ alkyl, C₁₋₆ alkoxy, amino, C₁₋₆ alkylamino, or di(C₁₋₆ alkyl)amino, the C₁₋₆ alkyl, hydroxy C₁₋₆ alkyl, C₁₋₆ alkoxy, C₁₋₆ alkylamino, or di(C₁₋₆ alkyl)amino is optionally substituted with one or more R';
or, R² and R^{1a}together with the atoms to which they are respectively attached to form a 3-12 membered heterocyclyl or 5-12 membered heteroaryl, the 3-12 membered heterocyclyl or 5-12 membered heteroaryl is optionally substituted with one or more R';
or, R³ and R^{1a}together with the atoms to which they are respectively attached to form a 3-12 membered heterocyclyl or 5-12 membered heteroaryl, the 3-12 membered heterocyclyl or 5-12 membered heteroaryl is optionally substituted with one or more R';
or, R² and R^{3a}together with the atoms to which they are respectively attached to form a 3-12 membered cycloalkyl, 3-12 membered heterocyclyl, 6-12 membered aryl, or 5-12 membered heteroaryl, the 3-12 membered cycloalkyl, 3-12 membered heterocyclyl, 6-12 membered aryl, or 5-12 membered heteroaryl is optionally substituted with one or more R';
or, R³ and R^{3a}together with the atoms to which they are respectively attached to form a 3-12 membered cycloalkyl, 3-12 membered heterocyclyl, 6-12 membered aryl, or 5-12 membered heteroaryl, the 3-12 membered cycloalkyl, 3-12 membered heterocyclyl, 6-12 membered aryl, or 5-12 membered heteroaryl is optionally substituted with one or more R';
or, R^{2a} and R^{3a}together with the carbon atom to which they are attached to form a 3-12 membered cycloalkyl or 3-12 membered heterocyclyl, the 3-12 membered cycloalkyl or 3-12 membered heterocyclyl is optionally substituted with one or more R';
Y is N or C;
W¹ is absent, O, or NH;
U¹ is N or C;
U² is N or C;
U⁶ is N or CH;
U⁹ is absent or is N, CR¹, O, S, or C(=O);
U¹⁰ is CR¹';
U¹¹ is N, CR¹, O, S, or C(=O);
U¹² is -N(R^{3a}')-, -C(R^{3a}')=C(R^{3a}')-;
R²⁰, R⁴⁰, R⁵⁰, R⁶⁰ are each independently hydrogen, deuterium, halogen, CN, amino, hydroxyl, C₁₋₆ alkyl, halo C₁₋₆ alkyl, C₁₋₆ alkoxy, C₁₋₆ alkylamino, di(C₁₋₆ alkyl)amino, C₀₋₂ alkyl-3-12 membered cycloalkyl, or C₀₋₂ alkyl-3-12 membered heterocyclyl, or oxo (=O), the C₁₋₆ alkyl, halo C₁₋₆ alkyl, C₁₋₆ alkoxy, C₁₋₆ alkylamino, di(C₁₋₆ alkyl)amino, C₀₋₂ alkyl-3-12 membered cycloalkyl, or C₀₋₂ alkyl-3-12 membered heterocyclyl is optionally substituted with one or more deuterium, hydroxyl, C₁₋₃ alkyl, or halogen;
or, two R²⁰together with the atoms to which they are respectively attached to form a 3-6 membered cycloalkyl or 3-7 membered heterocyclyl, the 3-6 membered cycloalkyl or 3-7 membered heterocyclyl is optionally substituted with one or more R';
each R³⁰ is independently hydrogen, deuterium, amino, C₁₋₆ alkyl, halo C₁₋₆ alkyl, C₀₋₂ alkyl-3-12 membered cycloalkyl, or C₀₋₂ alkyl-3-12 membered heterocyclyl, the C₁₋₆ alkyl, halo C₁₋₆ alkyl, C₀₋₂ alkyl-3-12 membered cycloalkyl, or C₀₋₂ alkyl-3-12 membered heterocyclyl is optionally substituted with one or more deuterium, CN, hydroxyl, or halogen;
or, two R³⁰together with the atoms to which they are respectively attached to form a 3-6 membered cycloalkyl or 3-7 membered heterocyclyl, the 3-6 membered cycloalkyl or 3-7 membered heterocyclyl is optionally substituted with one or more R';
R^{3a}' is hydrogen, deuterium, C₁₋₆ alkyl, C₂₋₆ alkenyl, C₂₋₆ alkynyl, C₀₋₆ alkyl-3-12 membered cycloalkyl, C₀₋₆ alkyl-3-12 membered heterocyclyl, OR^{e1}, the C₁₋₆ alkyl, C₂₋₆ alkenyl, C₂₋₆ alkynyl, C₀₋₆ alkyl-3-12 membered cycloalkyl, C₀₋₆ alkyl-3-12 membered heterocyclyl is optionally substituted with one or more R';
R^{3b}' is hydrogen, deuterium, halogen, CN, NO₂, C₁₋₆ alkyl, C₂₋₆ alkenyl, C₂₋₆ alkynyl, C₀₋₆ alkyl-3-12 membered cycloalkyl, C₀₋₆ alkyl-3-12 membered heterocyclyl, the C₁₋₆ alkyl, C₂₋₆ alkenyl, C₂₋₆ alkynyl, C₀₋₆ alkyl-3-12 membered cycloalkyl, C₀₋₆ alkyl-3-12 membered heterocyclyl is optionally substituted with one or more R';
R¹' is hydrogen, deuterium, halogen, CN, NO₂, C₁₋₆ alkyl, halo C₁₋₆ alkyl, C₁₋₆ alkoxy, C₂₋₆ alkenyl, C₂₋₆ alkynyl, 3-12 membered cycloalkyl, 3-12 membered heterocyclyl, 6-10 membered aryl, or 5-10 membered heteroaryl, the C₁₋₆ alkyl, halo C₁₋₆ alkyl, C₁₋₆ alkoxy, C₂₋₆ alkenyl, C₂₋₆ alkynyl, 3-12 membered cycloalkyl, 3-12 membered heterocyclyl, 6-10 membered aryl, or 5-10 membered heteroaryl is optionally substituted with one or more R';
m is 0, 1, 2, 3, or 4;
p is each independently 1 or 2;
q is 1, 2, or 3.

In other specific embodiments,
ring A is pyridine, pyrimidine, pyridazine, pyrazine, triazine, or thiazole;
ring M is pyridine, pyrimidine, pyridazine, imidazole, pyrazole, thiazole, oxazole, triazole, benzene, or pyridine N-oxide;
ring E is 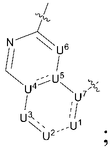
L₂ is methylene, ethylene, propylene, butylene, pentylene, vinylene, propenylene, butenylene, or ethyleneoxy, the methylene, ethylene, propylene, butylene, pentylene, vinylene, propenylene, butenylene, or ethyleneoxy is optionally substituted with one or more identical or different R⁵;
X₁ is absent or is NR^{1a}, -C(R^{2a})(R^{3a})-, or O;
X₂ is -C(R^{2a})(R^{3a})-, O, C(=O), S(=O), or S(=O)₂;
each R¹ is independently hydrogen, deuterium, halogen, methyl, ethyl, isopropyl, or cyclopropyl;
each R² is independently hydrogen, deuterium, halogen, CN, methyl, ethyl, isopropyl, vinyl, ethynyl, difluoromethyl, trifluoromethyl, methoxy, ethoxy, isopropoxy, amino, methylamino, dimethylamino, ethylamino, or diethylamino, the methyl, ethyl, isopropyl, vinyl, ethynyl, difluoromethyl, methoxy, ethoxy, isopropoxy, amino, methylamino, dimethylamino, ethylamino, or diethylamino is optionally substituted with one or more deuterium, halogen, hydroxyl, methoxy, ethoxy, or cyclopropyl;
R³ is hydrogen, deuterium, halogen, methyl, ethyl, cyclopropyl, dimethylamino, C(=O)CH₃, C(=O)-cyclopropyl, C(=O)N(CH₃)₂, or S(=O)₂-cyclopropyl;
R⁴ is 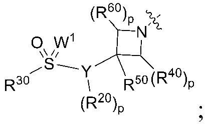
R⁵ is hydrogen, deuterium, halogen, CN, hydroxyl, hydroxyethyl, amino, methylamino, dimethylamino, cyclopropylamino, methyl, ethyl, difluoromethyl, trifluoromethyl, difluoroethyl, trifluoroethyl, isopropyl, cyclopropyl, methoxy, ethoxy, difluoromethoxy, trifluoromethoxy, difluoroethoxy, trifluoroethoxy, cyclopropoxy, or oxo;
or, two R⁵ together with the atoms to which they are respectively attached to form a cyclopropyl, cyclobutyl, cyclopentyl, cyclohexyl, oxiranyl, oxetanyl, tetrahydrofuranyl, pyranyl, azetidinyl, or piperidinyl, the cyclopropyl, cyclobutyl, cyclopentyl, cyclohexyl, oxiranyl, oxetanyl, tetrahydrofuranyl, pyranyl, azetidinyl, or piperidine is optionally substituted with one or more R';
R^{1a} and R^{2a} are each independently hydrogen, deuterium, F, Cl, methyl, ethyl, isopropyl, cyclopropyl, cyclobutyl, cyclopentyl, cyclohexyl, difluoromethyl, trifluoromethyl, difluoroethyl, trifluoroethyl, or hydroxyethyl;
R^{3a} is hydrogen, F, Cl, Br, methyl, ethyl, isopropyl, cyclopropyl, cyclobutyl, cyclopentyl, cyclohexyl, difluoromethyl, trifluoromethyl, difluoroethyl, trifluoroethyl, hydroxyethyl, amino, methylamino, dimethylamino, methoxy, ethoxy, or isopropoxy;
or, R^{2a} and R^{3a} together with the carbon atom to which they are attached to form a cyclopropyl, cyclobutyl, cyclopentyl, cyclohexyl, oxiranyl, or oxetanyl, the cyclopropyl, cyclobutyl, cyclopentyl, cyclohexyl, oxiranyl, or oxetanyl is optionally substituted with one or more R';
W¹ is absent, O, or NH;
Y is N or C; provided that when Y is C, ring M is pyridine, pyrimidine, oxazole, thiazole, imidazole, triazole, or pyridine N-oxide;
--- indicates absent or a single bond;
U¹ is N or C;
U² is N or C;
U³, U⁴, and U⁵ are each independently C;
U⁶ is N or CH;
U⁷ is C;
R²⁰, R⁴⁰, R⁵⁰, and R⁶⁰ are each independently hydrogen, deuterium, F, Cl, Br, CN, hydroxyl, amino, methyl, ethyl, isopropyl, cyclopropyl, cyclobutyl, cyclopentyl, cyclohexyl, difluoromethyl, trifluoromethyl, difluoroethyl, trifluoroethyl, hydroxyethyl, methylamino, dimethylamino, ethylamino, diethylamino, methoxy, ethoxy, or oxo, the amino, methyl, ethyl, isopropyl, cyclopropyl, cyclobutyl, cyclopentyl, cyclohexyl, difluoroethyl, trifluoroethyl, hydroxyethyl, methylamino, dimethylamino, ethylamino, diethylamino, methoxy, or ethoxy is optionally substituted with one or more halogen, methyl, or deuterium;
each R³⁰ is independently hydrogen, amino, methyl, ethyl, isopropyl, cyclopropyl, cyclobutyl, cyclopentyl, cyclohexyl, difluoromethyl, trifluoromethyl, difluoroethyl, trifluoroethyl, or hydroxyethyl, the amino, methyl, ethyl, isopropyl, cyclopropyl, cyclobutyl, cyclopentyl, cyclohexyl, difluoromethyl, difluoroethyl, trifluoroethyl, or hydroxyethyl is optionally substituted with one or more halogen or deuterium;
R' is deuterium, halogen, CN, hydroxyl, hydroxyethyl, amino, methylamino, dimethylamino, cyclopropylamino, methyl, ethyl, isopropyl, difluoromethyl, trifluoromethyl, difluoroethyl, trifluoroethyl, cyclopropyl, methoxy, ethoxy, difluoromethoxy, trifluoromethoxy, difluoroethoxy, trifluoroethoxy, cyclopropoxy, or oxo;
m, n, and t are each independently 0, 1, 2, 3, or 4;
p is each independently 1 or 2.

In other specific embodiments, in Formula IV,
L₂ is methylene, ethylene, propylene, butylene, pentylene, vinylene, propenylene, butenylene, or ethyleneoxy, the methylene, ethylene, propylene, butylene, pentylene, vinylene, propenylene, butenylene, or ethyleneoxy is optionally substituted with one or more identical or different R⁵;
X₁ is absent or is NR^{1a}, -C(R^{2a})(R^{3a})-, or O;
X₂ is -C(R^{2a})(R^{3a})-, O, C(=O), S(=O), or S(=O)₂;
each R¹ is independently hydrogen, deuterium, halogen, methyl, ethyl, isopropyl, vinyl, ethynyl, or cyclopropyl;
each R² is independently hydrogen, deuterium, halogen, CN, methyl, ethyl, isopropyl, vinyl, ethynyl, difluoromethyl, trifluoromethyl, methoxy, ethoxy, isopropoxy, amino, methylamino, dimethylamino, ethylamino, or diethylamino, the methyl, ethyl, isopropyl, vinyl, ethynyl, difluoromethyl, methoxy, ethoxy, isopropoxy, amino, methylamino, dimethylamino, ethylamino, or diethylamino is optionally substituted with one or more deuterium, halogen, hydroxyl, methoxy, ethoxy, or cyclopropyl;
R⁵ is hydrogen, deuterium, halogen, CN, hydroxyl, hydroxyethyl, amino, methylamino, dimethylamino, cyclopropylamino, methyl, ethyl, difluoromethyl, trifluoromethyl, difluoroethyl, trifluoroethyl, isopropyl, cyclopropyl, methoxy, ethoxy, difluoromethoxy, trifluoromethoxy, difluoroethoxy, trifluoroethoxy, cyclopropoxy, or oxo;
at least two R⁵ together with the atoms to which they are attached to form cyclopropyl, cyclobutyl, cyclopentyl, cyclohexyl, oxiranyl, oxetanyl, tetrahydrofuranyl, pyranyl, azetidinyl, or piperidinyl, the cyclopropyl, cyclobutyl, cyclopentyl, cyclohexyl, oxiranyl, oxetanyl, tetrahydrofuranyl, pyranyl, azetidinyl, or piperidine is optionally substituted with one or more R';
R^{1a} and R^{2a} are each independently hydrogen, deuterium, F, Cl, methyl, ethyl, isopropyl, cyclopropyl, cyclobutyl, cyclopentyl, cyclohexyl, difluoromethyl, trifluoromethyl, difluoroethyl, trifluoroethyl, or hydroxyethyl;
R^{3a} is hydrogen, F, Cl, Br, methyl, ethyl, isopropyl, cyclopropyl, cyclobutyl, cyclopentyl, cyclohexyl, difluoromethyl, trifluoromethyl, difluoroethyl, trifluoroethyl, hydroxyethyl, amino, methylamino, dimethylamino, methoxy, ethoxy, or isopropoxy;
or, R^{2a} and R^{3a} together with the carbon atom to which they are attached to form a cyclopropyl, cyclobutyl, cyclopentyl, cyclohexyl, oxiranyl, or oxetanyl, the cyclopropyl, cyclobutyl, cyclopentyl, cyclohexyl, oxiranyl, or oxetanyl is optionally substituted with one or more R';
W¹ is absent, O, or NH;
U¹ is N or C;
U² is N or C;
U⁶ is N or CH;
U⁸ is N, CR¹, O, S, or C(=O);
U⁹ is absent or is N, CR¹, O, S, or C(=O);
U¹¹ is N, CR¹, O, S, or C(=O);
R²⁰, R⁴⁰, R⁵⁰, and R⁶⁰ are each independently hydrogen, deuterium, F, Cl, Br, CN, hydroxyl, amino, methyl, ethyl, isopropyl, cyclopropyl, cyclobutyl, cyclopentyl, cyclohexyl, difluoromethyl, trifluoromethyl, difluoroethyl, trifluoroethyl, hydroxyethyl, methylamino, dimethylamino, ethylamino, diethylamino, methoxy, ethoxy, or oxo, the amino, methyl, ethyl, isopropyl, cyclopropyl, cyclobutyl, cyclopentyl, cyclohexyl, difluoroethyl, trifluoroethyl, hydroxyethyl, methylamino, dimethylamino, ethylamino, diethylamino, methoxy, or ethoxy is optionally substituted with one or more halogen, methyl, or deuterium;
each R³⁰ is independently hydrogen, amino, methyl, ethyl, isopropyl, cyclopropyl, cyclobutyl, cyclopentyl, cyclohexyl, difluoromethyl, trifluoromethyl, difluoroethyl, trifluoroethyl, or hydroxyethyl, the amino, methyl, ethyl, isopropyl, cyclopropyl, cyclobutyl, cyclopentyl, cyclohexyl, difluoromethyl, difluoroethyl, trifluoroethyl, or hydroxyethyl is optionally substituted with one or more halogen or deuterium;
R^{3a}' is hydrogen, deuterium, methyl, ethyl, propyl, trideuteriomethyl, difluoromethyl, trifluoromethyl, difluoroethyl, trifluoroethyl, vinyl, ethynyl, cyclopropyl, cyclobutyl, oxiranyl, oxetanyl, aziridinyl, or azetidinyl, the methyl, ethyl, propyl, vinyl, ethynyl, cyclopropyl, cyclobutyl, oxiranyl, oxetanyl, aziridinyl, or azetidinyl is optionally substituted with one or more groups selected from: deuterium, fluorine, chlorine, bromine, CN, methyl, ethyl, isopropyl, cyclopropyl, cyclobutyl, and oxo;
R^{3b}' is hydrogen, deuterium, fluorine, chlorine, bromine, CN, NO₂, methyl, ethyl, propyl, trideuteriomethyl, difluoromethyl, trifluoromethyl, difluoroethyl, trifluoroethyl, vinyl, ethynyl, cyclopropyl, cyclobutyl, oxiranyl, oxetanyl, aziridinyl, or azetidinyl, the methyl, ethyl, propyl, vinyl, ethynyl, cyclopropyl, cyclobutyl, oxiranyl, oxetanyl, aziridinyl, or azetidinyl is optionally substituted with one or more groups selected from: deuterium, fluorine, chlorine, bromine, CN, methyl, ethyl, isopropyl, cyclopropyl, cyclobutyl, and oxo;
R' is deuterium, halogen, CN, hydroxyl, hydroxyethyl, amino, methylamino, dimethylamino, cyclopropylamino, methyl, ethyl, isopropyl, difluoromethyl, trifluoromethyl, difluoroethyl, trifluoroethyl, cyclopropyl, methoxy, ethoxy, difluoromethoxy, trifluoromethoxy, difluoroethoxy, trifluoroethoxy, cyclopropoxy, or oxo;
m is 0, 1, 2, 3, or 4;
p is each independently 1 or 2.

In other specific embodiments, in Formula V,
L₂ is methylene, ethylene, propylene, butylene, pentylene, vinylene, propenylene, butenylene, or ethyleneoxy, the methylene, ethylene, propylene, butylene, pentylene, vinylene, propenylene, butenylene, or ethyleneoxy is optionally substituted with one or more identical or different R⁵;
X₁ is absent or is NR^{1a}, -C(R^{2a})(R^{3a})-, or O;
X₂ is -C(R^{2a})(R^{3a})-, O, C(=O), S(=O), or S(=O)₂;
each R¹ is independently hydrogen, deuterium, halogen, methyl, ethyl, isopropyl, vinyl, ethynyl, or cyclopropyl;
each R² is independently hydrogen, deuterium, halogen, CN, methyl, ethyl, isopropyl, vinyl, ethynyl, difluoromethyl, trifluoromethyl, methoxy, ethoxy, isopropoxy, amino, methylamino, dimethylamino, ethylamino, or diethylamino, the methyl, ethyl, isopropyl, vinyl, ethynyl, difluoromethyl, methoxy, ethoxy, isopropoxy, amino, methylamino, dimethylamino, ethylamino, or diethylamino is optionally substituted with one or more deuterium, halogen, hydroxyl, methoxy, ethoxy, or cyclopropyl;
R⁵ is hydrogen, deuterium, halogen, CN, hydroxyl, hydroxyethyl, amino, methylamino, dimethylamino, cyclopropylamino, methyl, ethyl, difluoromethyl, trifluoromethyl, difluoroethyl, trifluoroethyl, isopropyl, cyclopropyl, methoxy, ethoxy, difluoromethoxy, trifluoromethoxy, difluoroethoxy, trifluoroethoxy, cyclopropoxy, or oxo;
R^{1a} and R^{2a} are each independently hydrogen, deuterium, F, Cl, methyl, ethyl, isopropyl, cyclopropyl, cyclobutyl, cyclopentyl, cyclohexyl, difluoromethyl, trifluoromethyl, difluoroethyl, trifluoroethyl, or hydroxyethyl;
R^{3a} is hydrogen, F, Cl, Br, methyl, ethyl, isopropyl, cyclopropyl, cyclobutyl, cyclopentyl, cyclohexyl, difluoromethyl, trifluoromethyl, difluoroethyl, trifluoroethyl, hydroxyethyl, amino, methylamino, dimethylamino, methoxy, ethoxy, or isopropoxy;
or, R^{2a} and R^{3a} together with the carbon atom to which they are attached to form a cyclopropyl, cyclobutyl, cyclopentyl, cyclohexyl, oxiranyl, or oxetanyl, the cyclopropyl, cyclobutyl, cyclopentyl, cyclohexyl, oxiranyl, or oxetanyl is optionally substituted with one or more R';
W¹ is absent, O, or NH;
U¹ is N or C;
U² is N or C;
U⁶ is N or CH;
U⁹ is absent or is N, CR¹, O, S, or C(=O);
U¹⁰ is CR¹';
U¹¹ is N, CR¹, O, S, or C(=O);
R²⁰, R⁴⁰, R⁵⁰, and R⁶⁰ are each independently hydrogen, deuterium, F, Cl, Br, CN, hydroxyl, amino, methyl, ethyl, isopropyl, cyclopropyl, cyclobutyl, cyclopentyl, cyclohexyl, difluoromethyl, trifluoromethyl, difluoroethyl, trifluoroethyl, hydroxyethyl, methylamino, dimethylamino, ethylamino, diethylamino, methoxy, ethoxy, or oxo, the amino, methyl, ethyl, isopropyl, cyclopropyl, cyclobutyl, cyclopentyl, cyclohexyl, difluoroethyl, trifluoroethyl, hydroxyethyl, methylamino, dimethylamino, ethylamino, diethylamino, methoxy, or ethoxy is optionally substituted with one or more halogen, methyl, or deuterium;
each R³⁰ is independently hydrogen, amino, methyl, ethyl, isopropyl, cyclopropyl, cyclobutyl, cyclopentyl, cyclohexyl, difluoromethyl, trifluoromethyl, difluoroethyl, trifluoroethyl, or hydroxyethyl, the amino, methyl, ethyl, isopropyl, cyclopropyl, cyclobutyl, cyclopentyl, cyclohexyl, difluoromethyl, difluoroethyl, trifluoroethyl, or hydroxyethyl is optionally substituted with one or more halogen or deuterium;
R^{3a}' is hydrogen, deuterium, methyl, ethyl, propyl, trideuteriomethyl, difluoromethyl, trifluoromethyl, difluoroethyl, trifluoroethyl, vinyl, ethynyl, cyclopropyl, cyclobutyl, oxiranyl, oxetanyl, aziridinyl, or azetidinyl, the methyl, ethyl, propyl, vinyl, ethynyl, cyclopropyl, cyclobutyl, oxiranyl, oxetanyl, aziridinyl, or azetidinyl is optionally substituted with one or more groups selected from: deuterium, fluorine, chlorine, bromine, CN, methyl, ethyl, isopropyl, cyclopropyl, cyclobutyl, and oxo;
R^{3b}' is hydrogen, deuterium, fluorine, chlorine, bromine, CN, NO₂, methyl, ethyl, propyl, trideuteriomethyl, difluoromethyl, trifluoromethyl, difluoroethyl, trifluoroethyl, vinyl, ethynyl, cyclopropyl, cyclobutyl, oxiranyl, oxetanyl, aziridinyl, or azetidinyl, the methyl, ethyl, propyl, vinyl, ethynyl, cyclopropyl, cyclobutyl, oxiranyl, oxetanyl, aziridinyl, or azetidinyl is optionally substituted with one or more groups selected from: deuterium, fluorine, chlorine, bromine, CN, methyl, ethyl, isopropyl, cyclopropyl, cyclobutyl, and oxo;
R¹' is hydrogen, deuterium, fluorine, chlorine, bromine, CN, NO₂, hydroxyl, hydroxyethyl, amino, methylamino, dimethylamino, ethylamino, diethylamino, cyclopropylamino, methyl, ethyl, isopropyl, vinyl, ethynyl, trideuteriomethyl, difluoromethyl, trifluoromethyl, difluoroethyl, trifluoroethyl, cyclopropyl, methoxy, ethoxy, cyclopropoxy, or oxo;
R' is deuterium, halogen, CN, hydroxyl, hydroxyethyl, amino, methylamino, dimethylamino, cyclopropylamino, methyl, ethyl, isopropyl, difluoromethyl, trifluoromethyl, difluoroethyl, trifluoroethyl, cyclopropyl, methoxy, ethoxy, difluoromethoxy, trifluoromethoxy, difluoroethoxy, trifluoroethoxy, cyclopropoxy, or oxo;
m is 0, 1, 2, 3, or 4;
p is each independently 1 or 2.

In other specific embodiments, in Formula VI or Formula VII,
L₂ is methylene, ethylene, propylene, butylene, pentylene, vinylene, propenylene, butenylene, or ethyleneoxy, the methylene, ethylene, propylene, butylene, pentylene, vinylene, propenylene, butenylene, or ethyleneoxy is optionally substituted with one or more identical or different R⁵;
X₁ is absent or is NR^{1a}, -C(R^{2a})(R^{3a})-, or O;
X₂ is -C(R^{2a})(R^{3a})-, O, C(=O), S(=O), or S(=O)₂;
each R² is independently hydrogen, deuterium, halogen, CN, methyl, ethyl, isopropyl, vinyl, ethynyl, difluoromethyl, trifluoromethyl, methoxy, ethoxy, isopropoxy, amino, methylamino, dimethylamino, ethylamino, or diethylamino, the methyl, ethyl, isopropyl, vinyl, ethynyl, difluoromethyl, methoxy, ethoxy, isopropoxy, amino, methylamino, dimethylamino, ethylamino, or diethylamino is optionally substituted with one or more deuterium, halogen, hydroxyl, methoxy, ethoxy, or cyclopropyl;
each R⁵ is independently hydrogen, deuterium, halogen, CN, hydroxyl, hydroxyethyl, amino, methylamino, dimethylamino, cyclopropylamino, methyl, ethyl, difluoromethyl, trifluoromethyl, difluoroethyl, trifluoroethyl, isopropyl, cyclopropyl, methoxy, ethoxy, difluoromethoxy, trifluoromethoxy, difluoroethoxy, trifluoroethoxy, cyclopropoxy, or oxo;
or, two R⁵ together with the atoms to which they are respectively attached to form a cyclopropyl, cyclobutyl, cyclopentyl, cyclohexyl, oxiranyl, oxetanyl, tetrahydrofuranyl, pyranyl, azetidinyl, piperidinyl, or morpholinyl, the cyclopropyl, cyclobutyl, cyclopentyl, cyclohexyl, oxiranyl, oxetanyl, tetrahydrofuranyl, pyranyl, azetidinyl, piperidinyl, or morpholinyl is optionally substituted with one or more R';
R^{1a} and R^{2a} are each independently hydrogen, deuterium, halogen, methyl, ethyl, isopropyl, cyclopropyl, cyclobutyl, cyclopentyl, cyclohexyl, difluoromethyl, trifluoromethyl, difluoroethyl, trifluoroethyl, or hydroxyethyl;
R^{3a} is hydrogen, F, Cl, Br, methyl, ethyl, isopropyl, cyclopropyl, cyclobutyl, cyclopentyl, cyclohexyl, difluoromethyl, trifluoromethyl, difluoroethyl, trifluoroethyl, hydroxyethyl, amino, methylamino, dimethylamino, methoxy, ethoxy, or isopropoxy;
or, R^{2a} and R^{3a} together with the carbon atom to which they are attached to form a cyclopropyl, cyclobutyl, cyclopentyl, cyclohexyl, oxiranyl, or oxetanyl, the cyclopropyl, cyclobutyl, cyclopentyl, cyclohexyl, oxiranyl, or oxetanyl is optionally substituted with one or more R';
Y is N or C;
W¹ is absent, O, or NH;
U¹ is N or C;
U² is N or C;
U⁶ is N or CH;
U⁹ is absent or is N, CR¹, O, S, or C(=O);
U¹⁰ is CR¹';
U¹¹ is N, CR¹, O, S, or C(=O);
U¹² is -N(R^{3a}')-, -C(R^{3a}')=C(R^{3a}')-;
R²⁰, R⁴⁰, R⁵⁰, and R⁶⁰ are each independently hydrogen, deuterium, F, Cl, Br, CN, hydroxyl, amino, methyl, ethyl, isopropyl, cyclopropyl, cyclobutyl, cyclopentyl, cyclohexyl, difluoromethyl, trifluoromethyl, difluoroethyl, trifluoroethyl, hydroxyethyl, methylamino, dimethylamino, ethylamino, diethylamino, methoxy, ethoxy, or oxo, the amino, methyl, ethyl, isopropyl, cyclopropyl, cyclobutyl, cyclopentyl, cyclohexyl, difluoroethyl, trifluoroethyl, hydroxyethyl, methylamino, dimethylamino, ethylamino, diethylamino, methoxy, or ethoxy is optionally substituted with one or more halogen, methyl, or deuterium;
each R³⁰ is independently hydrogen, amino, methyl, ethyl, isopropyl, cyclopropyl, cyclobutyl, cyclopentyl, cyclohexyl, difluoromethyl, trifluoromethyl, difluoroethyl, trifluoroethyl, or hydroxyethyl, the amino, methyl, ethyl, isopropyl, cyclopropyl, cyclobutyl, cyclopentyl, cyclohexyl, difluoromethyl, difluoroethyl, trifluoroethyl, or hydroxyethyl is optionally substituted with one or more halogen or deuterium;
or, two R²⁰together with the atoms to which they are respectively attached to form a cyclopropyl, cyclobutyl, cyclopentyl, or cyclohexyl, the cyclopropyl, cyclobutyl, cyclopentyl, or cyclohexyl is optionally substituted with one or more methyl, ethyl, isopropyl, cyclopropyl, difluoromethyl, difluoroethyl, trifluoromethyl, trifluoroethyl, or halogen;
R^{3a}' is hydrogen, deuterium, methyl, ethyl, propyl, trideuteriomethyl, difluoromethyl, trifluoromethyl, difluoroethyl, trifluoroethyl, vinyl, ethynyl, cyclopropyl, cyclobutyl, oxiranyl, oxetanyl, aziridinyl, or azetidinyl, the methyl, ethyl, propyl, vinyl, ethynyl, cyclopropyl, cyclobutyl, oxiranyl, oxetanyl, aziridinyl, or azetidinyl is optionally substituted with one or more groups selected from: deuterium, fluorine, chlorine, bromine, CN, methyl, ethyl, isopropyl, cyclopropyl, cyclobutyl, and oxo;
R^{3b}' is hydrogen, deuterium, fluorine, chlorine, bromine, CN, NO₂, methyl, ethyl, propyl, trideuteriomethyl, difluoromethyl, trifluoromethyl, difluoroethyl, trifluoroethyl, vinyl, ethynyl, cyclopropyl, cyclobutyl, oxiranyl, oxetanyl, aziridinyl, or azetidinyl, the methyl, ethyl, propyl, vinyl, ethynyl, cyclopropyl, cyclobutyl, oxiranyl, oxetanyl, aziridinyl, or azetidinyl is optionally substituted with one or more groups selected from: deuterium, fluorine, chlorine, bromine, CN, methyl, ethyl, isopropyl, cyclopropyl, cyclobutyl, and oxo;
R¹' is hydrogen, deuterium, fluorine, chlorine, bromine, CN, NO₂, hydroxyl, hydroxyethyl, amino, methylamino, dimethylamino, ethylamino, diethylamino, cyclopropylamino, methyl, ethyl, isopropyl, vinyl, ethynyl, trideuteriomethyl, difluoromethyl, trifluoromethyl, difluoroethyl, trifluoroethyl, cyclopropyl, methoxy, ethoxy, cyclopropoxy, or oxo;
R' is deuterium, halogen, CN, hydroxyl, hydroxyethyl, amino, methylamino, dimethylamino, cyclopropylamino, methyl, ethyl, isopropyl, difluoromethyl, trifluoromethyl, difluoroethyl, trifluoroethyl, cyclopropyl, methoxy, ethoxy, difluoromethoxy, trifluoromethoxy, difluoroethoxy, trifluoroethoxy, cyclopropoxy, or oxo;
m is 0, 1, 2, 3, or 4;
p is each independently 1 or 2;
q is 1, 2, or 3.

In still other specific embodiments,
ring A is selected from the following groups: 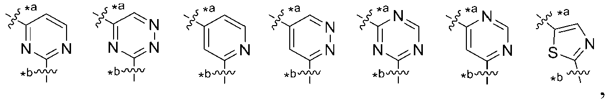 wherein *^{a} indicates the connection site to the N atom of NH, and *^{b} indicates the connection site to ring M;
ring M is selected from the following groups: 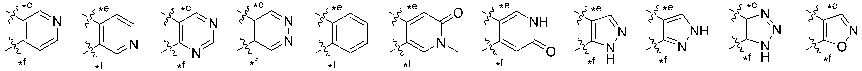 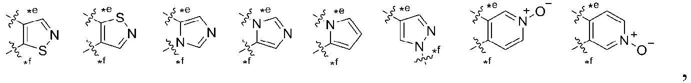 wherein *^{e} indicates the connection site to ring A, and *^{f} indicates the connection site to X₂;
ring E is 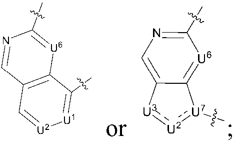
L₂ is methylene, ethylene, propylene, butylene, pentylene, vinylene, propenylene, butenylene, or ethyleneoxy, the methylene, ethylene, propylene, butylene, pentylene, vinylene, propenylene, butenylene, or ethyleneoxy is optionally substituted with one or more identical or different R⁵;
X₁ is absent or is NR^{1a}, -C(R^{2a})(R^{3a})-, or O;
X₂ is -C(R^{2a})(R^{3a})-, O, C(=O), or S(=O)₂;
each R¹ is independently hydrogen, deuterium, halogen, methyl, ethyl, or cyclopropyl;
each R² is independently hydrogen, deuterium, halogen, CN, methyl, ethyl, difluoromethyl, trifluoromethyl, or methoxy, the methyl, ethyl, or methoxy is optionally substituted with one or more halogen;
R³ is hydrogen, deuterium, halogen, methyl, ethyl, cyclopropyl, dimethylamino, C(=O)N(CH₃)₂, or S(=O)₂-cyclopropyl;
R⁴ is 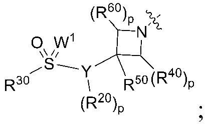
R⁵ is deuterium, halogen, methyl, ethyl, difluoromethyl, trifluoromethyl, difluoroethyl, trifluoroethyl, cyclopropyl, or oxo;
or, two R⁵ together with the atoms to which they are respectively attached to form a cyclopropyl, cyclobutyl, cyclopentyl, or oxetanyl, the cyclopropyl, cyclobutyl, cyclopentyl, or oxetanyl is optionally substituted with one or more deuterium, halogen, methyl, ethyl, or cyclopropyl;
R^{1a} and R^{2a} are each independently hydrogen, deuterium, F, Cl, methyl, ethyl, cyclopropyl, difluoromethyl, trifluoromethyl, difluoroethyl, trifluoroethyl, or hydroxyethyl;
R^{3a} is hydrogen, F, Cl, Br, methyl, ethyl, cyclopropyl, difluoromethyl, trifluoromethyl, difluoroethyl, trifluoroethyl, hydroxyethyl, amino, methylamino, dimethylamino, methoxy, ethoxy, or isopropoxy;
or, R^{2a} and R^{3a} together with the carbon atom to which they are attached to form a cyclopropyl, cyclobutyl, oxiranyl, or oxetanyl, the cyclopropyl, cyclobutyl, oxiranyl, or oxetanyl is optionally substituted with one or more halogen, methyl, or hydroxyl;
U¹ is N or C;
U² is N or C;
U³, U⁴, and U⁵ are each independently C;
U⁶ is N or CH;
U⁷ is C;
R²⁰, R⁴⁰, R⁵⁰, and R⁶⁰ are each independently hydrogen, deuterium, F, Cl, Br, CN, hydroxyl, amino, methyl, ethyl, isopropyl, difluoromethyl, trifluoromethyl, deuterated methyl, or oxo;
each R³⁰ is independently hydrogen, amino, methyl, ethyl, isopropyl, cyclopropyl, difluoromethyl, trifluoromethyl, difluoroethyl, trifluoroethyl, or hydroxyethyl;
W¹ is O;
Y is N or C;
--- or -- indicates absent or a single bond;
m, n, and t are each independently 0, 1, 2, 3, or 4;
p is each independently 1 or 2.

In still other specific embodiments,
ring A is selected from the following groups: 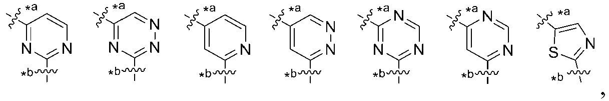 wherein *^{a} indicates the connection site to the N atom of NH, and *^{b} indicates the connection site to ring M;
ring M is selected from the following groups: 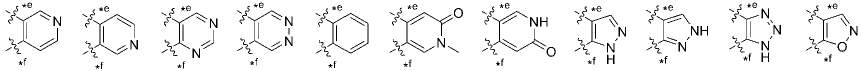 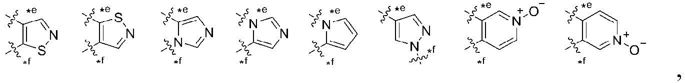 wherein *^{e} indicates the connection site to ring A, and *^{f} indicates the connection site to X₂;
ring E is selected from 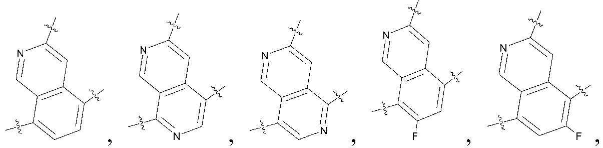 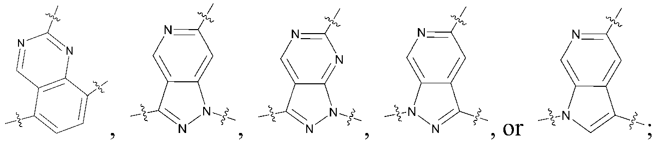
L₂ is methylene, ethylene, propylene, butylene, pentylene, vinylene, propenylene, butenylene, or ethyleneoxy, the methylene, ethylene, propylene, butylene, pentylene, vinylene, propenylene, butenylene, or ethyleneoxy is optionally substituted with one or more identical or different R⁵;
X₁ is absent or is NR^{1a}, -C(R^{2a})(R^{3a})-, or O;
X₂ is -C(R^{2a})(R^{3a})-, O, C(=O), or S(=O)₂;
each R¹ is independently hydrogen, deuterium, halogen, methyl, ethyl, or cyclopropyl;
each R² is independently hydrogen, deuterium, halogen, CN, methyl, ethyl, difluoromethyl, trifluoromethyl, or methoxy, the methyl, ethyl, or methoxy is optionally substituted with one or more halogen;
R³ is hydrogen, deuterium, halogen, methyl, ethyl, cyclopropyl, dimethylamino, C(=O)N(CH₃)₂, or S(=O)₂-cyclopropyl;
R⁴ is selected from 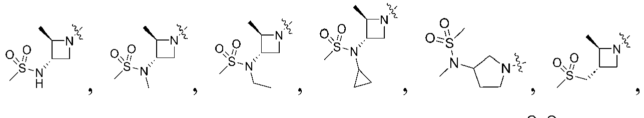 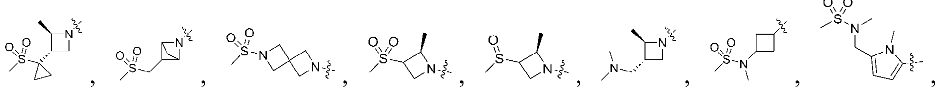 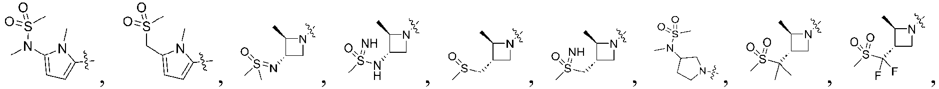 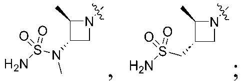
R⁵ is deuterium, halogen, methyl, ethyl, difluoromethyl, trifluoromethyl, difluoroethyl, trifluoroethyl, cyclopropyl, or oxo;
or, two R⁵together with the atoms to which they are respectively attached to form a cyclopropyl, cyclobutyl, cyclopentyl, or oxetanyl, the cyclopropyl, cyclobutyl, cyclopentyl, or oxetanyl is optionally substituted with one or more deuterium, halogen, methyl, ethyl, or cyclopropyl;
R^{1a} and R^{2a} are each independently hydrogen, deuterium, F, Cl, methyl, ethyl, cyclopropyl, difluoromethyl, trifluoromethyl, difluoroethyl, trifluoroethyl, or hydroxyethyl;
R^{3a} is hydrogen, F, Cl, Br, methyl, ethyl, cyclopropyl, difluoromethyl, trifluoromethyl, difluoroethyl, trifluoroethyl, hydroxyethyl, amino, methylamino, dimethylamino, methoxy, ethoxy, or isopropoxy;
or, R^{2a} and R^{3a}together with the carbon atom to which they are attached to form a cyclopropyl, cyclobutyl, oxiranyl, or oxetanyl, the cyclopropyl, cyclobutyl, oxiranyl, or oxetanyl is optionally substituted with one or more halogen, methyl, or hydroxyl;
m, n, and t are each independently 0, 1, 2, 3, or 4.

In still other specific embodiments, in Formula VI or Formula VII,
L₂ is 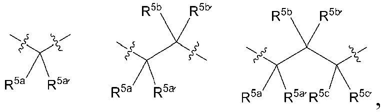 wherein R^{5a}, R^{5b}, R^{5c}, R^{5a}, R^{5b'}, and R^{5c'} are each independently R⁵; R⁵ is hydrogen, deuterium, F, Cl, Br, CN, hydroxyl, hydroxyethyl, amino, methylamino, dimethylamino, cyclopropylamino, methyl, ethyl, difluoromethyl, trifluoromethyl, difluoroethyl, trifluoroethyl, isopropyl, cyclopropyl, methoxy, ethoxy, difluoromethoxy, trifluoromethoxy, difluoroethoxy, trifluoroethoxy, cyclopropoxy, or oxo;
or, R^{5a} and R^{5a'}together with the atoms to which they are attached to form a cyclopropyl, cyclobutyl, cyclopentyl, cyclohexyl, oxiranyl, oxetanyl, tetrahydrofuranyl, pyranyl, azetidinyl, piperidinyl, or morpholinyl, the cyclopropyl, cyclobutyl, cyclopentyl, cyclohexyl, oxiranyl, oxetanyl, tetrahydrofuranyl, pyranyl, azetidinyl, piperidinyl, or morpholinyl is optionally substituted with one or more R';
or, R^{5b} and R^{5b'}together with the atoms to which they are attached to form a cyclopropyl, cyclobutyl, cyclopentyl, cyclohexyl, oxiranyl, oxetanyl, tetrahydrofuranyl, pyranyl, azetidinyl, piperidinyl, or morpholinyl, the cyclopropyl, cyclobutyl, cyclopentyl, cyclohexyl, oxiranyl, oxetanyl, tetrahydrofuranyl, pyranyl, azetidinyl, piperidinyl, or morpholinyl is optionally substituted with one or more R';
or, R^{5c} and R^{5c'}together with the atoms to which they are attached to form a cyclopropyl, cyclobutyl, cyclopentyl, cyclohexyl, oxiranyl, oxetanyl, tetrahydrofuranyl, pyranyl, azetidinyl, piperidinyl, or morpholinyl, the cyclopropyl, cyclobutyl, cyclopentyl, cyclohexyl, oxiranyl, oxetanyl, tetrahydrofuranyl, pyranyl, azetidinyl, piperidinyl, or morpholinyl is optionally substituted with one or more R';
or, R^{5a} and R^{5b}together with the atoms to which they are attached to form a cyclopropyl, cyclobutyl, cyclopentyl, cyclohexyl, oxiranyl, oxetanyl, tetrahydrofuranyl, pyranyl, azetidinyl, piperidinyl, or morpholinyl, the cyclopropyl, cyclobutyl, cyclopentyl, cyclohexyl, oxiranyl, oxetanyl, tetrahydrofuranyl, pyranyl, azetidinyl, piperidinyl, or morpholinyl is optionally substituted with one or more R';
or, R^{5a} and R^{5c}together with the atoms to which they are attached to form a cyclopropyl, cyclobutyl, cyclopentyl, cyclohexyl, oxiranyl, oxetanyl, tetrahydrofuranyl, pyranyl, azetidinyl, piperidinyl, or morpholinyl, the cyclopropyl, cyclobutyl, cyclopentyl, cyclohexyl, oxiranyl, oxetanyl, tetrahydrofuranyl, pyranyl, azetidinyl, piperidinyl, or morpholinyl is optionally substituted with one or more R';
or, R^{5b} and R^{5c}together with the atoms to which they are attached to form a cyclopropyl, cyclobutyl, cyclopentyl, cyclohexyl, oxiranyl, oxetanyl, tetrahydrofuranyl, pyranyl, azetidine, piperidinyl, or morpholinyl, said cyclopropyl, cyclobutyl, cyclopentyl, cyclohexyl, oxiranyl, oxetanyl, tetrahydrofuranyl, pyranyl, azetidinyl, piperidinyl, or morpholinyl is optionally substituted with one or more R';
X₁ is absent or is NR^{1a}, -C(R^{2a})(R^{3a})-, or O;
X₂ is -C(R^{2a})(R^{3a})-, O, C(=O), S(=O), or S(=O)₂;
each R² is independently hydrogen, deuterium, halogen, CN, methyl, ethyl, isopropyl, vinyl, ethynyl, difluoromethyl, trifluoromethyl, methoxy, ethoxy, isopropoxy, amino, methylamino, dimethylamino, ethylamino, or diethylamino, the methyl, ethyl, isopropyl, vinyl, ethynyl, difluoromethyl, methoxy, ethoxy, isopropoxy, amino, methylamino, dimethylamino, ethylamino, or diethylamino is optionally substituted with one or more deuterium, halogen, hydroxyl, methoxy, ethoxy, or cyclopropyl;
each R⁵ is independently hydrogen, deuterium, halogen, CN, hydroxyl, hydroxyethyl, amino, methylamino, dimethylamino, cyclopropylamino, methyl, ethyl, difluoromethyl, trifluoromethyl, difluoroethyl, trifluoroethyl, isopropyl, cyclopropyl, methoxy, ethoxy, difluoromethoxy, trifluoromethoxy, difluoroethoxy, trifluoroethoxy, cyclopropoxy, or oxo;
or, two R⁵ together with the atoms to which they are respectively attached to form a cyclopropyl, cyclobutyl, cyclopentyl, cyclohexyl, oxiranyl, oxetanyl, tetrahydrofuranyl, pyranyl, azetidinyl, piperidinyl, or morpholinyl, the cyclopropyl, cyclobutyl, cyclopentyl, cyclohexyl, oxiranyl, oxetanyl, tetrahydrofuranyl, pyranyl, azetidinyl, piperidinyl, or morpholinyl is optionally substituted with one or more R';
R^{1a} and R^{2a} are each independently hydrogen, deuterium, halogen, methyl, ethyl, isopropyl, cyclopropyl, cyclobutyl, cyclopentyl, cyclohexyl, difluoromethyl, trifluoromethyl, difluoroethyl, trifluoroethyl, or hydroxyethyl;
R^{3a} is hydrogen, F, Cl, Br, methyl, ethyl, isopropyl, cyclopropyl, cyclobutyl, cyclopentyl, cyclohexyl, difluoromethyl, trifluoromethyl, difluoroethyl, trifluoroethyl, hydroxyethyl, amino, methylamino, dimethylamino, methoxy, ethoxy, or isopropoxy;
or, R^{2a} and R^{3a}together with the carbon atom to which they are attached to form a cyclopropyl, cyclobutyl, cyclopentyl, cyclohexyl, oxiranyl, or oxetanyl, the cyclopropyl, cyclobutyl, cyclopentyl, cyclohexyl, oxiranyl, or oxetanyl is optionally substituted with one or more R';
Y is N or C;
W¹ is absent, O, or NH;
U¹ is N or C;
U² is N or C;
U⁶ is N or CH;
U⁹ is absent or is N, CR¹, O, S, or C(=O);
U¹⁰ is CR¹';
U¹¹ is N, CR¹, O, S, or C(=O);
U¹² is -N(R^{3a}')-, -C(R^{3a}')=C(R^{3a}')-;
R²⁰, R⁴⁰, R⁵⁰, and R⁶⁰ are each independently hydrogen, deuterium, F, Cl, Br, CN, hydroxyl, amino, methyl, ethyl, isopropyl, cyclopropyl, cyclobutyl, cyclopentyl, cyclohexyl, difluoromethyl, trifluoromethyl, difluoroethyl, trifluoroethyl, hydroxyethyl, methylamino, dimethylamino, ethylamino, diethylamino, methoxy, ethoxy, or oxo, the amino, methyl, ethyl, isopropyl, cyclopropyl, cyclobutyl, cyclopentyl, cyclohexyl, difluoroethyl, trifluoroethyl, hydroxyethyl, methylamino, dimethylamino, ethylamino, diethylamino, methoxy, or ethoxy is optionally substituted with one or more halogen, methyl, or deuterium;
each R³⁰ is independently hydrogen, amino, methyl, ethyl, isopropyl, cyclopropyl, cyclobutyl, cyclopentyl, cyclohexyl, difluoromethyl, trifluoromethyl, difluoroethyl, trifluoroethyl, or hydroxyethyl, the amino, methyl, ethyl, isopropyl, cyclopropyl, cyclobutyl, cyclopentyl, cyclohexyl, difluoromethyl, difluoroethyl, trifluoroethyl, or hydroxyethyl is optionally substituted with one or more halogen or deuterium;
or, two R²⁰ together with the atoms to which they are respectively attached to form a cyclopropyl, cyclobutyl, cyclopentyl, or cyclohexyl, the cyclopropyl, cyclobutyl, cyclopentyl, or cyclohexyl is optionally substituted with one or more methyl, ethyl, isopropyl, cyclopropyl, difluoromethyl, difluoroethyl, trifluoromethyl, trifluoroethyl, or halogen;
R^{3a}' is hydrogen, deuterium, methyl, ethyl, propyl, trideuteriomethyl, difluoromethyl, trifluoromethyl, difluoroethyl, trifluoroethyl, vinyl, ethynyl, cyclopropyl, cyclobutyl, oxiranyl, oxetanyl, aziridinyl, or azetidinyl, the methyl, ethyl, propyl, vinyl, ethynyl, cyclopropyl, cyclobutyl, oxiranyl, oxetanyl, aziridinyl, or azetidinyl is optionally substituted with one or more groups selected from: deuterium, fluorine, chlorine, bromine, CN, methyl, ethyl, isopropyl, cyclopropyl, cyclobutyl, and oxo;
R^{3b}' is hydrogen, deuterium, fluorine, chlorine, bromine, CN, NO₂, methyl, ethyl, propyl, trideuteriomethyl, difluoromethyl, trifluoromethyl, difluoroethyl, trifluoroethyl, vinyl, ethynyl, cyclopropyl, cyclobutyl, oxiranyl, oxetanyl, aziridinyl, or azetidinyl, the methyl, ethyl, propyl, vinyl, ethynyl, cyclopropyl, cyclobutyl, oxiranyl, oxetanyl, aziridinyl, or azetidinyl is optionally substituted with one or more groups selected from: deuterium, fluorine, chlorine, bromine, CN, methyl, ethyl, isopropyl, cyclopropyl, cyclobutyl, and oxo;
R¹' is hydrogen, deuterium, fluorine, chlorine, bromine, CN, NO₂, hydroxyl, hydroxyethyl, amino, methylamino, dimethylamino, ethylamino, diethylamino, cyclopropylamino, methyl, ethyl, isopropyl, vinyl, ethynyl, trideuteriomethyl, difluoromethyl, trifluoromethyl, difluoroethyl, trifluoroethyl, cyclopropyl, methoxy, ethoxy, cyclopropoxy, or oxo;
R' is deuterium, halogen, CN, hydroxyl, hydroxyethyl, amino, methylamino, dimethylamino, cyclopropylamino, methyl, ethyl, isopropyl, difluoromethyl, trifluoromethyl, difluoroethyl, trifluoroethyl, cyclopropyl, methoxy, ethoxy, difluoromethoxy, trifluoromethoxy, difluoroethoxy, trifluoroethoxy, cyclopropoxy, or oxo;
m is 0, 1, 2, 3, or 4;
p is each independently 1 or 2;
q is 1, 2, or 3.

In still other specific embodiments,
ring A is selected from the following groups: 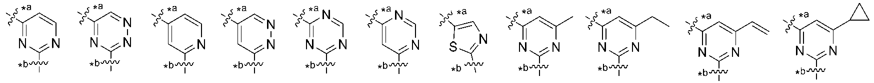 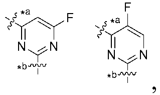 wherein *^{a} indicates the connection site to the N atom of NH, and *^{b} indicates the connection site to ring M;
ring M is selected from the following groups: 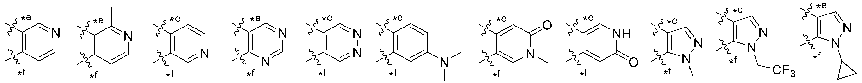 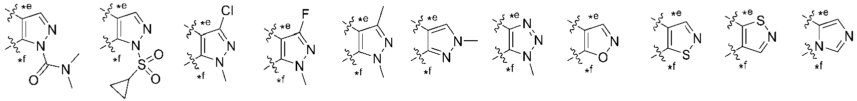 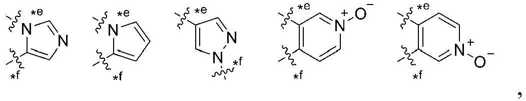 wherein *^{e} indicates the connection site to ring A, and *^{f} indicates the connection site to X₂;
ring E is selected from 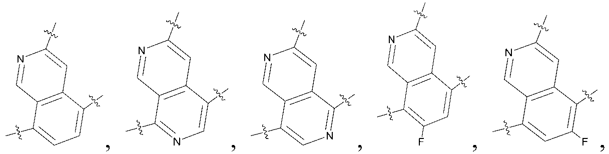 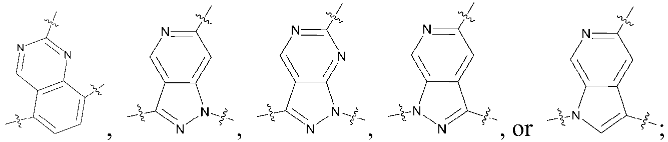
L₂ is methylene, ethylene, propylene, butylene, pentylene, vinylene, propenylene, butenylene, or ethyleneoxy, the methylene, ethylene, propylene, butylene, pentylene, vinylene, propenylene, butenylene, or ethyleneoxy is optionally substituted with one or more identical or different R⁵;
X₁ is absent or is NR^{1a}, -C(R^{2a})(R^{3a})-, or O;
X₂ is -C(R^{2a})(R^{3a})-, O, C(=O), or S(=O)₂;
each R¹ is independently hydrogen, deuterium, halogen, methyl, ethyl, or cyclopropyl;
each R² is independently hydrogen, deuterium, halogen, CN, methyl, ethyl, difluoromethyl, trifluoromethyl, or methoxy, the methyl, ethyl, or methoxy is optionally substituted with one or more halogen;
R³ is hydrogen, deuterium, halogen, methyl, ethyl, cyclopropyl, dimethylamino, C(=O)N(CH₃)₂, or S(=O)₂-cyclopropyl;
R⁴ is selected from 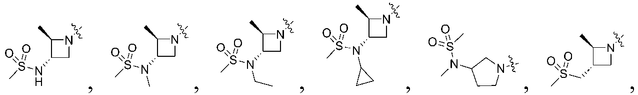 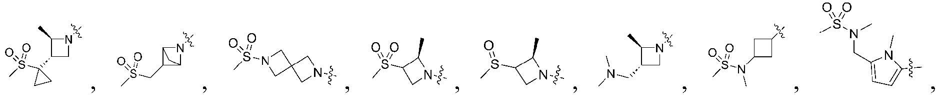 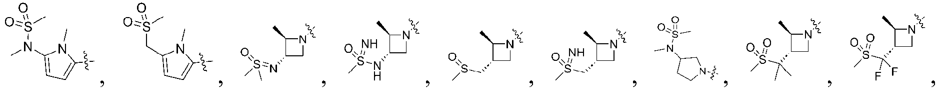 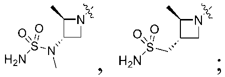
R⁵ is deuterium, halogen, methyl, ethyl, difluoromethyl, trifluoromethyl, difluoroethyl, trifluoroethyl, cyclopropyl, or oxo;
or, two R⁵together with the atoms to which they are respectively attached to form a cyclopropyl, cyclobutyl, cyclopentyl, or oxetanyl, the cyclopropyl, cyclobutyl, cyclopentyl, or oxetanyl is optionally substituted with one or more deuterium, halogen, methyl, ethyl, or cyclopropyl;
R^{1a} and R^{2a} are each independently hydrogen, deuterium, F, Cl, methyl, ethyl, cyclopropyl, difluoromethyl, trifluoromethyl, difluoroethyl, trifluoroethyl, or hydroxyethyl;
R^{3a} is hydrogen, F, Cl, Br, methyl, ethyl, cyclopropyl, difluoromethyl, trifluoromethyl, difluoroethyl, trifluoroethyl, hydroxyethyl, amino, methylamino, dimethylamino, methoxy, ethoxy, or isopropoxy;
or, R^{2a} and R^{3a}together with the carbon atom to which they are attached to form a cyclopropyl, cyclobutyl, oxiranyl, or oxetanyl, the cyclopropyl, cyclobutyl, oxiranyl, or oxetanyl is optionally substituted with one or more halogen, methyl, or hydroxyl;
m, n, and t are each independently 0, 1, 2, 3, or 4.

In some more specific embodiments, the compounds of Formula I to V are selected from the following compounds:

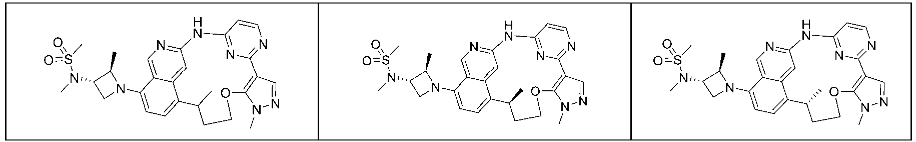

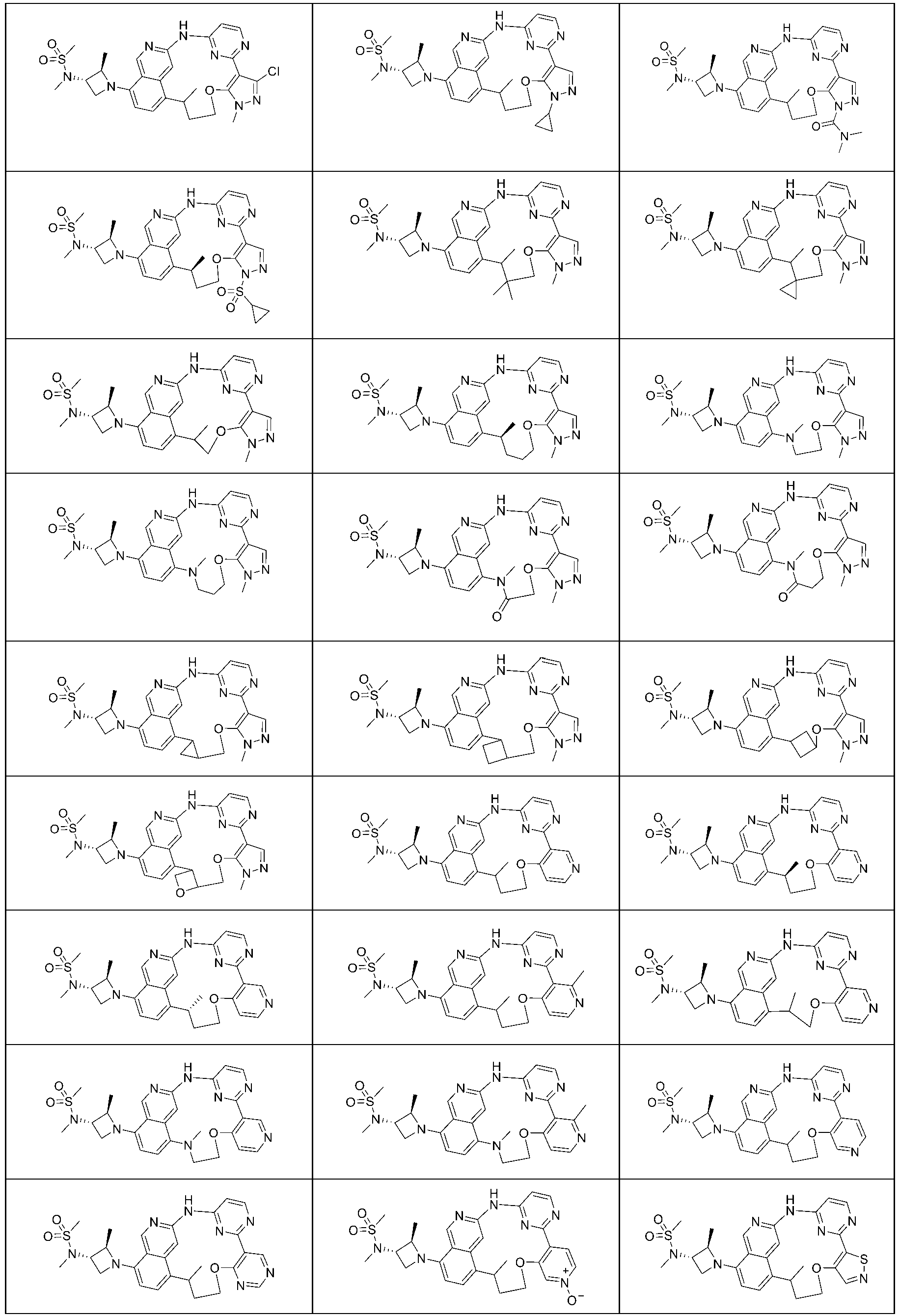

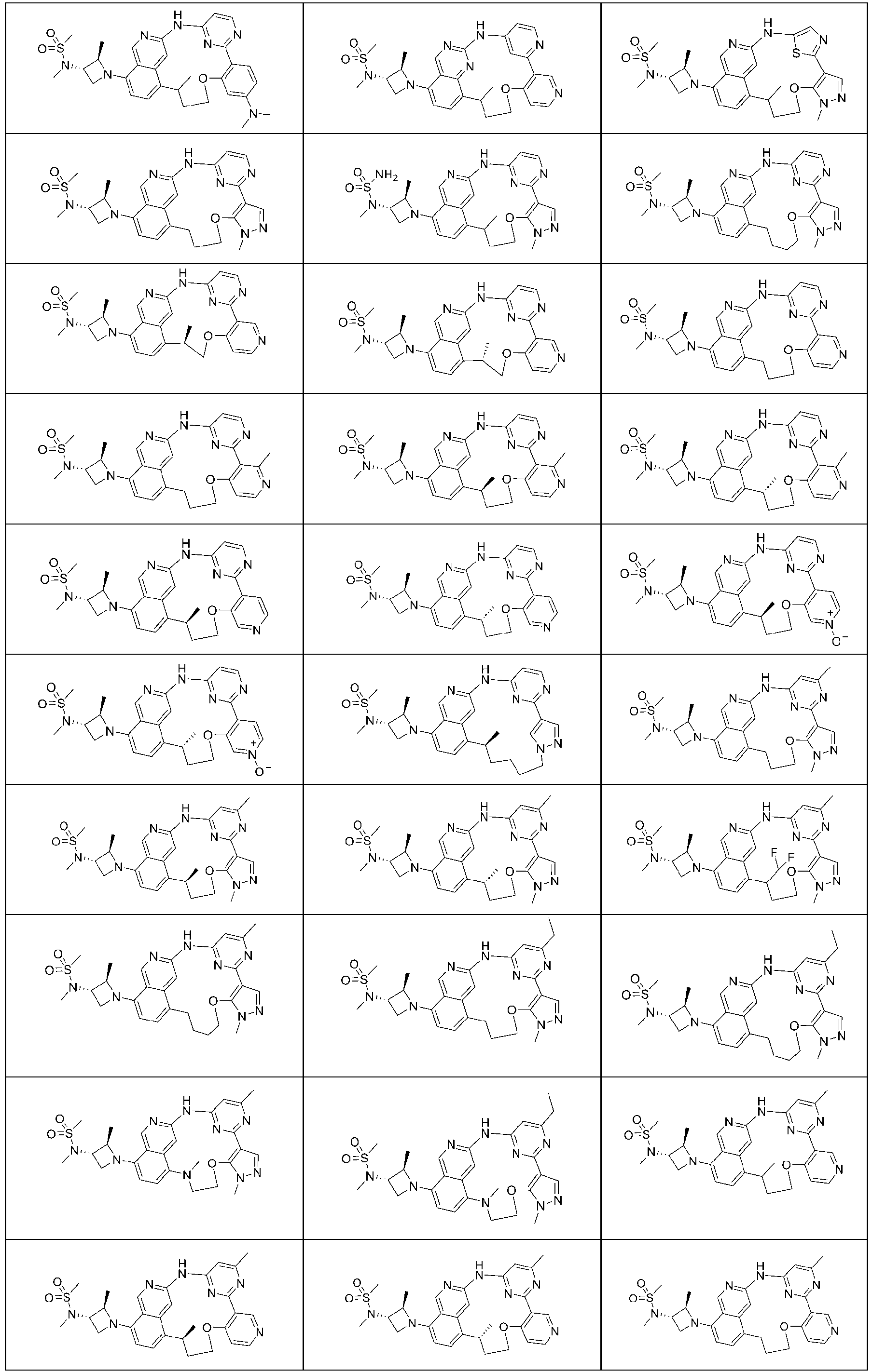

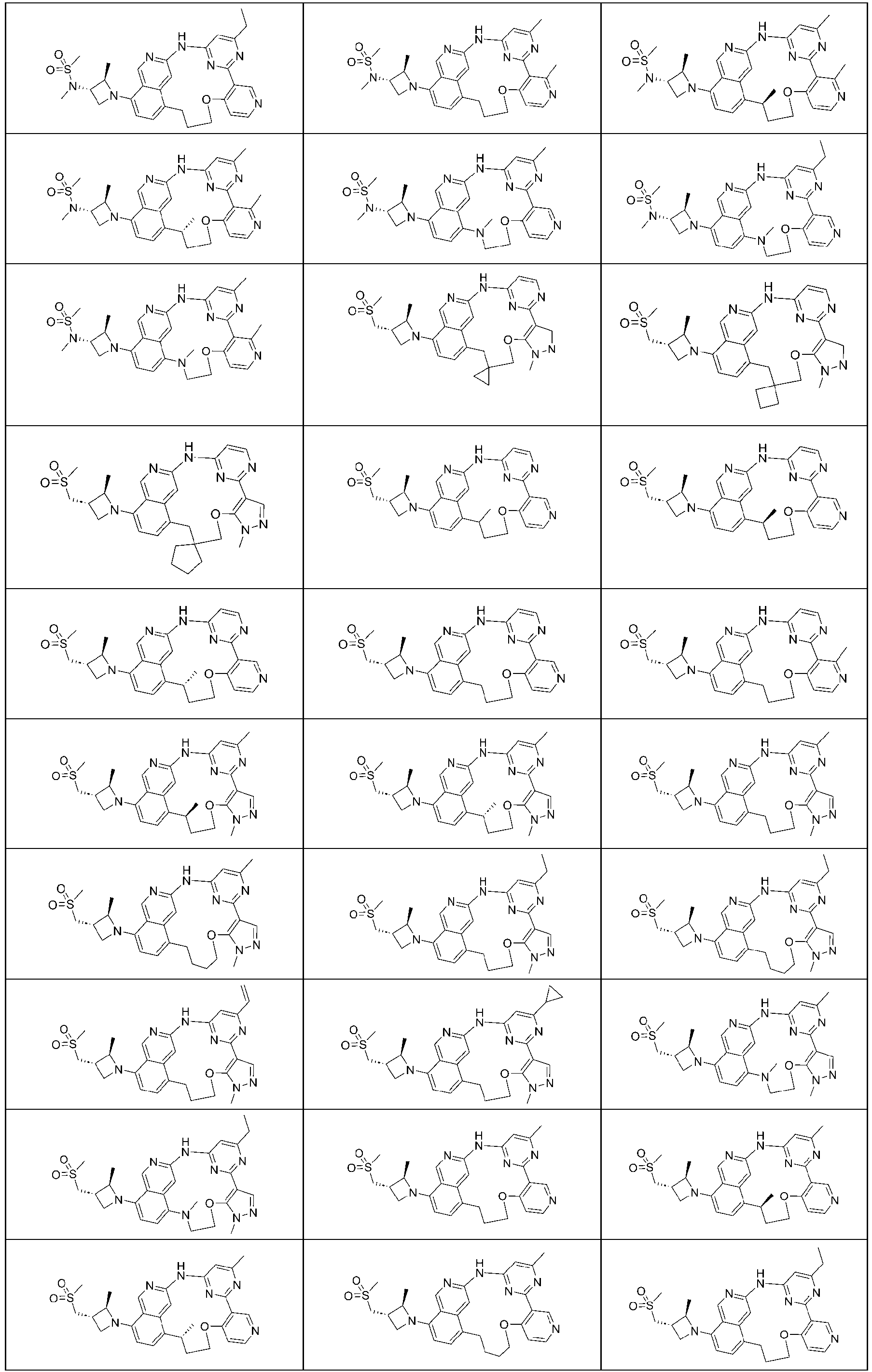

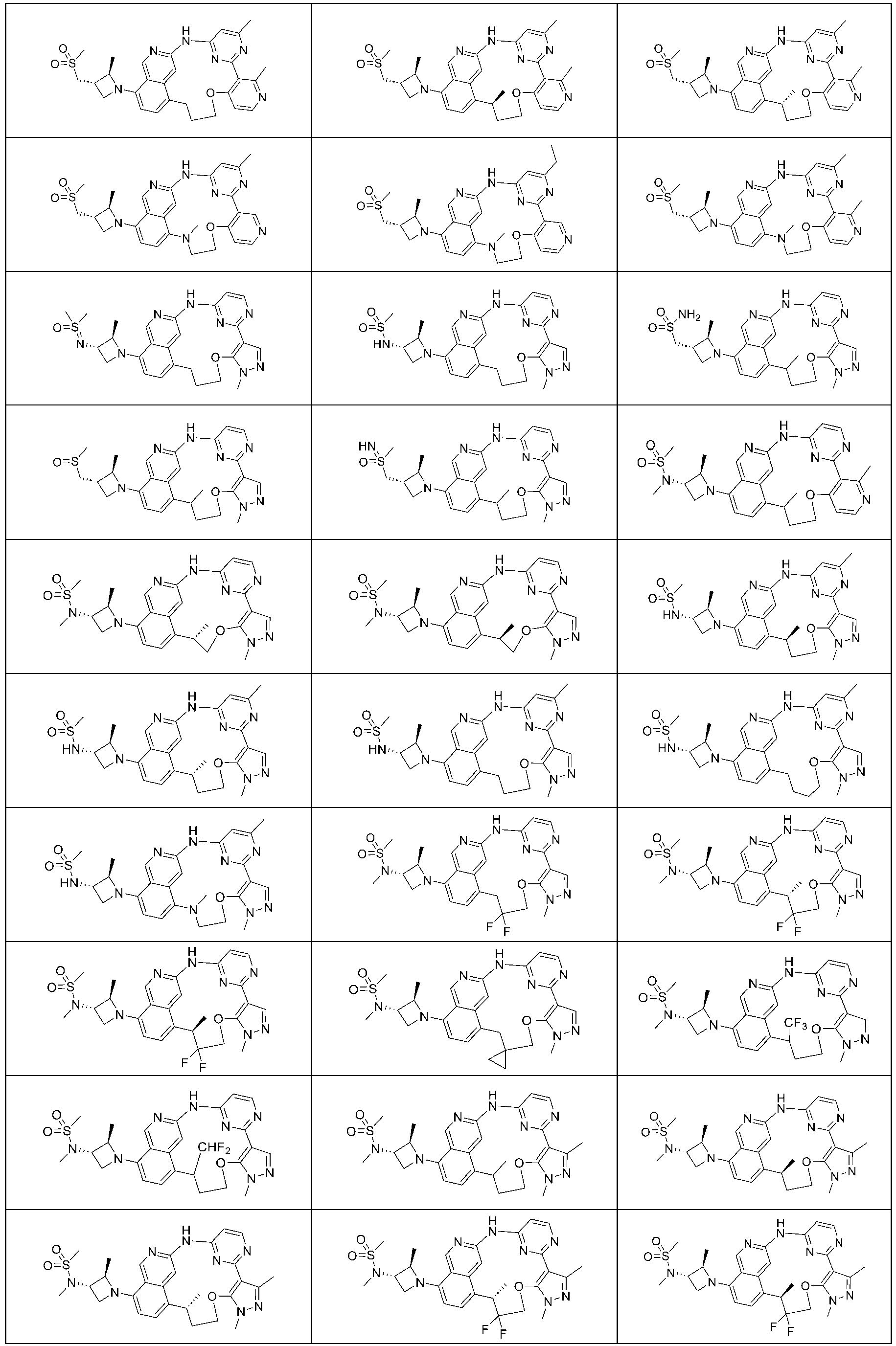

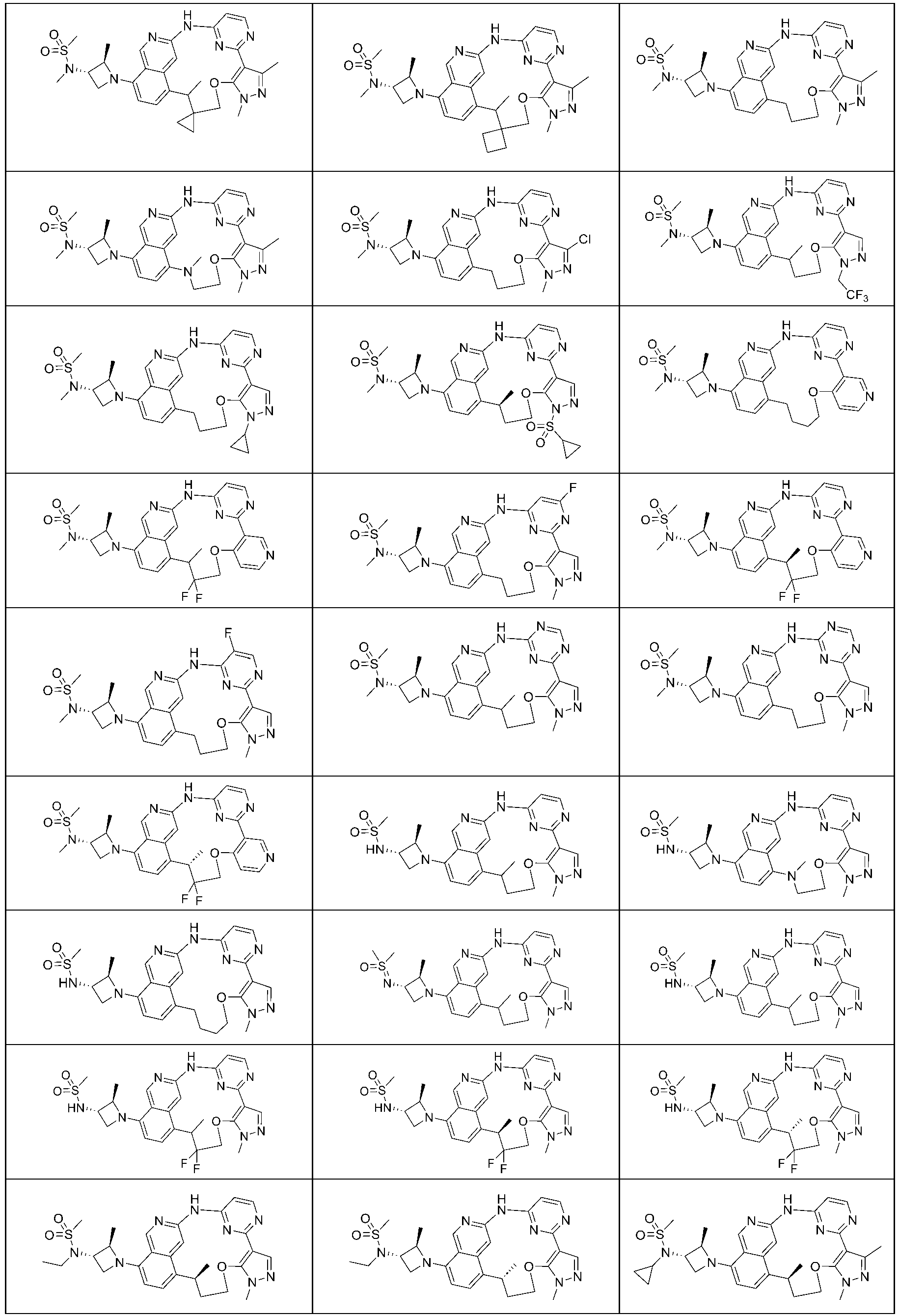

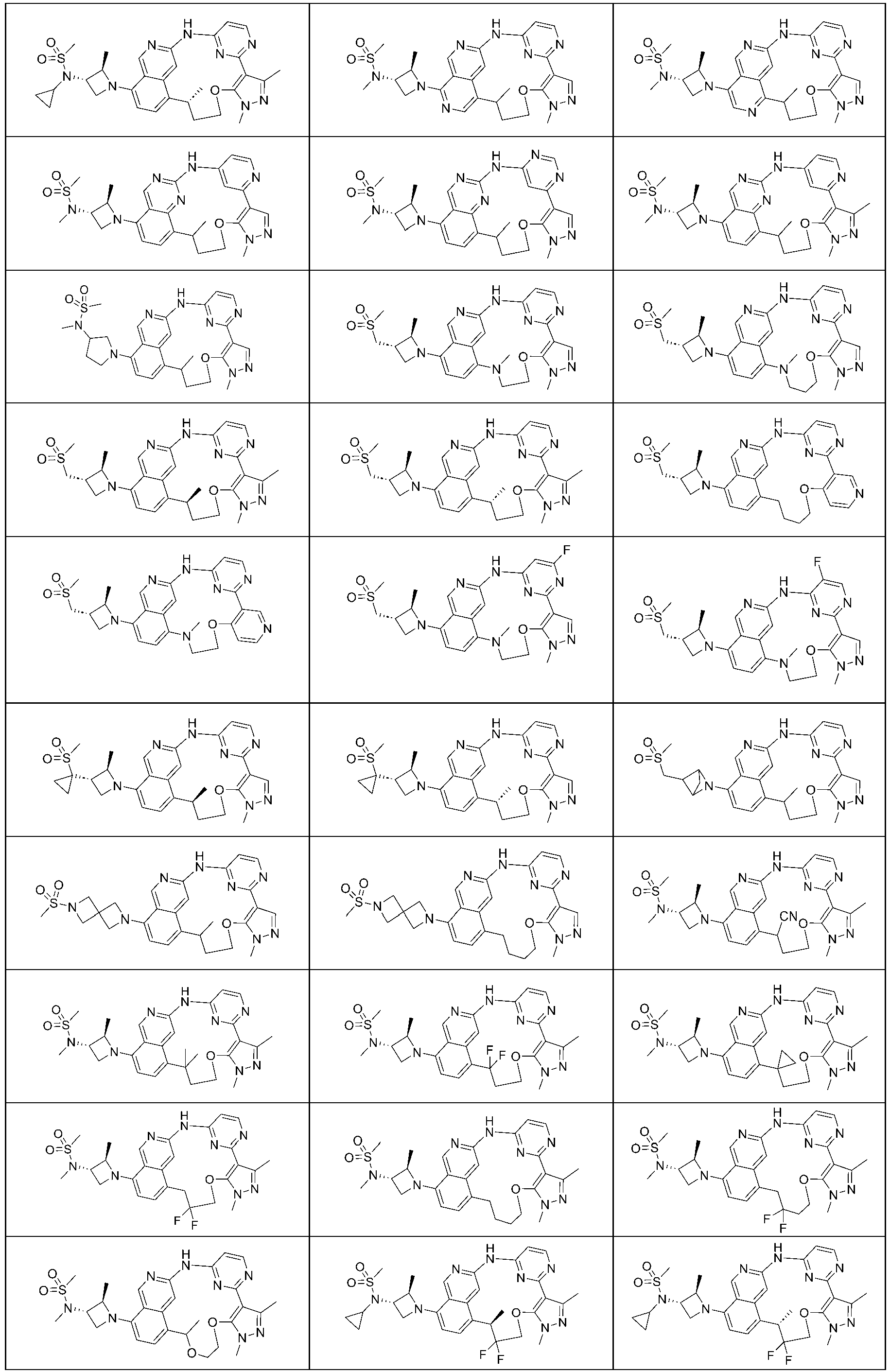

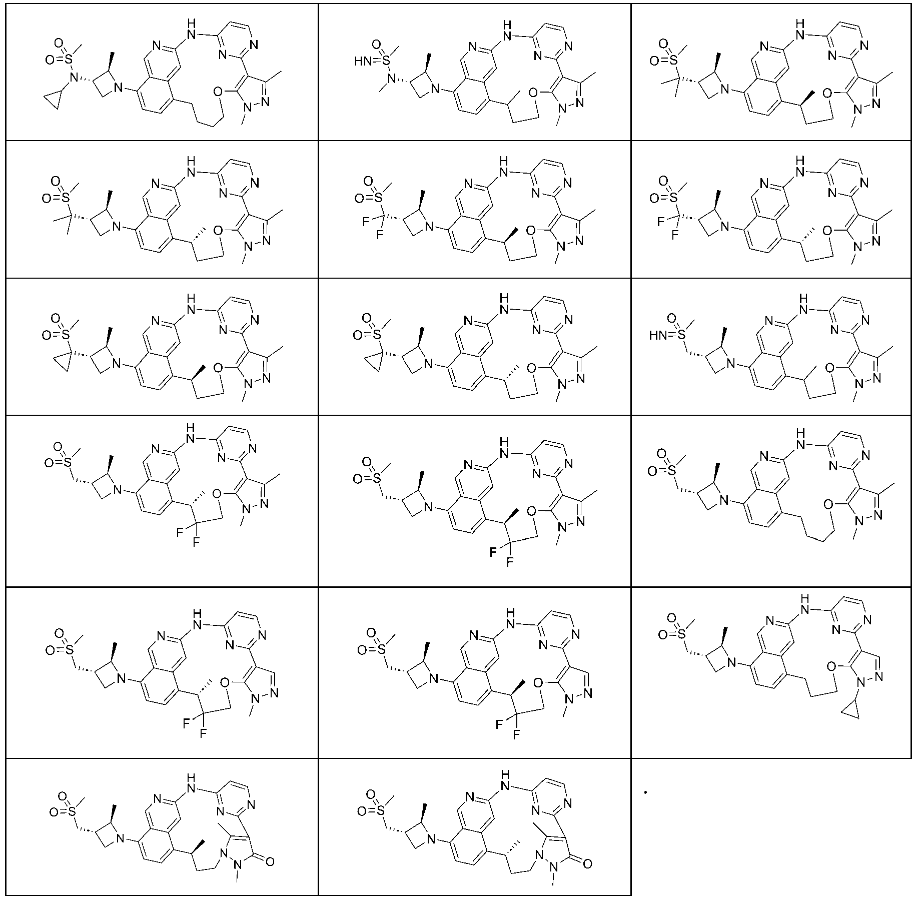

In a second aspect, in certain embodiments, the present invention provides a method for preparing a compound of Formula II, the method comprising the following reaction steps:
Compound II-1 and compound II-2 undergo a coupling reaction to give compound II-3;
Compound II-3 and compound II-4 undergo a coupling reaction to afford compound II-5;
Compound II-5 is deprotected to give compound II-6;
Compound II-6 undergoes a Mitsunobu reaction to afford the compound represented by Formula II; 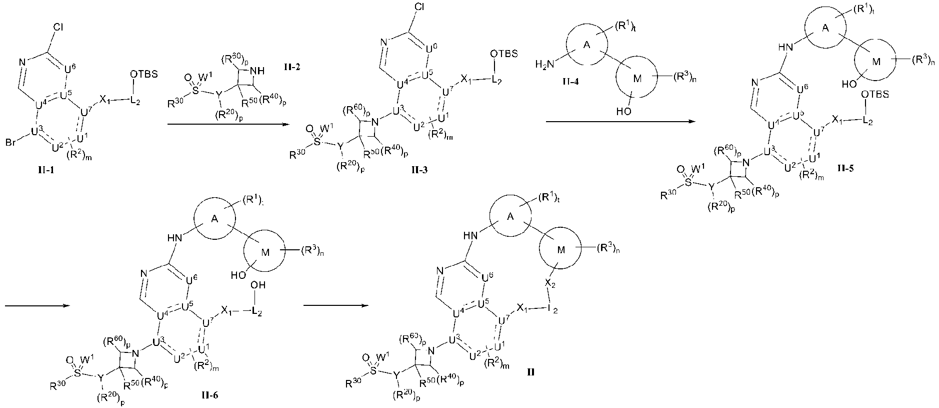
wherein A, M, X₁, X₂, L₂, R¹, R², R³, R²⁰, R³⁰, R⁴⁰, R⁵⁰, R⁶⁰, W¹, U¹, U², U³, U⁴, U⁵, U⁶, U⁷, Y, m, n, t, and p are as defined above.

In certain embodiments, the present invention provides a method for preparing a compound of Formula III, the method comprising the following reaction steps:
Compound III-1 and compound III-2 undergo a coupling reaction to give compound III-3;
Compound III-3 and compound II-4 undergo a coupling reaction to give compound III-5;
Compound III-5 is deprotected to afford compound III-6;
Compound III-6 undergoes a Mitsunobu reaction to afford the compound of Formula III; 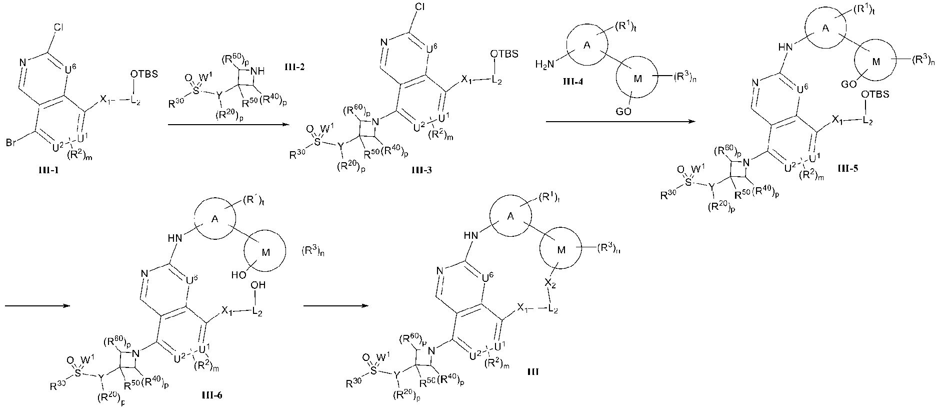
wherein A, M, X₁, X₂, L₂, R¹, R², R³, R²⁰, R³⁰, R⁴⁰, R⁵⁰, R⁶⁰, W¹, Y, U¹, U², U⁶, m, n, t, and p are as defined above;
G is a protecting group selected from PMB, SEM, methyl, Boc, Cbz.

In certain embodiments, the present invention provides a method for preparing a compound of Formula IV, the method comprising the following reaction steps:
Compound IV-1 and compound IV-2 undergo a coupling reaction to give compound IV-3;
Compound IV-3 and compound IV-4 undergo a coupling reaction to afford compound IV-5;
Compound IV-5 is deprotected to give compound IV-6;
Compound IV-6 undergoes a Mitsunobu reaction to afford the compound of Formula IV; 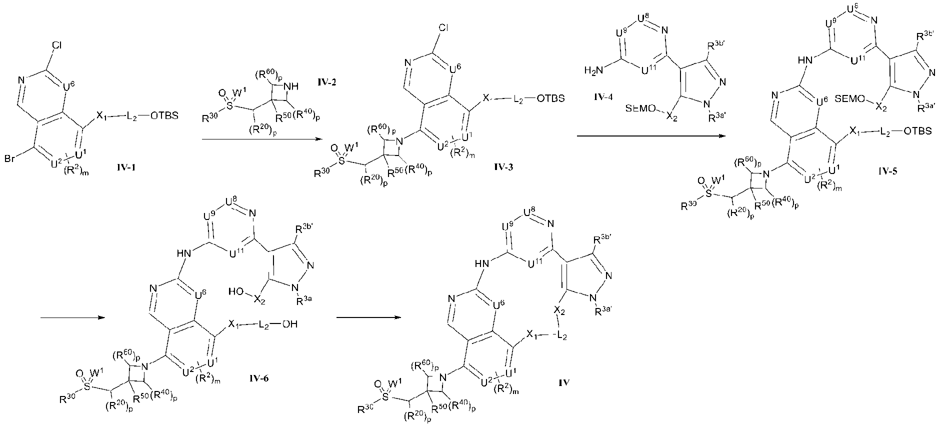
wherein X₁, X₂, L₂, R¹, R², R²⁰, R³⁰, R⁴⁰, R⁵⁰, R⁶⁰, W¹, U¹, U², U⁶, U⁸, U⁹, U¹¹, R^{3a}', R^{3b}', m, and p are as defined above.

In certain embodiments, the present invention provides a method for preparing a compound of Formula V, the method comprising the following reaction steps:
Compound V-1 and compound V-2 undergo a coupling reaction to afford compound V-3;
Compound V-3 and compound V-4 undergo a coupling reaction to give compound V-5;
Compound V-5 is deprotected to give compound V-6;
Compound V-6 undergoes a Mitsunobu reaction to afford the compound of Formula V; 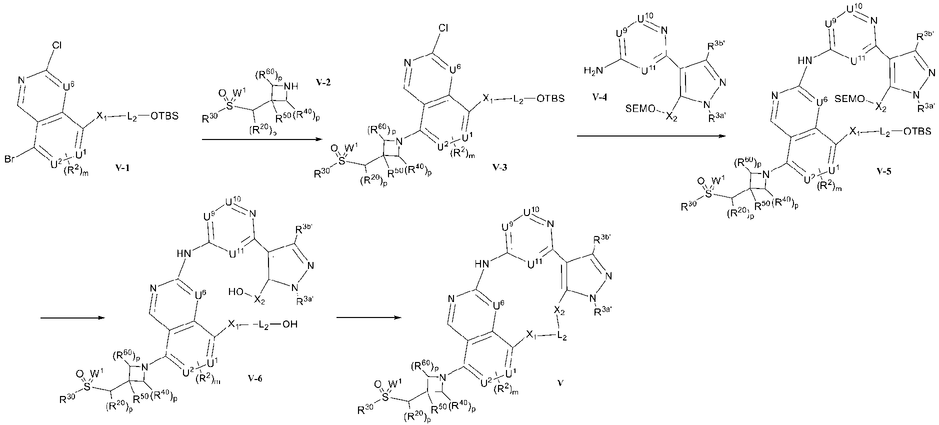
wherein X₁, X₂, L₂, R¹, R², R²⁰, R³⁰, R⁴⁰, R⁵⁰, R⁶⁰, W¹, U¹, U², U⁶, U⁹, U¹⁰, U¹¹, R^{3a}', R^{3b}', m, and p are as defined above.

In certain embodiments, the present invention provides a method for preparing a compound of Formula VII, the method comprising the following reaction steps:
Compound VII-1 and compound VII-2 undergo a coupling reaction to give compound VII-3;
Compound VII-3 and compound VII-4 undergo a coupling reaction to afford compound VII-5;
Compound VII-5 is deprotected to give compound VII-6;
Compound VII-6 undergoes a Mitsunobu reaction to afford the compound of Formula VII; 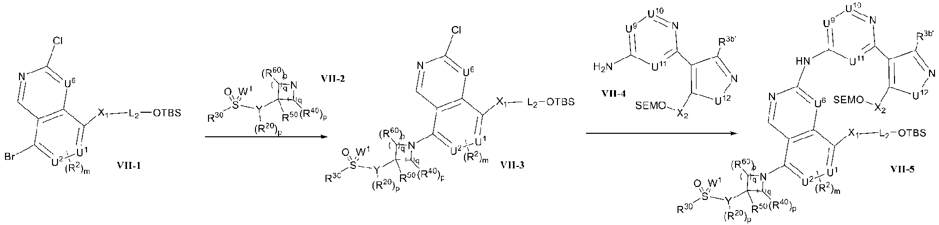 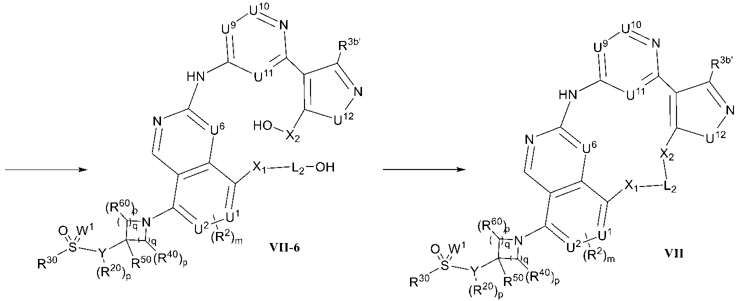
wherein X₁, X₂, L₂, R¹, R², R²⁰, R³⁰, R⁴⁰, R⁵⁰, R⁶⁰, W¹, U¹, U², U⁶, U⁹, U¹⁰, U¹¹, U¹², R^{3a}', R^{3b}', m, and p are as defined above.

In certain embodiments, the present invention provides another method for preparing intermediate compound VII-3, the method comprising the following reaction steps:
Compound VII-7 and compound VII-2 undergo a coupling reaction to form compound VII-8;
Compound VII-8 is iodinated to give compound VII-9;
Compound VII-9 and compound VII-10 or VII-11 undergo a Suzuki coupling reaction to afford compound VII-12;
Compound VII-12 is reduced to give compound VII-13;
Compound VII-13 is protected to afford the compound of Formula VII-3; 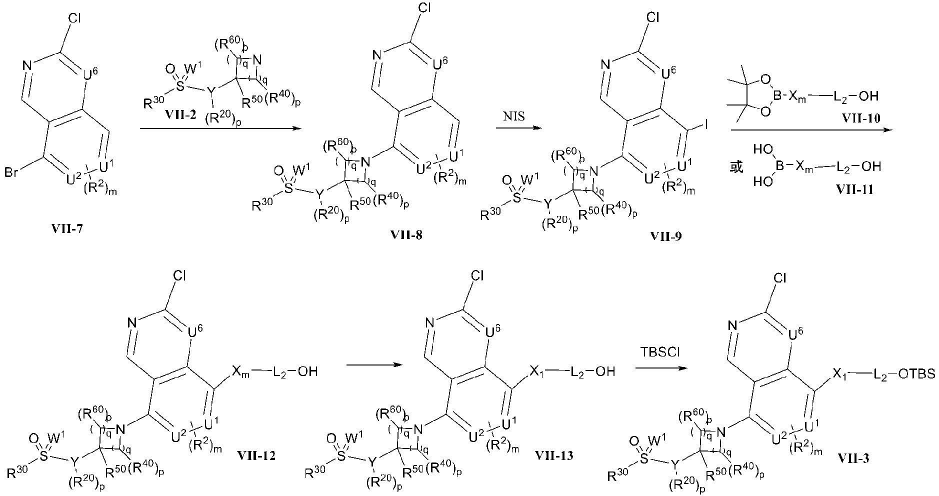
Wherein Xₘ is X₁, or X₁ containing a double bond;
X₁, X₂, L₂, R¹, R², R²⁰, R³⁰, R⁴⁰, R⁵⁰, R⁶⁰, W¹, U¹, U², U⁶, U⁹, U¹⁰, U¹¹, U¹², R^{3a}', R^{3b}', m, and p are as defined above.

In a third aspect, the present invention provides a pharmaceutical composition comprising the above-mentioned compound or its pharmaceutically acceptable salt, stereoisomer, racemate, tautomer, isotope-labeled derivative, deuterated derivative, N-oxide, prodrug, hydrate, or solvate thereof, and a pharmaceutically acceptable carrier or excipient;
preferably, the pharmaceutical composition is a tablet, capsule, pill, granule, powder, suppository, injection, solution, suspension, ointment, patch, lotion, drop, liniment, or spray.

In a fourth aspect, the present invention provides use of the above-mentioned compound or a pharmaceutically acceptable salt, stereoisomer, racemate, tautomer, isotope-labeled derivative, deuterated derivative, N-oxide, prodrug, hydrate, or solvate thereof, or a pharmaceutical composition comprising the above-mentioned compound or a pharmaceutically acceptable salt, stereoisomer, racemate, tautomer, isotopically labeled compound, deuterated compound, N-oxide, prodrug, hydrate, or solvate thereof and a pharmaceutically acceptable carrier or excipient, in the treatment of a disease mediated by EGFR abnormality;
preferably, the disease is a neoplastic disease;
more preferably, the neoplastic disease includes: head and neck cancer, nasopharyngeal carcinoma, melanoma, bladder cancer, esophageal cancer, renal cancer, breast cancer, colorectal cancer, ovarian cancer, cervical cancer, pancreatic cancer, glioma, prostate cancer, leukemia, lymphoma, gastric cancer, lung cancer, liver cancer, gastrointestinal stromal tumor, thyroid cancer, squamous cell carcinoma, cholangiocarcinoma, endometrial cancer, multiple myeloma, mesothelioma, atherosclerosis, or pulmonary fibrosis.

In a fifth aspect, the present invention provides a method for treating a tumor in a patient in need thereof, the method comprising administering to the patient a therapeutically effective amount of the above-mentioned compound or its pharmaceutically acceptable salt, stereoisomer, racemate, tautomer, isotope-labeled derivative, deuterated derivative, N-oxide, prodrug, hydrate, or solvate thereof, or the pharmaceutical composition.

The patient is preferably a mammal, and the mammal is preferably a human.

In certain embodiments, the route of administration includes: oral, mucosal, sublingual, ocular, topical, parenteral, rectal, intracisternal, vaginal, intraperitoneal, intravesical, or nasal administration.

In certain embodiments, the tumor includes: head and neck cancer, melanoma, bladder cancer, esophageal cancer, anaplastic large cell lymphoma, renal cancer, breast cancer, colorectal cancer, ovarian cancer, cervical cancer, pancreatic cancer, glioma, glioblastoma, prostate cancer, leukemia, lymphoma, non-Hodgkin's lymphoma, gastric cancer, lung cancer, liver cancer, gastrointestinal stromal tumor, thyroid cancer, squamous cell carcinoma, cholangiocarcinoma, endometrial cancer, multiple myeloma, or mesothelioma.

The compound of the present invention or its pharmaceutically acceptable salt, stereoisomer, racemate, tautomer, isotopically labeled compound, deuterated compound, N-oxide, prodrug, hydrate, or solvate, or the pharmaceutical composition, can be delivered into the body by any suitable route, such as, by oral, intravenous injection, intranasal, topical, intramuscular, intradermal injection, transdermal administration, or subcutaneous routes.

In certain embodiments, the compound provided in the present invention or its pharmaceutically acceptable salt, stereoisomer, racemate, tautomer, isotope-labeled derivate, deuterated derivate, N-oxide, prodrug, hydrate, or solvate, or the pharmaceutical composition, can be formulated into dosage forms suitable for drug delivery, administered via injection routes (such as subcutaneous, intravenous, intramuscular, intra-arterial, intrathecal, intracapsular, intraorbital, intracardiac, intradermal, intraperitoneal, intratracheal, epicutaneous, intra-articular, subcapsular, subarachnoid, intraspinal, intrasternal, and/or infusion) and non-injection routes (such as oral, enteral, buccal, nasal, intranasal, mucosal, epidermal, patch, dermal, ophthalmic, pulmonary, sublingual, rectal, vaginal, or topical epidermal administration).

Suitable dosage forms include (but are not limited to) dosage forms for injection such as emulsions, solutions, and suspensions; dosage forms for oral use such as tablets, capsules, pills, dragees, powders, and granules; dosage forms for topical or transdermal administration such as sprays, ointments, pastes, creams, lotions, gels, solutions, medicated patches, and inhalants; and dosage forms for vaginal or rectal administration such as suppositories. These dosage forms can be prepared under suitable conditions according to the compound and suitable excipients. The preparation methods and processes are well-known, such as those provided in Remington: The Science and Practice of Pharmacy (Gennaro ed. 20th edition, Williams & Wilkins PA, USA) (2000).

In certain embodiments, EGFR abnormality refers to EGFR mutation, EGFR amplification, or EGFR overexpression.

Preferably, EGFR mutation refers to deletion, alteration, and/or insertion of other amino acids at any position in the EGFR sequence. It generally occurs in exons 18-21 within the intracellular domain or exons 2-16 within the extracellular domain.

More preferably, EGFR mutation is a mutation in the kinase domain of exons 18-21, including but not limited to point mutations in exon 18 such as E709K/Q/A/G/V, G719C/S/A/R, G724S, etc.; deletions, point mutations, and/or insertion mutations in exon 19 such as delE746_A750, delE746_E749, A750P, delE746_A750insQP, delE746_A750insRP, etc.; point mutations and insertion mutations in exon 20 such as S768I, V769_D770insASV, D770_N771insSVD, D770_N771insG, A763_Y764insFQEA, etc.; point mutations in exon 21 such as L858R, L861Q/R, etc.; also including secondary resistance mutations such as T790M, L792H, G796R/S/D, C797S/G/N, L718P/Q/V, etc.; furthermore, EGFR mutations also include combinations of these mutations.

In certain embodiments, the compound or pharmaceutical composition of the present invention can be administered simultaneously with one or more substances having additional pharmacological activity substances, thereby achieving additive or even synergistic effects in the body. For example, the compound of the present invention can be combined with an additional pharmacological activity substance into one pharmaceutical composition, or administered simultaneously as separate compositions, or administered sequentially as separate compositions. Additional pharmacological activity Substances that can be administered simultaneously with the compound of the present invention for treatment of cancer include, but are not limited to: 1) EGFR family inhibitors, monoclonal/bispecific antibodies or ADCs , such as: osimertinib, almonertinib, furmonertinib, befotertinib, rezivertinib, rilertinib, olmutinib, lazertinib, PLB1004, afatinib, dacomitinib, erlotinib, gefitinib, icotinib, cetuximab, panitumumab, amivantamab, lapatinib, neratinib, tucatinib, trastuzumab, pertuzumab, margetuximab, ado-trastuzumab emtansine, fam-trastuzumab deruxtecan; 2) inhibitors targeting downstream pathways or other pathway, including monoclonal/bispecific antibodies or ADCs, wherein the targets include but not limited to MEK, RET, PI3K, mTOR, c-Met, PARP, or mitotic kinase inhibitors (such as CDK4/6), for example: trametinib, binimetinib, selumetinib, pralsetinib, idelalisib, copanlisib, duvelisib, alpelisib, umbralisib, parsaclisib, rapamycin, temsirolimus, everolimus, capmatinib, tepotinib, savolitinib, glumetinib, bozitinib, cabozantinib, emibetuzumab, telisotuzumab, niraparib, palbociclib, ribociclib, abemaciclib; 3) anti-angiogenic drugs, such as bevacizumab, aflibercept, ramucirumab, nintedanib; 4) apoptosis inducers (such as Bcl-2), for example: obatoclax, venetoclax; 5) chemotherapeutic drugs, for example: fluorouracil, doxorubicin, daunorubicin, tamoxifen, leuprorelin, goserelin, flutamide, nilutamide, finasteride, dexamethasone, aminoglutethimide, amsacrine, anastrozole, asparaginase, BCG vaccine, bicalutamide, bleomycin, busulfan, camptothecin, capecitabine, carboplatin, cisplatin, carmustine, chlorambucil, cladribine, colchicine, cyclophosphamide, cyproterone, cytarabine, dacarbazine, dienestrol, diethylstilbestrol, docetaxel, adriamycin, epirubicin, estradiol, estramustine, etoposide, exemestane, filgrastim, fludarabine, fludrocortisone, fluoxymesterone, gemcitabine, teniposide, testosterone, titanocene dichloride, topotecan, tretinoin, vinblastine, hydroxyurea, idarubicin, ifosfamide, irinotecan, letrozole, leucovorin, pentostatin, Plicamycin,, procarbazine, raltitrexed, porfimer sodium, rituximab, streptozocin, suramin, levamisole, lomustine, mechlorethamine, medroxyprogesterone, megestrol, melphalan, mercaptopurine, mesna, methotrexate, mitomycin, mitotane, mitoxantrone, nocodazole, octreotide, paclitaxel, pamidronate, thioguanine, thiotepa, , vincristine, vindesine, vinorelbine, pemetrexed.

In certain embodiments, the compound provided by the present invention can be used concurrently with immunotherapeutic agents. Suitable immunotherapeutic agents include: PD-1 inhibitors, PD-L1 inhibitors, CTLA-4 inhibitors, such as durvalumab; tumor cell multidrug resistance reversal agents (such as verapamil), mycophenolate mofetil, thalidomide, cyclosporine, and monoclonal antibodies.

In certain embodiments, the compound provided by the present invention can be used concurrently with non-chemical methods for cancer treatment. In certain embodiments, the compound provided by the present invention can be used concurrently with radiation therapy. In certain embodiments, the compound provided by the present invention can be used in combination with surgery, tumor hyperthermia, focused ultrasound therapy, cryotherapy, or a combination of the above therapies.

The technical solutions of the present invention will be clearly and completely described below in conjunction with the embodiments of the present invention. Obviously, the described embodiments are part of, rather than all of, the embodiments of the present invention. Based on the embodiments of the present invention, all other embodiments obtained by those skilled in the art without making creative efforts shall fall within the scope of protection of the present invention.

It is particularly noted that similar substitutions and modifications made to the present invention that are obvious to those skilled in the art are deemed to be included in the present invention. Relevant personnel can obviously modify or appropriately change and combine the methods and applications described herein without departing from the content, spirit, and scope of the present invention to realize and apply the technology of the present invention. Obviously, the described embodiments are only part of , rather than all of, the embodiments of the present invention.

Unless specific the conditions are indicated in the present invention, all operations are carried out under conventional conditions or the conditions recommended by the manufacturer. The active pharmaceutical ingredients or excipients, as well as the reagents or instruments used whose manufacturers are not specified, are all conventional products commercially available.

Unless otherwise indicated, the following definitions are set forth to illustrate and define the meaning and scope of the various terms used to describe the present invention.
--- indicates absence or a single bond.
" 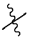 " indicates a point of attachment.

The minimum and maximum number of carbon atom content in a hydrocarbon group is indicated by a prefix, for example, the prefix (C_{a-b})alkyl means any alkyl containing "a" to "b" carbon atoms. Thus, for example, (C₁₋₆)alkyl means an alkyl containing 1 to 6 carbon atoms. The alkyl is branched or straight-chain.

The atoms described in the compounds of the present invention include their isotopes, for example, hydrogen can be deuterium or tritium.

"Bridged ring" refers to a polycyclic group in which any two rings share two non-directly connected atoms. It may contain one or more double bonds, provided that no ring has a fully conjugated π-electron system. The ring atoms may be all carbon atoms or one or more ring atoms may be selected from N, O, S, SO, or SO₂. It is preferably a 7-10 membered ring.

"Spiro ring" refers to a polycyclic group in which any two rings share one carbon atom. It may contain one or more double bonds, but no ring has a fully conjugated π-electron system. The ring atoms may be all carbon atoms or one or more ring atoms may be selected from N, O, S, SO, or SO₂. It is preferably a 5-10 membered ring.

Fused ring: refers to a polycyclic group in which each ring shares two adjacent atoms with other rings in the system.

Depending on the number of rings, it can be classified as bicyclic, tricyclic, tetracyclic, or polycyclic groups, preferably bicyclic, tricyclic, or tetracyclic, more preferably bicyclic or tricyclic.

A cyclic group may be bond to another group in various ways. If the bonding mode is not specified, s all possible modes are included. For example, "pyridyl" includes 2-, 3-, or 4-pyridyl, and "thienyl" includes 2- or 3-thienyl.

"Alkyl" refers to a straight-chain or branched, monovalent saturated hydrocarbon group, including but not limited to methyl, ethyl, n-propyl, isopropyl, n-butyl, isobutyl, sec-butyl, tert-butyl, n-pentyl, isopentyl, neopentyl, n-hexyl, etc. Preferably C₁₋₈ alkyl. More preferably C₁₋₆ alkyl. More preferably C₁₋₄ alkyl. Alkyl may be substituted with one or more substituents or unsubstituted. When substituted, the substituent(s) may substitute may be at any point of attachment. The substituent(s) are preferably the following groups:

Deuterium, halogen, cyano, nitro, azido, hydroxyl, C₁₋₁₀ alkyl, C₂₋₁₀ alkenyl, C₂₋₁₀ alkynyl, C₂₋₆ heteroalkenyl, 3-12 membered saturated or unsaturated cycloalkyl, 3-12 membered saturated or unsaturated heterocyclyl, C₁₋₁₀ alkoxy, halo C₁₋₁₀ alkyl, halo C₁₋₁₀ alkoxy, deuterated C₁₋₁₀ alkyl, deuterated C₁₋₁₀ alkoxy, 6-10 membered aryl, 5-10 membered heteroaryl, oxo (=O).

"Heteroalkyl" refers to a straight-chain or branched, monovalent saturated hydrocarbon group in which one or more carbon atoms are replaced by one or more heteroatoms selected from O, N, S, S(=O), S(=O)₂, or Si, including but not limited to -CH₂-CH₂-O-CH₃, -CH₂-CH₂-NH-CH₃, -CH₂-CH₂-N(CH₃)-CH₃, -CH₂-S-CH₂-CH₃, -O-CH₂-CH₃.

"Heteroalkylene" refers to a divalent group of heteroalkyl, wherein the heteroatom(s) may occupy one or both ends of the chain, including but not limited to -CH₂-CH₂-O-CH₂-,-CH₂-CH₂-NH-CH₂-, -CH₂-CH₂-N(CH₃)-CH₂-, -CH₂-S-CH₂-CH₂-, -CH₂-CH₂-O-, -O-CH₂-CH₂-O-, -CH₂-CH₂-N-, -N-CH₂-CH₂-N-.

"Cycloalkyl" refers to a saturated or partially unsaturated monocyclic or polycyclic cyclic hydrocarbon group, which can be combined with other groups. Monocyclic cyclic hydrocarbon groups include but are not limited to cyclopropyl, cyclobutyl, cyclopentyl, cyclohexyl, cycloheptyl, cyclooctyl. Preferably 3-8 membered cycloalkyl. More preferably 3-6 membered cycloalkyl. More preferably 3-5 membered cycloalkyl. Polycyclic cyclic hydrocarbon groups include bicyclic, spiro, fused, or bridged cyclic hydrocarbon groups, including but not limited to the following groups:

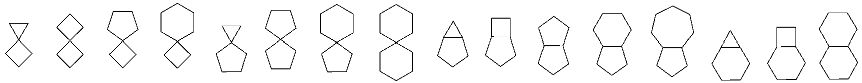

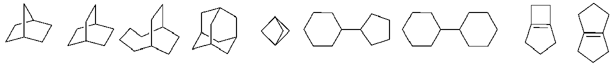

"Cycloalkenyl" refers to a partially unsaturated monocyclic or polycyclic cyclic hydrocarbon group having at least one carbon-carbon double bond, but not forming a fully conjugated π-electron system, which can be combined with other groups. Monocyclic cycloalkenyl groups include, but are not limited to, cyclopropenyl, cyclobutenyl, cyclopentenyl, cyclohexenyl, cyclohexadienyl, cycloheptenyl, cycloheptatrienyl, cyclooctenyl, etc. Preferably 3-8 membered cycloalkenyl. More preferably 3-6 membered cycloalkenyl. More preferably 5-6 membered cycloalkenyl. Polycyclic cycloalkenyl groups include bicyclic, spiro, fused, or bridged cycloalkenyl groups. Including but not limited to the following groups:

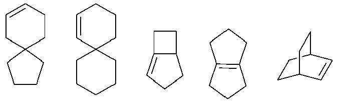

The cycloalkyl or cycloalkenyl may be fused to an aryl, heteroaryl, or heterocyclyl, including but not limited to tetrahydronaphthyl, benzocycloheptyl, etc.

"Alkenyl" refers to a straight-chain, branched, or cyclic hydrocarbon group containing one or more carbon-carbon double bonds, including but not limited to vinyl, propenyl, (E)-2-methylethenyl, (Z)-2-methylethenyl, (E)-but-2-enyl, (Z)-but-2-enyl, (E)-but-1-enyl, (Z)-but-1-enyl. Preferably C₂₋₆ alkenyl. More preferably C₂₋₄ alkenyl.

"Heteroalkenyl" refers to an alkenyl in which one or more carbon atoms are replaced by one or more heteroatoms selected from O, N, S, S(=O), S(=O)₂, or Si, including but not limited to -CH=CH-O-CH₃, -CH=CH-NH-CH₃, -CH-CH-N(CH₃)-CH₃, -CH₂-S-CH=CH₂.

"Heteroalkenylene" refers to a divalent group of heteroalkenyl, wherein the heteroatom(s) may occupy one or both ends of the chain, including but not limited to -CH=CH-O-CH₂-, -CH=CH-NH-CH₂-, -CH=CH-N(CH₃)-CH₂-, -CH₂-S-CH=CH-, -CH=CH-O-, -O-CH=CH-O-, -CH=CH-N-, -N-CH=CH-N-.

"Alkynyl" refers to a straight-chain, branched, or cyclic hydrocarbon group containing one or more carbon-carbon triple bonds, including but not limited to ethynyl, prop-1-ynyl, prop-2-ynyl, but-1-ynyl, but-2-ynyl, but-3-ynyl. Preferably C₂₋₆ alkynyl. More preferably C₂₋₄ alkynyl.

"Alkylene" refers to a straight-chain or branched, divalent saturated hydrocarbon group, i.e., an alkyl group in which one hydrogen atom is further substituted, including but not limited to "methylene" refers to -CH₂-, "ethylene" refers to -CH₂CH₂-, "propylene" refers to-CH₂CH₂CH₂-, "butylene" refers to -CH₂CH₂CH₂CH₂- or -CH₂CH(CH₃)CH₂-.

"Alkenylene" refers to a straight-chain or branched, divalent alkenyl, i.e., an alkenyl group in which one hydrogen atom is further substituted, including but not limited to "vinylene" refers to -CH=CH-, "propenylene" refers to -CH=CHCH₂-, "butenylene" refers to-CH=CHCH₂CH₂- or -CH₂CH=CHCH₂- or -CH=C(CH₃)CH₂-, etc.

"Alkynylene" refers to a straight-chain or branched, divalent alkynyl, i.e., an alkynyl group in which one hydrogen atom is further substituted, including but not limited to "ethynylene" refers to -C≡C-, "propynylene" refers to -C≡CCH₂-, "butynylene" refers to-CH₂C≡CCH₂- or -C≡CCH₂CH₂-, etc.

"Halogen" refers to fluorine, chlorine, bromine, or iodine, preferably fluorine, chlorine, and bromine.

"Haloalkyl" refers to an alkyl as defined herein, wherein one or more hydrogens have been replaced by the same or different halogen. Including but not limited to -CH₂Cl,-CHF₂, -CH₂CCl₃, -CH₂CHF₂, -CH₂CF₃, perfluoroalkyl (e.g., -CF₃), etc.

"Alkylamino" refers to NH₃ substituted with an alkyl. Including but not limited to methylamino, ethylamino, propylamino, isopropylamino, etc.

"Dialkylamino" refers to a group having the structure N(C₁₋₆ alkyl)₂. Including but not limited to dimethylamino, diethylamino, methyl(ethyl)amino, dipropylamino, diisopropylamino, etc.

"Aryl" refers to a monocyclic or polycyclic carbocyclic system having one or more fused or non-fused aromatic rings, including but not limited to phenyl, naphthyl, indenyl. Preferably a 6-10 membered monocyclic or bicyclic aromatic group. More preferably phenyl or naphthyl. Most preferably phenyl.

"Heterocyclyl" refers to a group of a 4-12 membered non-aromatic monocyclic or polycyclic ring system having ring carbon atoms and 1-4 ring heteroatoms, including saturated rings having only single bonds and unsaturated rings having at least one double bond (C=C, C=N, or N=N), but not forming a fully conjugated π-electron system. The heteroatoms are independently selected from N, O, S, oxidized nitrogen (NO), sulfoxide, S(O)(=NH), and sulfonyl. Polycyclic systems include fused, bridged, or spiro ring systems. Monocyclic heterocyclyl moieties include, but are not limited to aziridinyl, azetidinyl, oxetanyl, pyrrolidinyl, piperidinyl, piperazinyl, homopiperazinyl, oxopiperidinyl, oxopiperazinyl, oxohomopiperazinyl, tetrahydrofuranyl, imidazolinyl, morpholinyl, oxazolidinyl, isoxazolidinyl, thiazolidinyl, isothiazolidinyl, quinuclidinyl, thiadiazolidinyl, dihydrofuranyl, dihydropyranyl, tetrahydropyranyl, thiomorpholinyl, thiomorpholinyl sulfoxide, thiomorpholinyl sulfone, etc. Preferably 4-7 membered heterocyclyl. More preferably 4-6 membered heterocyclyl. Polycyclic heterocyclyl moieties include, but are not limited to 2-azabicyclo[2.2.1]heptyl, 2-oxa-5-azabicyclo[2.2.1]heptyl, 2,5-diazabicyclo[2.2.1]heptyl, 1-azabicyclo[2.2.2]octyl, 3-azabicyclo[3.2.1]octyl, 3,8-diazabicyclo[3.2.1]octyl, 6-oxa-2-azabicyclo[3.2.1]octyl, 6-oxa-3-azabicyclo[3.2.1]octyl, 8-oxa-3-azabicyclo[3.2.1]octyl, 8-azabicyclo[3.2.1]octyl, 8-azabicyclo[5.1.0]octyl, hexahydro-1H-furo[3,4-b]pyrrolyl, hexahydro-1H-furo[3,4-c]pyrrolyl, 2-oxa-6-azaspiro[3.3]heptyl, 5-oxa-2-azaspiro[3.4]octyl, 6-oxa-2-azaspiro[3.4]octyl, 1-oxa-7-azaspiro[3.5]nonan-7-yl, 1,4-dioxa-8-azaspiro[4.5]decan-8-yl, and 1,4-dioxa-9-azaspiro[5.5]undecan-9-yl, etc. Examples of unsaturated heterocyclyl moieties include, but are not limited to:

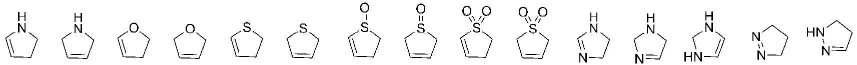

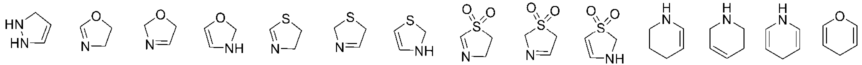

The heterocyclyl may be fused to an aryl, heteroaryl, or cycloalkyl, including but not limited to the following groups:

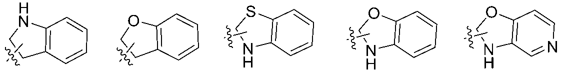

The heterocyclyl is optionally substituted or unsubstituted, and when substituted, the substituents are preferably one or more groups selected from: deuterium, halogen, CN, nitro, hydroxyl, azido, C₁₋₁₀ alkyl, C₂₋₁₀ alkenyl, C₂₋₁₀ alkynyl, C₂₋₆ heteroalkenyl, 3-12 membered saturated or unsaturated cycloalkyl, 3-12 membered saturated or unsaturated heterocyclyl, halo C₁₋₁₀ alkyl, C₁₋₁₀ alkoxy, halo C₁₋₁₀ alkoxy, deuterated C₁₋₁₀ alkyl, deuterated C₁₋₁₀ alkoxy, 6-10 membered aryl, 5-10 membered heteroaryl, and oxo (=O).

"Heteroaryl" refers to a substituted or unsubstituted 5-membered or 6-membered monocyclic heteroaromatic ring system, or a substituted or unsubstituted 9-membered or 10-membered fused or bicyclic heteroaromatic ring system, which contains 1-4 heteroatoms independently selected from N, O, or S, with the remaining ring atoms being carbon atoms. Heteroaryl moieties include, but are not limited to thienyl, furyl, imidazolyl, isoxazolyl, oxazolyl, pyrazolyl, pyrrolyl, thiadiazolyl, oxadiazolyl, triazolyl, pyridyl, pyrazinyl, pyridazinyl, pyrimidinyl, indolyl, indazolyl, quinolinyl, isoquinolinyl, benzimidazolyl, or benzothiazolyl.

The heteroaryl may be fused to an aryl, heterocyclyl, or cycloalkyl, including but not limited to the following groups:

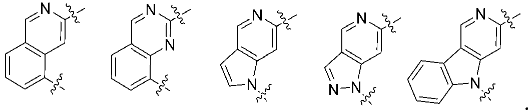

The heteroaryl is optionally substituted or unsubstituted, and when substituted, the substituents are preferably one or more groups selected from: deuterium, halogen, CN, nitro, hydroxyl, azido, C₁₋₁₀ alkyl, C₁₋₁₀ alkoxy, C₂₋₁₀ alkenyl, C₂₋₁₀ alkynyl, C₂₋₆ heteroalkenyl, 3-12 membered saturated or unsaturated cycloalkyl, 3-12 membered saturated or unsaturated heterocyclyl, halo C₁₋₁₀ alkyl, halo C₁₋₁₀ alkoxy, deuterated C₁₋₁₀ alkyl, deuterated C₁₋₁₀ alkoxy, 6-10 membered aryl, 5-10 membered heteroaryl, and oxo (=O).

"Alkoxy" refers to a straight-chain or branched, monovalent, saturated alkyl bonded to an oxygen atom, including but not limited to methoxy, ethoxy, propoxy, butoxy, isobutoxy, tert-butoxy, and other similar groups. Preferably C₁₋₈ alkoxy. More preferably C₁₋₆ alkoxy. More preferably C₁₋₄ alkoxy.

"Cycloalkoxy" refers to -O-cycloalkyl, wherein cycloalkyl is as defined above. Preferably C₃₋₈ cycloalkoxy. Including but not limited to cyclopropoxy, cyclobutoxy, cyclopentoxy, cyclohexoxy, etc.

"Heterocyclyloxy" refers to -O-heterocyclyl, wherein heterocyclyl is as defined above. Including but not limited to azetidinyloxy, oxetanyloxy, pyrrolidinyloxy, oxacyclohexyloxy, piperidinyloxy, etc.

"Pharmaceutically acceptable salt" refers to conventional acid addition salts or base addition salts that retain the biological effectiveness and properties of the compound of Formula I, formed from suitable non-toxic organic or inorganic acids or organic or inorganic bases. Examples of acid addition salts include those derived from inorganic acids and those derived from organic acids, such as hydrochloric acid, hydrobromic acid, hydroiodic acid, sulfuric acid, sulfamic acid, phosphoric acid, and nitric acid; and those derived from organic acid, for example, acetic acid, propionic acid, glycolic acid, oxalic acid, stearic acid, ascorbic acid, p-toluenesulfonic acid, salicylic acid, methanesulfonic acid, ethanesulfonic acid, succinic acid, citric acid, maleic acid, hydroxymaleic acid, lactic acid, fumaric acid, tartaric acid, malic acid, hydroxyethanesulfonic acid, benzenesulfonic acid, trifluoroacetic acid, mandelic acid, etc. Examples of base addition salts include those derived from inorganic bases such as ammonium salts, calcium salts, iron salts, aluminum salts, sodium salts, potassium salts, zinc salts, magnesium salts; and those derived from organic base includes salts of primary, secondary, or tertiary amines, such as trimethylamine, triethylamine, tripropylamine, diethanolamine, ethylenediamine, ethanolamine, etc. The chemical modification of a pharmaceutical compound (i.e., a drug) into a salt is a well known technique to pharmacists for obtaining improved physical and chemical stability, hygroscopicity, flowability, and solubility of the compound.

"Prodrug" refers to a structure that can be converted in vivo into the compounds of the present invention and their pharmaceutically acceptable salts i.

"N-oxide" refers to a compound in which, when the compound contains an amine functional group or a heteroaryl group containing an N atom, one or more N atom can be oxidized to form a compound containing N⁺, preferably an N-oxide of a tertiary amine or an N-oxide of an N-containing heteroaryl.

"Hydrate" refers to an association formed with a certain amount of water.

"Solvate" refers to an association formed between one or more solvent molecules and a compound of the present invention. Solvents that form solvates include, but are not limited to methanol, ethanol, isopropanol, ethyl acetate, acetic acid, etc.

"Pharmaceutical composition" refers to a mixture of one or more of the compounds of the present invention or their pharmaceutically acceptable salts, solvates, hydrates, or prodrugs with other chemical components, such as pharmaceutically acceptable carriers, excipients, or diluents. The purpose of a pharmaceutical composition is to facilitate administration to an animal. Pharmaceutical compositions may include pharmaceutically acceptable excipients to mimic physiological conditions, such as pH adjusters and buffers, toxicity modifiers, such as sodium acetate, sodium chloride, potassium chloride, calcium chloride, sodium lactate.

"Pharmaceutically acceptable carrier" refers to a pharmaceutically acceptable substance, composition, or vehicle, such as a liquid or solid filler, diluent, excipient, solvent, or encapsulating material, which participates in loading or transporting the compound involved in the present invention from one location, body fluid, tissue, organ (internal or external), or body part to another location, body fluid, organ (internal or external), or body part. A pharmaceutically acceptable carrier can be a medium, diluent, excipient, or other material without excessive toxic or side effects and suitable for contact with animal tissues.

Some substances that are pharmaceutically acceptable carriers include: (1) sugars, such as lactose, glucose, and sucrose; (2) starches, such as corn starch and potato starch; (3) cellulose and its derivatives, such as sodium carboxymethyl cellulose, ethyl cellulose, cellulose acetate; (4) powdered tragacanth; (5) maltose; (6) gelatin; (7) talc; (8) excipients, such as cocoa butter and suppository wax; (9) oils, such as peanut oil, cottonseed oil, safflower oil, sesame oil, olive oil, corn oil, and soybean oil; (10) glycols, such as propylene glycol; (11) polyols, such as glycerol, sorbitol, mannitol, and polyethylene glycol; (12) esters such as ethyl oleate, ethyl laurate; (13) agar; (14) buffers, such as magnesium hydroxide and aluminum hydroxide; (15) alginic acid; (16) sterile, pyrogen-free water; (17) physiological saline; (18) Ringer's solution; (19) alcohols, such as ethanol and propanol; (20) phosphate buffer; (21) other non-toxic, compatible substances used in pharmaceutical dosage forms, such as acetone.

Each pharmaceutically acceptable carrier should be compatible with the other ingredients, for example, forming a formulation with the compounds provided herein, without excessive toxicity, irritation, allergic reactions, immunogenicity, or other problems or complications to living biological tissues or organs, and have a reasonable benefit/risk ratio.

Pharmaceutical compositions can be prepared into any suitable dosage form, such as solid dosage forms (e.g., tablets, capsules, powders, granules, etc.) and liquid dosage forms (e.g., aqueous solutions, emulsions, elixirs, syrups, etc.). The methods for preparing pharmaceutical compositions are well known and can be prepared according to conventional techniques, such as those provided in Remington, The Science and Practice of Pharmacy (Gennaro ed. 20th edition, Williams & Wilkins PA, USA) (2000).

### DETAILED DESCRIPTION

### Group 1 Preparation Examples: Preparation of Intermediates A1-A9

### Intermediate A1. Preparation of 8-bromo-5-(4-((tertbutyldimethylsilyl)oxy)butan-2-yl)-3-chloroisoquinoline

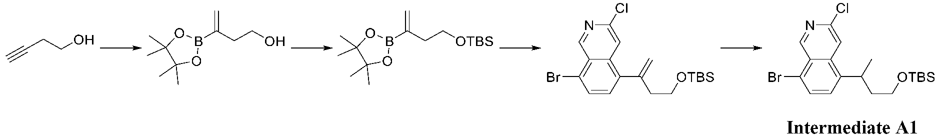

**Step 1:** Pd(PPh₃)₄ (8.2 g, 7.1 mmol) and bis(pinacolato)diboron (21.7 g, 85.6 mmol) were dissolved in toluene (50 mL), the atmosphere was replaced with nitrogen, but-3-yn-1-ol (5.0 g, 71.3 mmol) and acetic acid (3.6 mL, 62.8 mmol) were added, and the reaction mixture was pre-stirred at r.t. for 5 min, then heated to 80 °C under microwave irradiation and stired for 0.5 h. After completion of the reaction, the mixture was cooled to r.t. and concentrated to give a crude product. The crude product was purified by silica gel column chromatography eluting with a gradient of ethyl acetate/petroleum ether (0-15%) to afford 3-(4,4,5,5-tetramethyl-1,3,2-dioxaborolan-2-yl)but-3-en-1-ol (colorless oil, 12 g, 84.9%). MS(ESI⁺)m/z = 199.2 [M+H]⁺.

**Step 2:** 3-(4,4,5,5-Tetramethyl-1,3,2-dioxaborolan-2-yl)but-3-en-1-ol (12.0 g, 60.6 mmol) was dissolved in dichloromethane (100 mL), the atmosphere was replaced with nitrogen, imidazole (8.2 g, 121.2 mmol) and TBSCl (13.7 g, 90.9 mmol) were added sequentially, and the mixture was stirred at r.t. for 16 h. After completion of the reaction, water (50 mL) was added to quench the reaction. The mixture was extracted with dichloromethane (50 mL × 3). The combined organic layers were dried over anhydrous sodium sulfate, filtered, and the filtrate was concentrated to give a crude product. The crude product was purified by silica gel column chromatography eluting with a gradient of ethyl acetate/petroleum ether (0-15%) to afford tert-butyldimethyl((3-(4,4,5,5-tetramethyl-1,3,2-dioxaborolan-2-yl)but-3-en-1-yl)oxy)silane (yellow oil, 12 g, 63.4%). MS(ESI⁺)m/z = 313.2 [M+H]⁺.

**Step 3:** tert-Butyldimethyl((3-(4,4,5,5-tetramethyl-1,3,2-dioxaborolan-2-yl)but-3-en-1-yl)oxy)silane (6.0 g, 19.2 mmol), 8-bromo-3-chloro-5-iodoisoquinoline (8.5 g, 23.1 mmol), Pd(dppf)Cl₂ (1.4 g, 1.9 mmol), and potassium carbonate (8.0 g, 57.6 mmol) were dissolved in 1,4-dioxane (60 mL) and water (6 mL), the atmosphere was replaced with nitrogen, and the mixture was heated to 60 °C and stirred for 5 h. After completion of the reaction, the mixture was cooled to r.t., and water (50 mL) was added to quench the reaction. The mixture was extracted with ethyl acetate (50 mL × 3). The combined organic layers were dried over anhydrous sodium sulfate, filtered, and the filtrate was concentrated to give a crude product. The crude product was purified by silica gel column chromatography eluting with a gradient of ethyl acetate/petroleum ether (0-10%) to afford 8-bromo-5-(4-((tertbutyldimethylsilyl)oxy)but-1-en-2-yl)-3-chloroisoquinoline (yellow oil, 3 g, 36.6%). MS(ESI⁺)m/z = 428.0 [M+H]⁺.

**Step 4:** 8-Bromo-5-(4-((tert-butyldimethylsilyl)oxy)but-1-en-2-yl)-3-chloroisoquinoline (3.0 g, 7.0 mmol) was dissolved in ethyl acetate (150 mL), platinum(IV) oxide (638.4 mg, 2.8 mmol) was added slowly, the atmosphere was replaced with hydrogen, and the mixture was stirred at r.t. for 8 h. After completion of the reaction, the reaction mixture was filtered, and the filtrate was concentrated to afford 8-bromo-5-(4-((tertbutyldimethylsilyl)oxy)butan-2-yl)-3-chloroisoquinoline (yellow solid, 2.5 g, crude). MS(ESI⁺)m/z = 430.0 [M+H]⁺.

### Intermediate A2. Preparation of 8-bromo-5-(1-((tertbutyldimethylsilyl)oxy)propan-2-yl)-3-chloroisoquinoline

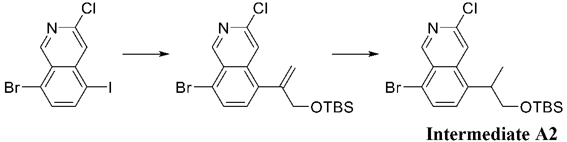

Using 8-bromo-3-chloro-5-iodoisoquinoline and tert-butyldimethyl((2-(4,4,5,5-tetramethyl-1,3,2-dioxaborolan-2-yl)allyl)oxy)silane as starting materials, 8-bromo-5-(1-((tert-butyldimethylsilyl)oxy)propan-2-yl)-3-chloroisoquinoline (Intermediate A2) was prepared according to the methods of Step 3 and Step 4 for the synthesis of Intermediate A1. MS(ESI⁺)m/z = 416.1 [M+H]⁺.

### Intermediate A3. Preparation of N-(2-((tert-butyldimethylsilyl)oxy)ethyl)-3-chloro-8-iodo-N-methylisoquinolin-5-amine

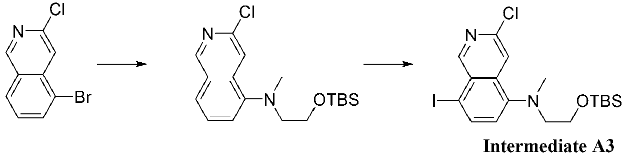

**Step 1:** Under a nitrogen atmosphere , 5-bromo-3-chloroisoquinoline (1.0 g, 4.1 mmol), N-[2-(tert-butyldimethylsiloxy)ethyl]methylamine (1.2 g, 6.2 mmol), cesium carbonate (4.1 g, 12.2 mmol), 1,4-dioxane (10 mL), Xphos Pd G₃ (0.4 g, 0.4 mmol), and Xphos (0.2 g, 0.41 mmol) were added sequentially to a 40 mL single-necked flask. The mixture was heated to 90 °C and stirred for 1 h. After completion of the reaction, the mixture was cooled to r.t., and water (30 mL) was added to quench the reaction. The mixture was extracted with ethyl acetate (100 mL × 3). The combined organic layers were washed with brine (50 mL) and dried over anhydrous sodium sulfate, filtered, and the filtrate was concentrated. The residue was purified by silica gel column chromatography eluting with ethyl acetate/petroleum ether (0-15%) to afford N-(2-((tert-butyldimethylsilyl)oxy)ethyl)-3-chloro-N-methylisoquinolin-5-amine (white solid, 700 mg, 48.3%). MS(ESI⁺)m/z = 351.2 [M+H]⁺.

**Step 2:** Under a nitrogen atmosphere, N-(2-((tert-butyldimethylsilyl)oxy)ethyl)-3-chloro-N-methylisoquinolin-5-amine (700 mg, 2.0 mmol) was dissolved in N,N-dimethylformamide (7 mL) in a 40 mL single-necked flask, N-iodosuccinimide (899.9 mg, 4.0 mmol) was added, and the mixture was heated to 60 °C and stirred for 1 h. After completion of the reaction, the mixture was cooled to r.t., and water (10 mL) was added to quench the reaction. The mixture was extracted with ethyl acetate (30 mL × 3). The combined organic layers were washed with brine (15 mL) and dried over anhydrous sodium sulfate, filtered, and the filtrate was concentrated. The residue was purified by silica gel column chromatography eluting with ethyl acetate/petroleum ether (0-15%) to afford N-(2-((tertbutyldimethylsilyl)oxy)ethyl)-3-chloro-8-iodo-N-methylisoquinolin-5-amine (white solid, 700 mg, 73.5%). MS(ESI⁺)m/z = 477.1 [M+H]⁺.

### Intermediate A4. Preparation of N-(3-((tert-butyldimethylsilyl)oxy)propyl)-3-chloro-8-iodo-N-methylisoquinolin-5-amine

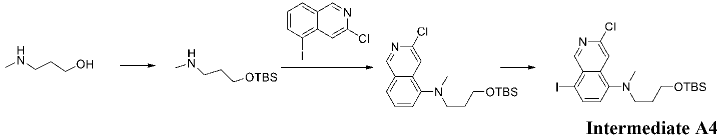

**Step 1:** 3-(Methylamino)propan-1-ol (5.0 g, 56.1 mmol) was dissolved in dichloromethane (100 mL) in a 250 mL reaction flask, then imidazole (11.5 g, 168.3 mmol) was added, and the mixture was cooled to 0 °C. TBSCl (12.6 g, 84.2 mmol) was added with stirring, and the reaction mixture was stirred at r.t. for 1 h. After completion of the reaction, water (50 mL) was added to quench the reaction. The mixture was extracted with dichloromethane (50 mL × 3). The combined organic layers were dried over anhydrous sodium sulfate, filtered, and the filtrate was concentrated. The residue was purified by silica gel column chromatography eluting with ethyl acetate/petroleum ether (0-30%) to afford 3-((tertbutyldimethylsilyl)oxy)-N-methylpropan-1-amine (yellow oil, 5.5 g, 49%). MS(ESI⁺)m/z = 204 [M+H]⁺.

**Step 2 and Step 3:** Using 3-((tert-butyldimethylsilyl)oxy)-N-methylpropan-1-amine and 3-chloro-5-iodoisoquinoline as starting materials, N-(3-((tertbutyldimethylsilyl)oxy)propyl)-3-chloro-8-iodo-N-methylisoquinolin-5-amine (Intermediate A4) was prepared according to the method for the synthesis of Intermediate A3.

### Intermediate A5. Preparation of 8-bromo-3-chloro-5-(3-((tetrahydro-2H-pyran-2-yl)oxy)propyl)isoquinoline

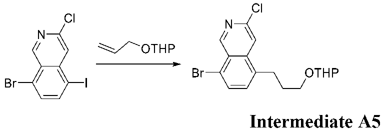

Under a nitrogen atmosphere, 8-bromo-3-chloro-5-iodoisoquinoline (2.0 g, 5.5 mmol) was dissolved in N,N-dimethylformamide (20 mL) in a 40 mL reaction flask. 2-(allyloxy)tetrahydro-2H-pyran (3.1 g, 21.8 mmol), Pd₂(dba)₃ (0.5 g, 0.55 mmol), triphenylphosphine (2.9 g, 10.9 mmol), tetramethylammonium chloride (1.2 g, 5.4 mmol), and potassium phosphate (3.5 g, 16.4 mmol) were added sequentially. The mixture was heated to 80 °C and stirred for 4 h. After completion of the reaction, the mixture was cooled to r.t., and water (30 mL) was added to quench the reaction. The mixture was extracted with ethyl acetate (40 mL × 3). The combined organic layers were washed with brine (15 mL) and dried over anhydrous sodium sulfate, filtered, and the filtrate was concentrated. The residue was purified by silica gel column chromatography eluting with ethyl acetate/petroleum ether (0-8%) to afford 8-bromo-3-chloro-5-(3-((tetrahydro-2H-pyran-2-yl)oxy)propyl)isoquinoline (white solid, 800 mg, 38.2%). MS(ESI⁺)m/z = 384.1 [M+H]⁺.

### Intermediate A6. Preparation of 3-(8-bromo-3-chloroisoquinolin-5-yl)-2,2-difluoropropan-1-ol

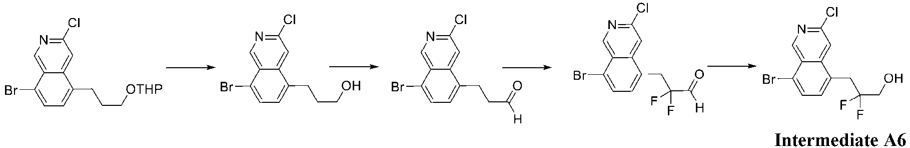

**Step** 1: 8-Bromo-3-chloro-5-(3-((tetrahydro-2H-pyran-2-yl)oxy)propyl)isoquinoline (Intermediate A5, 5.0 g, 13.1 mmol) was dissolved in methanol (50 mL) in a 100 mL reaction flask, then p-toluenesulfonic acid (11.23 g, 65.3 mmol) was added, and the mixture was heated to 60 °C and stirred for 1 h. After completion of the reaction, the mixture was cooled to r.t. and concentrated. The residue was purified by preparative liquid chromatography eluting with acetonitrile/water (0-30%) to afford 3-(8-bromo-3-chloroisoquinolin-5-yl)propan-1-ol (white solid, 3.7 g, 95.1%). MS(ESI⁺)m/z = 300.0 [M+H]⁺.

**Step 2:** 3-(8-Bromo-3-chloroisoquinolin-5-yl)propan-1-ol (1.0 g, 3.3 mmol) was dissolved in dichloromethane (10 mL) in a 100 mL reaction flask. Dess-Martin reagent (2.8 g, 6.7 mmol) was added under an ice bath. The reaction mixture was stirred at r.t. for 3 h. After completion of the reaction, brine (30 mL) was added. The mixture was extracted with dichloromethane (20 mL × 3). The combined organic layers were washed with brine (30 mL) and dried over anhydrous sodium sulfate, filtered, and the filtrate was concentrated. The residue was purified by silica gel column chromatography eluting with methanol/dichloromethane (0-20%) to afford 3-(8-bromo-3-chloroisoquinolin-5-yl)propanal (yellow oil, 880 mg, 88%). MS(ESI⁺)m/z = 298.1 [M+H]⁺.

**Step 3:** 3-(8-Bromo-3-chloroisoquinolin-5-yl)propanal (880 mg, 3.0 mmol) was dissolved in tetrahydrofuran (5 mL) in a 100 mL reaction flask. L-Proline (0.85 g, 7.4 mmol) and N-fluorobenzenesulfonimide (1.9 g, 5.9 mmol) were added sequentially. The reaction mixture was stirred at r.t. for 2 h. After completion of the reaction, an aqueous potassium bicarbonate solution (5 mL) was added to quench the reaction. The mixture was extracted with dichloromethane (15 mL × 3). The combined organic layers were dried over anhydrous sodium sulfate, filtered, and the filtrate was concentrated. The residue was purified by silica gel column chromatography eluting with ethyl acetate/petroleum ether (0-60%) to afford 3-(8-bromo-3-chloroisoquinolin-5-yl)-2,2-difluoropropanal (yellow solid, 360 mg, 36.6%). MS(ESI⁺)m/z = 334.0 [M+H]⁺.

**Step 4:** 3-(8-Bromo-3-chloroisoquinolin-5-yl)-2,2-difluoropropanal (360 mg, 1.1 mmol) was dissolved in methanol (20 mL) in a 100 mL reaction flask. The mixture was cooled to 0 °C, and sodium borohydride (86.7 mg, 2.2 mmol) was added slowly. The reaction mixture was stirred at r.t. for 4 h. After completion of the reaction, water (30 mL) was added to quench the reaction. The mixture was extracted with dichloromethane (100 mL × 3). The combined organic layers were washed with brine (10 mL) and dried over anhydrous sodium sulfate, filtered, and the filtrate was concentrated. The residue was purified by preparative liquid chromatography eluting with acetonitrile/0.1% aqueous sodium bicarbonate solution (0-25%) to afford 3-(8-bromo-3-chloroisoquinolin-5-yl)-2,2-difluoropropan-1-ol (yellow solid, 200 mg, 55.6%). MS(ESI⁺)m/z = 336.1 [M+H]⁺.

### Intermediate A7. Preparation of 3-(8-bromo-3-chloroisoquinolin-5-yl)-2,2-difluorobutan-1-ol

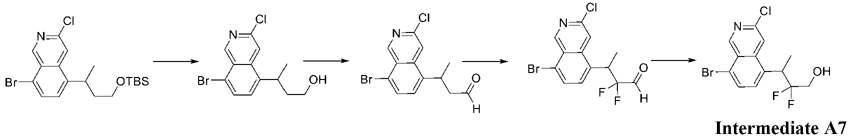

**Step** 1: 8-Bromo-5-(4-((tert-butyldimethylsilyl)oxy)butan-2-yl)-3-chloroisoquinoline (Intermediate A1, 2.0 g, 4.7 mmol) was dissolved in tetrahydrofuran (20 mL) in a 100 mL reaction flask. The mixture was cooled to 0 °C, and TBAF (4.7 mL, 1 M in THF, 4.7 mmol) was added with stirring. After the addition, the reaction mixture was stirred at r.t. for 1 h. After completion of the reaction, water (100 mL) was added to quench the reaction. The mixture was extracted with ethyl acetate (100 mL × 3). The combined organic layers were dried over anhydrous sodium sulfate, filtered, and the filtrate was concentrated. The residue was purified by silica gel column chromatography eluting with ethyl acetate/petroleum ether (0-60%) to afford 3-(8-bromo-3-chloroisoquinolin-5-yl)butan-1-ol (white solid, 1.2 g, 81%).

**Step 2, Step 3, and Step 4:** Using 3-(8-bromo-3-chloroisoquinolin-5-yl)butan-1-ol as the starting material, 3-(8-bromo-3-chloroisoquinolin-5-yl)-2,2-difluorobutan-1-ol (Intermediate A7) was prepared according to the methods of Step 2, Step 3, and Step 4 for the synthesis of Intermediate A6. MS(ESI⁺)m/z = 347.6 [M+H]⁺.

### Intermediate A8. Preparation of 4-(8-bromo-3-chloroisoquinolin-5-yl)butan-1-ol

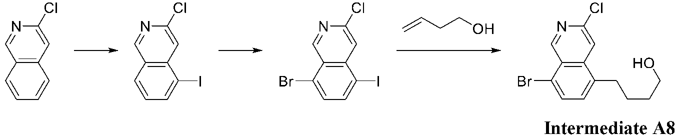

**Step 1:** 3-Chloroisoquinoline (100.0 g, 0.61 mol) was dissolved in trifluoromethanesulfonic acid (2 L). The mixture was cooled to -10 °C, and N-iodosuccinimide (151.3 g, 0.67 mol) was added slowly. The mixture was stirred at -10 °C for 1 h. After completion of the reaction, ice water (10 L) was slowly added to the reaction mixture, filtered, and the filter cake was collected and dissolved in ethyl acetate (800 mL). The pH was adjusted to 7 with aqueous ammonia. The mixture was extracted with ethyl acetate (800 mL × 2). The combined organic layers were dried over anhydrous sodium sulfate, filtered, and the filtrate was concentrated. The residue was purified by silica gel column chromatography eluting with a gradient of ethyl acetate/petroleum ether (0-20%) to afford 3-chloro-5-iodoisoquinoline (pale yellow solid, 150 g, 84.7%). MS(ESI⁺)m/z = 290.0 [M+H]⁺.

**Step 2:** At 0 °C, 3-chloro-5-iodoisoquinoline (150.0 g, 0.52 mol) was dissolved in concentrated sulfuric acid (2 L). N-Bromosuccinimide (92.2 g, 0.52 mol) was added in portions slowly. The mixture was stirred at 0 °C for 0.5 h. After completion of the reaction, ice water (4 L) was slowly added to the reaction mixture, filtered, and the filter cake was collected and dissolved in ethyl acetate (5 L). The solution was dried over anhydrous sodium sulfate, filtered, and the filtrate was concentrated. The residue was purified by silica gel column chromatography eluting with a gradient of ethyl acetate/petroleum ether (0-20%) to afford 8-bromo-3-chloro-5-iodoisoquinoline (pale yellow solid, 90 g, 47.4%). MS(ESI⁺)m/z = 369.9 [M+H]⁺.

**Step 3:** Under a nitrogen atmosphere, 8-bromo-3-chloro-5-iodoisoquinoline (3.0 g, 8.14 mmol) was dissolved in DMF (250 mL). But-3-en-1-ol (2.4 g, 33.4 mmol), an aqueous solution of tetramethylammonium hydroxide (2.6 g, 28.5 mmol), Pd₂(dba)₃ (175 mg, 0.19 mmol), triphenylphosphine (750.8 mg, 2.86 mmol), and potassium phosphate (6.1 g, 28.62 mmol) were added sequentially. The mixture was heated to 80 °C and stirred for 4 h. After completion of the reaction, the mixture was cooled to r.t. Water (40 mL) was added to the reaction mixture. The mixture was extracted with ethyl acetate (250 mL × 3). The combined organic layers were washed with brine (30 mL) and dried over anhydrous sodium sulfate, filtered, and the filtrate was concentrated. The residue was purified by silica gel column chromatography eluting with a gradient of ethyl acetate/petroleum ether (0-35%) to afford 4-(8-bromo-3-chloroisoquinolin-5-yl)butan-1-ol (white solid, 420 mg, 16.3%).

### Intermediate A9. Preparation of 8-bromo-5-(3-((tertbutyldimethylsilyl)oxy)propyl)-3-chloroisoquinoline

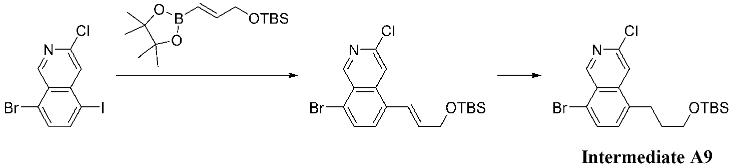

**Step 1:** Using 8-bromo-3-chloro-5-iodoisoquinoline (4.0 g, 10.86 mmol) and 3-(tert-butyldimethylsilyloxy)prop-1-en-1-ylboronic acid pinacol ester (3.90 g, 13.03 mmol) as starting materials, 8-bromo-5-(3-((tert-butyldimethylsilyl)oxy)prop-1-en-1-yl)-3-chloroisoquinoline (yellow solid, 800 mg, 17.8%) was prepared according to the method of Step 3 for the synthesis of Intermediate A1. MS(ESI⁺)m/z = 412.1 [M+H]⁺.

**Step 2:** Under a nitrogen atmosphere, 8-bromo-5-(3-((tertbutyldimethylsilyl)oxy)prop-1-en-1-yl)-3-chloroisoquinoline (800.0 mg, 1.94 mmol) was dissolved in ethyl acetate (50 mL). Rhodium on carbon (997.1 mg, 9.69 mmol) was added. The atmosphere was replaced with hydrogen, and the mixture was stirred at r.t. for 2 h. After completion of the reaction, filtered through Celite. The filtrate was concentrated. The residue was purified by silica gel column chromatography eluting with a gradient of ethyl acetate/petroleum ether (1-10%) to afford 8-bromo-5-(3-((tert-butyldimethylsilyl)oxy)propyl)-3-chloroisoquinoline (white solid, 200 mg, crude product). MS(ESI⁺)m/z = 300.1 [M-TBS+H]⁺.

### Group 2 Preparation Examples: Preparation of Intermediates B1-B11

### Intermediate B1. Preparation of 4-(4-aminopyrimidin-2-yl)-2-methyl-1-((2-(trimethylsilyl)ethoxy)methyl)-1,2-dihydro-3H-pyrazol-3-one

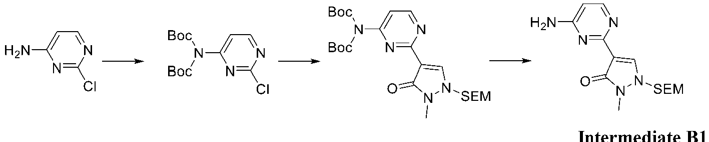

**Step 1:** 2-Chloropyrimidin-4-amine (20.0 g, 154.4 mmol) was dissolved in tetrahydrofuran (200 mL). The atmosphere was replaced with nitrogen, and the mixture was cooled to 0 °C. DMAP (1.9 g, 15.4 mmol), triethylamine (45.9 g, 453.9 mmol), and Boc₂O (100.0 g, 458.5 mmol) were added sequentially. The mixture was stirred at r.t. for 16 h. After completion of the reaction, water (100 mL) was added to the reaction mixture to quench the reaction. The mixture was extracted with ethyl acetate (100 mL × 3). The combined organic layers were dried over anhydrous sodium sulfate, filtered, and the filtrate was concentrated to afford a crude product. The crude product was purified by silica gel column chromatography eluting with a gradient of ethyl acetate/petroleum ether (0-50%) to afford tert-butyl(tert-butoxycarbonyl)(2-chloropyrimidin-4-yl)carbamate (white solid, 50 g, 98.2%). MS(ESI⁺)m/z = 330.0 [M+H]⁺.

**Step** 2: 4-Bromo-2-methyl-1-((2-(trimethylsilyl)ethoxy)methyl)-1,2-dihydro-3H-pyrazol-3-one (10.0 g, 0.033 mol), tert-butyl(tert-butoxycarbonyl)(2-chloropyrimidin-4-yl)carbamate (53.7 g, 0.16 mol), bis(pinacolato)diboron (41.3 g, 0.16 mol), Pd(Amphos)₂Cl₂ (4.6 g, 6.5 mmol), and sodium carbonate (17.2 g, 0.16 mol) were dissolved in acetonitrile (100 mL) and water (10 mL). The atmosphere was replaced with nitrogen, and the mixture was heated to 100 °C and stirred for 4 h. After completion of the reaction, the mixture was cooled to r.t. Water (100 mL) was added to the reaction mixture to quench the reaction. The mixture was extracted with ethyl acetate (100 mL × 3). The combined organic layers were dried over anhydrous sodium sulfate, filtered, and the filtrate was concentrated to afford a crude product. The crude product was purified by high-performance preparative liquid chromatography (mobile phase: acetonitrile/water (0.1% ammonia)) to afford tert-butyl(tert-butoxycarbonyl)(2-(2-methyl-3-oxo-1-((2-(trimethylsilyl)ethoxy)methyl)-2,3-dihydro-1H-pyrazol-4-yl)pyrimidin-4-yl)carbamate (white solid, 3.4 g, 20.0%). MS(ESI⁺)m/z = 522.3 [M+H]⁺.

**Step** 3: tert-Butyl(tert-butoxycarbonyl)(2-(2-methyl-3-oxo-1-((2-(trimethylsilyl)ethoxy)methyl)-2,3-dihydro-1H-pyrazol-4-yl)pyrimidin-4-yl)carbamate (3.4 g, 6.5 mmol) was dissolved in methanol (40 mL). An solution of sodium hydroxide (521.4 mg, 13.0 mmol) in water (40 mL) was added slowly. The atmosphere was replaced with nitrogen, and the mixture was heated to 80 °C and stirred for 24 h. After completion of the reaction, the mixture was cooled to r.t. Water (50 mL) was added to the reaction mixture to quench the reaction. The mixture was extracted with ethyl acetate (50 mL × 3). The combined organic layers were dried over anhydrous sodium sulfate, filtered, and the filtrate was concentrated to afford 4-(4-aminopyrimidin-2-yl)-2-methyl-1-((2-(trimethylsilyl)ethoxy)methyl)-1,2-dihydro-3H-pyrazol-3-one (white solid, 2 g, 95.5%). MS(ESI⁺)m/z = 322.0 [M+H]⁺.

### Intermediate B2. Preparation of 2-(4-((4-methoxybenzyl)oxy)-2-methylpyridin-3-yl)pyrimidin-4-amine

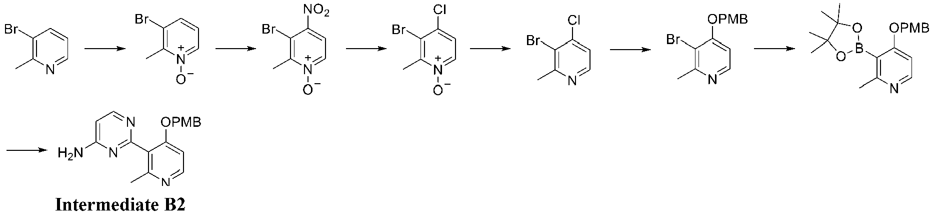

**Step 1:** At 0 °C, 3-bromo-2-methylpyridine (25.0 g, 145.33 mmol) was dissolved in dichloromethane (200 mL) in a 500 mL single-necked flask. m-Chloroperoxybenzoic acid (35.0 g, 202.83 mmol) was added slowly. The reaction mixture was stirred at 30 °C overnight. After completion of the reaction, the reaction mixture was slowly poured into ice water. The pH was adjusted to 10 with saturated sodium sulfite solution. The mixture was extracted with ethyl acetate (1.2 L × 3). The combined organic layers were dried over anhydrous sodium sulfate, filtered, and the filtrate was concentrated. The residue was purified by silica gel column chromatography eluting with methanol/dichloromethane (0-10%) to afford 3-bromo-2-methylpyridine 1-oxide (white solid, 25.0 g, 91.5%). MS(ESI⁺)m/z = 188.0 [M+H]⁺.

**Step 2:** At 0 °C, 3-bromo-2-methylpyridine 1-oxide (25.0 g, 132.96 mmol) was dissolved in concentrated sulfuric acid (200 mL) in a 500 mL single-necked flask. Concentrated nitric acid (51.8 g, 534.34 mmol) was added slowly. The mixture was heated to 90 °C and stirred overnight. After completion of the reaction, the reaction mixture was cooled to r.t. and slowly poured into ice water to quench the reaction. Saturated sodium carbonate solution was added to adjust the pH to 9. The mixture was extracted with ethyl acetate (1.2 L × 3). The combined organic layers were washed with brine (500 mL) and dried over anhydrous sodium sulfate, filtered, and the filtrate was concentrated to afford 3-bromo-2-methyl-4-nitropyridine 1-oxide (yellow solid, 15.2 g, 49.1%). MS(ESI⁺)m/z = 232.9 [M+H]⁺.

**Step 3:** At r.t., 3-bromo-2-methyl-4-nitropyridine 1-oxide (15.2 g, 65.23 mmol) was dissolved in acetonitrile (150 mL) in a 500 mL single-necked flask. Phosphorus oxychloride (50.0 g, 326.11 mmol) was added dropwise slowly. The mixture was heated to 80 °C and stirred overnight. After completion of the reaction, the reaction mixture was cooled to 0 °C. Water (100 mL) was added to quench the reaction. The mixture was extracted with ethyl acetate (800 mL × 3). The combined organic layers were washed with brine (50 mL) and dried over anhydrous sodium sulfate, filtered, and the filtrate was concentrated. The residue was purified by silica gel column chromatography eluting with methanol/dichloromethane (0-20%) to afford 3-bromo-4-chloro-2-methylpyridine 1-oxide (yellow solid, 11.6 g, 80.1%). MS(ESI⁺)m/z = 224.0 [M+H]⁺.

**Step 4:** At r.t., 3-bromo-4-chloro-2-methylpyridine 1-oxide (11.5 g, 51.69 mmol), ethanol (120 mL), water (40 mL), ammonium chloride (27.8 g, 519.72 mmol), and iron powder (14.6 g, 261.44 mmol) were added sequentially to a 500 mL single-necked flask. The mixture was heated to 80 °C and stirred for 2 h. After completion of the reaction, the mixture was cooled to r.t. and filtered. The filter cake was washed with dichloromethane. Water (100 mL) was added to the filtrate for dilution, and the mixture was extracted with dichloromethane (800 mL × 3). The combined organic layers were dried over anhydrous sodium sulfate, filtered, and the filtrate was concentrated. The residue was purified by silica gel column chromatography eluting with methanol/dichloromethane (0-20%) to afford 3-bromo-4-chloro-2-methylpyridine (yellow oil, 5.4 g, 50.6%). MS(ESI⁺)m/z = 206.0 [M+H]⁺.

**Step 5:** At 0 °C, sodium hydride (1.2 g, 30.00 mmol, 60%) and N,N-dimethylformamide (80 mL) were added sequentially to a 250 mL single-necked flask. 4-methoxybenzyl alcohol (4.0 g, 28.77 mmol) was added in portions, followed by the addition of 3-bromo-4-chloro-2-methylpyridine (5.4 g, 26.15 mmol). The mixture was heated to 80 °C and stirred for 2 h. After completion of the reaction, the mixture was cooled to r.t. Water (50 mL) was added to quench the reaction. The mixture was extracted with ethyl acetate (100 mL × 3). The combined organic layers were washed with brine (50 mL) and dried over anhydrous sodium sulfate, filtered, and the filtrate was concentrated. The residue was purified by silica gel column chromatography eluting with ethyl acetate/petroleum ether (0-15%) to afford 3-bromo-4-((4-methoxybenzyl)oxy)-2-methylpyridine (white solid, 5.8 g, crude). MS(ESI⁺)m/z = 308.0 [M+H]⁺.

**Step 6:** At r.t., 3-bromo-4-((4-methoxybenzyl)oxy)-2-methylpyridine (5.8 g, 18.82 mmol) was dissolved in 1,4-dioxane (120 mL) in a 250 mL single-necked flask. Bis(pinacolato)diboron (33.6 g, 132.32 mmol), potassium acetate (5.6 g, 57.06 mmol), and [1,1'-bis(diphenylphosphino)ferrocene]palladium(II) dichloride dichloromethane complex (1.5 g, 1.84 mmol) were successively added. Under a nitrogen atmosphere, the mixture was heated to 100 °C and stirred for 2 days. After completion of the reaction, the mixture was cooled to r.t. Water (100 mL) was added for dilution, and the mixture was extracted with ethyl acetate (500 mL × 3). The combined organic layers were dried over anhydrous sodium sulfate, filtered, and the filtrate was concentrated. The residue was purified by silica gel column chromatography eluting with methanol/dichloromethane (0-20%) to afford 4-((4-methoxybenzyl)oxy)-2-methyl-3-(4,4,5,5-tetramethyl-1,3,2-dioxaborolan-2-yl)pyridine (brown oil, 3.7 g, 55.4%). MS(ESI⁺)m/z = 356.2 [M+H]⁺.

**Step 7:** At r.t., 4-((4-methoxybenzyl)oxy)-2-methyl-3-(4,4,5,5-tetramethyl-1,3,2-dioxaborolan-2-yl)pyridine (3.7 g, 10.42 mmol), 1,4-dioxane (70 mL), water (7 mL), 2-bromopyrimidin-4-amine (1.2 g, 6.90 mmol), potassium carbonate (2.9 g, 20.84 mmol), and tetrakis(triphenylphosphine)palladium(0) (0.8 g, 0.69 mmol) were added sequentially to a 250 mL single-necked flask. Under a nitrogen atmosphere, the mixture was heated to 100 °C and stirred overnight. After completion of the reaction, the mixture was cooled to r.t. Water (100 mL) was added for dilution, and the mixture was extracted with ethyl acetate (500 mL × 3). The combined organic layers were dried over anhydrous sodium sulfate, filtered, and the filtrate was concentrated. The residue was purified by silica gel column chromatography eluting with methanol/dichloromethane (0-20%) to afford 2-(4-((4-methoxybenzyl)oxy)-2-methylpyridin-3-yl)pyrimidin-4-amine (yellow solid, 270.0 mg, 12.1%). MS(ESI⁺)m/z = 323.1 [M+H]⁺.

### Intermediate B3. Preparation of 2-(4-methoxypyridin-3-yl)pyrimidin-4-amine

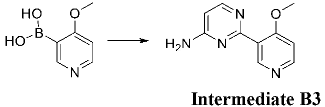

Using (4-methoxypyridin-3-yl)boronic acid and 2-bromopyrimidin-4-amine as starting materials, 2-(4-methoxypyridin-3-yl)pyrimidin-4-amine (Intermediate B3) was prepared according to the method of Step 7 for the synthesis of Intermediate B2. MS(ESI⁺)m/z = 203.1 [M+H]⁺.

### Intermediate B4. Preparation of 2-(4-(benzyloxy)pyridin-3-yl)pyrimidin-4-amine

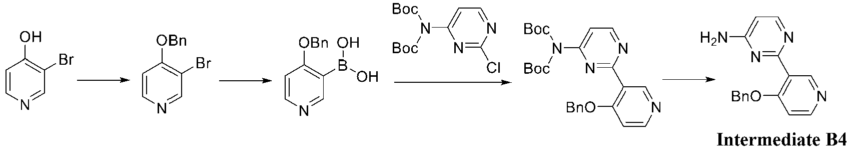

**Step 1:** 3-Bromopyridin-4-ol (50 g, 289 mmol) was dissolved in acetonitrile (500 mL) in a 1000 mL three-necked round-bottom flask. Potassium carbonate (80 g, 581 mmol) was added, followed by dropwise addition of benzyl bromide (54.4 g, 318 mmol). The reaction mixture was stirred at r.t. for 12 h. After completion of the reaction, water (100 mL) was added. The mixture was extracted with ethyl acetate (300 mL × 2). The combined organic layers were washed with brine (50 mL) and dried over anhydrous sodium sulfate, filtered, and the filtrate was concentrated. The residue was purified by silica gel column chromatography eluting with methanol/dichloromethane (0-5%) to afford 4-(benzyloxy)-3-bromopyridine (white solid, 60 g, 78.9%). MS(ESI⁺)m/z = 264.0 [M+H]⁺.

**Step 2:** Under a nitrogen atmosphere, 4-(benzyloxy)-3-bromopyridine (10 g, 38.0 mmol) was dissolved in 1,4-dioxane (500 mL) in a 1000 mL three-necked round-bottom flask. Pd(dppf)Cl₂ (3.11 g, 3.8 mmol), potassium acetate (11.2 g, 114.1 mmol), and bis(pinacolato)diboron (19.3 mg, 76.1 mmol) were successively added. The mixture was heated to 110 °C and stirred for 1 h. After completion of the reaction, the mixture was cooled to r.t. The reaction mixture was directly purified by preparative liquid chromatography eluting with acetonitrile/water (0-5%) to afford (4-(benzyloxy)pyridin-3-yl)boronic acid (yellow solid, 6 g, 68.6%). MS(ESI⁺)m/z = 230.1 [M+H]⁺.

**Step 3:** Under a nitrogen atmosphere, (4-(benzyloxy)pyridin-3-yl)boronic acid (6.0 g, 26.2 mmol) was dissolved in a mixture of 1,4-dioxane and water (550 mL, 10/1, v/v) in a 1000 mL three-necked round-bottom flask. tert-Butyl(tert-butoxycarbonyl)(2-chloropyrimidin-4-yl)carbamate (12.9 g, 39.3 mmol), Pd(dppf)Cl₂ (2.1 g, 2.6 mmol), and potassium carbonate (10.9 g, 78.6 mmol) were successively added. The mixture was heated to 90 °C and stirred for 1 h. After completion of the reaction, the mixture was cooled to r.t. Water (100 mL) was added, and the mixture was extracted with ethyl acetate (500 mL × 3). The combined organic layers were washed with brine (50 mL) and dried over anhydrous sodium sulfate, filtered, and the filtrate was concentrated. The residue was purified by silica gel column chromatography eluting with methanol/dichloromethane (0-5%) to afford tert-butyl(tert-butoxycarbonyl)(2-(4-(benzyloxy)pyridin-3-yl)pyrimidin-4-yl)carbamate (yellow oil, 6.0 g, 48%). MS(ESI⁺)m/z = 379.2 [M-100+H]⁺.

**Step 4:** tert-Butyl(tert-butoxycarbonyl)(2-(4-(benzyloxy)pyridin-3-yl)pyrimidin-4-yl)carbamate (6.0 g, 12.6 mmol) was dissolved in dichloromethane (50 mL) in a 100 mL three-necked round-bottom flask. Trifluoroacetic acid (10 mL) was added, and the reaction mixture was stirred at r.t. for 1 h. After completion of the reaction, the mixture was concentrated to afford 2-(4-(benzyloxy)pyridin-3-yl)pyrimidin-4-amine (brown solid, 4.0 g, crude). MS(ESI⁺)m/z = 279.2 [M+H]⁺.

### Intermediate B5. Preparation of 2-(4-fluoropyridin-3-yl)pyrimidin-4-amine

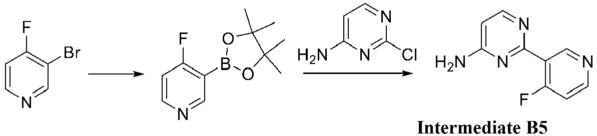

**Step 1:** Under a nitrogen atmosphere, 3-bromo-4-fluoropyridine (1 g, 5.7 mmol) was dissolved in 1,4-dioxane (20 mL) in a 100 mL reaction flask. Bis(pinacolato)diboron (2.8 g, 11.0 mmol), Pd(dppf)Cl₂ (415 mg, 0.57 mmol), and potassium acetate (1.7 g, 17.3 mmol) were successively added. The mixture was heated to 90 °C and stirred for 5 h. After completion of the reaction, the mixture was cooled to r.t. Water (5 mL) was added to quench the reaction. The mixture was concentrated to afford 4-fluoro-3-(4,4,5,5-tetramethyl-1,3,2-dioxaborolan-2-yl)pyridine (black oil, 1 g, 78%). The crude product was used directly in the next step without purification.

**Step 2:** Under a nitrogen atmosphere, 4-fluoro-3-(4,4,5,5-tetramethyl-1,3,2-dioxaborolan-2-yl)pyridine (950 mg, 4.3 mmol) was dissolved in a mixture of 1,4-dioxane and water (20 mL, 9/1, v/v) in a 100 mL reaction flask. 2-Chloropyrimidin-4-amine (1.1 g, 8.5 mmol), Pd(dppf)Cl₂ (312 mg, 0.43 mmol), and potassium carbonate (1.8 g, 13.0 mmol) were successively added. The mixture was heated to 100 °C and stirred for 1 h. After completion of the reaction, the mixture was cooled to r.t. Water (20 mL) was added to quench the reaction. The mixture was extracted with ethyl acetate (100 mL × 3). The combined organic layers were washed with brine (30 mL) and dried over anhydrous sodium sulfate, filtered, and the filtrate was concentrated. The residue was purified by silica gel column chromatography eluting with methanol/dichloromethane (0-31%) to afford 2-(4-fluoropyridin-3-yl)pyrimidin-4-amine (white solid, 310 mg, 31%). MS(ESI⁺)m/z = 191.1 [M+H]⁺.

### Intermediate B6. Preparation of 4-(4-aminopyrimidin-2-yl)-2,5-dimethyl-1-((2-(trimethylsilyl)ethoxy)methyl)-1,2-dihydro-3H-pyrazol-3-one

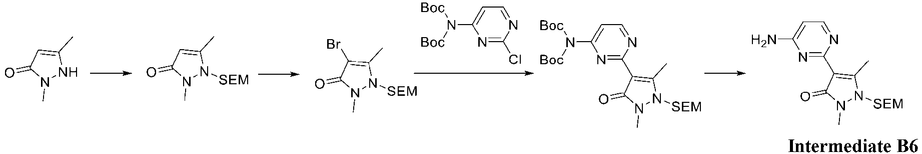

**Step 1:** 2,5-Dimethyl-1,2-dihydro-3H-pyrazol-3-one (50.0 g, 446.4 mmol) was dissolved in dichloromethane (1500 mL) in a 2000 mL round-bottom flask. Potassium carbonate (308.0 g, 2232.1 mmol) was added, followed by slow dropwise addition of 2-(trimethylsilyl)ethoxymethyl chloride (111.2 g, 669.6 mmol) under an ice bath. The mixture was stirred at r.t. for 16 h. After completion of the reaction, water (500 mL) was added. The mixture was extracted with dichloromethane (1000 mL × 3). The combined organic layers were washed with brine (200 mL) and dried over anhydrous sodium sulfate, filtered, and the filtrate was concentrated. The residue was purified by silica gel column chromatography eluting with methanol/dichloromethane (0-5%) to afford 2,5-dimethyl-1-((2-(trimethylsilyl)ethoxy)methyl)-1,2-dihydro-3H-pyrazol-3-one (yellow oil, 50 g, 46.3%). MS(ESI⁺)m/z = 243.2 [M+H]⁺.

**Step 2:** 2,5-Dimethyl-1-((2-(trimethylsilyl)ethoxy)methyl)-1,2-dihydro-3H-pyrazol-3-one (50.0 g, 206.6 mmol) was dissolved in chloroform (1000 mL) in a 100 mL round-bottom flask. The temperature was cooled to 0 °C, and N-bromosuccinimide (43.9 g, 247.9 mmol) was added in portions. After addition, the mixture was stirred at 25 °C for 2 h. After completion of the reaction, saturated sodium thiosulfate (1000 mL) was added to quench the reaction. The mixture was extracted with dichloromethane (500 mL × 3). The combined organic layers were washed with brine (1000 mL) and dried over anhydrous sodium sulfate, filtered, and the filtrate was concentrated. The residue was purified by silica gel column chromatography eluting with methanol/dichloromethane (0-2%) to afford 4-bromo-2,5-dimethyl-1-((2-(trimethylsilyl)ethoxy)methyl)-1,2-dihydro-3H-pyrazol-3-one (yellow solid, 40 g, 60.3 %). MS(ESI⁺)m/z = 321.1 [M+H]⁺.

**Step 3** and **Step 4:** Using 4-bromo-2,5-dimethyl-1-((2-(trimethylsilyl)ethoxy)methyl)-1,2-dihydro-3H-pyrazol-3-one and tert-butyl(tert-butoxycarbonyl)(2-chloropyrimidin-4-yl)carbamate as starting materials, 4-(4-aminopyrimidin-2-yl)-2,5-dimethyl-1-((2-(trimethylsilyl)ethoxy)methyl)-1,2-dihydro-3H-pyrazol-3-one (Intermediate B6) was prepared according to the methods of Step 2 and Step 3 for the synthesis of Intermediate B1. MS(ESI⁺)m/z = 336.3 [M+H]⁺.

### Intermediate B7. Preparation of 2-(4-methoxypyridin-3-yl)-6-methylpyrimidin-4-amine

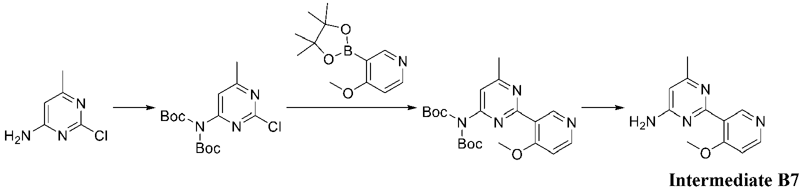

**Step 1:** 2-Chloro-6-methylpyrimidin-4-amine(24.0 g, 0.17 mol) was dissolved in THF (300 mL). DMAP (1.0 g, 8.36 mmol) and Boc₂O (73.0 g, 0.33 mol) were successively added. The mixture was stirred at r.t. for 4 h. After completion of the reaction, the reaction mixture was concentrated. Water (200 mL) was added to the residue. The mixture was extracted with ethyl acetate (200 mL × 3). The combined organic layers were dried over anhydrous sodium sulfate, filtered, and the filtrate was concentrated. The residue was purified by silica gel column chromatography eluting with ethyl acetate/petroleum ether (10%) to afford tert-butyl(tert-butoxycarbonyl)(2-chloro-6-methylpyrimidin-4-yl)carbamate (yellow solid, 50 g, 87.0%). MS(ESI⁺)m/z = 344.2 [M+H]⁺.

**Step 2 and Step 3:** Using tert-butyl(tert-butoxycarbonyl)(2-chloro-6-methylpyrimidin-4-yl)carbamate and 4-methoxypyridine-3-boronic acid pinacol ester as starting materials, 2-(4-methoxypyridin-3-yl)-6-methylpyrimidin-4-amine (Intermediate B7, light brown solid) was prepared according to the methods of Step 3 and Step 4 for the synthesis of Intermediate B4. MS(ESI⁺)m/z = 217.2 [M+H]⁺.

### Intermediate B8. Preparation of 6-ethyl-2-(4-methoxypyridin-3-yl)pyrimidin-4-amine trifluoroacetate

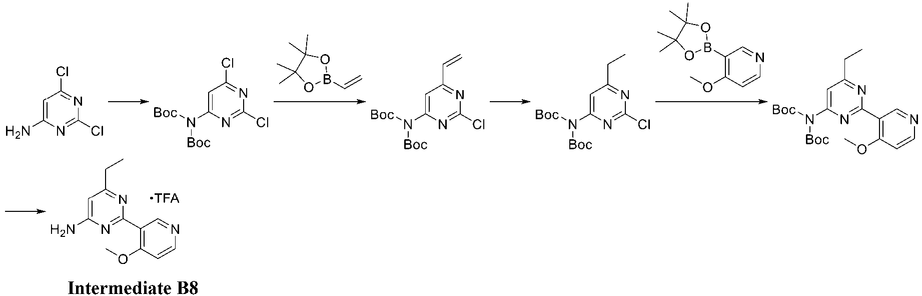

**Step 1:** Using 2,6-dichloropyrimidin-4-amine and Boc₂O as starting materials, tert-butyl(tert-butoxycarbonyl)(2,6-dichloropyrimidin-4-yl)carbamate (white solid) was prepared according to the method of Step 1 for the synthesis of Intermediate B7. MS(ESI⁺)m/z = 364.0 [M+H]⁺.

**Step 2:** Under a nitrogen atmosphere, tert-butyl(tert-butoxycarbonyl)(2,6-dichloropyrimidin-4-yl)carbamate (50.0 g, 137.3 mmol) was dissolved in a mixture of 1,4-dioxane/water (550 mL v/v, 10/1). Vinylboronic acid pinacol ester (23.3 g, 151.1 mmol), Pd(dppf)Cl₂ (10.0 g, 13.7 mmol), and K₂CO₃ (38.0 g, 275.0 mmol) were successively added. The temperature was warmed to 100 °C and stirred for 1 h. After completion of the reaction, the mixture was cooled to r.t. Water (500 mL) was added to the reaction mixture. The mixture was extracted with ethyl acetate (5000 mL × 3). The combined organic layers were dried over anhydrous sodium sulfate, filtered, and the filtrate was concentrated. The residue was purified by silica gel column chromatography using ethyl acetate/petroleum ether (0-20%) as eluent to afford tert-butyl(tert-butoxycarbonyl)(2-chloro-6-vinylpyrimidin-4-yl)carbamate (white oil, 27 g, 55.3%). MS(ESI⁺)m/z = 356.1 [M+H]⁺.

**Step 3:** Under a nitrogen atmosphere, tert-butyl(tert-butoxycarbonyl)(2-chloro-6-vinylpyrimidin-4-yl)carbamate (23.0 g, 64.64 mmol) was dissolved in ethyl acetate (230 mL). Platinum(IV) oxide (5.9 g, 25.86 mmol) was added. The atmosphere was replaced with hydrogen, and the mixture was stirred at r.t. for 4 h. After completion of the reaction, filtered through Celite. The filter cake was washed with ethyl acetate (200 mL). The filtrate was concentrated to afford tert-butyl(tert-butoxycarbonyl)(2-chloro-6-ethylpyrimidin-4-yl)carbamate (black solid, 22.8 g, 98.6%). MS(ESI⁺)m/z = 358.2 [M+H]⁺.

**Step 4:** Under a nitrogen atmosphere, tert-butyl(tert-butoxycarbonyl)(2-chloro-6-ethylpyrimidin-4-yl)carbamate (2.0 g, 5.59 mmol) was dissolved in a mixture of 1,4-dioxane and water (10/1 v/v, 44 mL). Pd(dppf)Cl₂ (409.0 mg, 0.56 mmol), Cs₂CO₃ (3.6 g, 11.18 mmol), and 4-methoxypyridine-3-boronic acid pinacol ester (1.4 g, 6.15 mmol) were successively added. The temperature was warmed to 100 °C and stirred for 1 h. After completion of the reaction, the mixture was cooled to r.t. Water (100 mL) was added to the reaction mixture. The mixture was extracted with ethyl acetate (100 mL × 2). The combined organic layers were dried over anhydrous sodium sulfate, filtered, and the filtrate was concentrated. The residue was purified by silica gel column chromatography eluting with ethyl acetate/petroleum ether (20%) to afford tert-butyl(tert-butoxycarbonyl)(6-ethyl-2-(4-methoxypyridin-3-yl)pyrimidin-4-yl)carbamate (colorless oil, 1.7 g, 70.6%). MS(ESI⁺)m/z = 431.2 [M+H]⁺.

**Step 5:** Using tert-butyl(tert-butoxycarbonyl)(6-ethyl-2-(4-methoxypyridin-3-yl)pyrimidin-4-yl)carbamate (1.7 g, 3.95 mmol) as the starting material, 6-ethyl-2-(4-methoxypyridin-3-yl)pyrimidin-4-amine trifluoroacetate (yellow solid, 1.2 g, 88.3%) was prepared according to the method of Step 4 for the synthesis of Intermediate B4. MS(ESI⁺)m/z = 231.2 [M+H]⁺.

### Intermediate B9. Preparation of 4-(4-amino-6-cyclopropylpyrimidin-2-yl)-2-methyl-1-((2-(trimethylsilyl)ethoxy)methyl)-1,2-dihydro-3H-pyrazol-3-one

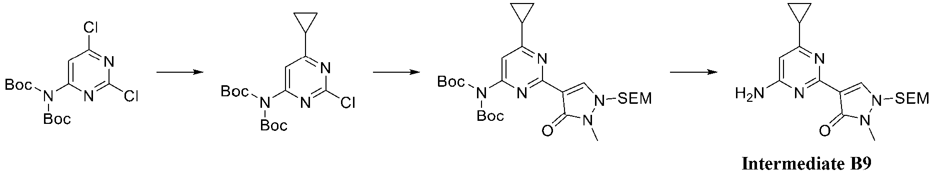

**Step 1:** tert-Butyl(tert-butoxycarbonyl)(2,6-dichloropyrimidin-4-yl)carbamate (30.0 g, 82.4 mmol) was dissolved in THF (30 mL). Fe(acac)₃ (5.8 g, 16.5 mmol) was added. The temperature was cooled to 0 °C, and cyclopropylmagnesium bromide (23.9 g, 164.7 mmol) was added dropwise. The mixture was stirred at r.t. for 2 h. After completion of the reaction, the temperature was cooled to 0 °C. Water (80 mL) was added to the reaction mixture, filtered, and the filter cake was washed with ethyl acetate (100 mL). The filtrate was extracted with ethyl acetate (100 mL × 2). The combined organic layers were dried over anhydrous sodium sulfate, filtered, and the filtrate was concentrated. The residue was purified by silica gel column chromatography eluting with ethyl acetate/petroleum ether (5%) to afford tert-butyl(tert-butoxycarbonyl)(2-chloro-6-cyclopropylpyrimidin-4-yl)carbamate (yellow oil, 23 g, 75.5%). MS(ESI⁺)m/z = 370.1 [M+H]⁺.

**Step 2:** Using tert-butyl(tert-butoxycarbonyl)(2-chloro-6-cyclopropylpyrimidin-4-yl)carbamate (6.0 g, 16.27 mmol) and 4-bromo-2-methyl-1-((2-(trimethylsilyl)ethoxy)methyl)-1,2-dihydro-3H-pyrazol-3-one (2.5 g, 8.1 mmol) as starting materials, tert-butyl(tert-butoxycarbonyl)(6-cyclopropyl-2-(2-methyl-3-oxo-1-((2-(trimethylsilyl)ethoxy)methyl)-2,3-dihydro-1H-pyrazol-4-yl)pyrimidin-4-yl)carbamate (yellow solid, 1.9 g, 41.6%) was prepared according to the method of Step 2 for the synthesis of Intermediate B1. MS(ESI⁺)m/z = 462.2 [M-100+H]⁺.

**Step 3:** tert-Butyl(tert-butoxycarbonyl)(6-cyclopropyl-2-(2-methyl-3-oxo-1-((2-(trimethylsilyl)ethoxy)methyl)-2,3-dihydro-1H-pyrazol-4-yl)pyrimidin-4-yl)carbamate (1.5 g, 2.67 mmol) was dissolved in hexafluoroisopropanol (60 mL). TFA (8.5 g, 74.77 mmol) was added, and the mixture was stirred at r.t. for 12 h. After completion of the reaction, the pH was adjusted to 8 with a saturated sodium bicarbonate solution. The mixture was extracted with ethyl acetate (50 mL × 3). The combined organic layers were dried over anhydrous sodium sulfate, filtered, and the filtrate was concentrated. The residue was purified by silica gel column chromatography eluting with methanol/dichloromethane (1/10) to afford 4-(4-amino-6-cyclopropylpyrimidin-2-yl)-2-methyl-1-((2-(trimethylsilyl)ethoxy)methyl)-1,2-dihydro-3H-pyrazol-3-one (yellow solid, 865 mg, 89.6%). MS(ESI⁺)m/z = 362.2 [M+H]⁺.

### Intermediate B10. Preparation of 4-(4-amino-6-methylpyrimidin-2-yl)-2-methyl-1-((2-(trimethylsilyl)ethoxy)methyl)-1,2-dihydro-3H-pyrazol-3-one

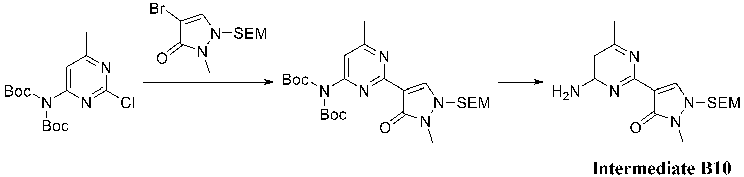

Using tert-butyl(tert-butoxycarbonyl)(2-chloro-6-methylpyrimidin-4-yl)carbamate and 4-bromo-2-methyl-1-((2-(trimethylsilyl)ethoxy)methyl)-1,2-dihydro-3H-pyrazol-3-one as starting materials, 4-(4-amino-6-methylpyrimidin-2-yl)-2-methyl-1-((2-(trimethylsilyl)ethoxy)methyl)-1,2-dihydro-3H-pyrazol-3-one (yellow solid) was prepared according to the methods of Steps 2 and 3 for the synthesis of B1. MS(ESI⁺)m/z = 336.2 [M+H]⁺.

### Intermediate B11. Preparation of 2-(4-((4-methoxybenzyl)oxy)-2-methylpyridin-3-yl)-6-methylpyrimidin-4-amine

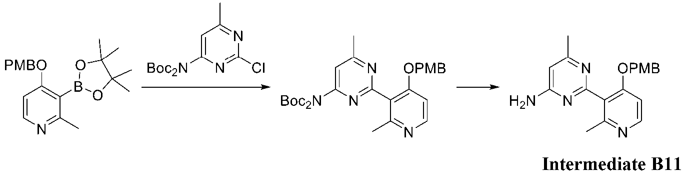

**Step 1:** Under a nitrogen atmosphere, 4-((4-methoxybenzyl)oxy)-2-methyl-3-(4,4,5,5-tetramethyl-1,3,2-dioxaborolan-2-yl)pyridine(800.0 mg, 2.39 mmol) was dissolved in a mixture of 1,4-dioxane and water (11 mL, 10/1, v/v). tert-Butyl(tert-butoxycarbonyl)(2-chloro-6-methylpyrimidin-4-yl)carbamate(2.3 g, 6.68 mmol), Pd(dppf)Cl₂ (0.18 g, 0.23 mmol), and potassium carbonate (0.94 g, 6.77 mmol) were added sequentially. The temperature was warmed to 100 °C and stirred for 1 h. After completion of the reaction, the mixture was cooled to r.t. Brine (30 mL) was added. The mixture was extracted with ethyl acetate (30 mL × 3). The combined organic layers were washed with brine (30 mL), dried over anhydrous sodium sulfate, filtered, and the filtrate was concentrated. The residue was purified by silica gel column chromatography eluting with ethyl acetate/petroleum ether (5%-20%) to afford tert-butyl(tert-butoxycarbonyl)(2-(4-((4-methoxybenzyl)oxy)-2-methylpyridin-3-yl)-6-methylpyrimidin-4-yl)carbamate (yellow oil, 200 g, 16.7%). MS(ESI⁺)m/z = 537.1 [M+H]⁺.

**Step 2:** In a 250 mL reaction flask, tert-butyl(tert-butoxycarbonyl)(2-(4-((4-methoxybenzyl)oxy)-2-methylpyridin-3-yl)-6-methylpyrimidin-4-yl)carbamate (200 mg, 0.37 mmol) was dissolved in methanol (5 mL). A sodium hydroxide solution (5 mL, 1.5 M) was added dropwise. The temperature was warmed to 80 °C and stirred for 12 h. After completion of the reaction, the mixture was cooled to r.t. The reaction mixture was extracted with dichloromethane (50 mL × 3). The combined organic layers were washed with brine (30 mL), dried over anhydrous sodium sulfate, filtered, and the filtrate was concentrated to afford 2-(4-((4-methoxybenzyl)oxy)-2-methylpyridin-3-yl)-6-methylpyrimidin-4-amine (yellow solid, 100 mg, 80 %). MS(ESI⁺)m/z = 337.2 [M+H]⁺.

### Group 3 of Preparation Examples: Preparation of Intermediates C1-C8

### Intermediate C1. Preparation of (2R,3S)-3-(methylsulfonylmethyl)-2-methylazetidine trifluoroacetate

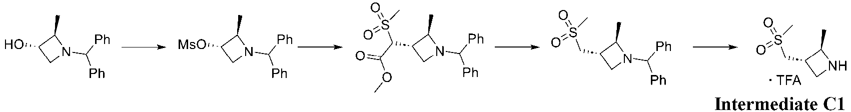

**Step 1:** To a 40 mL single-necked flask were added (2R,3S)-1-(diphenylmethyl)-2-methylazetidin-3-ol (5 g, 19.7 mmol) and dichloromethane (60 mL)sequentially. Triethylamine (6 g, 59.2 mmol) and methanesulfonyl chloride (3.39 g, 29.6 mmol) were added sequentially at 0 °C. The atmosphere was replaced with nitrogen, and the mixture was stirred at r.t. for 12 h. Water (30 mL) was added to quench the reaction. The mixture was extracted with dichloromethane (20 mL × 3). The combined organic layers were dried over anhydrous sodium sulfate, filtered, and the filtrate was concentrated to afford (2R,3S)-1-(diphenylmethyl)-2-methylazetidin-3-yl methanesulfonate (pale yellow oil, 5 g, 76.4%). MS(ESI⁺)m/z = 332.05 [M+H]⁺.

**Step 2:** To a 250 mL single-necked flask were added (2R,3S)-1-(diphenylmethyl)-2-methylazetidin-3-yl methanesulfonate (5 g, 15.09 mmol) and N,N-dimethylformamide (60 mL) sequentially. Sodium hydride (1.1 g, 45.3 mmol) was added at 0 °C. The atmosphere was replaced with nitrogen, and the mixture was stirred at 0 °C for 0.5 h. Then, methyl 2-(methylsulfonyl)acetate (5.7 g, 37.7 mmol) was added. The temperature was warmed to 80 °C and stirred for 12 h. After completion of the reaction, the mixture was cooled to r.t. Water (10 mL) was added to quench the reaction. The mixture was extracted with ethyl acetate (20 mL × 3). The combined organic layers were dried over anhydrous sodium sulfate, filtered, and the filtrate was concentrated to afford the crude product. The crude product was purified by silica gel column chromatographyusing ethyl acetate/petroleum ether (0-46%) as eluent to afford methyl 2-[(2R,3S)-1-(diphenylmethyl)-2-methylazetidin-3-yl]-2-(methylsulfonyl)acetate (white solid, 4 g, 68.4%). MS(ESI⁺)m/z = 388.05 [M+H]⁺.

**Step 3:** To a 250 mL single-necked flask were added methyl 2-[(2R,3S)-1-(diphenylmethyl)-2-methylazetidin-3-yl]-2-(methylsulfonyl)acetate (4 g, 10.32 mmol) and N,N-dimethylformamide (40 mL) sequentially. Lithium chloride (3.5 g, 82.6 mmol) was added with stirring. The temperature was warmed to 150 °C and stirred for 4 h. The reaction mixture was cooled to r.t. Water (30 mL) was added to quench the reaction. The mixture was extracted with ethyl acetate (20 mL × 3). The combined organic layers were dried over anhydrous sodium sulfate, filtered, and the filtrate was concentrated to afford the crude product. The crude product was purified by silica gel column chromatography using ethyl acetate/petroleum ether (0-52%) as eluent to afford (2R,3S)-1-(diphenylmethyl)-3-(methylsulfonylmethyl)-2-methylazetidine (off-white solid, 2 g, 58.8%). MS(ESI⁺)m/z = 330.10 [M+H]⁺.

**Step 4:** To a 50 mL single-necked flask were added (2R,3S)-1-(diphenylmethyl)-3-(methylsulfonylmethyl)-2-methylazetidine (700 mg, 2.13 mmol), methanol (10.5 mL), and trifluoroacetic acid (0.35 mL) sequentially. Added palladium hydroxide (208.9 mg, 1.49 mmol) while standing. The atmosphere was replaced with hydrogen, and the mixture was stirred at r.t. for 12 h, filtered, and the filtrate was concentrated to afford (2R,3S)-3-(methylsulfonylmethyl)-2-methylazetidine trifluoroacetate (colorless semi-oily solid, 300 mg, 86.5%). MS(ESI⁺)m/z = 164.05 [M+H]⁺.

### Intermediate C2. Preparation of N-((2R,3S)-2-methylazetidin-3-yl)methanesulfonamide trifluoroacetate

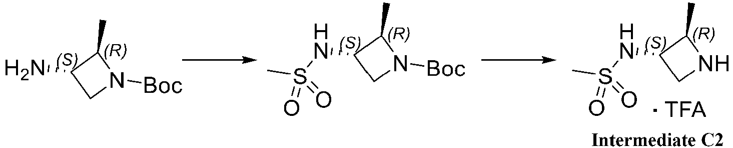

**Step 1:** Under a nitrogen atmosphere, tert-butyl (2R,3S)-3-amino-2-methylazetidine-1-carboxylate (300 mg, 1.61 mmol) was dissolved in dichloromethane (5 mL), and then triethylamine (489.0 mg, 4.83 mmol) was added. The temperature was cooled to 0 °C, and methanesulfonyl chloride (184.5 mg, 1.61 mmol) was added dropwise slowly with stirring. The temperature was warmed to r.t. and stirred for 1 h. After the reaction was complete as monitored by LCMS, the mixture was concentrated. The residue was purified by silica gel column chromatography using ethyl acetate/petroleum ether (1/1) as eluent to afford tert-butyl (2R,3S)-2-methyl-3-(methylsulfonamido)azetidine-1-carboxylate (white solid, 300 mg, 70.5%). MS(ESI⁺)m/z = 165.10 [M-100+H]⁺.

**Step 2:** tert-Butyl (2R,3S)-2-methyl-3-(methylsulfonamido)azetidine-1-carboxylate (300 mg, 1.13 mmol) was dissolved in dichloromethane/trifluoroacetic acid (3/1, 6 mL) in a 40 mL single-necked flask. The mixture was stirred at r.t. for 30 min. After the reaction was complete as monitored by LCMS, the mixture was concentrated at low temperature to afford N-((2R,3S)-2-methylazetidin-3-yl)methanesulfonamide (yellow oil, 400 mg, crude). MS(ESI⁺)m/z = 165.05 [M+H]⁺.

### Intermediate C3. Preparation of N-methyl-N-((2R,3S)-2-methylazetidin-3-yl)methanesulfonamide trifluoroacetate

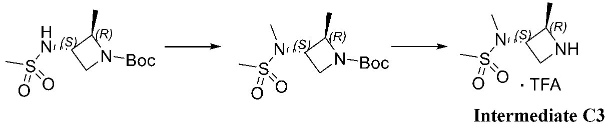

**Step 1:** tert-Butyl (2R,3S)-2-methyl-3-(methylsulfonamido)azetidine-1-carboxylate (450 mg, 1.7 mmol) was dissolved in DMF (4.79 mL). Sodium hydride (61.28 mg, 2.553 mmol) was added at 0 °C with stirring. The atmosphere was replaced with nitrogen, and stirring was continued for 0.5 h. Then, iodomethane (289.96 mg, 2.042 mmol) was added, and the mixture was stirred at r.t. for 1 h. Water (50 mL) was added to quench the reaction. The mixture was extracted with ethyl acetate (50 mL × 3). The combined organic layers were dried over anhydrous sodium sulfate, filtered, and the filtrate was concentrated to afford the crude product. The crude product was purified by silica gel column chromatography using ethyl acetate/petroleum ether (0-50%) as eluent to afford tert-butyl (2R,3S)-2-methyl-3-(N-methylmethylsulfonamido)azetidine-1-carboxylate (colorless transparent oil, 380 mg, 80.2%). MS(ESI⁺)m/z = 179.1 [M-100+H]⁺.

**Step 2:** Using tert-butyl (2R,3S)-2-methyl-3-(N-methylmethylsulfonamido)azetidine-1-carboxylate as starting material, N-methyl-N-((2R,3S)-2-methylazetidin-3-yl)methanesulfonamide trifluoroacetate was prepared according to the method of Step 2 for the synthesis of Intermediate C2. MS(ESI⁺)m/z = 179.2 [M+H]⁺.

### Intermediate C4. Preparation of 2-(methylsulfonyl)-2,6-diazaspiro[3.3]heptane

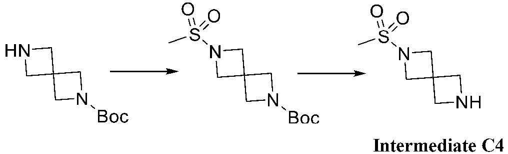

**Step 1:** tert-Butyl 2,6-diazaspiro[3.3]heptane-2-carboxylate (1.0 g, 5.0 mmol) was dissolved in dichloromethane (10 mL) in a 40 mL reaction flask. Triethylamine (1.5 g, 15.2 mmol) was added, followed by slow addition of Ms₂O (1.3 g, 7.5 mmol). The temperature was warmed to 100 °C and stirred for 1 h. After the reaction was complete, the mixture was cooled to r.t. Water (100 mL) was added to quench the reaction. The mixture was extracted with dichloromethane (150 mL × 3). The combined organic layers were dried over anhydrous sodium sulfate, filtered, and the filtrate was concentrated. The residue was purified by silica gel column chromatography using ethyl acetate/petroleum ether (0-60%) as eluent to afford tert-butyl 6-(methylsulfonyl)-2,6-diazaspiro[3.3]heptane-2-carboxylate (yellow oily solid, 600 mg, 46%). MS(ESI⁺)m/z = 277 [M+H]⁺.

**Step 2:** tert-Butyl 6-(methylsulfonyl)-2,6-diazaspiro[3.3]heptane-2-carboxylate (600 mg, 2.17 mmol) was dissolved in dichloromethane (6 mL). The temperature was cooled to 0 °C, and trifluoroacetic acid (4 mL) was added slowly. The mixture was stirred at r.t. for 1 h. After the reaction was complete, the mixture was concentrated. The residue was purified by high-performance preparative liquid chromatography (Column: XBridge BEH Prep OBD Amide Column, 19*250 mm, 5 µm; Mobile phase: acetonitrile/water (10 mmol/L NH₄HCO₃); Flow rate: 60 mL/min; Gradient: 5%-95% 7 min; Wavelength: 254/220 nm) to afford 2-(methylsulfonyl)-2,6-diazaspiro[3.3]heptane (yellow oil, 250 mg, 65%). MS(ESI⁺)m/z = 176.8 [M+H]⁺.

### Intermediate C5. Preparation of dimethyl(((2R,3S)-2-methylazetidin-3-yl)imino)-λ⁶-sulfoxide trifluoroacetate

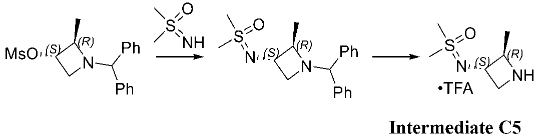

**Step 1:** (2R,3S)-1-(Diphenylmethyl)-2-methylazetidin-3-yl methanesulfonate (10.0 g, 30.17 mmol) was dissolved in acetonitrile (100 mL). Cs₂CO₃ (19.7 g, 60.34 mmol) and dimethyl sulfoximide (4.2 g, 45.26 mmol) were added sequentially. The temperature was warmed to 80 °C and stirred for 2 h. After the reaction was complete, the mixture was cooled to r.t. Water (200 mL) was added to the reaction mixture. The mixture was extracted with ethyl acetate (200 mL × 2). The combined organic layers were dried over anhydrous sodium sulfate, filtered, and the filtrate was concentrated. The residue was purified by silica gel column chromatography using ethyl acetate/petroleum ether (1/1) as eluent to afford (((2R,3S)-1-diphenylmethyl-2-methylazetidin-3-yl)imino)dimethyl-λ⁶-sulfoxide (white solid, 6 g, 60.5%). MS(ESI⁺)m/z = 329.1 [M+H]⁺.

**Step 2:** Using (((2R,3S)-1-diphenylmethyl-2-methylazetidin-3-yl)imino)dimethyl-λ⁶-sulfoxide (6.0 g, 18.27 mmol) as starting material, dimethyl(((2R,3S)-2-methylazetidin-3-yl)imino)-λ⁶-sulfanone trifluoroacetate (white solid, 5 g, 99.1%) was prepared according to the method of Step 4 for the synthesis of Intermediate C1.

### Intermediate C6. Preparation of N-methyl-N-(pyrrolidin-3-yl)methanesulfonamide hydrochloride

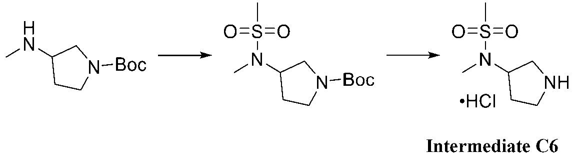

**Step 1:** Using 1-Boc-3-methylaminopyrrolidine (1.3 g, 6.49 mmol) and Ms₂O (1.7 g, 9.74 mmol) as starting materials, tert-butyl 3-(N-methylmethylsulfonamido)pyrrolidine-1-carboxylate (yellow solid, 2 g, crude) was prepared according to the method of Step 1 for the synthesis of Intermediate C4.

**Step 2:** tert-Butyl 3-(N-methylmethylsulfonamido)pyrrolidine-1-carboxylate (2.1 g, 7.54 mmol) was dissolved in DCM (21 mL). A solution of hydrogen chloride in 1,4-dioxane (4 M, 11 mL, 45.26 mmol) was added, and the mixture was stirred at r.t. for 2 h. After the reaction was complete, the mixture was concentrated to afford N-methyl-N-(pyrrolidin-3-yl)methanesulfonamide hydrochloride (yellow solid, 1.3 g, yield over two steps: 88.8%).

### Intermediate C7. Preparation of N-cyclopropyl-N-((2R,3S)-2-methylazetidin-3-yl)methanesulfonamide trifluoroacetate

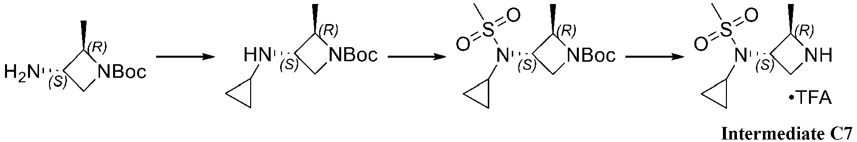

**Step 1:** tert-Butyl (2R,3S)-3-(cyclopropylamino)-2-methylazetidine-1-carboxylate (1.0 g, 5.37 mmol) was dissolved in methanol (10 mL). Sodium cyanoborohydride (1.0 g, 16.11 mmol) and (1-ethoxycyclopropoxy)trimethylsilane (2.8 g, 16.11 mmol) were added. The temperature was warmed to 60 °C and stirred for 12 h. After the reaction was complete, the mixture was cooled to r.t. Water (50 mL) was added to the reaction mixture. The mixture was extracted with ethyl acetate (50 mL × 3). The combined organic layers were dried over anhydrous sodium sulfate, filtered, and the filtrate was concentrated to afford tert-butyl (2R,3S)-3-(cyclopropylamino)-2-methylazetidine-1-carboxylate (white oil, 500 mg, crude).

**Step 2:** tert-Butyl (2R,3S)-3-(cyclopropylamino)-2-methylazetidine-1-carboxylate (500 mg, 2.2 mmol) was dissolved in dichloromethane (5 mL). Triethylamine (447 mg, 4.4 mmol) was added, and the temperature was cooled to 0 °C. Methanesulfonic anhydride (920 mg, 5.28 mmol) was added slowly. The mixture was warmed to r.t. and stirred for 2 h. After the reaction was complete, the temperature was cooled to 0 °C. Water (50 mL) was added to the reaction mixture. The mixture was extracted with dichloromethane (50 mL × 3). The combined organic layers were dried over anhydrous sodium sulfate, filtered, and the filtrate was concentrated to afford tert-butyl (2R,3S)-3-(N-cyclopropylmethylsulfonamido)-2-methylazetidine-1-carboxylate (white oil, 350 mg, 52%).

**Step 3:** Using tert-butyl (2R,3S)-3-(N-cyclopropylmethylsulfonamido)-2-methylazetidine-1-carboxylate as starting material, N-cyclopropyl-N-((2R,3S)-2-methylazetidin-3-yl)methanesulfonamide trifluoroacetate (black oil) was prepared according to the method of Step 2 for the synthesis of Intermediate C4.

### Intermediate C8. Preparation of N-ethyl-N-((2R,3S)-2-methylazetidin-3-yl)methanesulfonamide trifluoroacetate

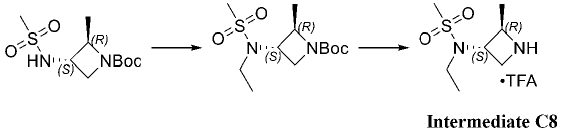

**Step 1:** Under a nitrogen atmosphere, tert-butyl (2R,3S)-2-methyl-3-(methylsulfonamido)azetidine-1-carboxylate (595.0 mg, 2.25 mmol) was dissolved in acetonitrile (10 mL). Cesium carbonate (2.2 g, 6.75 mmol) and iodoethane (421.3 mg, 2.70 mmol) were added sequentially. The temperature was warmed to 100 °C and stirred for 1 h. After the reaction was complete, the mixture was cooled to r.t. Water (20 mL) was added to the reaction mixture. The mixture was extracted with ethyl acetate (100 mL × 3). The combined organic layers were washed with brine (30 mL), dried over anhydrous sodium sulfate, filtered, and the filtrate was concentrated. The residue was purified by silica gel column chromatography using methanol/dichloromethane (0-50%) as eluent to afford tert-butyl (2R,3S)-3-(N-ethylmethylsulfonamido)-2-methylazetidine-1-carboxylate (white solid, 500 mg, 76.0%). MS(ESI⁺)m/z = 293.3 [M+H]⁺.

**Step 2:** Using tert-butyl (2R,3S)-3-(N-ethylmethylsulfonamido)-2-methylazetidine-1-carboxylate (500.0 mg, 1.71 mmol) as starting material, N-ethyl-N-((2R,3S)-2-methylazetidin-3-yl)methanesulfonamide trifluoroacetate (yellow oil, 320 mg, crude) was prepared according to the method of Step 2 for the synthesis of Intermediate C4. MS(ESI⁺)m/z = 193.2 [M+H]⁺.

### Group 4 of Preparation Examples: Preparation of Final Products

### Example 1 Preparation of N-((2S,3R)-1-(1¹,5-dimethyl-1¹H-8-oxa-3-aza-4(3,5)-isoquinolin-2(2,4)-pyrimidin-1(4,5)-pyrazolcyclooctan-4⁸-yl)-2-methylazetidin-3-yl)-N-methylmethanesulfonamide (Final Product 1), N-methyl-N-((2R,3S)-2-methyl-1-(1¹-methyl-1¹H-9-oxa-3-aza-4(3,5)-isoquinolin-2(2,4)-pyrimidin-1(4,5)-pyrazolcyclononan-4⁸-yl)azetidin-3-yl)methanesulfonamide (Final Product 36), N-((2S,3R)-1-((S)-1¹,5-dimethyl-1¹H-8-oxa-3-aza-4(3,5)-isoquinolin-2(2,4)-pyrimidin-1(4,5)-pyrazolcyclooctan-4⁸-yl)-2-methylazetidin-3-yl)-N-methylmethanesulfonamide and N-((2S,3R)-1-((R)-1¹,5-dimethyl-1¹H-8-oxa-3-aza-4(3,5)-isoquinolin-2(2,4)-pyrimidin-1(4,5)-pyrazolcyclooctan-4⁸-yl)-2-methylazetidin-3-yl)-N-methylmethanesulfonamide

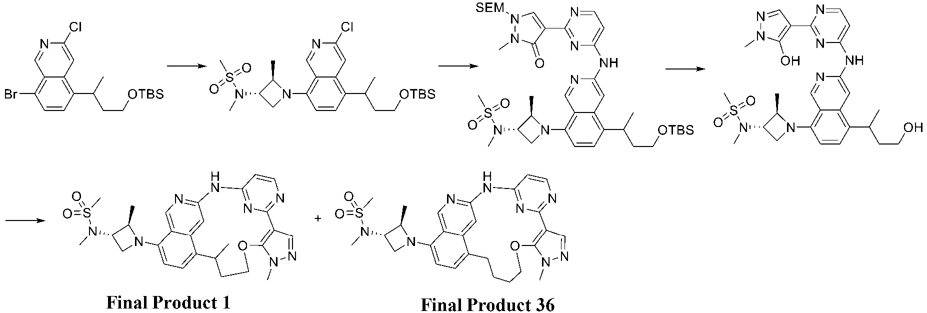

**Step 1:** Intermediate A1 (1.5 g, 3.5 mmol), Intermediate C3 (866.5 mg, 3.2 mmol), Xantphos Pd G₄ (337.9 mg, 0.35 mmol), and cesium carbonate (3.4 g, 10.5 mmol) were dissolved in 1,4-dioxane (15 mL). The atmosphere was replaced with nitrogen, the temperature was warmed to 100 °C and stirred for 1 h. After the reaction was complete, the mixture was cooled to r.t. Water (50 mL) was added to the reaction mixture to quench the reaction. The mixture was extracted with ethyl acetate (50 mL × 3). The combined organic layers were dried over anhydrous sodium sulfate, filtered, and the filtrate was concentrated to afford a crude product. The crude product was purified by silica gel column chromatography using methanol/dichloromethane (0-10%) as eluent to afford N-((2R,3S)-1-(5-(4-((tertbutyldimethylsilyl)oxy)butan-2-yl)-3-chloroisoquinolin-8-yl)-2-methylazetidin-3-yl)-N-methylmethanesulfonamide (yellow solid, 750 mg, 40.8%). MS(ESI⁺)m/z = 526.1 [M+H]⁺.

**Step 2:** N-((2R,3S)-1-(5-(4-((tert-butyldimethylsilyl)oxy)butan-2-yl)-3-chloroisoquinolin-8-yl)-2-methylazetidin-3-yl)-N-methylmethanesulfonamide (100.0 mg, 0.19 mmol), Intermediate B1 (73.3 mg, 0.23 mmol), EPhos Pd G₄ (17.5 mg, 0.019 mmol), Ephos (10.16 mg, 0.019 mmol), and cesium carbonate (185.8 mg, 0.57 mmol) were dissolved in 1,4-dioxane (5 mL). The atmosphere was replaced with nitrogen, the temperature was warmed to 100 °C and stirred for 1 h. After the reaction was complete, the mixture was cooled to r.t. Water (50 mL) was added to the reaction mixture to quench the reaction. The mixture was extracted with ethyl acetate (50 mL × 3). The combined organic layers were dried over anhydrous sodium sulfate, filtered, and the filtrate was concentrated to afford a crude product. The crude product was purified by silica gel column chromatography using methanol/dichloromethane (0-10%) as eluent to afford N-((2R,3S)-1-(5-(4-((tertbutyldimethylsilyl)oxy)butan-2-yl)-3-((2-(2-methyl-3-oxo-1-((2-(trimethylsilyl)ethoxy)methyl)-2,3-dihydro-1H-pyrazol-4-yl)pyrimidin-4-yl)amino)isoquinolin-8-yl)-2-methylazetidin-3-yl)-N-methylmethanesulfonamide (yellow solid, 100 mg, 64.9%). MS(ESI⁺)m/z = 811.6 [M+H]⁺. The reaction conditions for this step could also be: Brettphos Pd G₄, Cs₂CO₃, 1,4-dioxane, 100 °C.

**Step 3:** N-((2R,3S)-1-(5-(4-((tert-butyldimethylsilyl)oxy)butan-2-yl)-3-((2-(2-methyl-3-oxo-1-((2-(trimethylsilyl)ethoxy)methyl)-2,3-dihydro-1H-pyrazol-4-yl)pyrimidin-4-yl)amino)isoquinolin-8-yl)-2-methylazetidin-3-yl)-N-methylmethanesulfonamide (90.0 mg, 0.11 mmol) was dissolved in tetrahydrofuran (3 mL). The atmosphere was replaced with nitrogen, TBAF (58.0 mg, 0.22 mmol) was added, the temperature was warmed to 70 °C and stirred for 1 h. After the reaction was complete, the mixture was cooled to r.t. Water (5 mL) was added to the reaction mixture to quench the reaction. The mixture was extracted with dichloromethane (5 mL × 3). The combined organic layers were dried over anhydrous sodium sulfate, filtered, and the filtrate was concentrated to afford a crude product. The crude product was purified by silica gel column chromatography using methanol/dichloromethane (0-20%) as eluent to afford N-((2R,3S)-1-(5-(4-hydroxybutan-2-yl)-3-((2-(5-hydroxy-1-methyl-1H-pyrazol-4-yl)pyrimidin-4-yl)amino)isoquinolin-8-yl)-2-methylazetidin-3-yl)-N-methylmethanesulfonamide (yellow solid, 50 mg, 79.5%). MS(ESI⁺)m/z = 567.3 [M+H]⁺.

**Step 4:** N-((2R,3S)-1-(5-(4-hydroxybutan-2-yl)-3-((2-(5-hydroxy-1-methyl-1H-pyrazol-4-yl)pyrimidin-4-yl)amino)isoquinolin-8-yl)-2-methylazetidin-3-yl)-N-methylmethanesulfonamide (50.0 mg, 0.088 mmol) was dissolved in toluene (2 mL). The atmosphere was replaced with nitrogen, CMBP (42.6 mg, 0.18 mmol) was added, the temperature was warmed to 110 °C, and stirred for 1 h. After the reaction was complete, the mixture was cooled to r.t. Water (5 mL) was added to the reaction mixture to quench the reaction. The mixture was extracted with dichloromethane (5 mL × 3). The combined organic layers were dried over anhydrous sodium sulfate, filtered, and the filtrate was concentrated to afford a crude product. The crude product was purified by high-performance preparative liquid chromatography (Column: Xselect CSH C18 OBD Column, 30*150 mm, 5 µm; Mobile phase: acetonitrile/water (0.1% formic acid); Flow rate: 60 mL/min; Gradient: 12%-42%-10 min; Wavelength: 254/220 nm) to afford N-((2S,3R)-1-(1¹,5-dimethyl-1¹H-8-oxa-3-aza-4(3,5)-isoquinolin-2(2,4)-pyrimidin-1(4,5)-pyrazolcyclooctan-4⁸-yl)-2-methylazetidin-3-yl)-N-methylmethanesulfonamide (Final Product 1) and N-methyl-N-((2R,3S)-2-methyl-1-(1¹-methyl-1¹H-9-oxa-3-aza-4(3,5)-isoquinolin-2(2,4)-pyrimidin-1(4,5)-pyrazolcyclononan-4⁸-yl)azetidin-3-yl)methanesulfonamide (Final Product 36).

**Final Product 1:** Pale yellow solid, MS(ESI⁺)m/z = 549.3 [M+H]⁺. ¹H NMR (300 MHz, DMSO-*d*₆) *δ* 10.18 (d, *J =* 11.7 Hz, 1H), 9.17-9.05 (m, 2H), 8.28 (dd, *J =* 4.8, 0.9 Hz, 1H), 7.94 (d, *J =* 2.5 Hz, 1H), 7.53 (d, *J =* 8.1 Hz, 1H), 6.90-6.81 (m, 1H), 6.76-6.58 (m, 1H), 4.66 (t, *J* = 7.4 Hz, 1H), 4.46-4.33 (m, 2H), 4.22 (q, *J =* 6.7 Hz, 1H), 3.87-3.72 (m, 5H), 2.96 (d, *J* = 2.1 Hz, 3H), 2.82 (d, *J =* 3.3 Hz, 3H), 2.28-2.10 (m, 1H), 2.02-1.79 (m, 2H), 1.58-1.47 (m, 3H), 1.47-1.39 (m, 3H).

**Final Product 36:** Off-white solid, MS(ESI⁺)m/z = 549.3 [M+H]⁺. ¹H NMR (300 MHz, DMSO-*d*₆) *δ* 10.21 (s, 1H), 9.10 (s, 1H), 9.00 (s, 1H), 8.31 (d, *J =* 5.8 Hz, 1H), 7.89 (s, 1H), 7.38 (d, *J =* 7.7 Hz, 1H), 6.91 (d, *J* = 5.8 Hz, 1H), 6.61 (d, *J =* 7.8 Hz, 1H), 4.64 (t, *J* = 7.4 Hz, 1H), 4.43 (t, *J* = 6.2 Hz, 1H), 4.37-4.28 (m, 1H), 4.27-4.14 (m, 2H), 3.84 (t, *J* = 7.2 Hz, 1H), 3.75 (s, 3H), 3.29-3.21 (m, 1H), 3.19-3.08 (m, 1H), 2.95 (s, 3H), 2.82 (s, 3H), 2.00-1.71 (m, 4H), 1.41 (d, *J* = 5.9 Hz, 3H).

Final Product 1 was separated by a chiral preparative column (Column: CHIRALPAK IG 2*25 cm, 5 µm; Mobile phase A: cyclohexane/dichloromethane (0.5% 2 M NH₃-MeOH) = 3/1, Mobile phase B: EtOH; Flow rate: 20 mL/min; Wavelength: 220/254 nm) to afford **Final Product 2**. The specific configuration of **Final Product 2** is uncertain.

### Final Product 2:

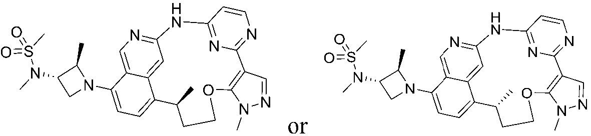

Pale yellow solid, MS(ESI⁺)m/z = 549.3 [M+H]⁺. ¹H NMR (300 MHz, DMSO-*d*₆) *δ* 10.19 (s, 1H), 9.09 (d, *J =* 14.7 Hz, 2H), 8.28 (d, *J =* 5.8 Hz, 1H), 7.92 (s, 1H), 7.52 (d, *J =* 8.0 Hz, 1H), 6.83 (d, *J =* 5.9 Hz, 1H), 6.68 (d, *J =* 8.1 Hz, 1H), 4.64 (t, *J =* 7.4 Hz, 1H), 4.53-4.37 (m, 3H), 4.26-4.14 (m, 1H), 3.84-3.78 (m, 5H), 2.94 (s, 3H), 2.80 (s, 3H), 2.22-1.88 (m, 2H), 1.49 (d, *J =* 6.8 Hz, 3H), 1.41 (d, *J =* 5.9 Hz, 3H).

### Example 2-21 Preparation of Final Products 10, 12, 13, 78-79, 84, 110-111, 117, 119, 124, 139, 145, 148, 154-155, 166-167, 176, 187, 190

Using Intermediates A1-A9, Intermediates B1-B10, and Intermediates C1-C8 as starting materials, Final Products10, 12, 13, 78-79, 84, 110-111, 117, 119, 124, 139, 145, 148, 154-155, 166-167, 176, 187, 190 were prepared according to the synthetic method of Example 1. As shown in Table 1. The coupling conditions for the first step could also be: Pd₂(dba)₃, BINAP, tBuONa, toluene, 90-100 °C or Pd₂(dba)₃, Xantphos, Cs₂CO₃, 1,4-dioxane, 100 °C. The deprotection reaction conditions for the third step could also be: TBAF, TMEDA, THF, 70 °C.

**Table 1 Characterization Data for Final Products 10, 12, 13, 78-79, 84, 110-111, 117, 119, 124, 139, 145, 148, 154-155, 166-167, 176, 187, 190**

| **Final Product** | **Inter mediat e** | **Structure of Final Product** | **NMR or MS** |
|---|---|---|---|
| **Final Product 10** | **A2** | 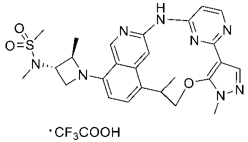 Purified by high-performance preparative liquid chromatography, Mobile phase: acetonitrile/water (0.1% trifluoroacetic acid). | MS(ESI⁺)m/z = 535.3 [M+H]⁺. ¹H NMR (300 MHz, DMSO-*d*₆) *δ* 10.88 (s, 1H), 9.16-8.98 (m, 2H), 8.33 (d, *J* = 6.4 Hz, 1H), 7.94 (s, 1H), 7.57-7.42 (m, 1H), 7.31-6.93 (m, 1H), 6.92-6.84 (m, 1H), 6.76-6.63 (m, 1H), 4.90-4.79 (m, 1H), 4.78-4.62 (m, 1H), 4.52-4.32 (m, 1H), 4.26-4.14 (m, 1H), 4.04-3.93 (m, 1H), 3.91-3.85 (m, 1H), 3.83 (s, 1H), 3.79 (d, *J =* 2.8 Hz, 3H), 2.95 (d, *J =* 2.8 Hz, 3H), 2.86-2.78 (m, 3H), 1.72 (d, *J =* 6.8 Hz, 3H), 1.44-1.39 (m, 3H). |
| | **B1** | | |
| | **C3** | | |
| **Final Product 12** | **A3** | 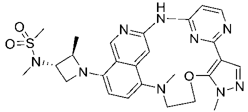 | MS(ESI⁺)m/z = 550.2 [M+H]⁺. ¹H NMR (300 MHz, DMSO-*d*₆) *δ* 10.19 (s, 1H), 9.20 (s, 1H), 9.07 (s, 1H), 8.30 (d, *J =* 5.8 Hz, 1H), 7.97 (s, 1H), 7.20 (d, *J* = 8.2 Hz, 1H), 6.85 (d, *J* = 5.8 Hz, 1H), 6.65 (d, *J =* 8.3 Hz, 1H), 4.69-4.54 (m, 3H), 4.50-4.30 (m, 1H), 4.26-4.13 (m, 1H), 3.84 (s, 3H), 3.74 (t, *J =* 7.1 Hz, 1H), 3.37-3.32 (m, 2H), 2.95 (s, 3H), 2.92 (s, 3H), 2.83 (s, 3H), 1.40 (d, *J =* 5.9 Hz, 3H). |
| | **B1** | | |
| | **C3** | | |
| **Final Product 13** | **A4** | 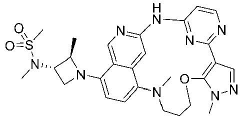 The coupling conditions for the first step were: Pd₂(dba)₃, BINAP, tBuONa, toluene, 90 °C. | MS (ESI⁺)m/z = 564.2 [M+H]⁺. ¹H NMR (300 MHz, Methanol-*d₄*) *δ* 9.28 (s, 1H), 9.12 (s, 1H), 8.47-8.21 (m, 1H), 7.95 (s, 1H), 7.48-7.30 (m, 1H), 7.02-6.55 (m, 2H), 4.67-4.52 (m, 3H), 4.52-4.37 (m, 2H), 4.34-4.04 (m, 1H), 3.98-3.62 (m, 4H), 3.11-2.81 (m, 10H), 2.39-1.82 (m, 2H), 1.53 (s, 3H). |
| | **B1** | | |
| | **C3** | | |
| **Final Product 78** | **A9** | 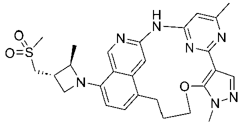 | Yellow solid. MS (ESI⁺) m/z = 534.3 [M+H]⁺. ¹H NMR (300 MHz, DMSO-*d*₆) *δ* 10.06 (s, 1H), 9.06-8.96 (m, 2H), 7.91 (s, 1H), 7.41 (d, *J =* 7.8 Hz, 1H), 6.67 (s, 1H), 6.53 (d, *J* = 7.9 Hz, 1H), 4.66 (t, *J* = 7.5 Hz, 1H), 4.46-4.23 (m, 2H), 4.23-4.13 (m, 1H), 3.76 (s, 3H), 3.69-3.58 (m, 1H), 3.57-3.42 (m, 2H), 3.23-3.12 (m, 1H), 3.10-3.01 (m, 1H), 2.98 (s, 3H), 2.94-2.81 (m, 1H), 2.31 (s, 3H), 2.28-2.21 (m, 1H), 2.09-1.98 (m, 1H), 1.42 (d, *J =* 6.0 Hz, 3H). |
| | **B10** | | |
| | **C1** | | |
| **Final Product 79** | **A8** | 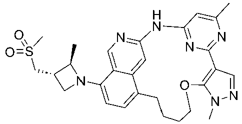 | Yellow solid. MS (ESI⁺) m/z = 548.2 [M+H]⁺. ¹H NMR (400 MHz, DMSO-*d*₆) *δ* 10.07 (s, 1H), 9.06 (s, 1H), 8.98 (s, 1H), 7.88 (s, 1H), 7.35 (d, *J =* 7.8 Hz, 1H), 6.75 (s, 1H), 6.51 (d, *J =* 7.8 Hz, 1H), 4.67 (t, *J* = 7.6 Hz, 1H), 4.36-4.27 (m, 1H), 4.25-4.15 (m, 2H), 3.74 (s, 3H), 3.70-3.62 (m, 1H), 3.62-3.54 (m, 2H), 3.26-3.22 (m, 1H), 3.19-3.09 (m, 1H), 3.00 (s, 3H), 2.95-2.85 (m, 1H), 2.33 (s, 3H), 1.92-1.75 (m, 4H), 1.43 (d, *J =* 6.0 Hz, 3H). |
| | **B10** | | |
| | **C1** | | |
| **Final Product 84** | **A3** | 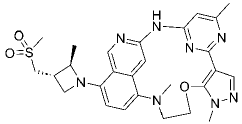 The coupling conditions for the first step were: Pd₂(dba)₃, BINAP, tBuONa, toluene, 90 °C. | Yellow solid. MS (ESI⁺) m/z = 549.2 [M+H]⁺. ¹H NMR (300 MHz, Methanol-*d₄*) *δ* 9.15 (s, 1H), 8.98 (s, 1H), 8.03 (s, 1H), 7.15 (d, *J =* 8.1 Hz, 1H), 6.61-6.48 (m, 2H), 4.59-4.37 (m, 4H), 4.20-4.10 (m, 1H), 3.77 (s, 3H), 3.57-3.26 (m, 5H), 2.96-2.88 (m, 6H), 2.30 (s, 3H), 1.38 (d, *J =* 6.0 Hz, 3H). |
| | **B10** | | |
| | **C1** | | |
| **Final Product 110** | **A6** | 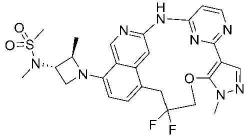 | White solid. MS (ESI⁺) m/z = 571.2 [M+H]⁺. ¹H NMR (300 MHz, Chloroform-d) *δ* 9.09 (s, 1H), 8.97 (s, 1H), 8.48-8.36 (m, 2H), 7.57 (d, *J =* 7.9 Hz, 1H), 6.72-6.60 (m, 2H), 4.67 (t, *J =* 7.5 Hz, 2H), 4.53-4.39 (m, 2H), 4.36-4.24 (m, 2H), 3.97 (t, *J =* 7.1 Hz, 2H), 3.90 (s, 3H), 2.93 (s, 3H), 2.89 (s, 3H), 1.54 (d, *J* = 6.0 Hz, 3H). |
| | **B1** | | |
| | **C3** | | |
| **Final Product 111** | | 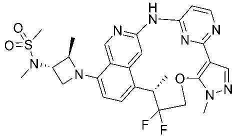 | Chiral separation: Column: CHIRALPAK IFColumn, 2*25 mm, 5 µm; Mobile phase A: Hex (0.5% 2 M NH₃-MeOH); Mobile phase B: EtOH/DCM = 1/1; Flow rate: 20 mL/min; Gradient: isocratic 40; Wavelength: 254/220 nm. |
| | **A7** | | Off-white solid. MS (ESI⁺) m/z = 585.3 [M+H]⁺. ¹H NMR (300 MHz, Acetonitrile-*d₃*) *δ* 9.12 (s, 1H), 8.91 (s, 1H), 8.37 (s, 1H), 8.26 (d, *J =* 5.7 Hz, 1H), 7.99 (s, 1H), 7.62 (d, *J =* 8.1 Hz, 1H), 6.74 (d, *J =* 8.2 Hz, 1H), 6.67 (d, *J =* 5.8 Hz, 1H), 4.95-4.43 (m, 4H), 4.35-4.09 (m, 2H), 3.91 (t, *J =* 7.1 Hz, 1H), 3.83 (s, 3H), 2.86 (d, *J =* 9.9 Hz, 6H), 1.68-1.56 (m, 3H), 1.46 (d, *J =* 6.1 Hz, 3H). ¹⁹F NMR (282 MHz, Acetonitrile-*d₃*) *δ* -102.95, -103.86, -109.21, -110.11. |
| | **B1** | or | |
| | **C3** | 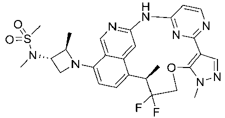 | |
| **Final Product 117** | | 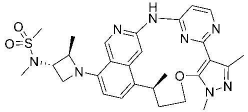 | Chiral separation: Column: CHIRALPAK IE Column, 2*25 mm, 5 µm; Mobile phase A: n-Hexane (0.5% 2 M NH₃-MeOH); Mobile phase B: Ethanol/DCM (1/1); Flow rate: 20 mL/min; Wavelength: 254 nm. |
| | **A1** | | Off-white solid. MS (ESI⁺) m/z = 563.4 [M+H]⁺. ¹H NMR (300 MHz, Chloroform-d) *δ* 9.30 (s, 1H), 9.04 (s, 1H), 8.85 (s, 1H), 8.45 (d, *J =* 5.7 Hz, 1H), 7.61 (d, *J =* 8.0 Hz, 1H), 6.67 (d, *J =* 8.0 Hz, 1H), 6.59 (d, *J =* 5.8 Hz, 1H), 4.62 (t, *J =* 7.4 Hz, 1H), 4.52-4.40 (m, 2H), 4.37-4.16 (m, 2H), 4.11-3.98 (m, 1H), 3.92 (t*, J* = 7.1 Hz, 1H), 3.80 (s, 3H), 2.93 (s, 3H), 2.88 (s, 3H), 2.60 (s, 3H), 2.32-2.16 (m, 1H), 2.16-2.08 (m, 1H), 1.62-1.48 (m, 6H). |
| | **B6** | or | |
| | **C3** | 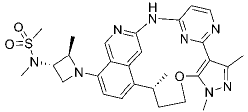 | |
| **Final Product 119** | | 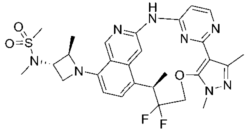 | Chiral separation: Column: CHIRALPAK IF, 2*25 cm, 5 µm; Mobile phase A: n-Hexane (0.5% 2 M NH₃-MeOH); Mobile phase B: Ethanol/DCM (1/1); Flow rate: 20 mL/min; Wavelength: 220/254 nm. |
| | | or | Off-white solid. MS (ESI⁺) m/z = 599.2 [M+H]⁺. ¹H NMR (300 MHz, DMSO-*d*₆) *δ* 10.16 (s, 1H), 9.14 (s, 1H), 8.78 (s, 1H), 8.36 (d, *J =* 5.7 Hz, 1H), 7.68 (d, *J* = 8.1 Hz, 1H), 6.84 (d, *J =* 5.8 Hz, 1H), 6.76 (d, *J =* 8.2 Hz, 1H), 4.72 (t, *J =* 7.5 Hz, 1H), 4.65-4.31 (m, 4H), 4.28-4.19 (m, 1H), 3.92 (t, *J* = 7.2 Hz, 1H), 3.77 (s, 3H), 2.97 (s, 3H), 2.84 (s, 3H), 2.45 (s, 3H), 1.65-1.51 (m, 3H), 1.43 (d, *J =* 6.0 Hz, 3H). |
| | **A7** | 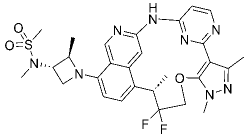 The deprotection reaction conditions for the third step were: TBAF, TMEDA, THF, 0 °C. | |
| | **B6** | | |
| | **C3** | | |
| **Final Product 124** | **A4** | 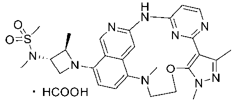 | Yellow solid. MS (ESI⁺) m/z = 564.2 [M+H]⁺. ¹H NMR (400 MHz, DMSO-*d*₆) *δ* 10.10 (s, 1H), 9.28 (s, 1H), 9.05 (s, 1H), 8.46 (s, 1H), 8.33 (d, *J =* 6.0 Hz, 1H), 7.21 (d, *J =* 8.0 Hz, 1H), 6.85 (d, *J =* 5.6 Hz, 1H), 6.66 (d, *J =* 8.0 Hz, 1H), 4.68-4.47 (m, 3H), 4.42-4.31 (m, 1H), 4.21-4.13 (m, 1H), 3.78 (s, 3H), 3.78-3.71 (m, 1H), 2.95-2.88 (m, 6H), 2.83 (s, 3H), 2.45 (s, 3H), 2.02-1.92 (m, 2H), 1.39 (d, *J =* 6.0 Hz, 3H). |
| | **B6** | | |
| | **C3** | | |
| **Final Product 139** | **A8** | 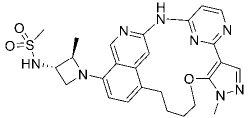 | Yellow solid. MS (ESI⁺) m/z = 535.1 [M+H]⁺. ¹H NMR (400 MHz, DMSO-*d*₆) *δ* 10.20 (s, 1H), 9.07 (s, 1H), 8.99 (s, 1H), 8.32 (d, *J =* 5.8 Hz, 1H), 7.90 (s, 1H), 7.75 (d, *J* = 8.4 Hz, 1H), 7.38 (d, *J =* 7.7 Hz, 1H), 6.91 (d, *J =* 5.8 Hz, 1H), 6.57 (d, *J* = 7.7 Hz, 1H), 4.79 (t, *J =* 7.2 Hz, 1H), 4.36-4.28 (m, 1H), 4.27-4.19 (m, 1H), 4.19-4.10 (m, 1H), 3.97-3.90 (m, 1H), 3.75 (s, 3H), 3.57 (t, *J =* 6.9 Hz, 1H), 3.32-3.22 (m, 1H), 3.21-3.10 (m, 1H), 2.97 (s, 3H), 1.95-1.75 (m, 4H), 1.43 (d, *J =* 6.1 Hz, 3H). |
| | **B1** | | |
| | **C2** | | |
| **Final Product 145** | **A1** | 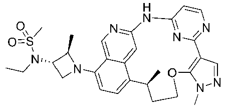 | Off-white solid. MS (ESI⁺) m/z = 563.4 [M+H]⁺. ¹H NMR (300 MHz, Chloroform-d) *δ* 9.28 (s, 1H), 9.10 (s, 1H), 8.37 (d, *J =* 5.9 Hz, 2H), 8.32-8.17 (m, 1H), 7.56 (d, *J =* 8.0 Hz, 1H), 6.69 (d, *J =* 8.0 Hz, 1H), 6.56 (d, *J* = 6.0 Hz, 1H), 4.66 (t, *J =* 7.2 Hz, 1H), 4.55-4.28 (m, 4H), 4.10-3.98 (m, 1H), 3.93-3.82 (m, 4H), 3.49-3.30 (m, 2H), 2.92 (s, 3H), 2.37-2.26 (m, 2H), 1.61-1.49 (m, 6H), 1.30 (*t, J =* 7.2 Hz, 3H). |
| | **B1** | or | |
| | **C8** | 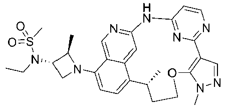 | |
| **Final Product 148** | | 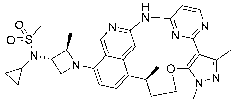 | Chiral separation: Column: CHIRALPAK IA-3, 4.6*50 mm, 3 µm; Mobile phase A: n-Hexane (0.1% diethylamine); Mobile phase B: Ethanol/DCM (1/1); Flow rate: 1 mL/min; Wavelength: 254 nm. |
| | **A1** | | Off-white solid. MS (ESI⁺) m/z = 589.2 [M+H]⁺. ¹H NMR (400 MHz, Acetonitrile-*d₃*) *δ* 9.14-8.97 (m, 2H), 8.22 (d, *J* = 5.8 Hz, 1H), 8.03 (s, 1H), 7.51-7.38 (m, 1H), 6.68-6.56 (m, 1H), 6.53 (d, *J* = 5.8 Hz, 1H), 5.94-5.78 (m, 1H), 5.24-5.15 (m, 1H), 5.14-5.07 (m, 1H), 4.54 (q, *J =* 8.4 Hz, 1H), 4.43-4.08 (m, 4H), 3.90-3.86 (m, 2H), 3.86-3.78 (m, 1H), 3.75 (t, *J =* 6.8 Hz, 1H), 2.81 (s, 3H), 2.38 (s, 3H), 1.91-1.73 (m, 2H), 1.45-1.24 (m, 7H), 1.26-1.03 (m, 2H). |
| | **B6** | or | |
| | **C7** | 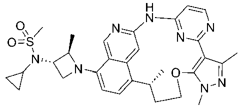 | |
| **Final Product 154** | **A1** | 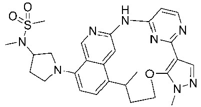 The deprotection reaction conditions for the third step were: TBAF, TMEDA, THF, 0 °C-60 °C. | Yellow solid. MS (ESI⁺) m/z = 549.4 [M+H]⁺. ¹H NMR (300 MHz, DMSO-*d*₆) *δ* 10.15 (s, 1H), 9.30 (d, *J =* 4.7 Hz, 1H), 9.14 (d, *J =* 4.1 Hz, 1H), 8.30 (d, *J =* 5.8 Hz, 1H), 7.95 (s, 1H), 7.61-7.50 (m, 1H), 6.94 (t, *J =* 8.0 Hz, 1H), 6.86 (dd, *J = 5.7,* 1.4 Hz, 1H), 4.65-4.28 (m, 3H), 3.94-3.73 (m, 4H), 3.58-3.19 (m, 3H), 2.96 (s, 3H), 2.91 (s, 3H), 2.33-2.21 (m, 1H), 2.20-2.14 (m, 2H), 2.02-1.88 (m, 2H), 1.51 (d, *J =* 6.8 Hz, 3H). |
| | **B1** | | |
| | **C6** | | |
| **Final Product 155** | **A3** | 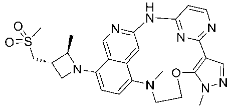 The coupling conditions for the first step were: Pd₂(dba)₃, BINAP, tBuONa, toluene, 90 °C. | Off-white solid. MS (ESI⁺) m/z = 535.2 [M+H]⁺. ¹H NMR (300 MHz, Methanol-*d₄*) *δ* 9.29 (s, 1H), 9.10 (s, 1H), 8.23 (d, *J =* 6.0 Hz, 1H), 8.08 (s, 1H), 7.32-7.21 (m, 1H), 6.80-6.71 (m, 1H), 6.71-6.58 (m, 1H), 4.72-4.56, (m, 3H), 4.31-4.16 (m, 1H), 3.89 (s, 3H), 3.64-3.37 (m, 5H), 3.09-2.92 (m, 7H), 1.48 (d, *J =* 6.1 Hz, 3H). |
| | **B1** | | |
| | **C1** | | |
| Final **Product 166** | **A1** | 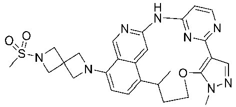 | Off-white solid. MS (ESI⁺) m/z = 547.3 [M+H]⁺. ¹H NMR (400 MHz, DMSO-*d*₆) *δ* 9.86 (s, 1H), 9.11-9.02 (m, 2H), 8.27 (d, *J =* 5.6 Hz, 1H), 7.93 (s, 1H), 7.48 (d, *J =* 8.0 Hz, 1H), 6.84 (d, *J =* 5.6 Hz, 1H), 6.44 (d, *J =* 8.0 Hz, 1H), 4.49-4.36 (m, 2H), 4.36-4.23 (m, 4H), 4.12 (s, 4H), 3.86-3.76 (m, 4H), 2.97 (s, 3H), 2.27-1.85 (m, 2H), 1.49 (d, *J* = 7.6 Hz, 3H). |
| | **B1** | | |
| | **C4** | | |
| **Final Product 167** | **A1** | 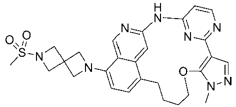 The coupling conditions for the first step were: Pd₂(dba)₃, Xantphos, Cs₂CO₃, 1,4-dioxane, 100 °C. | Off-white solid. MS (ESI⁺) m/z = 547.3 [M+H]⁺. ¹H NMR (400 MHz, DMSO-*d*₆) *δ* 9.06 (s, 1H), 8.94 (s, 1H), 8.28 (d, *J =* 5.8 Hz, 1H), 7.89 (s, 1H), 7.34 (d, *J* = 6.8 Hz, 1H), 7.23-7.18 (m, 1H), 6.89 (d, *J =* 5.8 Hz, 1H), 6.36 (d, *J =* 7.7 Hz, 1H), 4.37-4.26 (m, 6H), 4.12 (s, 4H), 3.73 (s, 3H), 2.97 (s, 3H), 2.01 (s, 2H), 1.92-1.71 (m, 4H). |
| | **B1** | | |
| | **C4** | | |
| **Final Product 176** | | 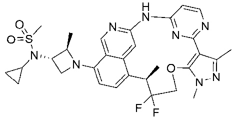 | Chiral separation: Column: CHIRALPAK ID, 2*25 cm, 5 µm; Mobile phase A: n-Hexane (0.5% 2 M NH₃-MeOH), Mobile phase B: Ethanol/DCM (1/1); Flow rate: 20 mL/min; Wavelength: 220/254 nm. |
| | **A7** | | White solid. MS (ESI⁺) m/z = 625.2 [M+H]⁺. ¹H NMR (300 MHz, DMSO-*d*₆) *δ* 10.17 (s, 1H), 9.15 (s, 1H), 8.79 (s, 1H), 8.36 (d, *J = 5.7* Hz, 1H), 7.67 (d, *J* = 8.1 Hz, 1H), 6.88-6.76 (m, 2H), 4.76-4.21 (m, 6H), 3.97 (t, *J =* 7.5 Hz, 1H), 3.77 (s, 3H), 3.06 (s, 3H), 2.45 (s, 3H), 2.43-2.34 (m, 1H), 1.56 (brs, 3H), 1.48 (d, *J* = 5.9 Hz, 3H), 0.94 (brs, 2H), 0.79 (d, *J =* 7.1 Hz, 2H). |
| | **B6** | or | |
| | **C7** | 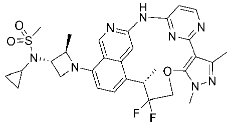 | |
| **Final Product 187** | | 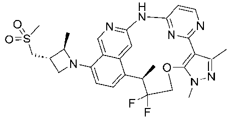 | Chiral separation: Column: CHIRALPAK IF, 2 *25 cm, 5 µm; Mobile phase A: n-Hexane (0.5% 2 M NH₃-MeOH); Mobile phase B: Ethanol/DCM (1/1); Flow rate: 20 mL/min; Wavelength: 220/254 nm. |
| | | or | Off-white solid. MS (ESI⁺) m/z = 584.1 [M+H]⁺. ¹H NMR (300 MHz, Acetonitrile-*d₃*) *δ* 9.14 (s, 1H), 8.81 (s, 1H), 8.34 (d, *J =* 5.8 Hz, 1H), 8.28 (s, 1H), 7.67 (d, *J =* 8.1 Hz, 1H), 6.79-6.61 (m, 2H), 4.75 (t, *J* = 7.7 Hz, 1H), 4.71-4.39 (m, 2H), 4.38-4.27 (m, 1H), 4.27-4.00 (m, 1H), 3.79 (s, 3H), 3.78-3.69 (m, 1H), 3.54-3.33 (m, 2H), 3.08-2.95 (m, 1H), 2.93 (s, 3H), 2.50 (s, 3H), 1.67-1.56 (m, 3H), 1.49 (d, *J =* 6.1 Hz, 3H). |
| | **A7** | 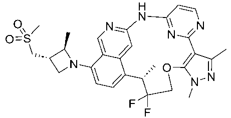 The deprotection reaction conditions for the third step were: TBAF, TMEDA, THF, 0 °C. | |
| | **B6** | | |
| | **C1** | | |
| Final Product 190 | | 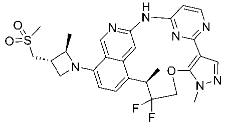 | Chiral separation: Column: CHIRALPAK IF, 2 *25 cm, 5 µm; Mobile phase A: n-Hexane (0.5% 2 M NH₃-MeOH); Mobile phase B: Ethanol/DCM (1/1); Flow rate: 20 mL/min; Wavelength: 220/254 nm. |
| | | or | Off-white solid. MS (ESI⁺) m/z = 570.3 [M+H]⁺. ¹H NMR (300 MHz, Acetonitrile-*d₃*) *δ* 9.02 (s, 1H), 8.78 (s, 1H), 8.26 (s, 1H), 8.17 (d, *J =* 5.8 Hz, 1H), 7.91 (s, 1H), 7.55 *(d, J=* 8.1 Hz, 1H), 6.60-6.54 (m, 2H), 4.75-4.61 (m, 2H), 4.53-4.39 (m, 1H), 4.26-4.16 (m, 1H), 4.13-4.00 (m, 1H), 3.73 (s, 3H), 3.69-3.61 (m, 1H), 3.41-3.24 (m, 2H), 2.96-2.86 (m, 1H), 2.83 (s, 3H), 1.53-1.45 (m, 3H), 1.36 (d, *J =* 6.1 Hz, 3H). |
| | **A7** | 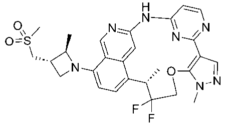 The deprotection reaction conditions for the third step were: TBAF, TMEDA, THF, 0 °C. | |
| | **B1** | | |
| | **C1** | | |

### Example 22 Preparation of Final Product 23 and Final Product 42

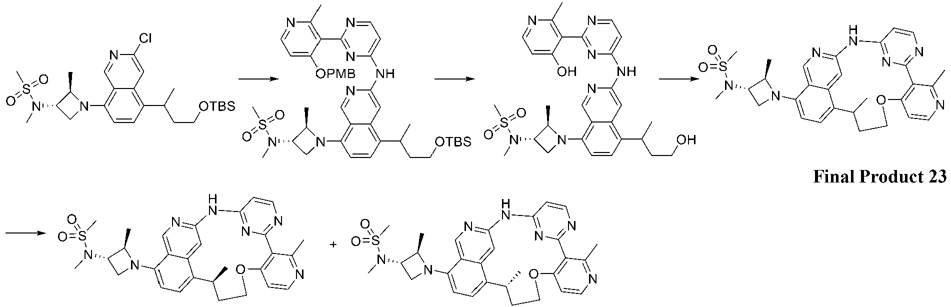

**Step 1:** Using N-((2R,3S)-1-(5-(4-((tert-butyldimethylsilyl)oxy)butan-2-yl)-3-chloroisoquinolin-8-yl)-2-methylazetidin-3-yl)-N-methylmethanesulfonamide and Intermediate B2 as starting materials, N-((2R,3S)-1-(5-(4-((tert-butyldimethylsilyl)oxy)butan-2-yl)-3-((2-(4-((4-methoxybenzyl)oxy)-2-methylpyridin-3-yl)pyrimidin-4-yl)amino)isoquinolin-8-yl)-2-methylazetidin-3-yl)-N-methylmethanesulfonamide was prepared according to the method of Step 2 in Example 1. MS (ESI⁺) m/z = 812.4 [M+H]⁺.

**Step 2:** N-((2R,3S)-1-(5-(4-((tert-butyldimethylsilyl)oxy)butan-2-yl)-3-((2-(4-((4-methoxybenzyl)oxy)-2-methylpyridin-3-yl)pyrimidin-4-yl)amino)isoquinolin-8-yl)-2-methylazetidin-3-yl)-N-methylmethanesulfonamide (550.0 mg, 0.68 mmol), dichloromethane (9 mL), and trifluoroacetic acid (3 mL) were added sequentially to a 25 mL single-necked flask at r.t. The mixture was stirred at r.t. for 1 h. After completion of the reaction, the mixture was concentrated. To the residue were successively added ethanol (4 mL), tetrahydrofuran (12 mL), water (4 mL), and lithium hydroxide (357.1 mg, 14.91 mmol) at r.t., and the mixture was stirred at r.t. for 2 h. After completion of the reaction, the mixture was diluted with water (10 mL) and extracted with ethyl acetate (150 mL × 3) and dichloromethane (150 mL × 4). The combined organic layers were dried over anhydrous sodium sulfate, filtered, and the filtrate was concentrated to afford N-((2R,3S)-1-(3-((2-(4-hydroxy-2-methylpyridin-3-yl)pyrimidin-4-yl)amino)-5-(4-hydroxybutan-2-yl)isoquinolin-8-yl)-2-methylazetidin-3-yl)-N-methylmethanesulfonamide (yellow solid, 450.0 mg, crude). MS (ESI⁺) m/z = 578.3 [M+H]⁺.

**Step 3:** Using N-((2R,3S)-1-(3-((2-(4-hydroxy-2-methylpyridin-3-yl)pyrimidin-4-yl)amino)-5-(4-hydroxybutan-2-yl)isoquinolin-8-yl)-2-methylazetidin-3-yl)-N-methylmethanesulfonamide as the starting material, N-((2R,3S)-1-(1²,5-dimethyl-8-oxa-3-aza-4(3,5)-isoquinolin-2(2,4)-pyrimidin-1(3,4)-pyridinacyclooctan-4⁸-yl)-2-methylazetidin-3-yl)-N-methylmethanesulfonamide (Final Product 23, crude) was prepared according to the method of Step 4 in Example 1. The crude Final Product 23 was purified by chiral preparative column separation (Column: CHIRALPAK IG 2*25 cm, 5 µm; Mobile phase: Cyclohexane (0.5% 2 M NH₃-MeOH) and EtOH: DCM = 1: 1; Flow rate: 20 mL/min; Wavelength: 220/254 nm) to afford **Final Product 42**. The specific configuration of **Final Product 42** is uncertain.

### Final Product 42:

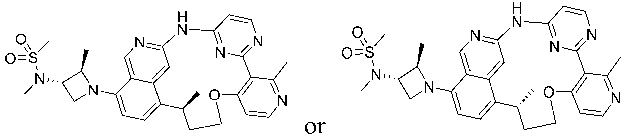

MS(ESI⁺)m/z = 560.2 [M+H]⁺. ¹H NMR (400 MHz, DMSO-*d*₆) *δ* 10.38 (s, 1H), 9.48 (s, 1H), 9.08 (s, 1H), 8.53 (d, *J =* 5.8 Hz, 1H), 8.38 (d, *J =* 5.9 Hz, 1H), 7.55-7.28 (m, 2H), 7.06 (d, *J =* 5.9 Hz, 1H), 6.61 (d, *J =* 8.0 Hz, 1H), 4.87 (t, *J =* 11.2 Hz, 1H), 4.67 (t, *J =* 7.4 Hz, 1H), 4.49-4.39 (m, 1H), 4.29-4.16 (m, 1H), 4.10-3.92 (m, 1H), 3.83 (t, *J =* 7.2 Hz, 1H), 3.30-3.20 (m, 1H), 2.95 (s, 3H), 2.82 (s, 3H), 2.50-2.48 (m, 1H), 2.38 (s, 3H), 2.14-2.00 (m, 1H), 1.48 (d, *J* = 6.6 Hz, 3H), 1.40 (d, *J =* 6.0 Hz, 3H).

### Example 23 Preparation of 1²,2⁶-dimethyl-4⁸-((2R,3S)-2-methyl-3-((methylsulfonyl)methyl)azetidin-1-yl)-8-oxa-3-aza-4(3,5)-isoquinolin-2(2,4)-pyrimidin-1(3,4)-pyridinacyclooctane (Final Product 91)

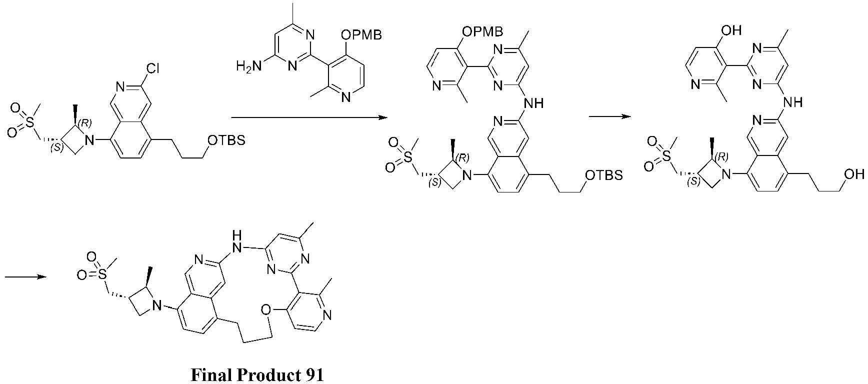

Using 5-(3-((tert-butyldimethylsilyl)oxy)propyl)-3-chloro-8-((2R,3S)-2-methyl-3-((methylsulfonyl)methyl)azetidin-1-yl)isoquinoline (prepared from Intermediate A9 and Intermediate C1 according to the method of the first step for the synthesis of final product 36) and Intermediate B11 as starting materials, Final Product 91 was prepared according to the synthesis method for Final Product 23 described above.

MS(ESI⁺)m/z = 545.2 [M+H]⁺. ¹H NMR (300 MHz, Chloroform-d) *δ* 9.36 (s, 1H), 9.06 (s, 1H), 8.50 (d, *J =* 6.3 Hz, 1H), 7.59 (s, 1H), 7.31 (d, *J* = 7.7 Hz, 1H), 7.09 (d, *J =* 6.5 Hz, 1H), 6.60 (s, 1H), 6.41 (d, *J =* 7.7 Hz, 1H), 4.71 (t, *J =* 7.5 Hz, 1H), 4.38-4.32 (m, 2H), 4.31-4.21 (m, 1H), 3.72 (t, *J =* 6.9 Hz, 1H), 3.46-3.26 (m, 2H), 3.14-2.99 (m, 3H), 2.97 (s, 3H), 2.72 (s, 3H), 2.53 (s, 3H), 2.19-2.04 (m, 2H), 1.52 (d, *J =* 6.0 Hz, 3H).

### Example 24 Preparation of 5-methyl-4⁸-((2R,3S)-2-methyl-3-((methylsulfonyl)methyl)azetidin-1-yl)-8-oxa-3-aza-4(3,5)-isoquinolin-2(2,4)-pyrimidin-1(3,4)-pyridinacyclooctane (Final Product 71)

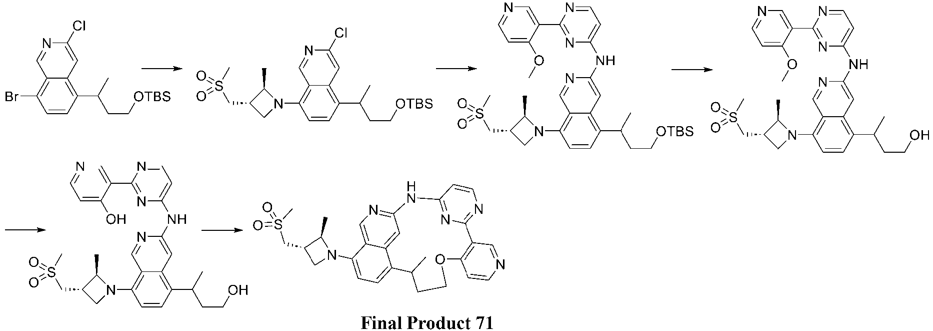

**Step 1 and Step 2:** 5-(4-((tert-butyldimethylsilyl)oxy)butan-2-yl)-N-(2-(4-methoxypyridin-3-yl)pyrimidin-4-yl)-8-((2R,3S)-2-methyl-3-((methylsulfonyl)methyl)azetidin-1-yl)isoquinolin-3-amine was prepared form intermediates A1, C1, and B3 according to the methods of Step 1 and Step 2 in Example 1 . MS (ESI⁺) m/z = 677.4 [M+H]⁺.

**Step 3:** Under a nitrogen atmosphere, 5-(4-((tert-butyldimethylsilyl)oxy)butan-2-yl)-N-(2-(4-methoxypyridin-3-yl)pyrimidin-4-yl)-8-((2R,3S)-2-methyl-3-((methylsulfonyl)methyl)azetidin-1-yl)isoquinolin-3-amine (190 mg, 0.28 mmol) was dissolved in tetrahydrofuran (5 mL) in a 40 mL single-necked flask. TBAF (1.1 mL, 1.12 mmol) was added under stirring. The temperature was warmed to 70 °C and stirred for 1 h. After cooling to r.t., the mixture was concentrated to afford a crude product. The crude product was purified by silica gel column chromatography using methanol/dichloromethane (0-20%) as eluent to obtain 3-(3-((2-(4-methoxypyridin-3-yl)pyrimidin-4-yl)amino)-8-((2R,3S)-2-methyl-3-((methylsulfonyl)methyl)azetidin-1-yl)isoquinolin-5-yl)butan-1-ol (yellow solid, 150 mg, 85.5%). MS (ESI⁺) m/z = 563.4 [M+H]⁺.

**Step 4:** Under a nitrogen atmosphere, 3-(3-((2-(4-methoxypyridin-3-yl)pyrimidin-4-yl)amino)-8-((2R,3S)-2-methyl-3-((methylsulfonyl)methyl)azetidin-1-yl)isoquinolin-5-yl)butan-1-ol (145 mg, 0.26 mmol) and tetrahydrofuran solution of lithium tri-sec-butylborohydride (1 M, 5 mL) were added sequentially to a 40 mL single-necked flask. The mixture was stirred at r.t. for 20 min. Methanol (5 mL) was added to quench the reaction. The mixture was concentrated to afford a crude product. The crude product was purified by high-performance preparative liquid chromatography using acetonitrile/water (0.1% ammonia) as eluent to obtain 3-(4-((5-(4-hydroxybutan-2-yl)-8-((2R,3S)-2-methyl-3-((methylsulfonyl)methyl)azetidin-1-yl)isoquinolin-3-yl)amino)pyrimidin-2-yl)pyridin-4-ol (white solid, 50 mg, 31.8%). MS (ESI⁺) m/z = 549.3 [M+H]⁺. The reaction conditions for this step can also be: NaH (60%), ethanethiol, DMF, 120 °C.

**Step 5:** Using 3-(4-((5-(4-hydroxybutan-2-yl)-8-((2R,3S)-2-methyl-3-((methylsulfonyl)methyl)azetidin-1-yl)isoquinolin-3-yl)amino)pyrimidin-2-yl)pyridin-4-ol as starting material, 5-methyl-4⁸-((2R,3S)-2-methyl-3-((methylsulfonyl)methyl)azetidin-1-yl)-8-oxa-3-aza-4(3,5)-isoquinolin-2(2,4)-pyrimidin-1(3,4)-pyridinacyclooctane (Final Product 71) was prepared according to the method of Step 4 in Example 1.

MS(ESI⁺)m/z = 531.2 [M+H]⁺. ¹H NMR (300 MHz, DMSO-*d*₆) *δ* 10.35 (d, *J =* 3.8 Hz, 1H), 9.45 (d, *J =* 6.5 Hz, 1H), 9.24 (d, *J =* 11.4 Hz, 1H), 9.07 (s, 1H), 8.50 (t, *J =* 5.7 Hz, 2H), 7.49-7.38 (m, 2H), 7.06 (d, *J =* 5.8 Hz, 1H), 6.54 (t, *J =* 7.0 Hz, 1H), 4.77-4.65 (m, 2H), 4.24-4.13 (m, 1H), 4.10-4.01 (m, 1H), 3.70-3.59 (m, 1H), 3.58-3.50 (m, 3H), 2.99 (s, 3H), 2.93-2.84 (m, 1H), 2.28-2.13 (m, 1H), 1.66-1.51 (m, 1H), 1.50-1.37 (m, 6H).

### Examples 25-26 Preparation of Final Products 58, 60

Final Products 58 and 60 were prepared form intermediates A1 or A9, Intermediate B7, and Intermediate C3 according to the preparation method of the above Final Product 71. As shown in Table 2.

**Table 2 Characterization Data of Final Products 58, 60**

| **Final Product** | **Inter mediate** | **Structure of Final Product** | **NMR or MS** |
|---|---|---|---|
| **Final Product 58** | **A1** | 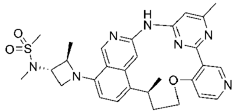 | Yellow solid. MS (ESI⁺) m/z = 560.1 [M+H]⁺. ¹H NMR (400 MHz, DMSO-*d*₆) *δ* 11.68 (s, 1H), 9.29-9.18 (m, 2H), 9.08 (s, 1H), 8.13 (dd, *J =* 7.6, 1.9 Hz, 1H), 7.69 (d, *J =* 8.1 Hz, 1H), 6.91 (s, 1H), 6.82 (t, *J* = 8.4 Hz, 2H), 4.73 (t, *J =* 7.6 Hz, 1H), 4.60-4.48 (m, 1H), 4.46-4.36 (m, 1H), 4.28-4.11 (m, 2H), 3.89 (t, *J* = 7.1 Hz, 1H), 3.72-3.67 (m, 1H), 2.97 (s, 3H), 2.84 (s, 3H), 2.63 (s, 3H), 2.42-2.30 (m, 1H), 2.02-1.91 (m, 1H), 1.43 (d, *J =* 6.0 Hz, 3H), 1.37 (d, *J* = 6.5 Hz, 3H). |
| | **B7** | or | |
| | **C3** | 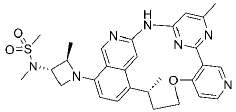 | |
| **Final Product 60** | **A9** | 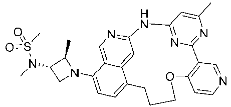 | White solid. MS (ESI⁺) m/z = 546.3 [M+H]⁺. ¹H NMR (400 MHz, DMSO-*d*₆) *δ* 10.50 (s, 1H), 9.46-9.37 (m, 2H), 9.10 (s, 1H), 8.84 (d, *J =* 6.6 Hz, 1H), 7.83 (d, *J* = 7.0 Hz, 1H), 7.44 (d, *J =* 7.7 Hz, 1H), 7.02 (s, 1H), 6.59 (d, *J =* 7.8 Hz, 1H), 4.67 (t, *J =* 7.5 Hz, 1H), 4.61-4.50 (m, 2H), 4.47-4.41 (m, 1H), 4.20 (q, *J* = 6.8 Hz, 1H), 3.84 (t, *J* = 7.2 Hz, 1H), 3.12-2.98 (m, 2H), 2.96 (s, 3H), 2.82 (s, 3H), 2.45 (s, 3H), 2.19-2.06 (m, 2H), 1.41 (d, *J =* 6.0 Hz, 3H). |
| | **B7** | | |
| | **C3** | | |

### Example 27 Preparation of N-methyl-N-((2R,3S)-2-methyl-1-(5-methyl-8-oxa-3-aza-4(3,5)-isoquinolin-2(2,4)-pyrimidin-1(3,4)-pyridinacyclooctan-4⁸-yl)azetidin-3-yl)methanesulfonamide (Final Product 20) and Final Product 22

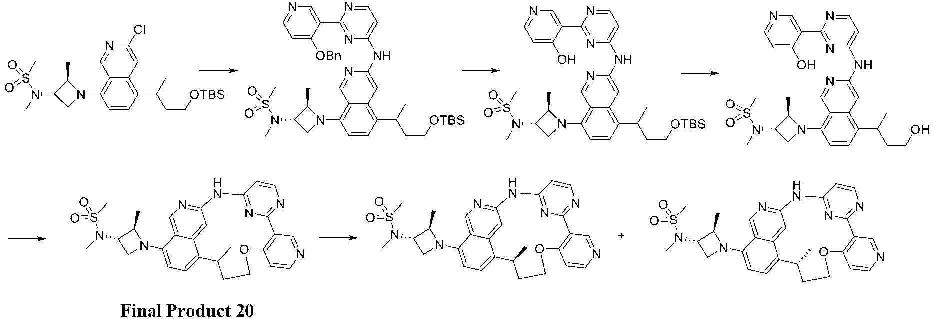

**Step 1:** Under a nitrogen atmosphere, N-((2R,3S)-1-(5-(4-((tertbutyldimethylsilyl)oxy)butan-2-yl)-3-chloroisoquinolin-8-yl)-2-methylazetidin-3-yl)-N-methylmethanesulfonamide (595 mg, 1.13 mmol) was dissolved in 1,4-dioxane (10 mL) in a 40 mL reaction flask. 2-(4-(Benzyloxy)pyridin-3-yl)pyrimidin-4-amine (Intermediate B4, 378 mg, 1.36 mmol), Josiphos (49 mg, 0.11 mmol), Josiphos Pd G₃ (105 mg, 0.11 mmol), and cesium carbonate (1.11 g, 3.4 mmol) were added sequentially. The mixture was heated to 100 °C and stirred for 1 h. After completion of the reaction, the mixture was cooled to r.t., and water (20 mL) was added to quench the reaction. The mixture was extracted with ethyl acetate (100 mL × 3). The combined organic layers were washed with brine (30 mL), dried over anhydrous sodium sulfate, filtered, and the filtrate was concentrated. The residue was purified by silica gel column chromatography eluting with a gradient of methanol/dichloromethane (0-50%) to afford N-((2R,3S)-1-(3-((2-(4-(benzyloxy)pyridin-3-yl)pyrimidin-4-yl)amino)-5-(4-((tert-butyldimethylsilyl)oxy)butan-2-yl)isoquinolin-8-yl)-2-methylazetidin-3-yl)-N-methylmethanesulfonamide (yellow solid, 560 mg, 64.4%). MS (ESI⁺) m/z = 768.4 [M+H]⁺.

**Step 2:** N-((2R,3S)-1-(3-((2-(4-(benzyloxy)pyridin-3-yl)pyrimidin-4-yl)amino)-5-(4-((tert-butyldimethylsilyl)oxy)butan-2-yl)isoquinolin-8-yl)-2-methylazetidin-3-yl)-N-methylmethanesulfonamide (595 mg, 0.73 mmol) was dissolved in ethanol (15 mL) in a 100 mL reaction flask. Ammonium formate (0.46 g, 73.01 mmol) and Pd/C (116 mg, 1.11 mmol) were added sequentially. The mixture was heated to 60 °C and stirred for 2 h. After completion of the reaction, the mixture was cooled to r.t., filtered through Celite, and the filtrate was dried over anhydrous sodium sulfate, filtered, and the filtrate was concentrated to afford N-((2R,3S)-1-(5-(4-((tert-butyldimethylsilyl)oxy)butan-2-yl)-3-((2-(4-hydroxypyridin-3-yl)pyrimidin-4-yl)amino)isoquinolin-8-yl)-2-methylazetidin-3-yl)-N-methylmethanesulfonamide (yellow oil, 320 mg, crude). MS (ESI⁺) m/z = 678.4 [M+H]⁺.

**Step 3**: Under a nitrogen atmosphere, N-((2R,3S)-1-(5-(4-((tertbutyldimethylsilyl)oxy)butan-2-yl)-3-((2-(4-hydroxypyridin-3-yl)pyrimidin-4-yl)amino)isoquinolin-8-yl)-2-methylazetidin-3-yl)-N-methylmethanesulfonamide (320 mg, 0.47 mmol) was dissolved in tetrahydrofuran (10 mL) in a 40 mL reaction flask. Triethylamine trihydrofluoride (0.11 g, 0.71 mmol) was added under an ice bath. The mixture was stirred at r.t. for 1 h. After completion of the reaction, the reaction mixture was cooled to 0 °C, and water (5 mL) was added to quench the reaction. The mixture was extracted with dichloromethane (30 mL × 3). The combined organic layers were dried over anhydrous sodium sulfate, filtered, and the filtrate was concentrated. The residue was purified by silica gel column chromatography eluting with ethyl acetate/petroleum ether (0-60%) to afford N-((2R,3S)-1-(5-(4-hydroxybutan-2-yl)-3-((2-(4-hydroxypyridin-3-yl)pyrimidin-4-yl)amino)isoquinolin-8-yl)-2-methylazetidin-3-yl)-N-methylmethanesulfonamide (yellow oil, 157 mg, 59.0%). MS (ESI⁺) m/z = 564.4 [M+H]⁺.

**Step 4:** Under a nitrogen atmosphere, N-((2R,3S)-1-(5-(4-hydroxybutan-2-yl)-3-((2-(4-hydroxypyridin-3-yl)pyrimidin-4-yl)amino)isoquinolin-8-yl)-2-methylazetidin-3-yl)-N-methylmethanesulfonamide (150 mg, 0.28 mmol) was dissolved in toluene (5 mL) in a 40 mL reaction flask. Cyanomethylenetributylphosphorane (0.67 g, 2.79 mmol) was then added. The mixture was heated to 100 °C and stirred for 1 h. After completion of the reaction, the mixture was cooled to r.t., and water (5 mL) was added to quench the reaction. The mixture was extracted with dichloromethane (10 mL × 3). The combined organic layers were dried over anhydrous sodium sulfate, filtered, and the filtrate was concentrated. The residue was purified by silica gel column chromatography eluting with methanol/dichloromethane (0-10%) to afford N-methyl-N-((2R,3S)-2-methyl-1-(5-methyl-8-oxa-3-aza-4(3,5)-isoquinolin-2(2,4)-pyrimidin-1(3,4)-pyridinacyclooctan-4⁸-yl)azetidin-3-yl)methanesulfonamide (crude). The crude product was subjected to chiral separation(Column: CHIRALPAK IE, 2*25 cm, 5 µm; Mobile phase A: methyl tert-butyl ether (0.5% 2 M NH₃-MeOH); Mobile phase B: methanol/dichloromethane (1/1); Flow rate: 20 mL/min; Wavelength: 254 nm; RT1(min): 17.375; RT2(min): 20.88) to afford N-methyl-N-((2R,3S)-2-methyl-1-((S)-5-methyl-8-oxa-3-aza-4(3,5)-isoquinolin-2(2,4)-pyrimidin-1(3,4)-pyridinacyclooctan-4⁸-yl)azetidin-3-yl)methanesulfonamide and N-methyl-N-((2R,3S)-2-methyl-1-((R)-5-methyl-8-oxa-3-aza-4(3,5)-isoquinolin-2(2,4)-pyrimidin-1(3,4)-pyridinacyclooctan-4⁸-yl)azetidin-3-yl)methanesulfonamide (Final Product 22). The specific configuration (R or S) of **Final Product 22** is uncertain.

### Final Product 22:

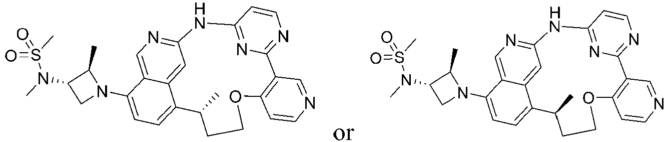

Off-white solid. MS (ESI⁺) m/z = 546.4 [M+H]⁺. ¹H NMR (300 MHz, Methanol-*d₄*) *δ* 9.48 (s, 1H), 9.09 (s, 1H), 9.03 (s, 1H), 8.40 (d, *J =* 6.0 Hz, 1H), 8.34 (d, *J =* 5.9 Hz, 1H), 7.42 (*d, J* = 7.9 Hz, 1H), 7.31 (d, *J =* 6.1 Hz, 1H), 6.84 (d, *J =* 5.9 Hz, 1H), 6.62 (d, *J =* 8.0 Hz, 1H), 4.70-4.64 (m, 1H), 4.52 (t, *J =* 7.4 Hz, 1H), 4.42-4.31 (m, 1H), 4.16 (q, *J =* 6.7 Hz, 1H), 4.03 (d, *J* = 9.3 Hz, 1H), 3.73 (t, *J =* 7.1 Hz, 1H), 3.57-3.42 (m, 1H), 2.87-2.79 (m, 6H), 2.31 (t, *J* = 13.4 Hz, 1H), 1.66-1.51 (m, 1H), 1.48-1.36 (m, 6H).

### Example 28 Preparation of Final Product 38

Final Product 38 was prepared from Intermediate A2, Intermediate B4, and Intermediate C3 according to the synthetic method for the synthesis of Final Product 22. The specific configuration of **Final Product 38** is uncertain.

### Final Product 38:

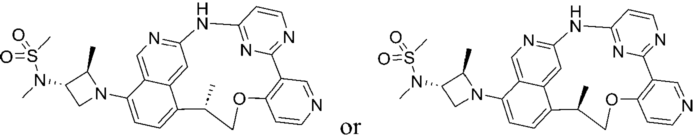

White solid. MS (ESI⁺) m/z = 532.1 [M+H]⁺. ¹H NMR (300 MHz, Chloroform-d) *δ* 9.46 (s, 1H), 9.19 (s, 1H), 9.07 (s, 1H), 8.65-8.54 (m, 2H), 8.31 (s, 1H), 7.55 (d, *J* = 8.0 Hz, 1H), 7.18 (d, *J =* 6.6 Hz, 1H), 6.77 (d, *J =* 5.8 Hz, 1H), 6.63 (d, *J* = 8.1 Hz, 1H), 4.65 (t, *J =* 7.6 Hz, 1H), 4.52-4.33 (m, 3H), 4.33-4.22 (m, 1H), 4.05-3.87 (m, 2H), 2.97-2.83 (m, 6H), 1.62 (d, *J= 7.4* Hz, 3H), 1.51 (d, *J =* 6.1 Hz, 3H).

### Example 29 Preparation of Final Product 73

Final Product 71 was prepared from Intermediate A1, Intermediate B4, and Intermediate C1 according to the synthetic method of above Final Product 22. Final Product 71 was purified by high-performance preparative liquid chromatography (Column: XBridge Prep Shield RP18 OBDColumn, 30*150 mm, 5 µm; Mobile phase: acetonitrile/water (0.1% formic acid); Flow rate: 60 mL/min; Gradient: 5%-35% -7 min; Wavelength: 254 nm) to afford Final Product 73. The specific configuration of **Final Product 73** is uncertain.

### Final Product 73:

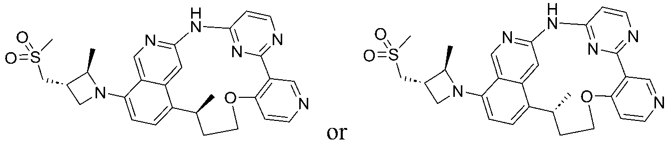

Yellow solid. MS (ESI⁺) m/z = 531.4 [M+H]⁺. ¹H NMR (300 MHz, Methanol-*d₄*) *δ* 9.40 (s, 1H), 9.06 (s, 1H), 8.98 (s, 1H), 8.38 (d, *J* = 6.0 Hz, 1H), 8.32 (d, *J* = 6.0 Hz, 1H), 7.34 (d, *J* = 7.9 Hz, 1H), 7.28 (d, *J* = 6.2 Hz, 1H), 6.81 (d, *J* = 5.9 Hz, 1H), 6.51 *(d, J =* 7.9 Hz, 1H), 4.69-4.47 (m, 3H), 4.25-4.12 (m, 1H), 3.98 (d, *J =* 9.5 Hz, 1H), 3.57-3.34 (m, 3H), 2.96-2.84 (m, 4H), 2.25 (t, *J =* 13.2 Hz, 1H), 1.61-1.47 (m, 1H), 1.46-1.33 (m, 6H).

### Example 30 Preparation of N-((2R,3S)-1-(9-oxa-3-aza-4(3,5)-isoquinolin-2(2,4)-pyrimidin-1(3,4)-pyridinacyclononan-4⁸-yl)-2-methylazetidin-3-yl)-N-methylmethanesulfonamide (Final Product 129)

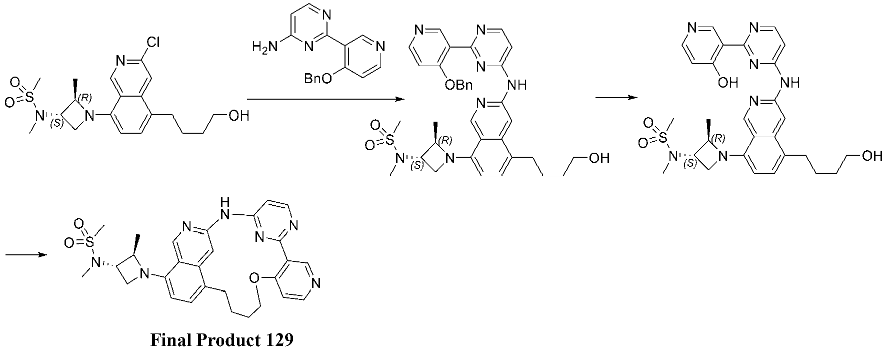

Final Product 129 was prepared from N-((2R,3S)-1-(3-chloro-5-(4-hydroxybutyl)isoquinolin-8-yl)-2-methylazetidin-3-yl)-N-methylmethanesulfonamide (prepared from Intermediate A8 and Intermediate C3 according to the method of Step 1 in Example 1) and Intermediate B4, sequentially following the methods of Step 1, Step 2, and Step 4 for the synthesis of Final Product 20.

MS(ESI⁺)m/z = 546.3 [M+H]⁺. ¹H NMR (400 MHz, DMSO-*d*₆) *δ* 10.26 (s, 1H), 9.08 (s, 1H), 8.95 (s, 1H), 8.67 (s, 1H), 8.42 (d, *J =* 5.6 Hz, 1H), 7.77-7.63 (m, 1H), 7.43 (d, *J =* 7.8 Hz, 1H), 6.92 (d, *J =* 5.6 Hz, 1H), 6.63 (d, *J =* 7.6 Hz, 1H), 6.26 (d, *J =* 7.6 Hz, 1H), 4.65 (t, *J* = 7.4 Hz, 1H), 4.48-4.41 (m, 1H), 4.25-4.17 (m, 3H), 3.83 (t, *J =* 7.2 Hz, 1H), 3.33-3.22 (m, 1H), 3.03-2.89 (m, 4H), 2.83 (s, 3H), 1.99-1.91 (m, 2H), 1.82-1.71 (m, 1H), 1.69-1.58 (m, 1H), 1.41 (d, *J* = 6.0 Hz, 3H).

### Example 31 Preparation of N-methyl-N-((2R,3S)-2-methyl-1-(5-methyl-8-oxa-3,5-diaza-4(3,5)-isoquinolin-2(2,4)-pyrimidin-1(3,4)-pyridinacyclooctan-4⁸-yl)azetidin-3-yl)methanesulfonamide (Final Product 25)

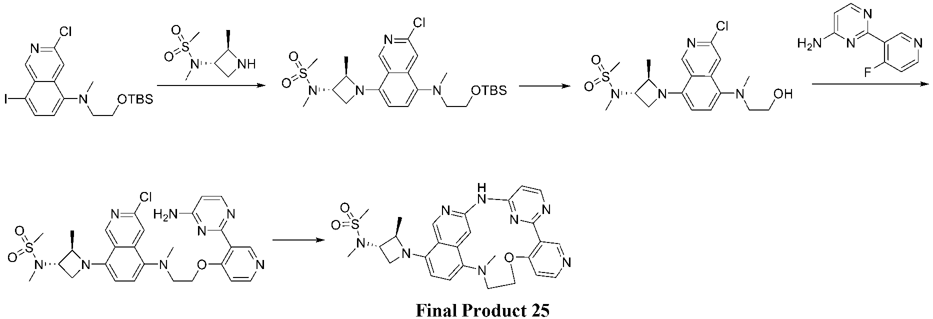

**Step 1:** Under a nitrogen atmosphere, N-(2-((tert-butyldimethylsilyl)oxy)ethyl)-3-chloro-8-iodo-N-methylisoquinolin-5-amine (Intermediate A3, 700 mg, 1.47 mmol) was dissolved in toluene (7 mL) in a 40 mL reaction flask. N-methyl-N-((2R,3S)-2-methylazetidin-3-yl)methanesulfonamide (Intermediate C3, 393 mg, 2.21 mmol), Pd₂(dba)₃ (134.6 mg, 0.15 mmol), BINAP (91.5 mg, 0.15 mmol), and sodium tert-butoxide (424.0 mg, 4.41 mmol) were added sequentially. The mixture was heated to 90 °C and stirred for 1 h. After completion of the reaction, the mixture was cooled to r.t., and brine (30 mL) was added. The mixture was extracted with ethyl acetate (30 mL × 3). The combined organic layers were washed with brine (30 mL), dried over anhydrous sodium sulfate, filtered, and the filtrate was concentrated. The residue was purified by silica gel column chromatography using ethyl acetate/petroleum ether (5-20%) as eluent to afford N-((2R,3S)-1-(5-((2-((tertbutyldimethylsilyl)oxy)ethyl)(methyl)amino)-3-chloroisoquinolin-8-yl)-2-methylazetidin-3-yl)-N-methylmethanesulfonamide (yellow oil, 500 mg, 64.7%). MS (ESI⁺) m/z = 527.3 [M+H]⁺.

**Step 2:** Under a nitrogen atmosphere, N-((2R,3S)-1-(5-((2-((tertbutyldimethylsilyl)oxy)ethyl)(methyl)amino)-3-chloroisoquinolin-8-yl)-2-methylazetidin-3-yl)-N-methylmethanesulfonamide (500 mg, 0.95 mmol) was dissolved in tetrahydrofuran (20 mL) in a 250 mL reaction flask. TBAF (2.35 mL, 1 M THF solution, 1.423 mmol) was added dropwise with stirring. The mixture was heated to 60 °C and stirred for 1 h. After completion of the reaction, the mixture was cooled to r.t., and water (50 mL) was added to quench the reaction. The mixture was extracted with dichloromethane (50 mL × 3). The combined organic layers were dried over anhydrous sodium sulfate, filtered, and the filtrate was concentrated. The residue was purified by silica gel column chromatography using ethyl acetate/petroleum ether (0-60%) as eluent to afford N-((2R,3S)-1-(3-chloro-5-((2-hydroxyethyl)(methyl)amino)isoquinolin-8-yl)-2-methylazetidin-3-yl)-N-methylmethanesulfonamide (yellow solid, 300 mg, 76.6%).

**Step 3:** Under a nitrogen atmosphere, 2-(4-fluoropyridin-3-yl)pyrimidin-4-amine (270 mg, 1.420 mmol) was dissolved in 1,4-dioxane (10 mL) in a 100 mL reaction flask. N-((2R,3S)-1-(3-chloro-5-((2-hydroxyethyl)(methyl)amino)isoquinolin-8-yl)-2-methylazetidin-3-yl)-N-methylmethanesulfonamide (290 mg, 0.70 mmol) and potassium hydroxide (239 mg, 4.3 mmol) were added sequentially. The mixture was heated to 100 °C and stirred for 4 h. After completion of the reaction, the mixture was cooled to r.t., and water (20 mL) was added to quench the reaction. The mixture was extracted with ethyl acetate (100 mL × 3). The combined organic layers were washed with brine (20 mL), dried over anhydrous sodium sulfate, filtered, and the filtrate was concentrated. The residue was purified by silica gel column chromatographyusing methanol/dichloromethane (0-15%) as eluent to afford N-((2R,3S)-1-(5-((2-((3-(4-aminopyrimidin-2-yl)pyridin-4-yl)oxy)ethyl)(methyl)amino)-3-chloroisoquinolin-8-yl)-2-methylazetidin-3-yl)-N-methylmethanesulfonamide (yellow solid, 130 mg, 31%). MS (ESI⁺) m/z = 583.3 [M+H]⁺.

**Step 4:** Under a nitrogen atmosphere, N-((2R,3S)-1-(5-((2-((3-(4-aminopyrimidin-2-yl)pyridin-4-yl)oxy)ethyl)(methyl)amino)-3-chloroisoquinolin-8-yl)-2-methylazetidin-3-yl)-N-methylmethanesulfonamide (110 mg, 0.19 mmol) was dissolved in 1,4-dioxane (5 mL) in a 40 mL reaction flask. Ephos (10 mg, 0.019 mmol), Ephos Pd G₄ (35 mg, 0.038 mmol), and cesium carbonate (184 mg, 0.57 mmol) were added sequentially. The mixture was heated to 100 °C and stirred for 1 h. After completion of the reaction, the mixture was cooled to r.t., and water (5 mL) was added to quench the reaction. The mixture was extracted with ethyl acetate (10 mL × 3). The combined organic layers were dried over anhydrous sodium sulfate, filtered, and the filtrate was concentrated. The residue was purified by silica gel column chromatography using methanol/dichloromethane (0-10%) as eluent to afford a crude product. The crude product was purified by high-performance preparative liquid chromatography (Column: XBridge Prep C18 OBD Column, 19*250 mm, 5 µm; Mobile phase: acetonitrile/water (0.1% ammonium bicarbonate); Flow rate: 25 mL/min; Gradient: 28%-50% -10 min; Wavelength: 254 nm, RT1(min): 8.99) to afford N-methyl-N-((2R,3S)-2-methyl-1-(5-methyl-8-oxa-3,5-diaza-4(3,5)-isoquinolin-2(2,4)-pyrimidin-1(3,4)-pyridinacyclooctan-4⁸-yl)azetidin-3-yl)methanesulfonamide (10.2 mg, 9.8%).

Yellow solid, MS (ESI⁺) m/z = 547.2 [M+H]⁺. ¹H NMR (300 MHz, Methanol-*d₄*) *δ* 9.74 (s, 1H), 9.28 (s, 1H), 9.12 (s, 1H), 8.51 (d, *J =* 6.0 Hz, 1H), 8.45 (d, *J* = 5.9 Hz, 1H), 7.45 (d, *J =* 6.1 Hz, 1H), 7.33 (d, *J* = 8.2 Hz, 1H), 6.94 (d, *J =* 5.9 Hz, 1H), 6.70 (d, *J =* 8.2 Hz, 1H), 4.86-4.83 (m, 3H), 4.66-4.52 (m, 2H), 4.50-4.38 (m, 1H), 4.25 (q, *J =* 6.8 Hz, 1H), 3.76 (t, *J* = 7.1 Hz, 1H), 3.00-2.78 (m, 9H), 1.48 (d, *J =* 6.1 Hz, 3H).

### Example 32 Preparation of N-methyl-N-((2R,3S)-2-methyl-1-(1¹-methyl-1¹H-8-oxa-3-aza-4(3,5)-isoquinolin-2(2,4)-pyrimidin-1(4,5)-pyrazolacyclooctan-4⁸-yl)azetidin-3-yl)methanesulfonamide (Final Product 34)

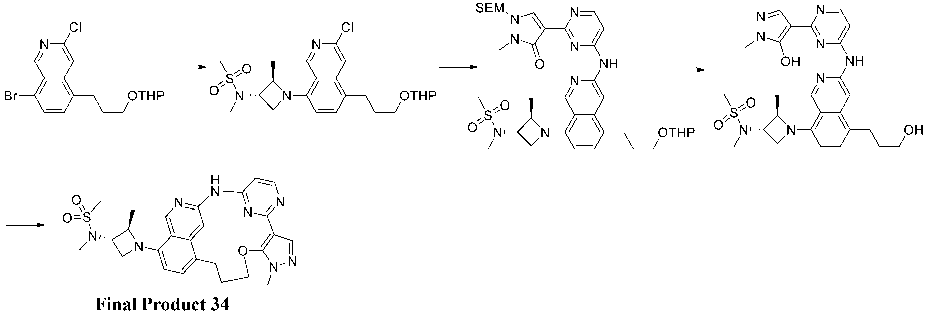

**Step 1 and Step 2:** N-methyl-N-((2R,3S)-2-methyl-1-(3-((2-(2-methyl-3-oxo-1-((2-(trimethylsilyl)ethoxy)methyl)-2,3-dihydro-1H-pyrazol-4-yl)pyrimidin-4-yl)amino)-5-(3-((tetrahydro-2H-pyran-2-yl)oxy)propyl)isoquinolin-8-yl)azetidin-3-yl)methanesulfonamide was prepared from Intermediate A5 and Intermediate C3, and Intermediate B1 according to the methods of Step 1 and Step 2 in Example 1. MS (ESI⁺) m/z = 767.4 [M+H]⁺.

**Step 3:** N-methyl-N-((2R,3S)-2-methyl-1-(3-((2-(2-methyl-3-oxo-1-((2-(trimethylsilyl)ethoxy)methyl)-2,3-dihydro-1H-pyrazol-4-yl)pyrimidin-4-yl)amino)-5-(3-((tetrahydro-2H-pyran-2-yl)oxy)propyl)isoquinolin-8-yl)azetidin-3-yl)methanesulfonamide (200 mg, 0.26 mmol) was dissolved in methanol (10 mL) in a 40 mL reaction flask. Then, p-toluenesulfonic acid (0.22 g, 1.30 mmol) was added. The mixture was heated to 60 °C and stirred for 16 h. After completion of the reaction, the mixture was cooled to r.t. The reaction mixture was purified by preparative liquid chromatography using acetonitrile/water (0-30%) as eluent to afford N-((2R,3S)-1-(3-((2-(5-hydroxy-1-methyl-1H-pyrazol-4-yl)pyrimidin-4-yl)amino)-5-(3-hydroxypropyl)isoquinolin-8-yl)-2-methylazetidin-3-yl)-N-methylmethanesulfonamide (yellow solid, 90 mg, 62.5%). MS (ESI⁺) m/z = 553.3 [M+H]⁺.

**Step 4:** Using N-((2R,3S)-1-(3-((2-(5-hydroxy-1-methyl-1H-pyrazol-4-yl)pyrimidin-4-yl)amino)-5-(3-hydroxypropyl)isoquinolin-8-yl)-2-methylazetidin-3-yl)-N-methylmethanesulfonamide as starting material, N-methyl-N-((2R,3S)-2-methyl-1-(1¹-methyl-1¹H-8-oxa-3-aza-4(3,5)-isoquinolin-2(2,4)-pyrimidin-1(4,5)-pyrazolacyclooctan-4⁸-yl)azetidin-3-yl)methanesulfonamide was prepared according to the method of Step 4 in Example 1.

Yellow solid. MS (ESI⁺) m/z= 535.4 [M+H]⁺. ¹H NMR (400 MHz, DMSO-*d*₆) *δ* 10.20 (s, 1H), 9.08 (s, 1H), 9.03 (s, 1H), 8.30 (d, *J =* 5.7 Hz, 1H), 7.95 (s, 1H), 7.46 (d, *J =* 7.8 Hz, 1H), 6.85 (d, *J =* 5.8 Hz, 1H), 6.65 (d, *J =* 7.9 Hz, 1H), 4.70-4.62 (m, 1H), 4.50-4.37 (m, 2H), 4.37-4.28 (m, 1H), 4.26-4.16 (m, 1H), 3.87-3.79 (m, 1H), 3.78 (s, 3H), 3.22-3.15 (m, 1H), 3.15-3.06 (m, 1H), 2.96 (s, 3H), 2.82 (s, 3H), 2.31-2.19 (m, 1H), 2.18-2.10 (m, 1H), 1.41 (d, *J* = 6.0 Hz, 3H).

### Example 33 Preparation of N-((2R,3S)-1-(8-oxa-3-aza-4(3,5)-isoquinolin-2(2,4)-pyrimidin-1(3,4)-pyridinacyclooctan-4⁸-yl)-2-methylazetidin-3-yl)-N-methylmethanesulfonamide (Final Product 39)

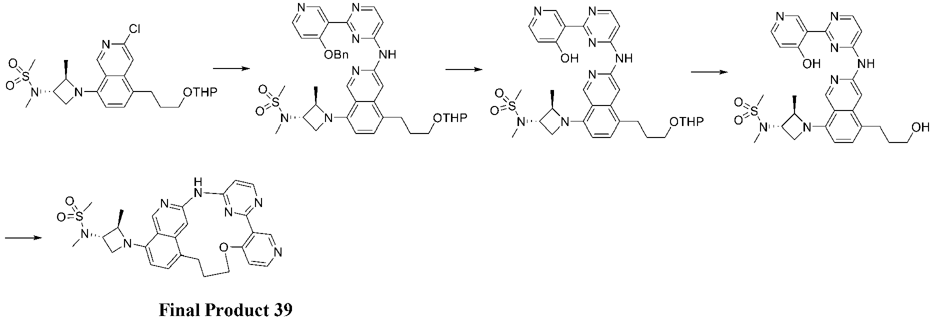

**Step 1:** Under a nitrogen atmosphere, N-((2R,3S)-1-(3-chloro-5-(3-((tetrahydro-2H-pyran-2-yl)oxy)propyl)isoquinolin-8-yl)-2-methylazetidin-3-yl)-N-methylmethanesulfonamide (300 mg, 0.62 mmol) was dissolved in 1,4-dioxane (8 mL) in a 40 mL reaction flask. Intermediate B4 (0.17 g, 0.62 mmol), Ephos Pd G₄ (0.06 g, 0.06 mmol), Ephos (0.03 g, 0.06 mmol), and cesium carbonate (0.61 g, 1.87 mmol) were added sequentially. The mixture was heated to 100 °C and stirred for 12 h. After completion of the reaction, the mixture was cooled to r.t., and water (30 mL) was added to quench the reaction. The mixture was extracted with dichloromethane (30 mL × 3). The combined organic layers were washed with brine (15 mL), dried over anhydrous sodium sulfate, filtered, and the filtrate was concentrated. The residue was purified by silica gel column chromatography using methanol/dichloromethane (0-15%) as eluent to afford N-((2R,3S)-1-(3-((2-(4-(benzyloxy)pyridin-3-yl)pyrimidin-4-yl)amino)-5-(3-((tetrahydro-2H-pyran-2-yl)oxy)propyl)isoquinolin-8-yl)-2-methylazetidin-3-yl)-N-methylmethanesulfonamide (white solid, 200 mg, 44.4%). MS (ESI⁺) m/z = 724.3 [M+H]⁺.

**Step 2:** Using N-((2R,3S)-1-(3-((2-(4-(benzyloxy)pyridin-3-yl)pyrimidin-4-yl)amino)-5-(3-((tetrahydro-2H-pyran-2-yl)oxy)propyl)isoquinolin-8-yl)-2-methylazetidin-3-yl)-N-methylmethanesulfonamide as starting material, N-((2R,3S)-1-(3-((2-(4-hydroxypyridin-3-yl)pyrimidin-4-yl)amino)-5-(3-((tetrahydro-2H-pyran-2-yl)oxy)propyl)isoquinolin-8-yl)-2-methylazetidin-3-yl)-N-methylmethanesulfonamide was prepared according to the method of Step 2 for the synthesis of Final Product 20. MS (ESI⁺) m/z = 634.3 [M+H]⁺.

**Step 3:** Using N-((2R,3S)-1-(3-((2-(4-hydroxypyridin-3-yl)pyrimidin-4-yl)amino)-5-(3-((tetrahydro-2H-pyran-2-yl)oxy)propyl)isoquinolin-8-yl)-2-methylazetidin-3-yl)-N-methylmethanesulfonamide as starting material, N-((2R,3S)-1-(5-(3-hydroxypropyl)-3-((2-(4-hydroxypyridin-3-yl)pyrimidin-4-yl)amino)isoquinolin-8-yl)-2-methylazetidin-3-yl)-N-methylmethanesulfonamide was prepared according to the method of Step 3 for the synthesis of Final Product 34. MS (ESI⁺) m/z = 550.1 [M+H]⁺.

**Step 4:** Using N-((2R,3S)-1-(5-(3-hydroxypropyl)-3-((2-(4-hydroxypyridin-3-yl)pyrimidin-4-yl)amino)isoquinolin-8-yl)-2-methylazetidin-3-yl)-N-methylmethanesulfonamide as starting material, N-((2R,3S)-1-(8-oxa-3-aza-4(3,5)-isoquinolin-2(2,4)-pyrimidin-1(3,4)-pyridinacyclooctan-4⁸-yl)-2-methylazetidin-3-yl)-N-methylmethanesulfonamide (Final Product 39) was prepared according to the method of Step 4 in Example 1.

MS(ESI⁺)m/z = 532.1 [M+H]⁺. ¹H NMR (300 MHz, DMSO-*d₆*) *δ* 10.68 (s, 1H), 9.60 (s, 1H), 9.17-9.05 (m, 2H), 8.43 (d, *J =* 6.1 Hz, 1H), 7.84-7.74 (m, 1H), 7.50 (d, *J =* 7.8 Hz, 1H), 6.95 (d, *J =* 6.1 Hz, 1H), 6.65 (d, *J* = 7.8 Hz, 1H), 6.39 (d, *J =* 7.5 Hz, 1H), 4.70 (t, *J* = 7.5 Hz, 1H), 4.55-4.41 (m, 1H), 4.28-4.11 (m, 3H), 3.87 (t, *J =* 7.2 Hz, 1H), 2.97 (s, 3H), 2.94-2.87 (m, 2H), 2.83 (s, 3H), 2.27-2.08 (m, 2H), 1.42 (d, *J =* 6.0 Hz, 3H).

### Example 34 Preparation of N-((2R,3S)-1-(1¹,5-dimethyl-1¹H-8-oxa-3-aza-4(3,5)-isoquinolin-2(2,4)-pyrimidin-1(4,5)-pyrazolacyclooctan-4⁸-yl)-2-methylazetidin-3-yl)methanesulfonamide (Final Product 137)

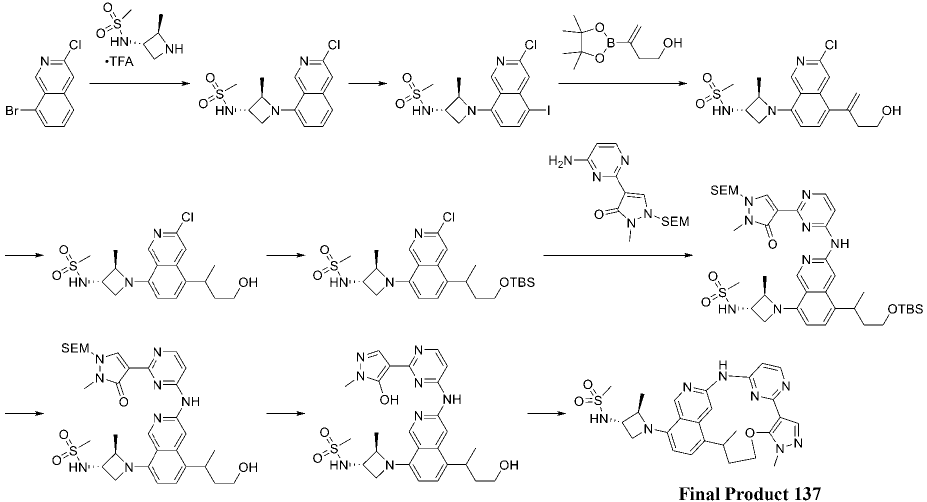

**Step 1:** Using 8-bromo-3-chloroisoquinoline (1.5 g, 6.19 mmol) and Intermediate C2 (1.9 g, 6.80 mmol) as starting materials, N-((2R,3S)-1-(3-chloroisoquinolin-8-yl)-2-methylazetidin-3-yl)methanesulfonamide was prepared according to the method of Step 1 for the synthesis of Final Product 1 (yellow solid, 800 mg, 39.7%). MS (ESI⁺) m/z = 326.1 [M+H]⁺.

**Step 2:** N-((2R,3S)-1-(3-chloroisoquinolin-8-yl)-2-methylazetidin-3-yl)methanesulfonamide (800.0 mg, 2.46 mmol) was dissolved in acetic acid (8 mL), and NIS (552.4 mg, 2.46 mmol) was added slowly. The mixture was stirred at r.t. for 5 min. After completion of the reaction, a sodium thiosulfate solution (200 mL) was added to the reaction mixture. The mixture was extracted with ethyl acetate (100 mL × 2). The combined organic layers were dried over anhydrous sodium sulfate, filtered, and the filtrate was concentrated. The residue was purified by silica gel column chromatography using ethyl acetate/petroleum ether (1/3) as eluent to afford N-((2R,3S)-1-(3-chloro-5-iodoisoquinolin-8-yl)-2-methylazetidin-3-yl)methanesulfonamide (yellow solid, 900 mg, 81.1%). MS (ESI⁺) m/z = 451.9 [M+H]⁺.

**Step 3:** Under a nitrogen atmosphere, N-((2R,3S)-1-(3-chloro-5-iodoisoquinolin-8-yl)-2-methylazetidin-3-yl)methanesulfonamide (900.0 mg, 1.99 mmol) was dissolved in a mixture of 1,4-dioxane and water (10/1 v/v, 11 mL). Pd(dppf)Cl₂ (145.8 mg, 0.20 mmol), K₂CO₃ (550.7 mg, 3.98 mmol), and 3-(4,4,5,5-tetramethyl-1,3,2-dioxaborolan-2-yl)but-3-en-1-ol (394.6 mg, 1.99 mmol) were added sequentially. The mixture was heated to 100 °C and stirred for 1 h. After completion of the reaction, the mixture was cooled to r.t., and water (200 mL) was added to the reaction mixture. The mixture was extracted with ethyl acetate (100 mL × 2). The combined organic layers were dried over anhydrous sodium sulfate, filtered, and the filtrate was concentrated. The residue was purified by silica gel column chromatography using ethyl acetate/petroleum ether (1/3) as eluent to afford N-((2R,3S)-1-(3-chloro-5-(4-hydroxybut-1-en-2-yl)isoquinolin-8-yl)-2-methylazetidin-3-yl)methanesulfonamide (yellow solid, 300 mg, 38.0%). MS (ESI⁺) m/z = 396.1 [M+H]⁺.

**Step 4:** Under a nitrogen atmosphere, N-((2R,3S)-1-(3-chloro-5-(4-hydroxybut-1-en-2-yl)isoquinolin-8-yl)-2-methylazetidin-3-yl)methanesulfonamide (300.0 mg, 0.76 mmol) was dissolved in ethyl acetate (5 mL), and platinum(IV) oxide (68.8 mg, 0.30 mmol) was added. The atmosphere was replaced with hydrogen, and the mixture was stirred at r.t. for 2 h. After completion of the reaction, the mixture was filtered, and the filtrate was concentrated to afford N-((2R,3S)-1-(3-chloro-5-(4-hydroxybutan-2-yl)isoquinolin-8-yl)-2-methylazetidin-3-yl)methanesulfonamide (yellow solid, 300 mg, 99.5%). MS (ESI⁺) m/z = 398.1 [M+H]⁺.

**Step 5:** N-((2R,3S)-1-(3-chloro-5-(4-hydroxybutan-2-yl)isoquinolin-8-yl)-2-methylazetidin-3-yl)methanesulfonamide (300.0 mg, 0.75 mmol) was dissolved in DCM (6 mL), and imidazole (154.0 mg, 2.26 mmol) was added. The mixture was cooled to 0 °C, and TBSCl (170.4 mg, 1.13 mmol) was added. The mixture was stirred at r.t. for 16 h. After completion of the reaction, water (20 mL) was added to the reaction mixture. The mixture was extracted with dichloromethane (20 mL × 3). The combined organic layers were washed with brine (20 mL), dried over anhydrous sodium sulfate, filtered, and the filtrate was concentrated. The residue was purified by silica gel column chromatography using ethyl acetate/petroleum ether (1/10) as eluent to afford N-((2R,3S)-1-(5-(4-((tert-butyldimethylsilyl)oxy)butan-2-yl)-3-chloroisoquinolin-8-yl)-2-methylazetidin-3-yl)methanesulfonamide (yellow solid, 300 mg, 77.7%). MS (ESI⁺) m/z = 512.2 [M+H]⁺.

**Step 6:** Under a nitrogen atmosphere, N-((2R,3S)-1-(5-(4-((tertbutyldimethylsilyl)oxy)butan-2-yl)-3-chloroisoquinolin-8-yl)-2-methylazetidin-3-yl)methanesulfonamide (300.0 mg, 0.59 mmol) was dissolved in 1,4-dioxane (5 mL). Brettphos Pd G₄ (107.8 mg, 0.12 mmol), Cs₂CO₃ (381.7 mg, 1.17 mmol), and 4-(4-aminopyrimidin-2-yl)-2-methyl-1-((2-(trimethylsilyl)ethoxy)methyl)-1,2-dihydro-3H-pyrazol-3-one (Intermediate B1, 188.3 mg, 0.59 mmol) were added sequentially. The mixture was heated to 100 °C and stirred for 2 h. After completion of the reaction, the mixture was cooled to r.t., filtered, and the filtrate was concentrated. The residue was purified by silica gel column chromatography using methanol/dichloromethane (1/10) as eluent to afford N-((2R,3S)-1-(5-(4-((tert-butyldimethylsilyl)oxy)butan-2-yl)-3-((2-(2-methyl-3-oxo-1-((2-(trimethylsilyl)ethoxy)methyl)-2,3-dihydro-1H-pyrazol-4-yl)pyrimidin-4-yl)amino)isoquinolin-8-yl)-2-methylazetidin-3-yl)methanesulfonamide (yellow solid, 200 mg, 42.8%). MS (ESI⁺) m/z = 797.3 [M+H]⁺.

**Step 7:** N-((2R,3S)-1-(5-(4-((tert-butyldimethylsilyl)oxy)butan-2-yl)-3-((2-(2-methyl-3-oxo-1-((2-(trimethylsilyl)ethoxy)methyl)-2,3-dihydro-1H-pyrazol-4-yl)pyrimidin-4-yl)amino)isoquinolin-8-yl)-2-methylazetidin-3-yl)methanesulfonamide (200.0 mg, 0.25 mmol) was dissolved in THF (1 mL), and hydrochloric acid (2 M, 2 mL, 0.50 mmol) was added dropwise. The mixture was stirred at r.t. for 1 h. After completion of the reaction, water (20 mL) was added to the reaction mixture. The mixture was extracted with ethyl acetate (20 mL × 2). The aqueous phase was adjusted to pH 13 with NaOH solution and then extracted with ethyl acetate (20 mL × 2). The combined organic layers were dried over anhydrous sodium sulfate, filtered, and the filtrate was concentrated to afford N-((2R,3S)-1-(5-(4-hydroxybutan-2-yl)-3-((2-(2-methyl-3-oxo-1-((2-(trimethylsilyl)ethoxy)methyl)-2,3-dihydro-1H-pyrazol-4-yl)pyrimidin-4-yl)amino)isoquinolin-8-yl)-2-methylazetidin-3-yl)methanesulfonamide (yellow-green oil, 200 mg, crude). MS (ESI⁺) m/z = 683.2 [M+H]⁺.

**Step 8:** N-((2R,3S)-1-(5-(4-hydroxybutan-2-yl)-3-((2-(2-methyl-3-oxo-1-((2-(trimethylsilyl)ethoxy)methyl)-2,3-dihydro-1H-pyrazol-4-yl)pyrimidin-4-yl)amino)isoquinolin-8-yl)-2-methylazetidin-3-yl)methanesulfonamide (200.0 mg, 0.29 mmol) was dissolved in DCM (1 mL), and TFA (1 mL) was added dropwise. The mixture was stirred at r.t. for 1 h. After completion of the reaction, the mixture was concentrated. The residue was adjusted to pH 13 with NaOH (1 M) solution, stirred for 1 h, and concentrated. The residue was purified by silica gel column chromatography using methanol/dichloromethane (1/10) as eluent to afford N-((2R,3S)-1-(3-((2-(5-hydroxy-1-methyl-1H-pyrazol-4-yl)pyrimidin-4-yl)amino)-5-(4-hydroxybutan-2-yl)isoquinolin-8-yl)-2-methylazetidin-3-yl)methanesulfonamide (yellow solid, 100 mg, yield over two steps: 72.1%). MS (ESI⁺) m/z = 553.2 [M+H]⁺.

**Step 9:** N-((2R,3S)-1-(3-((2-(5-hydroxy-1-methyl-1H-pyrazol-4-yl)pyrimidin-4-yl)amino)-5-(4-hydroxybutan-2-yl)isoquinolin-8-yl)-2-methylazetidin-3-yl)methanesulfonamide (80.0 mg, 0.14 mmol) was dissolved in THF (1 mL) and cooled to 0 °C. PPh₃ (113.9 mg, 0.43 mmol) and DBAD (129.5 mg, 0.43 mmol) were added sequentially. The mixture was stirred at r.t. for 1 h. After completion of the reaction, H₂SO₄ solution (1 M, 10 mL) was added to the reaction mixture. The mixture was extracted with ethyl acetate (10 mL × 2). The aqueous phase was adjusted to pH 13 with NaOH solution (1 M) and then extracted with ethyl acetate (10 mL × 2). The combined organic layers were dried over anhydrous sodium sulfate, filtered, and the filtrate was concentrated. The residue was purified by high-performance preparative liquid chromatography to afford N-((2R,3S)-1-(1¹,5-dimethyl-1¹H-8-oxa-3-aza-4(3,5)-isoquinolin-2(2,4)-pyrimidin-1(4,5)-pyrazolcyclooctan-4⁸-yl)-2-methylazetidin-3-yl)methanesulfonamide (yellow solid, 5 mg, 6.3%).

MS(ESI⁺)m/z = 534.8 [M+H]⁺. ¹H NMR (400 MHz, DMSO-*d*₆) *δ* 9.18-9.04 (m, 2H), 8.28-8.19 (m, 1H), 8.08 (s, 1H), 7.58 (dd, *J =* 8.0, 2.8 Hz, 1H), 6.97 (d, *J =* 5.4 Hz, 1H), 6.80-6.62 (m, 1H), 4.74 (t, *J =* 7.0 Hz, 1H), 4.60-4.48 (m, 1H), 4.39-4.29 (m, 1H), 4.19-4.12 (m, 1H), 4.12-3.81 (m, 3H), 3.74 (s, 2H), 3.60-3.52 (m, 1H), 2.94 (d, *J =* 3.8 Hz, 3H), 2.20-2.03 (m, 1H), 1.94-1.83 (m, 1H), 1.59-1.46 (m, 3H), 1.40 (t, *J=* 5.8 Hz, 3H).

### Examples 35-42 Preparation of Final Products 61, 77, 83, 86, 98, 123, 140, 157, 189, 193

Using intermediates B1-B10 and intermediates C1-C5 as starting materials, Final products 61, 77, 83, 86, 98, 123, 140, 157, 189, 193 were prepared according to the preparation method of above final product 137. As shown in Table 3. The coupling reaction conditions for the first step may also be: Xantphos Pd G₃, Cs₂CO₃, dioxane, 100 °C. The iodination reaction conditions for the second step may also be: NIS, acetonitrile, acetic acid, r.t. The reaction conditions for the TBS protection in the fifth step may also be: imidazole, TBSCl, DMAP, DCM. The coupling reaction conditions for the sixth step may also be: EPhos Pd G₄, Ephos, Cs₂CO₃, 1,4-dioxane, 100 °C. The deprotection reactions in the seventh and eighth steps can be completed in one step using TBAF, THF, 70 °C; when intermediates B3, B7, or B8 are used as starting materials, the demethylation reaction conditions for the eighth step may also be: DMF, NaH, ethanethiol, DMF, 120 °C. The Mitsunobu reaction conditions for the ninth step may also be: CMBP, toluene, 100-110 °C.

**Table 3 Characterization Data for Final Products 61, 77, 83, 86, 98, 123, 140, 157, 189, 193**

| **Final Product** | **Inter mediate** | **Structure of Final Product** | **NMR or MS** |
|---|---|---|---|
| **Final Product 61** | **B8** | 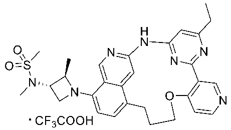 | Orange solid. MS (ESI⁺) m/z = 560.3 [M+H]⁺. ¹H NMR (400 MHz, DMSO-*d*₆) *δ* 10.49 (s, 1H), 9.41 (d, *J* = 10.7 Hz, 2H), 9.11 (s, 1H), 8.86 (d, *J =* 6.8 Hz, 1H), 7.86 *(d, J=* 6.8 Hz, 1H), 7.44 (d, *J* = 7.7 Hz, 1H), 7.05 (s, 1H), 6.59 (d, *J =* 7.8 Hz, 1H), 4.68 (t, *J =* 7.5 Hz, 1H), 4.61-4.50 (m, 2H), 4.49-4.40 (m, 1H), 4.24-4.16 (m, 1H), 3.91-3.81 (m, 1H), 3.13-2.99 (m, 2H), 2.96 (s, 3H), 2.82 (s, 3H), 2.73 (q, *J* = 7.5 Hz, 2H), 2.20-2.16 (m, 2H), 1.41 (d, *J =* 6.0 Hz, 3H), 1.29 (t, *J* = 7.5 Hz, 3H). |
| | **C3** | | |
| **Final Product 77** | **B10** | 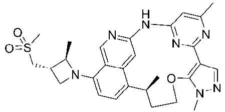 | Chiral separation: Column: CHIRALPAK IA 2*25 cm, 5 µm; Mobile phase A: n-hexane (0.5% 2 M NH₃-MeOH); Mobile phase B: ethanol/DCM (1/1); Flow rate: 20 mL/min; Wavelength: 220/254 nm. Yellow solid. MS (ESI⁺) m/z = 548.3 [M+H]⁺. ¹H NMR (300 MHz, DMSO-*d*₆) *δ* 10.41-10.08 (m, 1H), 9.11-9.05 (m, 2H), 8.00 (s, 1H), 7.51 (d, *J =* 8.0 Hz, 1H), 6.71 (s, 1H), 6.61 (d, *J =* 8.1 Hz, 1H), 4.66 (t, *J* = 7.5 Hz, 1H), 4.49-4.41 (m, 1H), 4.38-4.30 (m, 1H), 4.25-4.15 (m, 1H), 3.83 (s, 3H), 3.79-3.70 (m, 1H), 3.68-3.49 (m, 2H), 2.99 (s, 3H), 2.94-2.84 (m, 2H), 2.34 (s, 3H), 2.22-2.13 (m, 1H), 1.92-1.82 (m, 1H), 1.48 (d, *J* = 6.8 Hz, 3H), 1.41 (d, *J* = 6.1 Hz, 3H). |
| | **C1** | or 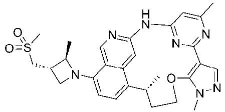 | |
| **Final Product 83** | **B9** | 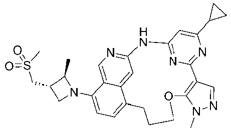 The coupling reaction conditions for the sixth step were: EPhos Pd G₄, Ephos, Cs₂CO₃, 1,4-dioxane. The Mitsunobu reaction conditions for the ninth step were: CMBP, toluene, 110 °C. | Yellow solid. MS (ESI⁺) m/z = 560.2 [M+H]⁺. ¹H NMR (300 MHz, DMSO-*d*₆) *δ* 9.97 (s, 1H), 9.03 (s, 1H), 8.99 (s, 1H), 7.88 (s, 1H), 7.41 (d, *J* = 7.8 Hz, 1H), 6.73 (s, 1H), 6.53 (d, *J =* 7.9 Hz, 1H), 4.67 (t, *J* = 7.5 Hz, 1H), 4.48-4.36 (m, 1H), 4.36-4.25 (m, 1H), 4.19 (t, *J =* 6.1 Hz, 1H), 3.76 (s, 3H), 3.65 (t, *J =* 7.1 Hz, 1H), 3.56-3.48 (m, 1H), 2.99 (s, 3H), 2.96-2.81 (m, 2H), 2.31-2.17 (m, 2H), 2.16-1.99 (m, 2H), 1.98-1.86 (m, 1H), 1.43 (d, *J =* 6.0 Hz, 3H), 1.05-0.99 (m, 2H), 0.98-0.91 (m, 2H). |
| | **C1** | | |
| **Final Product 86** | **B7** | 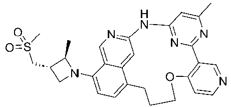 The Mitsunobu reaction conditions for the ninth step were: CMBP, toluene, 100 °C. | White solid. MS (ESI⁺) m/z = 531.1 [M+H]⁺. ¹H NMR (400 MHz, DMSO-*d*₆) *δ* 10.29 (s, 1H), 9.67 (s, 1H), 9.06 (s, 1H), 8.95 (s, 1H), 7.71 (d, *J* = 7.3 Hz, 1H), 7.43 (d, *J =* 7.8 Hz, 1H), 6.74 (s, 1H), 6.50 (d, *J* = 7.8 Hz, 1H), 6.29 (d, *J* = 7.5 Hz, 1H), 4.70 (t, *J =* 7.6 Hz, 1H), 4.21 (t, *J* = 6.2 Hz, 1H), 4.11 (s, 2H), 3.69 (t, *J* = 7.1 Hz, 1H), 3.61-3.46 (m, 2H), 3.00 (s, 3H), 2.93-2.76 (m, 3H), 2.39 (s, 3H), 2.24-2.19 (m, 2H), 1.43 (d, *J =* 6.0 Hz, 3H). |
| | **C1** | | |
| **Final Product 98** | **B1** | 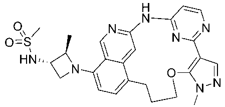 The reaction conditions for the second step were: NIS, acetonitrile, acetic acid, r.t. The reaction conditions for the ninth step were: CMBP, toluene, 100 °C. | Yellow solid. MS (ESI⁺) m/z = 521.2 [M+H]⁺. ¹H NMR (300 MHz, DMSO-*d*₆) *δ* 10.19 (s, 1H), 9.06 (s, 1H), 9.02 (s, 1H), 8.30 (d, *J* = 5.7 Hz, 1H), 7.94 (s, 1H), 7.79 (d, *J =* 8.5 Hz, 1H), 7.45 (d, *J* = 7.8 Hz, 1H), 6.85 (d, *J =* 5.8 Hz, 1H), 6.61 (d, *J =* 7.9 Hz, 1H), 4.79 (t, *J =* 7.1 Hz, 1H), 4.48-4.38 (m, 1H), 4.38-4.29 (m, 1H), 4.23-4.09 (m, 1H), 4.00-3.86 (m, 1H), 3.78 (s, 3H), 3.57 (t, *J =* 6.9 Hz, 1H), 2.96 (s, 3H), 2.33-2.20 (m, 2H), 2.14-1.98 (m, 2H), 1.43 (d, *J =* 6.1 Hz, 3H). |
| | **C2** | | |
| **Final Product 123** | **B6** | 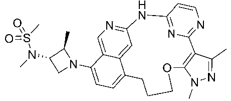 The reaction conditions for the sixth step were: EPhos Pd G₄, Ephos, Cs₂CO₃, 1,4-dioxane, 100 °C. The reaction conditions for the ninth step were: CMBP, toluene, 100 °C. | White solid. MS (ESI⁺) m/z = 549.2 [M+H]⁺. ¹H NMR (400 MHz, DMSO-*d*₆) *δ* 10.11 (s, 1H), 9.08 (s, 1H), 8.91 (s, 1H), 8.39-8.30 (m, 1H), 7.52-7.41 (m, 1H), 6.88-6.82 (m, 1H), 6.72-6.65 (m, 1H), 4.65 (t, *J* = 7.2 Hz, 1H), 4.49-4.20 (m, 4H), 3.84-3.81 (m, 1H), 3.69 (s, 3H), 2.95 (s, 3H), 2.82 (s, 3H), 2.43 (s, 3H), 2.35-2.20 (m, 1H), 2.20-1.87 (m, 3H), 1.48-1.34 (m, 3H). |
| | **C3** | | |
| **Final Product 140** | **B1** | 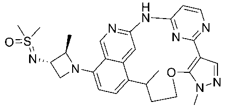 The reaction conditions for the second step were: NIS, acetonitrile, acetic acid, r.t. The reaction conditions for the fifth step were: imidazole, TBSCl, DMAP, DCM, r.t. | Orange solid. MS (ESI⁺) m/z = 533.3 [M+H]⁺. ¹H NMR (300 MHz, DMSO-*d*₆) *δ* 10.14 (d, *J =* 10.9 Hz, 1H), 9.16-9.01 (m, 2H), 8.29 (d, *J =* 5.7 Hz, 1H), 7.94 (d, *J =* 2.1 Hz*,* 1H), 7.50 (d, *J* = 7.9 Hz, 1H), 6.84 (dd, *J =* 5.8, 1.7 Hz, 1H), 6.62 (dd, *J =* 8.1, 2.8 Hz, 1H), 4.71 (t, *J* = 6.4 Hz, 1H), 4.51-4.29 (m, 2H), 4.06-3.90 (m, 2H), 3.88-3.74 (m, 4H), 3.49-3.40 (m, 1H), 3.09-2.98 (m, 6H), 2.29-2.08 (m, 1H), 2.05-1.74 (m, 1H), 1.52-1.47 (m, 3H), 1.44-1.32 (m, 3H). |
| | **C5** | | |
| **Final Product 157** | **B6** | 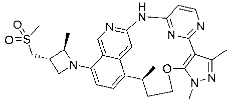 | Chiral separation: Column: CHIRALPAK IG, 2*25 cm, 5 µm; Mobile phase A: methyl tert-butyl ether (0.5% 2 M NH₃-MeOH); Mobile phase B: ethanol/DCM (1/1); Flow rate: 20 mL/min; Wavelength: 220/254 nm. |
| | **C1** | or | Off-white solid. MS (ESI⁺) m/z = 548.2 [M+H]⁺.¹H NMR (300 MHz, Acetonitrile-*d₃*) *δ* 9.12 (s, 1H), 9.10 (s, 1H), 8.34 (d, *J* = 5.8 Hz, 1H), 8.21 (s, 1H), 7.56 (d, *J* = 8.1 Hz, 1H), 6.67 (d, *J* = 6.1 Hz, 2H), 4.71 (t, *J =* 7.5 Hz, 1H), 4.48-4.36 (m, 1H), 4.33-4.21 (m, 2H), 3.98-3.89 (m, 1H), 3.76 (s, 3H), 3.71-3.66 (m, 1H), 3.53-3.33 (m, 2H), 3.03-2.94 (m, 1H), 2.92 (s, 3H), 2.50 (s, 3H), 1.58-1.44 (m, 8H). |
| | | 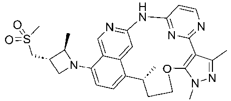 | |
| **Final Product 189** | **B6** | 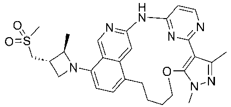 | Yellow solid. MS (ESI⁺) m/z = 548.1 [M+H]⁺. ¹H NMR (300 MHz, Acetonitrile-*d₃*) *δ* 9.05 (s, 1H), 8.99 (s, 1H), 8.36 (d, *J* = 5.8 Hz, 1H), 8.25 (s, 1H), 7.19 (s, 1H), 6.70 (d, *J =* 5.8 Hz, 1H), 5.56-5.49 (m, 1H), 4.32-4.18 (m, 2H), 3.72 (s, 3H), 3.61-3.43 (m, 2H), 3.31-3.11 (m, 3H), 2.97-2.85 (m, 5H), 2.51-2.40 (m, 4H), 1.94-1.76 (m, 3H), 1.36 (d, *J=* 7.1 Hz, 3H). |
| | **C1** | | |
| **Final Product 193** | **B6** | 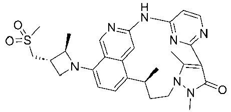 | Yellow solid. MS (ESI⁺) m/z = 548.2 [M+H]⁺. ¹H NMR (300 MHz, Acetonitrile-*d₃*) *δ* 9.05 (s, 1H), 8.99 (s, 1H), 8.33 (d, *J* = 5.8 Hz, 1H), 8.18 (s, 1H), 7.37 (s, 1H), 6.65 *(d, J=* 5.7 Hz, 1H), 5.60-5.50 (m, 1H), 4.51-4.38 (m, 1H), 4.27-4.18 (m, 1H), 3.91-3.80 (m, 1H), 3.75 (s, 3H), 3.58-3.49 (m, 2H), 3.25-3.12 (m, 1H), 3.02-2.87 (m, 5H), 2.52-2.41 (m, 4H), 2.16-2.08 (m, 2H), 1.52 (d, *J =* 7.0 Hz, 3H), 1.37 (d, *J* = 7.0 Hz, 3H). |
| | | or | |
| | **C1** | 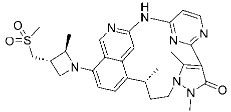 | |

### Preparation of Comparative Compound A

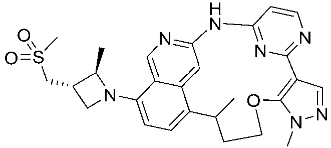

Comparative Compound A was prepared from Intermediate A1, Intermediate C1, and Intermediate B1 according to the synthesis method in Example 1. MS (ESI⁺) m/z = 534.2 [M+H]⁺. ¹H NMR (300 MHz, DMSO-*d*₆) *δ* 10.13 (s, 1H), 9.08 (d, *J =* 8.7 Hz, 2H), 8.29 (d, *J* = 5.8 Hz, 1H), 7.93 (s, 1H), 7.50 (d, *J =* 8.0 Hz, 1H), 6.83 (d, *J =* 5.8 Hz, 1H), 6.60 (d, *J =* 8.1 Hz, 1H), 4.67 (t, *J =* 7.5 Hz, 1H), 4.49-4.41 (m, 1H), 4.38-4.30 (m, 1H), 4.25-4.15 (m, 1H), 3.82 (s, 3H), 3.70-3.59 (m, 1H), 3.60-3.50 (m, 3H), 2.99 (s, 3H), 2.93-2.84 (m, 1H), 2.35-2.24 (m, 1H), 1.89-1.83 (m, 1H), 1.49 (d, *J = 7.2* Hz, 3H), 1.42 (d, *J =* 6.9 Hz, 3H).

### Experimental Examples

### I. Cell Proliferation Inhibition Assay

The CellTiter-Glo^{™} Luminescent Cell Viability Assay Kit uses luciferase as the detection reagent, and ATP is required for the luminescence process. The CellTiter-Glo^{™} reagent was added to the cell culture medium, and the luminescence signal was measured. The light signal is proportional to the amount of ATP in the system, which in turn is positively correlated with the number of viable cells. Therefore, by detecting ATP content using the CellTiter-Glo kit, cell proliferation can be assessed.

In this experiment, the celltiter-Glo (CTG) method was used to measure the proliferation inhibitory effects of the the previously prepared compounds in tumor cell lines Ba/F₃ (EGFR-L858R/C797S), Ba/F₃ (EGFR-L858R/T790M/C797S), NCI-H1975, PC9, Ba/F₃ EGFR wt, and A431, and the 50% inhibitory concentration (IC₅₀) was calculated.

### Experimental Design

Compounds were tested on the selected cells, with a vehicle control set. A total of 9 concentrations were tested, each in duplicate wells.

### Reagents and Consumables

| **Reagents** | **Supplier** | **Cat#** |
|---|---|---|
| Celltiter-Glo^{®} | Promega | G7573 |
| RPMI1640 | VivaCell | C3010-0500 |
| PC-9 | RIKEN | RCB4455 |
| NCI-H1975 | ZOXZ | ZQ0395 |
| Ba/F₃ (EGFR-L858R/C797S) | RIKEN | RCB0805 |
| Ba/F₃ (EGFR-L858R/T790M/C797S) | RIKEN | RCB0805 |
| Ba/F₃ (EGFR wt) | RIKEN | RCB0805 |
| A431 | RIKEN | RCB0202 |
| FBS | VivaCell | C04002-500 or C04001-500 |
| DMSO | Solarbio | D8371 |

| **Instruments and Consumables** | **Supplier** | **Cat# or Model** |
|---|---|---|
| 96-well clear flat-bottom plate | Biosharp | BS-MP-96W |
| CO₂ incubator | Thermo | HERA cell vios 160i |
| Counter | Count star | IC1000 |
| Inverted microscope | OPTEC | BDS400 |
| Multi-label microplate reader | Molecular Devices | SpectraMax Paradigm |

### Experimental Procedure

I. Cell Culture
   a) All cells were cultured according to the ATCC-recommended methods. Cells in the logarithmic growth phase were harvested. Cell viability was assessed and ensured to be above 90%.
   b) Cell culture medium: The cell concentration was adjusted, and 90 µL of cell suspension was added to each well of a 96-well plate.
   c) Cell culture conditions: Incubation at 37 °C, 5% CO₂, and 95% humidity.
II. Drug Dilution
   a) Drug stock solution: Compoundss were dissolved in DMSO to prepare 10 mM DMSO stock solutions.
   b) Drug storage: DMSO stock solutions were stored for short-term use (up to 3 months) in a desiccator at r.t. The remaining stock were stored at -20 °C for longer periods.
III. Drug Addition
   a) For testing antiproliferative activity in Ba/F₃ (EGFR-L858R/C797S), Ba/F₃ (EGFR-L858R/T790M/C797S), NCI-H1975, and PC9 cell lines, ccompounds were 3-fold serially diluted starting from 1 µM, giving 10 concentration points. For testing the antiproliferative activity in Ba/F₃ EGFR wt wild-type cells (with EGF stimulation) and A431 cell lines, compounds were 3-fold serially diluted starting from 10 µM, giving 10 concentration points.
   b) Positive control and DMSO vehicle controls were prepared.
   c) 10 µL of a 10x drug solution was added to each well of the 96-well plate containing cells, each drug concentration was tested in duplicate wells.
   d) The 96-well plate with added drugs were incubated at 37 °C, 5% CO₂, and 95% humidity for an additional 72 hours, followed by CTG analysis.
IV. Endpoint Plate Reading
   a) The CTG reagent was thawed, and 50 µL of CTG solution was added to each well and mixed.
   b) The plate was shaken on an orbital shaker for 2 minutes and then incubated at r.t. for 10 minutes.
   c) The luminescence signal was read using a microplate reader.

### Data Processing

Data were analyzed using GraphPad Prism 8.0 software. A nonlinear sigmoidal dose-response curvewas fitted to the data to obtain the ose-effect curve, from which the IC₅₀ values were calculated. 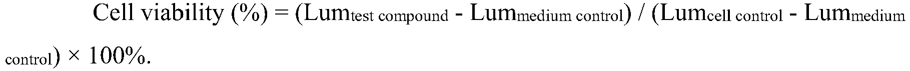 Cell viability (%) = (Lumtest compound - Lummedium control) / (Lumcell control - Lummedium control) × 100%.

### Experimental Results

**Table 4 Antiproliferative activity (IC₅₀ (nM)) of compounds against Ba/F₃ (EGFR-L858R/C797S), Ba/F₃ (EGFR-L858R/T790M/C797S), NCI-H1975, PC9, Ba/F₃ EGFR wt+EGF, and A431 Cell Lines**

| **Compound Number** | **NCI-H1975** | **PC9** | **Ba/F₃ EGFR LR/CS** | **Ba/F₃ EGFR LR/TM/CS** | **Ba/F₃ EGFR wt+EGF** | **A431** |
|---|---|---|---|---|---|---|
| **1** | 1.5 | 0.7 | 0.5 | 1.7 | 20 | 196 |
| **2** | 0.7 | 0.3 | 0.2 | 0.7 | 12 | 117 |
| **10** | / | 12 | 13 | / | / | 857 |
| **12** | 1.9 | 3.3 | 4.1 | 2.3 | 133 | 632 |
| **13** | 32 | 34 | / | / | / | / |
| **22** | 0.4 | 0.5 | 0.8 | 1.2 | 80 | 327 |
| **25** | 1.3 | 1.1 | 5.0 | 3.1 | 103 | 410 |
| **34** | 3.3 | 7.5 | 3.9 | 2.6 | 152 | 746 |
| **36** | 5.5 | 5.1 | 4 | 3 | 895 | 2126 |
| **38** | 36 | 10 | / | / | / | 3354 |
| **42** | 2.8 | 0.2 | 1.0 | 2.5 | 106 | 582 |
| **60** | 33 | 18 | 2.8 | 3.5 | 372 | 1240 |
| **71** | 1.5 | 0.5 | 0.8 | 1.4 | 80 | 468 |
| **73** | 0.3 | 0.3 | 0.4 | 0.9 | 63 | 395 |
| **77** | 28 | 9.8 | / | / | / | / |
| **110** | 9.9 | 17.4 | / | / | / | / |
| **111** | 1.1 | 2 | 0.4 | 1.1 | 145 | 613 |
| **117** | 0.5 | 0.4 | 0.14 | 0.6 | 104 | 363 |
| **119** | 1.4 | 2.4 | 0.5 | 1.8 | 138 | 775 |
| **123** | 10.2 | 8.3 | 0.9 | / | / | 876 |
| **124** | 11.4 | 5.8 | 1.2 | / | / | 1384 |
| **137** | / | 2.1 | 1.1 | / | / | 810 |
| **140** | / | 15.2 | / | / | / | 4187 |
| **145** | 0.6 | 0.3 | 0.3 | 0.5 | 189 | 612 |
| **148** | 0.9 | 0.5 | 0.6 | 1.2 | 210 | 653 |
| **154** | / | 23 | / | / | / | / |
| **155** | 51 | 40 | / | / | / | / |
| **157** | 1 | 0.6 | 0.9 | 1.5 | 160 | 548 |
| **166** | / | 30.6 | / | / | / | >10000 |
| **176** | 1.2 | 1.7 | 0.8 | 2.1 | 256 | 823 |
| **187** | 3.1 | 1 | 0.8 | 1.4 | 196 | 695 |
| **190** | 4.1 | 4.7 | 0.7 | 0.9 | 98 | 847 |
| **Comparative Compound A** | 0.5 | 5.3 | 0.7 | 1.3 | 19 | 154 |

| | | | | | | |
|---|---|---|---|---|---|---|
| Note: LR/CS refers to L858R/C797S; LR/TM/CS refers to L858R/T790M/C797S; /: not tested; | | | | | | |

The IC₅₀ values for the antiproliferative activity of the compounds of the present invention against Ba/F₃ (EGFR-L858R/C797S), Ba/F₃ (EGFR-L858R/T790M/C797S), NCI-H1975, PC9, Ba/F₃ EGFR wt+EGF, and A431 cell lines are shown in Table 4.

As can be seen from Table 4, the compounds of the present invention exhibited good antiproliferative activity against Ba/F₃ (EGFR-L858R/C797S), Ba/F₃ (EGFR-L858R/T790M/C797S), NCI-H1975, and PC9 cell lines, while showing weaker activity against the wild-type EGFR-expressing cell lines, Ba/F₃ EGFR wt+EGF and A431 This indicates that the compounds of the present invention have good inhibitory activity and selectivity against EGFR mutations -expressing cell lines. The data in Table 4 further indicates when the linker chain L₂ group between the isoquinoline and pyrazole bears a substituent (e.g., compounds 111, 119, 187, 190) or when the pyrazole 3-position bears a substituent (e.g., compound 117), the selectivity is enhanced.

### II. Cassette Pharmacokinetic Studies of Compounds in Mice

Objective: ICR mice were used as the test subjects, the LC/MS/MS method was employed to determine the plasma concentrations of compounds at various time points after oral gavage (PO) administration. The pharmacokinetic behavior of the compounds of the present invention in mice was studied and their pharmacokinetic profiles were evaluated.

Drug Preparation: Compounds were formulated as clear solutions using 10% NMP + 10% Solutol + 80% Saline as the vehicle for PO administration. The cassette dosing method was applied. The administration doses were: 10 mg/kg, 15 mg/kg, or 20 mg/kg. The resulting pharmacokinetic parameter are summarized in Table 5.

**Table 5 Cassette Pharmacokinetic Test Results in Mice**

| Compound No. | Dose mg/kg | Half-life T_{1/2} (h) | Maximum Concentration Cₘₐₓ (ng/mL) | Area Under the Curve AUC₀₋₂₄ₕ (ng·hr/mL) |
|---|---|---|---|---|
| **1** | 15 | 4.0 | 1413 | 10199 |
| **2** | 15 | 1.0 | 672 | 2154 |
| **34** | 15 | 4.7 | 1082 | 6648 |
| **42** | 15 | 3.0 | 2950 | 13075 |
| **111** | 10 | 6 | 964 | 10972 |
| **117** | 10 | 3.2 | 953 | 10741 |
| **119** | 15 | 5.1 | 1581 | 21410 |
| **154** | 10 | 3.0 | 2193 | 28665 |
| **176** | 10 | 4.6 | 2857 | 31408 |
| **187** | 20 | 4.2 | 4540 | 35072 |
| **190** | 10 | 5.4 | 1416 | 22063 |

As can be seen from Table 5, the compounds of the present invention exhibit good exposure.

### III. P-gp Substrate Evaluation Assay

### Experimental Design

### I. Cell Culture

High-glucose DMEM medium containing L-glutamine was used, supplemented with 10% fetal bovine serum, 0.1 mg/mL streptomycin, and 0.6 µg/mL penicillin.

MDCKII-MDR1 cells were cultured in T-75 cell culture flasks. The incubator was set at 37 °C, 5% CO₂, and 95% relative humidity. When cell confluency reached 70-90%, cells were ready for Transwell seeding.

Before cell seeding, 50 µL of cell culture medium was added to each well of the upper Transwell chamber, and 25 mL of cell culture medium was added to the lower plate. The culture plate was incubated at 37 °C, 5% CO₂ for 1 hour before seeding.

Cells were gently rinsed with 5 mL of PBS. PBS was discarded, and 1.5 mL of trypsin-EDTA was added, followed by incubation at 37 °C for 5 to 10 minutes until completely detached. Trypsinization was stopped by adding Serum-containing medium.

The cell suspension was transferred to a round-bottom centrifuge tube, and centrifuged at 120 × g for 10 minutes.

Cells were resuspended in culture medium to a final concentration of 1.56 × 10⁶ cells/mL.

### I. MDCKII-MDR1 Cell Seeding

Cell suspension (50 µL) was added to each well of the upper chamber of a 96-well Transwell plate, giving a final seeding density of 1.45 × 10⁵ cells/cm².

Medium was changed 24 hours after seeding. Cells were cultured for 4-8 days with medium replacement every other day.

### I. Evaluation ofMonolayer Integrity

After 4-8 days of culture, MDCKII-MDR1 and MDCKII cells should be fully confluent and differentiated, and were then ready for permeability assays.

Transendothelial electrical resistance (TEER) was measured using a voltohmmeter (Millipore, USA), and the resistance value for each well was recorded.

After measurement, the Transwell plate was returned to the incubator.

TEER was calculated as:
Measured resistance value (ohms) × membrane area (cm²) = TEER value (ohm·cm²)
Wells with TEER < 42 ohm·cm²were excluded from permeability tests.

### I. Drug Permeability Assay

The MDCKII-MDR1 Transwell plate was removed from the incubator. Cell monolayer was rinsed twice with HBSS (10 mM HEPES, pH 7.4) and incubated at 37 °C for 30 minutes.

For apical to the basolateral transport: 125 µL of the test compound or control compound in HBSS (10 mM HEPES, pH 7.4) buffer was added to the apical side (upper chamber), and 235 µL of HBSS (10 mM HEPES, pH 7.4) buffer was added to the basolateral side (lower chamber) per well.

For basolateral to apical transport: 285 µL of HBSS (10 mM HEPES, pH 7.4) buffer was added to the apical side(upper chamber), and75 µL of the test compound or control in HBSS (10 mM HEPES, pH 7.4) buffer was added and to basolateral side (lower chamber) per well.

The assembled apical and basolateral compartments were incubated at 37 °C for 2 hours.

After incubation, 50 µL samples were taken from both apical and basolateral compartments of each well and transferred to new tubes. Four volumes of acetonitrile containing internal standards (200 nM alprazolam, 200 nM labetalol, 200 nM diclofenac, 100 nM tolbutamide) were added, vortexed for 5 minutes, andcentrifuged at 3,220 g for 15 minutes. The supernatant (100 µL) was diluted with an equal volume of water and analyzed by LC-MS/MS. All samples were prepared in triplicate.

Monolayer integrity after 2 hours incubation was assessed by lucifer yellow leakage. The lucifer yellow stock solution was diluted in HBSS (10 mM HEPES, pH 7.4) buffer to a final concentration of 100 µM. 100 µL of the lucifer yellow solution was added to each apical well, and 300 µL of HBSS (10 mM HEPES, pH 7.4) buffer was added to each basolateral well. After incubation at 37 °C for 30 minutes, 80 µL samples were taken from both sides of each well and transferred to a new 96-well plate. Fluorescence was measured using a microplate reader at excitation 485 nm and emission 530 nm.

### Data Processing

Data was calculated using Excel. The apparent permeability coefficient (Pₐₚₚ, unit: cm/s) was calculated using the following formula: $\begin{matrix}Apparent Permeability Coefficient = \frac{Volume of Receiver compartment}{Membrane Area \times Incubation Time} \times \\ \frac{Drug Concentration in Receiver compartment}{Initial Drug Concentration in Donor compartment}\end{matrix}$

Wherein, the membrane area is 0.143 cm² (Transwell-96 well plate); and incubation time is in seconds (s).

The efflux ratio was calculated using the following formula:$Efflux Ratio = \frac{Apparent Permeability Coefficient \left(Basolateral to Apical\right)}{Apparent Permeability Coefficient \left(Apical to Basolateral\right)}$

**Table 6. Efflux Ratio of Test Compounds**

| **Final Product Number** | **ER (Efflux Ratio)** |
|---|---|
| **1** | 0.4 |
| **2** | 1.5 |
| **12** | 3.2 |
| **34** | 3.2 |
| **36** | 1.7 |
| **73** | 1.0 |
| **111** | 1.0 |
| **117** | 0.4 |
| **119** | 1.2 |
| **187** | 0.9 |

The descriptions of the above embodiments are only intended to facilitate understanding of the method and its core idea of the present invention. It should be noted that those of ordinary skilled in the art may also make certain improvements and modifications to the present invention without departing from the principles thereof, and such improvements and modifications also fall within the scope of protection of the present invention.

## Claims

1. An amino heteroaryl macrocyclic compound represented by Formula I, or a pharmaceutically acceptable salt, stereoisomer, racemate, tautomer, isotope-labeled derivative, deuterated derivative, N-oxide, prodrug, hydrate, or solvate thereof: 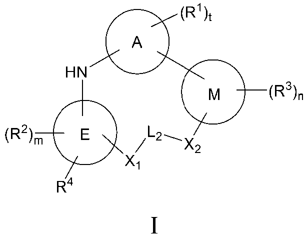 wherein, in Formula I,
ring A is a 5-12 membered heteroaryl, 6-12 membered aryl, or 3-12 membered heterocyclyl, the 5-12 membered heteroaryl, 6-12 membered aryl, or 3-12 membered heterocyclyl is connected to NH and ring M via two different atoms, respectively;
ring E is a 5-12 membered heteroaryl, 6-12 membered aryl, or 3-12 membered heterocyclyl, the 5-12 membered heteroaryl, 6-12 membered aryl, or 3-12 membered heterocyclyl is connected to NH and X₁ via two different atoms, respectively;
ring M is a 5-12 membered heteroaryl, 6-12 membered aryl, 3-12 membered heterocyclyl, 3-12 membered cycloalkyl, or 3-12 membered cycloalkenyl, the 5-12 membered heteroaryl, 6-12 membered aryl, 3-12 membered heterocyclyl, 3-12 membered cycloalkyl, or 3-12 membered cycloalkenyl is connected to ring A and to X₂ via two different atoms;
L₂ is C₁₋₆ alkylene, C₂₋₆ alkenylene, C₂₋₆ alkynylene, C₁₋₆ heteroalkylene containing 1-3 heteroatoms selected from N, O, S, C(=O), S(=O)(=W¹), or C₂₋₆ heteroalkenylene containing 1-3 heteroatoms selected from N, O, S, C(=O), S(=O)(=W¹), the C₁₋₆ alkylene, C₂₋₆ alkenylene, C₂₋₆ alkynylene, C₁₋₆ heteroalkylene containing 1-3 heteroatoms selected from N, O, S, C(=O), S(=O)(=W¹), or C₂₋₆ heteroalkenylene containing 1-3 heteroatoms selected from N, O, S, C(=O), S(=O)(=W¹) is optionally substituted with one or more identical or different R⁵;
X₁ and X₂ are each independently absent or are each independently NR^{1a}, -C(R^{2a})(R^{3a})-, O, S, C(=O), or S(=O)(=W¹);
each R¹ is independently hydrogen, deuterium, halogen, CN, NO₂, C₁₋₆ alkyl, halo C₁₋₆ alkyl, C₁₋₆ alkoxy, C₂₋₆ alkenyl, C₂₋₆ alkynyl, 3-12 membered cycloalkyl, 3-12 membered heterocyclyl, 6-10 membered aryl, or 5-10 membered heteroaryl, the C₁₋₆ alkyl, halo C₁₋₆ alkyl, C₁₋₆ alkoxy, C₂₋₆ alkenyl, C₂₋₆ alkynyl, 3-12 membered cycloalkyl, 3-12 membered heterocyclyl, 6-10 membered aryl, or 5-10 membered heteroaryl is optionally substituted with one or more R';
R² and R³ are each independently hydrogen, deuterium, halogen, CN, NO₂, C₁₋₆ alkyl, C₂-₆ alkenyl, C₂₋₆ alkynyl, C₀₋₆ alkyl-3-12 membered cycloalkyl, C₀₋₆ alkyl-3-12 membered heterocyclyl, C₀₋₆ alkyl-6-10 membered aryl, C₀₋₆ alkyl-5-10 membered heteroaryl, 3-12 membered cycloalkyl-C₁₋₆ alkyl, 3-12 membered heterocyclyl-C₁₋₆ alkyl, 6-10 membered aryl-C₁₋₆ alkyl, 5-10 membered heteroaryl-C₁₋₆ alkyl, OR^{e1}, OC(=O)R^{e1}, OC(=O)NR^{e1}R^{e2}, OS(=O)(=W¹)R^{e1}, OS(=O)(=W¹)NR^{e1}R^{e2}, SR^{e1}, S(=O)(=W¹)R^{e1}, S(=O)(=W¹)NR^{e1}R^{e2}, NR^{e1}R^{e2}, NR^{e1}C(=O)R^{e2}, NR^{e1}S(=O)(=W¹)R^{e2}, NR^{e1}C(=O)OR^{e2}, NR^{e1}C(=O)NR^{e2}, C(=O)R^{e1}, C(=O)NR^{e1}, C(=O)OR^{e1}, PR^{e1}R^{e2}, P(=O)R^{e1}R^{e2}, P(=O)2R^{e1}R^{e2}, P(=O)OR^{e2}, or oxo (=O), the C₁₋₆ alkyl, C₂₋₆ alkenyl, C₂₋₆ alkynyl, C₀₋₆ alkyl-3-12 membered cycloalkyl, C₀₋₆ alkyl-3-12 membered heterocyclyl, C₀₋₆ alkyl-6-10 membered aryl, C₀₋₆ alkyl-5-10 membered heteroaryl, 3-12 membered cycloalkyl-C₁₋₆ alkyl, 3-12 membered heterocyclyl-C₁₋₆ alkyl, 6-10 membered aryl-C₁₋₆ alkyl, and 5-10 membered heteroaryl-C₁₋₆ alkyl are optionally substituted with one or more R';
or, two R² together with the atoms to which they are respectively attached to form a 3-12 membered cycloalkyl, 3-12 membered cycloalkenyl, 3-12 membered heterocyclyl, 5-14 membered heteroaryl, or 6-14 membered aryl, the 3-12 membered cycloalkyl, 3-12 membered cycloalkenyl, 3-12 membered heterocyclyl, 5-14 membered heteroaryl, or 6-14 membered aryl is optionally substituted with one or more R';
R⁴ is R³⁰-S(=O)(W¹)-Y(R²⁰)ₚ-C₀₋₄ alkyl-4-14 membered heterocyclyl, (R³⁰)ₚN-Y(R²⁰)ₚ-C₀₋₄ alkyl-4-14 membered heterocyclyl, R³⁰-S(=O)(W¹)-Y(R²⁰)ₚ-C₀₋₄ alkyl-5-6 membered heteroaryl, or R³⁰-S(=O)(W¹)-Y(R²⁰)ₚ-C₀₋₄ alkyl-4-12 membered cycloalkyl, the R³⁰-S(=O)(W¹)-Y(R²⁰)ₚ-C₀₋₄ alkyl-4-14 membered heterocyclyl, (R³⁰)ₚN₋Y(R²⁰)ₚ-C₀₋₄ alkyl-4-14 membered heterocyclyl, R³⁰-S(=O)(W¹)-Y(R²⁰)ₚ-C₀₋₄ alkyl-5-6 membered heteroaryl, or R³⁰-S(=O)(W¹)-Y(R²⁰)ₚ-C₀₋₄ alkyl-4-12 membered cycloalkyl is optionally substituted with one or more R';
or, R⁴ is 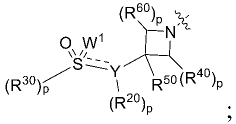
each R⁵ is independently hydrogen, deuterium, halogen, CN, NO₂, C₁₋₆ alkyl, C₂₋₆ alkenyl, C₂₋₆ alkynyl, C₀₋₆ alkyl-3-12 membered cycloalkyl, C₀₋₆ alkyl-3-12 membered heterocyclyl, C₀₋₆ alkyl-6-10 membered aryl, C₀₋₆ alkyl-5-10 membered heteroaryl, 3-12 membered cycloalkyl-C₁₋₆ alkyl, 3-12 membered heterocyclyl-C₁₋₆ alkyl, 6-10 membered aryl-C₁₋₆ alkyl, 5-10 membered heteroaryl-C₁₋₆ alkyl, OR^{e1}, OC(=O)R^{e1}, OC(=O)NR^{e1}R^{e2}, OS(=O)(=W¹)R^{e1}, OS(=O)(=W¹)NR^{e1}R^{e2}, SR^{e1}, S(=O)(=W¹)R^{e1}, S(=O)(=W¹)NR^{e1}R^{e2}, NR^{e1}R^{e2}, NR^{e1}C(=O)R^{e2}, NR^{e1}S(=O)(=W¹)R^{e2}, NR^{e1}C(=O)OR^{e2}, NR^{e1}C(=O)NR^{e2}, C(=O)R^{e1}, C(=O)NR^{e1}, C(=O)OR^{e1}, PR^{e1}R^{e2}, P(=O)R^{e1}R^{e2}, P(=O)₂R^{e1}R^{e2}, P(=O)OR^{e2}, or oxo (=O), the C₁₋₆ alkyl, C₂₋₆ alkenyl, C₂₋₆ alkynyl, C₀₋₆ alkyl-3-12 membered cycloalkyl, C₀₋₆ alkyl-3-12 membered heterocyclyl, C₀₋₆ alkyl-6-10 membered aryl, C₀₋₆ alkyl-5-10 membered heteroaryl, 3-12 membered cycloalkyl-C₁₋₆ alkyl, 3-12 membered heterocyclyl-C₁₋₆ alkyl, 6-10 membered aryl-C₁₋₆ alkyl, and 5-10 membered heteroaryl-C₁₋₆ alkyl are optionally substituted with one or more R';
or, two R⁵ together with the atoms to which they are respectively attached to form a 3-12 membered cycloalkyl, 6-12 membered aryl, 5-10 membered heteroaryl, 3-12 membered cycloalkenyl, or 3-12 membered heterocyclyl, the 3-12 membered cycloalkyl, 6-12 membered aryl, 5-10 membered heteroaryl, 3-12 membered cycloalkenyl, or 3-12 membered heterocyclyl is optionally substituted with one or more R';
R^{1a} and R^{2a} are each independently absent or are each independently hydrogen, deuterium, halogen, C₁₋₆ alkyl, halo C₁₋₆ alkyl, C₂₋₆ alkenyl, C₂₋₆ alkynyl, 3-12 membered cycloalkyl, 3-12 membered heterocyclyl, 6-10 membered aryl, 5-10 membered heteroaryl, S(=O)(=W¹)R^{e1}, S(=O)(=W¹)NR^{e1}R^{e2}, C(=O)R^{e1}, C(=O)NR^{e1}, C(=O)OR^{e1}, S(=O)(=W¹)NR^{e1}, P(=O)R^{e1}R^{e2}, P(=O)₂R^{e1}R^{e2}, or P(=O)OR^{e2}, the C₁₋₆ alkyl, halo C₁₋₆ alkyl, C₂₋₆ alkenyl, C₂₋₆ alkynyl, 3-12 membered cycloalkyl, 3-12 membered heterocyclyl, 6-10 membered aryl, or 5-10 membered heteroaryl is optionally substituted with one or more R';
or, R² and R^{1a} together with the atoms to which they are respectively attached to form a 3-12 membered heterocyclyl or 5-12 membered heteroaryl, the 3-12 membered heterocyclyl or 5-12 membered heteroaryl is optionally substituted with one or more R';
or, R³ and R^{1a} together with the atoms to which they are respectively attached to form a 3-12 membered heterocyclyl or 5-12 membered heteroaryl, the 3-12 membered heterocyclyl or 5-12 membered heteroaryl is optionally substituted with one or more R;
R^{3a} is hydrogen, deuterium, halogen, CN, NO₂, C₁₋₆ alkyl, C₂₋₆ alkenyl, C₂₋₆ alkynyl, 3-12 membered cycloalkyl, 3-12 membered heterocyclyl, 6-10 membered aryl, 5-10 membered heteroaryl, OR^{e1}, OC(=O)R^{e1}, OC(=O)NR^{e1}R^{e2}, OS(=O)(=W¹)R^{e1}, OS(=O)(=W¹)NR^{e1}R^{e2}, SR^{e1}, S(=O)(=W¹)R^{e1}, S(=O)(=W¹)NR^{e1}R^{e2}, NR^{e1}R^{e2}, NR^{e1}C(=O)R^{e2}, NR^{e1}C(=O)OR^{e2}, NR^{e1}C(=O)NR^{e2}, NR^{e1}S(=O)(=W¹)R^{e2}, C(=O)R^{e1}, C(=O)NR^{e1}, C(=O)OR^{e1}, PR^{e1}R^{e2}, P(=O)R^{e1}R^{e2}, P(=O)₂R^{e1}R^{e2}, P(=O)OR^{e2}, or oxo (=O), the C₁₋₆ alkyl, C₂₋₆ alkenyl, C₂₋₆ alkynyl, 3-12 membered cycloalkyl, 3-12 membered heterocyclyl, 6-10 membered aryl, or 5-10 membered heteroaryl is optionally substituted with one or more R';
or, R^{2a} and R^{3a} together with the carbon atom to which they are attached to form a 3-12 membered cycloalkyl or 3-12 membered heterocyclyl, the 3-12 membered cycloalkyl or 3-12 membered heterocyclyl is optionally substituted with one or more R';
or, R² and R^{3a} together with the atoms to which they are respectively attached to form a 3-12 membered cycloalkyl, 3-12 membered heterocyclyl, 6-12 membered aryl, or 5-12 membered heteroaryl, the 3-12 membered cycloalkyl, 3-12 membered heterocyclyl, 6-12 membered aryl, or 5-12 membered heteroaryl is optionally substituted with one or more R';
or, R³ and R^{3a} together with the atoms to which they are respectively attached to form a 3-12 membered cycloalkyl, 3-12 membered heterocyclyl, 6-12 membered aryl, or 5-12 membered heteroaryl, the 3-12 membered cycloalkyl, 3-12 membered heterocyclyl, 6-12 membered aryl, or 5-12 membered heteroaryl is optionally substituted with one or more R';
R' is deuterium, halogen, CN, NO₂, C₁₋₆ alkyl, halo C₁₋₆ alkyl, C₂₋₆ alkenyl, C₂₋₆ alkynyl, 3-12 membered cycloalkyl, 3-12 membered heterocyclyl, 6-10 membered aryl, 5-10 membered heteroaryl, OR^{f1}, OC(=O)R^{f1}, OC(=O)NR^{f1}R^{f2}, OS(=O)(=W¹)R^{f1}, OS(=O)(=W¹)NR^{f1}R^{f2}, SR^{f1}, S(=O)(=W¹)R^{f1}, S(=O)(=W¹)NR^{f1}R^{f2}, NR^{f1}R^{f2}, NR^{f1}C(=O)R^{f2}, NR^{f1}S(=O)(=W¹)R^{f2}, NR^{f1}C(=O)OR^{f2}, NR^{f1}C(=O)NR^{f2}, C(=O)R^{f1}, C(=O)NR^{f1}, C(=O)OR^{f1}, -(O)(OR^{f1})₂, PR^{f1}R^{f2}, P(=O)R^{f1}R^{f2}, P(=O)₂R^{f1}R^{f2}, P(=O)OR^{f2}, or oxo (=O);
R^{e1}, R^{e2}, R^{f1}, and R^{f2} are each independently hydrogen, deuterium, C₁₋₆ alkyl, halo C₁₋₆ alkyl, C₂₋₆ alkenyl, C₂₋₆ alkynyl, 3-12 membered cycloalkyl, 3-12 membered heterocyclyl, 6-10 membered aryl, or 5-10 membered heteroaryl, the C₁₋₆ alkyl, C₂₋₆ alkenyl, C₂₋₆ alkynyl, 3-12 membered cycloalkyl, 3-12 membered heterocyclyl, 6-10 membered aryl, or 5-10 membered heteroaryl is optionally substituted with one or more R";
or, R^{e1} and R^{e2} together with the atoms to which they are attached to form a 3-12 membered heterocyclyl, the 3-12 membered heterocyclyl is optionally substituted with one or more groups selected from: deuterium, halogen, CN, NO₂, C₁₋₆ alkyl, halo C₁₋₆ alkyl, C₂₋₆ alkenyl, C₂₋₆ alkynyl, 3-12 membered cycloalkyl, 3-12 membered heterocyclyl, 6-10 membered aryl, 5-10 membered heteroaryl, and OR^{g1};
or, R^{f1} and R^{f2} together with the atoms to which they are attached to form a 3-12 membered heterocyclyl, the 3-12 membered heterocyclyl is optionally substituted with one or more groups selected from: deuterium, halogen, CN, NO₂, C₁₋₆ alkyl, halo C₁₋₆ alkyl, C₂₋₆ alkenyl, C₂₋₆ alkynyl, 3-12 membered cycloalkyl, 3-12 membered heterocyclyl, 6-10 membered aryl, 5-10 membered heteroaryl, and OR^{g1};
R" is deuterium, halogen, CN, NO₂, C₁₋₆ alkyl, halo C₁₋₆ alkyl, C₂₋₆ alkenyl, C₂₋₆ alkynyl, 3-12 membered cycloalkyl, 3-12 membered heterocyclyl, 6-10 membered aryl, 5-10 membered heteroaryl, OR^{g1}, OC(=O)R^{g1}, OC(=O)NR^{g1}R^{g2}, OS(=O)(=W¹)R^{g1}, OS(=O)(=W¹)NR^{g1}R^{g2}, SR^{e1}, S(=O)(=W¹)R^{g1}, S(=O)(=W¹)NR^{g1}R^{g2}, NR^{g1}R^{g2}, NR^{g1}C(=O)R^{g2}, NR^{g1}S(=O)(=W¹)R^{g2}, NR^{g1}C(=O)OR^{g2}, NR^{g1}C(=O)NR^{g2}, C(=O)R^{g1}, C(=O)NR^{g1}, C(=O)OR^{g1}, -(O)(OR^{g1})₂, PR^{g1}R^{g2}, P(=O)R^{g1}R^{g2}, P(=O)₂R^{g1}R^{g2}, P(=O)OR^{g2}, or oxo (=O);
R^{g1} and R^{g2} are each independently hydrogen, deuterium, C₁₋₆ alkyl, halo C₁₋₆ alkyl, C₂₋₆ alkenyl, or 3-12 membered cycloalkyl;
W¹ is absent, O, or NR^{g1};
Y is N or C;
- - - represents a single bond or a double bond;
R³⁰ is hydrogen, deuterium, C₁₋₆ alkyl, halo C₁₋₆ alkyl, amino, C₁₋₆ alkylamino, di(C₁₋₆ alkyl)amino, C₃₋₆ cycloalkylamino, C₂₋₆ alkenyl, C₂₋₆ alkynyl, C₀₋₆ alkyl-3-12 membered cycloalkyl, or C₀₋₆ alkyl-3-12 membered heterocyclyl, the C₁₋₆ alkyl, halo C₁₋₆ alkyl, C₂₋₆ alkenyl, C₂₋₆ alkynyl, C₀₋₆ alkyl-3-12 membered cycloalkyl, and C₀₋₆ alkyl-3-12 membered heterocyclyl are optionally substituted with one or more R'; or, two R³⁰ together with the atoms to which they are respectively attached to form a 3-6 membered cycloalkyl or 3-7 membered heterocyclyl, the 3-6 membered cycloalkyl or 3-7 membered heterocyclyl is optionally substituted with one or more R';
R²⁰, R⁴⁰, R⁵⁰, and R⁶⁰ are each independently hydrogen, deuterium, halogen, CN, amino, hydroxyl, C₁₋₆ alkyl, halo C₁₋₆ alkyl, C₂₋₆ alkenyl, C₂₋₆ alkynyl, C₁₋₆ alkoxy, C₁₋₆ alkylamino, di(C₁₋₆ alkyl)amino, C₀₋₆ alkyl-3-12 membered cycloalkyl, C₀₋₆ alkyl-3-12 membered heterocyclyl, or oxo (=O), the C₁₋₆ alkyl, halo C₁₋₆ alkyl, C₂₋₆ alkenyl, C₂₋₆ alkynyl, C₁₋₆ alkoxy, C₁₋₆ alkylamino, di(C₁₋₆ alkyl)amino, C₀₋₆ alkyl-3-12 membered cycloalkyl, and C₀₋₆ alkyl-3-12 membered heterocyclyl are optionally substituted with one or more R'; or, two R²⁰ together with the atoms to which they are respectively attached to form a 3-6 membered cycloalkyl or 3-7 membered heterocyclyl, the 3-6 membered cycloalkyl or 3-7 membered heterocyclyl is optionally substituted with one or more R';
or, R²⁰ and R⁵⁰ together with the atoms to which they are respectively attached to form a 3-6 membered cycloalkyl or 3-7 membered heterocyclyl, the 3-6 membered cycloalkyl or 3-7 membered heterocyclyl is optionally substituted with one or more R';
or, R⁴⁰ and R⁶⁰ together with the atoms to which they are respectively attached to form a 3-6 membered cycloalkyl or 3-7 membered heterocyclyl, the 3-6 membered cycloalkyl or 3-7 membered heterocyclyl is optionally substituted with one or more R';
m, n, and t are each independently 0, 1, 2, 3, or 4;
p is each independently 1 or 2.

2. The amino heteroaryl macrocyclic compound or a pharmaceutically acceptable salt, stereoisomer, racemate, tautomer, isotope-labeled derivative, deuterated derivative, N-oxide, prodrug, hydrate, or solvate thereof according to claim 1, wherein, in Formula I, ring A is a 5-6 membered heteroaryl;
preferably, ring A is pyridine, pyrimidine, pyridazine, pyrazine, triazine, or thiazole;
more preferably, ring A is selected from the following groups: 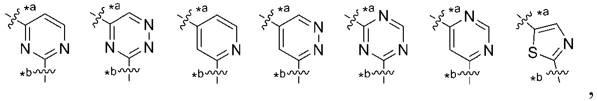 wherein *^{a} indicates the connection site to NH, and *^{b} indicates the connection site to ring M;
most preferably, ring A is selected from the following groups: 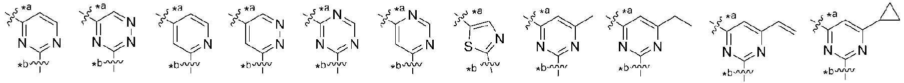 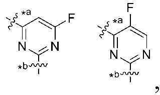 wherein *^{a} indicates the connection site to the N atom of NH, and *^{b} indicates the connection site to ring M;
further optionally, in Formula I, ring E is a 9-10 membered heteroaryl, the 9-10 membered heteroaryl is connected to NH and X₁ via two different atoms, respectively;
preferably, ring E is 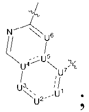
more preferably, ring E is 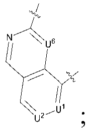
most preferably, ring E is selected from 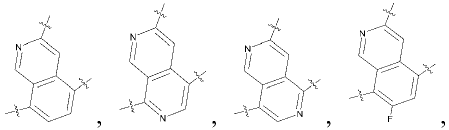 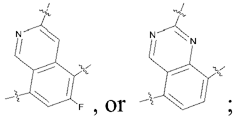
wherein --- or -- indicates absent or a single bond;
U¹ is absent, or is N, CH, or C;
U² is absent, or is N, CH, or C;
U³ is N, C, NR¹⁰⁰, O, S, or C(=O);
U⁴ and U⁵ are each independently N or C, and U⁴ and U⁵ are not both N simultaneously;
U⁶ is N or CH;
U⁷ is C, N, NR²⁰⁰, O, S, C(=O), C(=O)O, C(=O)NR³⁰⁰, OC(=O), or NR³⁰⁰C(=O);
R¹⁰⁰, R²⁰⁰, or R³⁰⁰ are each independently hydrogen, deuterium, C₁₋₆ alkyl, halo C₁₋₆ alkyl, C₀₋₆ alkyl-3-12 membered cycloalkyl, or C₀₋₆ alkyl-3-12 membered heterocyclyl;
when U¹ is absent, U² and U⁷ are connected by a single bond or a double bond; when U² is absent, U¹ and U³ are connected by a single bond or a double bond;
when U¹ and U² are N or CH, at most three of U¹, U², U³, U⁴, U⁵, and U⁷ are simultaneously N.

3. The amino heteroaryl macrocyclic compound or a pharmaceutically acceptable salt, stereoisomer, racemate, tautomer, isotope-labeled derivative, deuterated derivative, N-oxide, prodrug, hydrate, or solvate thereof according to claim 1 or 2, wherein, in Formula I, ring M is a 5-6 membered heteroaryl or benzene;
preferably, ring M is pyridine, pyrimidine, pyridazine, imidazole, pyrazole, thiazole, oxazole, triazole, benzene, or pyridine N-oxide;
more preferably, ring M is selected from the following groups: 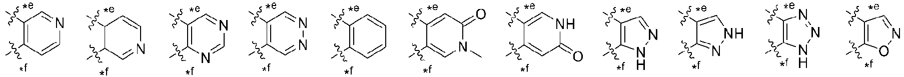 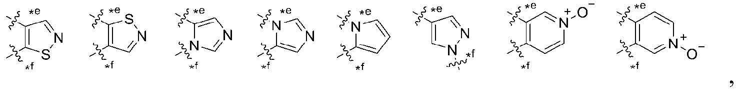 wherein *^{e} indicates the connection site to ring A, and *^{f} indicates the connection site to X₂;
most preferably, ring M is selected from the following groups: 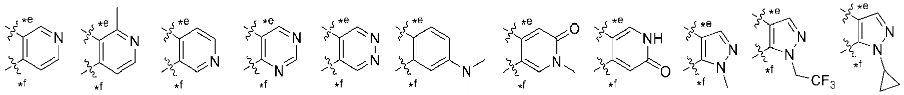 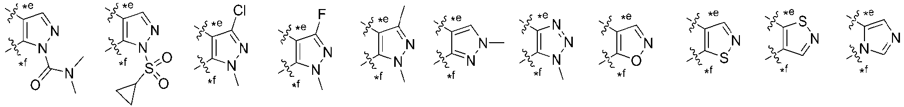 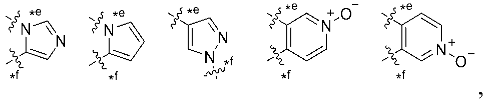 wherein *^{e} indicates the connection site to ring A, and *^{f} indicates the connection site to X₂.

4. The amino heteroaryl macrocyclic compound or a pharmaceutically acceptable salt, stereoisomer, racemate, tautomer, isotope-labeled derivative, deuterated derivative, N-oxide, prodrug, hydrate, or solvate thereof according to any one of claims 1 to 3, wherein, in Formula I, L₂ is C₁₋₆ alkylene, C₁₋₆ alkenylene, or C₁₋₆ heteroalkylene containing 1-3 heteroatoms selected from N, O, S, C(=O), and S(=O)(=W¹), the C₁₋₆ alkylene, C₁₋₆ alkenylene, or C₁₋₆ heteroalkylene containing 1-3 heteroatoms selected from N, O, S, C(=O), and S(=O)(=W¹) is optionally substituted with one or more identical or different R⁵;
preferably, L₂ is methylene, ethylene, propylene, butylene, pentylene, vinylene, propenylene, butenylene, or ethyleneoxy, the methylene, ethylene, propylene, butylene, pentylene, vinylene, propenylene, butenylene, or ethyleneoxy is optionally substituted with one or more identical or different R⁵;
wherein R⁵ is hydrogen, deuterium, halogen, CN, C₁₋₆ alkyl, C₀₋₂ alkyl-3-12 membered cycloalkyl, C₀₋₂ alkyl-3-12 membered heterocyclyl, C₁₋₆ alkoxy, or oxo, the C₁₋₆ alkyl, C₀₋₂ alkyl-3-12 membered cycloalkyl, C₀₋₂ alkyl-3-12 membered heterocyclyl, or C₁₋₆ alkoxy is optionally substituted with one or more R';
or, two R⁵ together with the atoms to which they are respectively attached to form a 3-6 membered cycloalkyl, phenyl, 4-7 membered heterocyclyl, 5-6 membered heterocyclyl, or 3-6 membered cycloalkenyl, the 3-6 membered cycloalkyl, phenyl, 4-7 membered heterocyclyl, 5-6 membered heterocyclyl, or 3-6 membered cycloalkenyl is optionally substituted with one or more R';
preferably, R⁵ is hydrogen, deuterium, halogen, CN, hydroxyl, hydroxyethyl, amino, methylamino, dimethylamino, cyclopropylamino, methyl, ethyl, difluoromethyl, trifluoromethyl, difluoroethyl, trifluoroethyl, isopropyl, cyclopropyl, methoxy, ethoxy, difluoromethoxy, trifluoromethoxy, difluoroethoxy, trifluoroethoxy, cyclopropoxy, or oxo;
or, two R⁵ together with the atoms to which they are respectively attached to form a cyclopropyl, cyclobutyl, cyclopentyl, cyclohexyl, oxiranyl, oxetanyl, tetrahydrofuranyl, pyranyl, azetidinyl, or piperidinyl, the cyclopropyl, cyclobutyl, cyclopentyl, cyclohexyl, oxiranyl, oxetanyl, tetrahydrofuranyl, pyranyl, azetidinyl, or piperidine is optionally substituted with one or more R';
more preferably, R⁵ is deuterium, halogen, methyl, ethyl, difluoromethyl, trifluoromethyl, difluoroethyl, trifluoroethyl, cyclopropyl, or oxo;
or, two R⁵ together with the atoms to which they are respectively attached to form a cyclopropyl, cyclobutyl, cyclopentyl, or oxetanyl, the cyclopropyl, cyclobutyl, cyclopentyl, or oxetanyl is optionally substituted with one or more deuterium, halogen, methyl, ethyl, or cyclopropyl;
more preferably, L₂ is 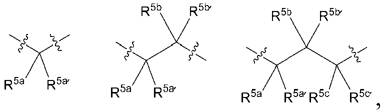 wherein R^{5a}, R^{5b}, R^{5c}, R^{5a'}, R^{5b'}, and R^{5c'} are each independently R⁵; preferably, R^{5a}, R^{5b}, R^{5c}, R^{5a'}, R^{5b'}, and R^{5c'} are each independently hydrogen, deuterium, halogen, CN, hydroxyl, C₁₋₆ alkyl, C₂₋₄ alkenyl, C₂₋₄ alkynyl, C₁₋₆ alkylamino, di(C₁₋₆ alkyl)amino, 3-6 membered cycloalkylamino, C₀₋₂ alkyl-3-6 membered cycloalkyl, C₀₋₂ alkyl-3-7 membered heterocyclyl, C₁₋₆ alkoxy, or oxo, the C₁₋₆ alkyl, C₂₋₄ alkenyl, C₂₋₄ alkynyl, C₁₋₆ alkylamino, di(C₁₋₆ alkyl)amino, 3-6 membered cycloalkylamino, C₀₋₂ alkyl-3-6 membered cycloalkyl, C₀₋₂ alkyl-3-7 membered heterocyclyl, or C₁₋₆ alkoxy is optionally substituted with one or more hydroxyl, amino, cyano, halogen, C₁₋₄ alkyl, 3-6 membered cycloalkyl, or oxo;
or, R^{5a} and R^{5a'} together with the atoms to which they are attached to form a 3-6 membered cycloalkyl, 3-7 membered heterocyclyl, or 3-6 membered cycloalkenyl, the 3-6 membered cycloalkyl, 3-7 membered heterocyclyl, or 3-6 membered cycloalkenyl is optionally substituted with one or more R';
or, R^{5b} and R^{5b'} together with the atoms to which they are attached to form a 3-6 membered cycloalkyl, 3-7 membered heterocyclyl, or 3-6 membered cycloalkenyl, the 3-6 membered cycloalkyl, 3-7 membered heterocyclyl, or 3-6 membered cycloalkenyl is optionally substituted with one or more R';
or, R^{5c} and R^{5c'} together with the atoms to which they are attached to form a 3-6 membered cycloalkyl, 3-7 membered heterocyclyl, or 3-6 membered cycloalkenyl, the 3-6 membered cycloalkyl, 3-7 membered heterocyclyl, or 3-6 membered cycloalkenyl is optionally substituted with one or more R';
or, R^{5a} and R^{5b} together with the atoms to which they are attached to form a 3-6 membered cycloalkyl, 3-7 membered heterocyclyl, or 3-6 membered cycloalkenyl, the 3-6 membered cycloalkyl, 3-7 membered heterocyclyl, or 3-6 membered cycloalkenyl is optionally substituted with one or more R';
or, R^{5a} and R^{5c} together with the atoms to which they are attached to form a 3-6 membered cycloalkyl, 3-7 membered heterocyclyl, or 3-6 membered cycloalkenyl, the 3-6 membered cycloalkyl, 3-7 membered heterocyclyl, or 3-6 membered cycloalkenyl is optionally substituted with one or more R';
or, R^{5b} and R^{5c} together with the atoms to which they are attached to form a 3-6 membered cycloalkyl, 3-7 membered heterocyclyl, or 3-6 membered cycloalkenyl, the 3-6 membered cycloalkyl, 3-7 membered heterocyclyl, or 3-6 membered cycloalkenyl is optionally substituted with one or more R';
more preferably, R^{5a}, R^{5b}, R^{5c}, R^{5a'}, R^{5b'}, and R^{5c'} are each independently hydrogen, deuterium, F, Cl, Br, CN, hydroxyl, hydroxyethyl, amino, methylamino, dimethylamino, cyclopropylamino, methyl, ethyl, difluoromethyl, trifluoromethyl, difluoroethyl, trifluoroethyl, isopropyl, cyclopropyl, methoxy, ethoxy, difluoromethoxy, trifluoromethoxy, difluoroethoxy, trifluoroethoxy, cyclopropoxy, or oxo;
or, R^{5a} and R^{5a'} together with the atoms to which they are attached to form a cyclopropyl, cyclobutyl, cyclopentyl, cyclohexyl, oxiranyl, oxetanyl, tetrahydrofuranyl, pyranyl, azetidinyl, piperidinyl, or morpholinyl, the cyclopropyl, cyclobutyl, cyclopentyl, cyclohexyl, oxiranyl, oxetanyl, tetrahydrofuranyl, pyranyl, azetidinyl, piperidinyl, or morpholinyl is optionally substituted with one or more R';
or, R^{5b} and R^{5b'} together with the atoms to which they are attached to form a cyclopropyl, cyclobutyl, cyclopentyl, cyclohexyl, oxiranyl, oxetanyl, tetrahydrofuranyl, pyranyl, azetidinyl, piperidinyl, or morpholinyl, the cyclopropyl, cyclobutyl, cyclopentyl, cyclohexyl, oxiranyl, oxetanyl, tetrahydrofuranyl, pyranyl, azetidinyl, piperidinyl, or morpholinyl is optionally substituted with one or more R';
or, R^{5c} and R^{5c'} together with the atoms to which they are attached to form a cyclopropyl, cyclobutyl, cyclopentyl, cyclohexyl, oxiranyl, oxetanyl, tetrahydrofuranyl, pyranyl, azetidinyl, piperidinyl, or morpholinyl, the cyclopropyl, cyclobutyl, cyclopentyl, cyclohexyl, oxiranyl, oxetanyl, tetrahydrofuranyl, pyranyl, azetidinyl, piperidinyl, or morpholinyl is optionally substituted with one or more R';
or, R^{5a} and R^{5b} together with the atoms to which they are attached to form a cyclopropyl, cyclobutyl, cyclopentyl, cyclohexyl, oxiranyl, oxetanyl, tetrahydrofuranyl, pyranyl, azetidinyl, piperidinyl, or morpholinyl, the cyclopropyl, cyclobutyl, cyclopentyl, cyclohexyl, oxiranyl, oxetanyl, tetrahydrofuranyl, pyranyl, azetidinyl, piperidinyl, or morpholinyl is optionally substituted with one or more R';
or, R^{5a} and R^{5c} together with the atoms to which they are attached to form a cyclopropyl, cyclobutyl, cyclopentyl, cyclohexyl, oxiranyl, oxetanyl, tetrahydrofuranyl, pyranyl, azetidinyl, piperidinyl, or morpholinyl, the cyclopropyl, cyclobutyl, cyclopentyl, cyclohexyl, oxiranyl, oxetanyl, tetrahydrofuranyl, pyranyl, azetidinyl, piperidinyl, or morpholinyl is optionally substituted with one or more R';
or, R^{5b} and R^{5c} together with the atoms to which they are attached to form a cyclopropyl, cyclobutyl, cyclopentyl, cyclohexyl, oxiranyl, oxetanyl, tetrahydrofuranyl, pyranyl, azetidine, piperidinyl, or morpholinyl, the cyclopropyl, cyclobutyl, cyclopentyl, cyclohexyl, oxiranyl, oxetanyl, tetrahydrofuranyl, pyranyl, azetidinyl, piperidinyl, or morpholinyl is optionally substituted with one or more R';
wherein R' is deuterium, halogen, CN, hydroxyl, hydroxyethyl, amino, methylamino, dimethylamino, cyclopropylamino, methyl, ethyl, isopropyl, difluoromethyl, trifluoromethyl, difluoroethyl, trifluoroethyl, cyclopropyl, methoxy, ethoxy, difluoromethoxy, trifluoromethoxy, difluoroethoxy, trifluoroethoxy, cyclopropoxy, or oxo.

5. The amino heteroaryl macrocyclic compound or pharmaceutically acceptable salt, stereoisomer, racemate, tautomer, isotope-labeled derivative, deuterated derivative, N-oxide, prodrug, hydrate, or solvate thereof according to any one of claims 1 to 4, wherein, in Formula I, each R¹ is independently hydrogen, deuterium, halogen, CN, C₁₋₆ alkyl, halo C₁₋₆ alkyl, C₁₋₆ alkoxy, C₂₋₄ alkenyl, C₂₋₄ alkynyl, 3-6 membered cycloalkyl, or oxo, the C₁₋₆ alkyl, halo C₁₋₆ alkyl, C₁₋₆ alkoxy, C₂₋₄ alkenyl, C₂₋₄ alkynyl, and 3-6 membered cycloalkyl are optionally substituted with one or more R';
preferably, each R¹ is independently hydrogen, deuterium, halogen, CN, hydroxyl, hydroxyethyl, amino, methylamino, dimethylamino, ethylamino, diethylamino, cyclopropylamino, methyl, ethyl, isopropyl, vinyl, ethynyl, difluoromethyl, trifluoromethyl, difluoroethyl, trifluoroethyl, cyclopropyl, methoxy, ethoxy, cyclopropoxy, or oxo;
preferably, each R² is independently hydrogen, deuterium, halogen, CN, C₁₋₆ alkyl, halo C₁₋₆ alkyl, C₂₋₆ alkenyl, C₂₋₆ alkynyl, C₁₋₆ alkoxy, C₁₋₆ alkoxy-C₁₋₆ alkyl, amino, C₁₋₄ alkylamino, di(C₁₋₄ alkyl)amino, C₀₋₆ alkyl-3-12 membered cycloalkyl, C₀₋₆ alkyl-3-12 membered heterocyclyl, 3-12 membered cycloalkyl-C₁₋₆ alkyl, 3-12 membered heterocyclyl-C₁₋₆ alkyl, or oxo (=O), the C₁₋₆ alkyl, halo C₁₋₆ alkyl, C₂₋₆ alkenyl, C₂₋₆ alkynyl, C₁₋₆ alkoxy, C₁₋₆ alkoxy-C₁₋₆ alkyl, amino, C₁₋₄ alkylamino, di(C₁₋₄ alkyl)amino, C₀₋₆ alkyl-3-12 membered cycloalkyl, C₀₋₆ alkyl-3-12 membered heterocyclyl, 3-12 membered cycloalkyl-C₁₋₆ alkyl, and 3-12 membered heterocyclyl-C₁₋₆ alkyl are optionally substituted with one or more deuterium, halogen, hydroxyl, methyl, ethyl, methoxy, ethoxy, or cyclopropyl;
preferably, each R² is independently hydrogen, deuterium, halogen, CN, methyl, ethyl, isopropyl, vinyl, ethynyl, difluoromethyl, trifluoromethyl, methoxy, ethoxy, isopropoxy, amino, methylamino, dimethylamino, ethylamino, or diethylamino, the methyl, ethyl, isopropyl, vinyl, ethynyl, difluoromethyl, methoxy, ethoxy, isopropoxy, amino, methylamino, dimethylamino, ethylamino, or diethylamino is optionally substituted with one or more deuterium, halogen, hydroxyl, methoxy, ethoxy, or cyclopropyl;
preferably, each R³ is independently hydrogen, deuterium, halogen, CN, C₁₋₆ alkyl, halo C₁₋₆ alkyl, C₂₋₄ alkenyl, C₂₋₄ alkynyl, C₁₋₆ alkoxy, C₁₋₆ alkoxy-C₁₋₆ alkyl, amino, C₁₋₄ alkylamino, di(C₁₋₄ alkyl)amino, C₀₋₆ alkyl-3-6 membered cycloalkyl, C₀₋₆ alkyl-3-6 membered heterocyclyl, 3-6 membered cycloalkyl-C₁₋₆ alkyl, 3-6 membered heterocyclyl-C₁₋₆ alkyl, oxo (=O), C(=O)R^{e1}, C(=O)NR^{e1}, or S(=O)₂R^{e1}, the C₁₋₆ alkyl, halo C₁₋₆ alkyl, C₂₋₄ alkenyl, C₂₋₄ alkynyl, C₁₋₆ alkoxy, C₁₋₆ alkoxy-C₁₋₆ alkyl, amino, C₁₋₄ alkylamino, di(C₁₋₄ alkyl)amino, C₀₋₆ alkyl-3-6 membered cycloalkyl, C₀₋₆ alkyl-3-6 membered heterocyclyl, 3-6 membered cycloalkyl-C₁₋₆ alkyl, or 3-6 membered heterocyclyl-C₁₋₆ alkyl is optionally substituted with one or more deuterium, F, Cl, hydroxyl, methyl, ethyl, methoxy, ethoxy, or cyclopropyl;
preferably, each R³ is independently hydrogen, deuterium, halogen, CN, hydroxyl, hydroxyethyl, amino, methylamino, ethylamino, dimethylamino, diethylamino, cyclopropylamino, methyl, ethyl, isopropyl, difluoromethyl, trifluoromethyl, difluoroethyl, trifluoroethyl, cyclopropyl, methoxy, ethoxy, cyclopropoxy, oxo, C(=O)R^{e1}, C(=O)NR^{e1}, or S(=O)₂R^{e1}; wherein R^{e1} is hydrogen, methyl, ethyl, amino, methylamino, ethylamino, dimethylamino, diethylamino, cyclopropylamino, cyclopropyl, cyclobutyl, cyclopentyl, or cyclohexyl;
more preferably, each R¹ is independently hydrogen, deuterium, halogen, methyl, ethyl, or cyclopropyl;
more preferably, each R² is independently hydrogen, deuterium, halogen, CN, methyl, ethyl, difluoromethyl, trifluoromethyl, or methoxy, the methyl, ethyl, or methoxy is optionally substituted with one or more halogen;
more preferably, R³ is hydrogen, deuterium, halogen, methyl, ethyl, cyclopropyl, dimethylamino, C(=O)CH₃, C(=O)-cyclopropyl, C(=O)N(CH₃)₂, or S(=O)₂-cyclopropyl.

6. The amino heteroaryl macrocyclic compound or pharmaceutically acceptable salt, stereoisomer, racemate, tautomer, isotope-labeled derivative, deuterated derivative, N-oxide, prodrug, hydrate, or solvate thereof according to any one of claims 1 to 5, wherein, in Formula I, X₁ and X₂ are each independently absent or are each independently NR^{1a}, -C(R^{2a})(R^{3a})-, O, C(=O), S(=O), or S(=O)₂;
preferably, X₁ is absent or is NR^{1a}, -C(R^{2a})(R^{3a})-, or O;
preferably, X₂ is -C(R^{2a})(R^{3a})-, O, C(=O), S(=O), or S(=O)₂;
wherein R^{1a} and R^{2a} are each independently hydrogen, deuterium, halogen, C₁₋₆ alkyl, halo C₁₋₆ alkyl, or 3-12 membered cycloalkyl, the C₁₋₆ alkyl, halo C₁₋₆ alkyl, or 3-12 membered cycloalkyl is optionally substituted with one or more R';
R^{3a} is hydrogen, deuterium, halogen, CN, C₁₋₆ alkyl, hydroxyl, hydroxy C₁₋₆ alkyl, C₁₋₆ alkoxy, amino, C₁₋₆ alkylamino, or di(C₁₋₆ alkyl)amino, the C₁₋₆ alkyl, hydroxy C₁₋₆ alkyl, C₁₋₆ alkoxy, C₁₋₆ alkylamino, or di(C₁₋₆ alkyl)amino is optionally substituted with one or more R';
preferably, R^{1a} and R^{2a} are each independently hydrogen, deuterium, F, Cl, methyl, ethyl, isopropyl, cyclopropyl, cyclobutyl, cyclopentyl, cyclohexyl, difluoromethyl, trifluoromethyl, difluoroethyl, trifluoroethyl, or hydroxyethyl;
or, R³ and R^{1a} together with the atoms to which they are attached to form pyrrole, imidazole, thiazole, oxazole, isothiazole, isoxazole, or pyrazole;
preferably, R^{3a} is hydrogen, F, Cl, Br, methyl, ethyl, isopropyl, cyclopropyl, cyclobutyl, cyclopentyl, cyclohexyl, difluoromethyl, trifluoromethyl, difluoroethyl, trifluoroethyl, hydroxyethyl, amino, methylamino, dimethylamino, methoxy, ethoxy, or isopropoxy;
or, R^{2a} and R^{3a} together with the carbon atom to which they are attached to form a 3-6 membered cycloalkyl or a 3-6 membered heterocyclyl, the 3-6 membered cycloalkyl or 3-6 membered heterocyclyl is optionally substituted with one or more R';
or, R^{2a} and R^{3a} together with the carbon atom to which they are attached to form a cyclopropyl, cyclobutyl, cyclopentyl, cyclohexyl, oxiranyl, or oxetanyl, the cyclopropyl, cyclobutyl, cyclopentyl, cyclohexyl, oxiranyl, or oxetanyl is optionally substituted with one or more R';
or, R^{2a} and R^{3a} together with the carbon atom to which they are attached to form cyclopropyl, cyclobutyl, oxiranyl, or oxetanyl, the cyclopropyl, cyclobutyl, oxiranyl, or oxetanyl is optionally substituted with one or more halogen, methyl, or hydroxy.

7. The amino heteroaryl macrocyclic compound or pharmaceutically acceptable salt, stereoisomer, racemate, tautomer, isotope-labeled derivative, deuterated derivative, N-oxide, prodrug, hydrate, or solvate thereof according to any one of claims 1 to 6, wherein, in Formula I,
R⁴ is R³⁰-S(=O)(W¹)-Y(R²⁰)ₚ-C₀₋₄ alkyl-4-7 membered heterocyclyl, (R³⁰)ₚN-Y(R²⁰)ₚ-C₀₋₄ alkyl-4-7 membered heterocyclyl, R³⁰-S(=O)(W¹)-Y(R²⁰)ₚ-C₀₋₄ alkyl-5-6 membered heteroaryl, or R³⁰-S(=O)(W¹)-Y(R²⁰)ₚ-C₀₋₄ alkyl-4-7 membered cycloalkyl, the R³⁰-S(=O)(W¹)-Y(R²⁰)ₚ-C₀₋₄ alkyl-4-7 membered heterocyclyl, (R³⁰)ₚN-Y(R²⁰)ₚ-C₀₋₄ alkyl-4-7 membered heterocyclyl, R³⁰-S(=O)(W¹)-Y(R²⁰)ₚ-C₀₋₄ alkyl-5-6 membered heteroaryl, or R³⁰-S(=O)(W¹)-Y(R²⁰)ₚ-C₀₋₄ alkyl-4-7 membered cycloalkyl is optionally substituted with one or more R'; or, R⁴ is 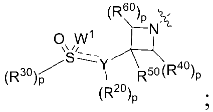
preferably, R⁴ is selected from 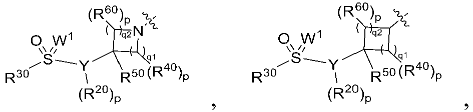 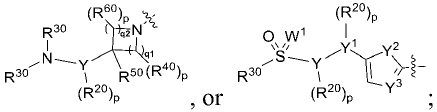
more preferably, R⁴ is 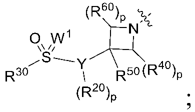
most preferably, R⁴ is selected from 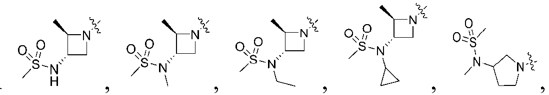 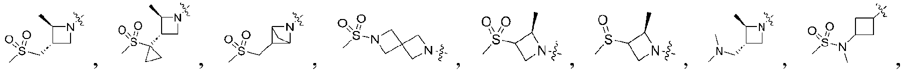 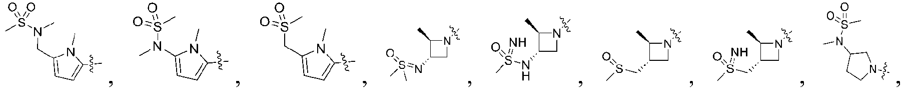 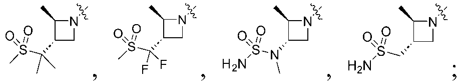
wherein, W¹ is absent, O, or NH;
Y is N or C;
Y¹ is absent or is N or C;
Y² and Y³ are each independently NR⁷⁰, N, CR⁷⁰, O, or S;
each R⁷⁰ is independently hydrogen, deuterium, amino, C₁₋₆ alkyl, halo C₁₋₆ alkyl, C₀₋₂ alkyl-3-12 membered cycloalkyl, or C₀₋₂ alkyl-3-12 membered heterocyclyl, the C₁₋₆ alkyl, halo C₁₋₆ alkyl, C₀₋₂ alkyl-3-12 membered cycloalkyl, or C₀₋₂ alkyl-3-12 membered heterocyclyl is optionally substituted with one or more deuterium, CN, hydroxyl, or halogen;
q1 or q2 are each independently 1, 2, or 3;
further optionally, each R³⁰ is independently hydrogen, deuterium, amino, C₁₋₆ alkyl, halo C₁₋₆ alkyl, C₀₋₂ alkyl-3-12 membered cycloalkyl, or C₀₋₂ alkyl-3-12 membered heterocyclyl, the C₁₋₆ alkyl, halo C₁₋₆ alkyl, C₀₋₂ alkyl-3-12 membered cycloalkyl, or C₀₋₂ alkyl-3-12 membered heterocyclyl is optionally substituted with one or more deuterium, CN, hydroxyl, or halogen;
preferably, each R³⁰ is independently hydrogen, amino, methyl, ethyl, isopropyl, cyclopropyl, cyclobutyl, cyclopentyl, cyclohexyl, difluoromethyl, trifluoromethyl, difluoroethyl, trifluoroethyl, or hydroxyethyl, the amino, methyl, ethyl, isopropyl, cyclopropyl, cyclobutyl, cyclopentyl, cyclohexyl, difluoromethyl, difluoroethyl, trifluoroethyl, or hydroxyethyl is optionally substituted with one or more halogen or deuterium;
or, two R³⁰ together with the atoms to which they are attached to form an azetidinyl, azepanyl, or azocanyl, the azetidinyl, azepanyl, or azocanyl is optionally substituted with one or more methyl, ethyl, isopropyl, cyclopropyl, difluoromethyl, difluoroethyl, trifluoromethyl, trifluoroethyl, or halogen;
preferably, R²⁰, R⁴⁰, R⁵⁰, and R⁶⁰ are each independently hydrogen, deuterium, halogen, CN, amino, hydroxyl, C₁₋₆ alkyl, halo C₁₋₆ alkyl, C₁₋₆ alkoxy, C₁₋₆ alkylamino, di(C₁₋₆ alkyl)amino, C₀₋₂ alkyl-3-12 membered cycloalkyl, C₀₋₂ alkyl-3-12 membered heterocyclyl, or oxo (=O), the C₁₋₆ alkyl, halo C₁₋₆ alkyl, C₁₋₆ alkoxy, C₁₋₆ alkylamino, di(C₁₋₆ alkyl)amino, C₀₋₂ alkyl-3-12 membered cycloalkyl, or C₀₋₂ alkyl-3-12 membered heterocyclyl is optionally substituted with one or more deuterium, hydroxyl, C₁₋₃ alkyl, or halogen;
preferably, R²⁰, R⁴⁰, R⁵⁰, and R⁶⁰ are each independently hydrogen, deuterium, F, Cl, Br, CN, hydroxyl, amino, methyl, ethyl, isopropyl, cyclopropyl, cyclobutyl, cyclopentyl, cyclohexyl, difluoromethyl, trifluoromethyl, difluoroethyl, trifluoroethyl, hydroxyethyl, methylamino, dimethylamino, ethylamino, diethylamino, methoxy, ethoxy, or oxo, the amino, methyl, ethyl, isopropyl, cyclopropyl, cyclobutyl, cyclopentyl, cyclohexyl, difluoroethyl, trifluoroethyl, hydroxyethyl, methylamino, dimethylamino, ethylamino, diethylamino, methoxy, or ethoxy is optionally substituted with one or more halogen, methyl, or deuterium;
or, two R²⁰ together with the atoms to which they are respectively attached to form a cyclopropyl, cyclobutyl, cyclopentyl, or cyclohexyl, the cyclopropyl, cyclobutyl, cyclopentyl, or cyclohexyl is optionally substituted with one or more methyl, ethyl, isopropyl, cyclopropyl, difluoromethyl, difluoroethyl, trifluoromethyl, trifluoroethyl, or halogen;
or, R²⁰ and R⁵⁰ together with the atoms to which they are respectively attached to form an azetidinyl, azepanyl, azocanyl, cyclobutyl, cyclopentyl, or cyclohexyl, the azetidinyl, azepanyl, azocanyl, cyclobutyl, cyclopentyl, or cyclohexyl is optionally substituted with one or more methyl, ethyl, isopropyl, cyclopropyl, difluoromethyl, difluoroethyl, trifluoromethyl, trifluoroethyl, halogen, or cyano;
or, R⁴⁰ and R⁶⁰ together with the atoms to which they are respectively attached to form an azetidinyl, azepanyl, or azocanyl, the azetidinyl, azepanyl, or azocanyl is optionally substituted with one or more methyl, ethyl, isopropyl, cyclopropyl, difluoromethyl, difluoroethyl, trifluoromethyl, trifluoroethyl, halogen, or cyano;
more preferably, each R³⁰ is independently hydrogen, amino, methyl, ethyl, isopropyl, cyclopropyl, difluoromethyl, trifluoromethyl, difluoroethyl, trifluoroethyl, or hydroxyethyl;
more preferably, R²⁰, R⁴⁰, R⁵⁰, and R⁶⁰ are each independently hydrogen, deuterium, F, Cl, Br, CN, hydroxyl, amino, methyl, ethyl, isopropyl, difluoromethyl, trifluoromethyl, deuterated methyl, or oxo.

8. The amino heteroaryl macrocyclic compound or pharmaceutically acceptable salt, stereoisomer, racemate, tautomer, isotope-labeled derivative, deuterated derivative, N-oxide, prodrug, hydrate, or solvate thereof according to any one of claims 1 to 7, the compound is selected from a structural formula represented by Formula II, Formula III, Formula IV, Formula V, Formula VI, or Formula VII 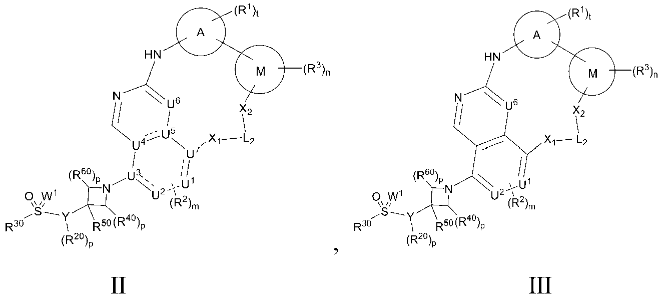 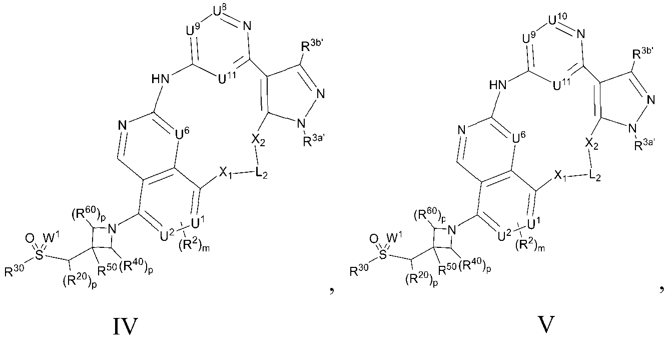 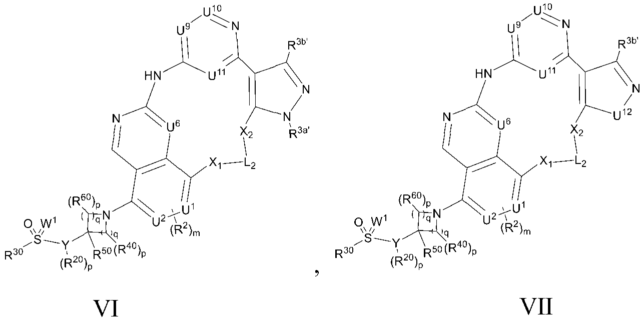
wherein, in Formula II, the definitions of A, M, X₁, X₂, L₂, R¹, R², R³, R²⁰, R³⁰, R⁴⁰, R⁵⁰, R⁶⁰, W¹, Y, m, n, t, p, U¹, U², U³, U⁴, U⁵, U⁶, and U⁷ are as defined in any one of claims 1 to 9;
wherein, in Formula III, the definitions of A, M, X₁, X₂, L₂, R¹, R², R³, R²⁰, R³⁰, R⁴⁰, R⁵⁰, R⁶⁰, W¹ Y, m, n, t, p, U¹, U², or U⁶ are as defined in any one of claims 1 to 9;
wherein, in Formula IV, the definitions of X₁, X₂, L₂, R¹, R², R⁵, R²⁰, R³⁰ R⁴⁰, R⁵⁰, R⁶⁰, W¹, m, p, U¹, U², or U⁶ are as defined in any one of claims 1 to 9;
U⁸ is N, CR¹, O, S, or C(=O);
U⁹ is absent or is N, CR¹, O, S, or C(=O);
U¹¹ is N, CR¹, O, S, or C(=O);
wherein, in Formula V, Formula VI, or Formula VII, the definitions of Y, X₁, X₂, L₂, R¹, R², R²⁰, R³⁰, R⁴⁰, R⁵⁰, R⁶⁰, W¹, m, p, U¹, U², or U⁶ are as defined in any one of claims 1 to 9;
U⁹ is absent or is N, CR¹, O, S, or C(=O);
U¹⁰ is CR¹';
U¹¹ is N, CR¹, O, S, or C(=O);
U¹² is -N(R^{3a}')-, -C(R^{3a}')=C(R^{3a}')-;
q is 1, 2, or 3;
R¹' is hydrogen, deuterium, halogen, CN, NO₂, C₁₋₆ alkyl, halo C₁₋₆ alkyl, C₁₋₆ alkoxy, C₂₋₆ alkenyl, C₂₋₆ alkynyl, 3-12 membered cycloalkyl, 3-12 membered heterocyclyl, 6-10 membered aryl, or 5-10 membered heteroaryl, the C₁₋₆ alkyl, halo C₁₋₆ alkyl, C₁₋₆ alkoxy, C₂₋₆ alkenyl, C₂₋₆ alkynyl, 3-12 membered cycloalkyl, 3-12 membered heterocyclyl, 6-10 membered aryl, or 5-10 membered heteroaryl is optionally substituted with one or more R';
preferably, R¹' is hydrogen, deuterium, fluorine, chlorine, bromine, CN, NO₂, hydroxyl, hydroxyethyl, amino, methylamino, dimethylamino, ethylamino, diethylamino, cyclopropylamino, methyl, ethyl, isopropyl, vinyl, ethynyl, trideuteromethyl, difluoromethyl, trifluoromethyl, difluoroethyl, trifluoroethyl, cyclopropyl, methoxy, ethoxy, cyclopropoxy, or oxo;
wherein, in Formula IV, Formula V, Formula VI, or Formula VII, R^{3a}' is hydrogen, deuterium, C₁₋₆ alkyl, C₂₋₆ alkenyl, C₂₋₆ alkynyl, C₀₋₆ alkyl-3-12 membered cycloalkyl, C₀₋₆ alkyl-3-12 membered heterocyclyl, or OR^{e1}, the C₁₋₆ alkyl, C₂₋₆ alkenyl, C₂₋₆ alkynyl, C₀₋₆ alkyl-3-12 membered cycloalkyl, or C₀₋₆ alkyl-3-12 membered heterocyclyl is optionally substituted with one or more R';
preferably, R^{3a}' is hydrogen, deuterium, methyl, ethyl, propyl, trideuteromethyl, difluoromethyl, trifluoromethyl, difluoroethyl, trifluoroethyl, vinyl, ethynyl, cyclopropyl, cyclobutyl, oxiranyl, oxetanyl, aziridinyl, or azetidinyl, the methyl, ethyl, propyl, vinyl, ethynyl, cyclopropyl, cyclobutyl, oxiranyl, oxetanyl, aziridinyl, or azetidinyl is optionally substituted with one or more groups selected from: deuterium, fluorine, chlorine, bromine, CN, methyl, ethyl, isopropyl, cyclopropyl, cyclobutyl, and oxo;
preferably, R^{3b}' is hydrogen, deuterium, halogen, CN, NO₂, C₁₋₆ alkyl, C₂₋₆ alkenyl, C₂₋₆ alkynyl, C₀₋₆ alkyl-3-12 membered cycloalkyl, or C₀₋₆ alkyl-3-12 membered heterocyclyl, the C₁₋₆ alkyl, C₂₋₆ alkenyl, C₂₋₆ alkynyl, C₀₋₆ alkyl-3-12 membered cycloalkyl, or C₀₋₆ alkyl-3-12 membered heterocyclyl is optionally substituted with one or more R';
preferably, R^{3b}' is hydrogen, deuterium, fluorine, chlorine, bromine, CN, NO₂, methyl, ethyl, propyl, trideuteromethyl, difluoromethyl, trifluoromethyl, difluoroethyl, trifluoroethyl, vinyl, ethynyl, cyclopropyl, cyclobutyl, oxiranyl, oxetanyl, aziridinyl, or azetidinyl, the methyl, ethyl, propyl, vinyl, ethynyl, cyclopropyl, cyclobutyl, oxiranyl, oxetanyl, aziridinyl, or azetidinyl is optionally substituted with one or more groups selected from: deuterium, fluorine, chlorine, bromine, CN, methyl, ethyl, isopropyl, cyclopropyl, cyclobutyl, and oxo.

9. The amino heteroaryl macrocyclic compound or a pharmaceutically acceptable salt, stereoisomer, racemate, tautomer, isotope-labeled derivative, deuterated derivative, N-oxide, prodrug, hydrate, or solvate thereof according to any one of claims 1 to 8, wherein the compound represented by any one of Formulas I to VII is selected from the following compounds:

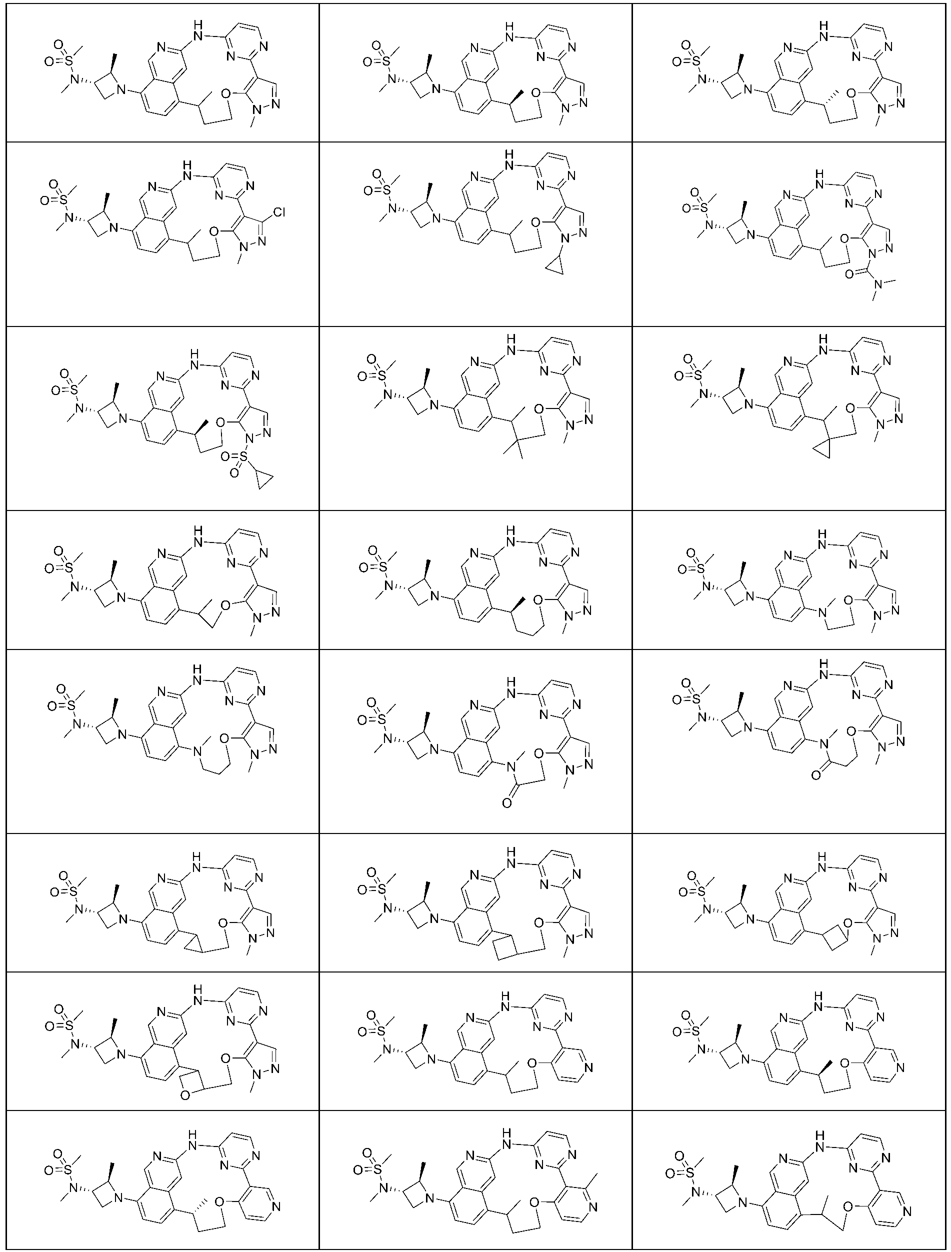

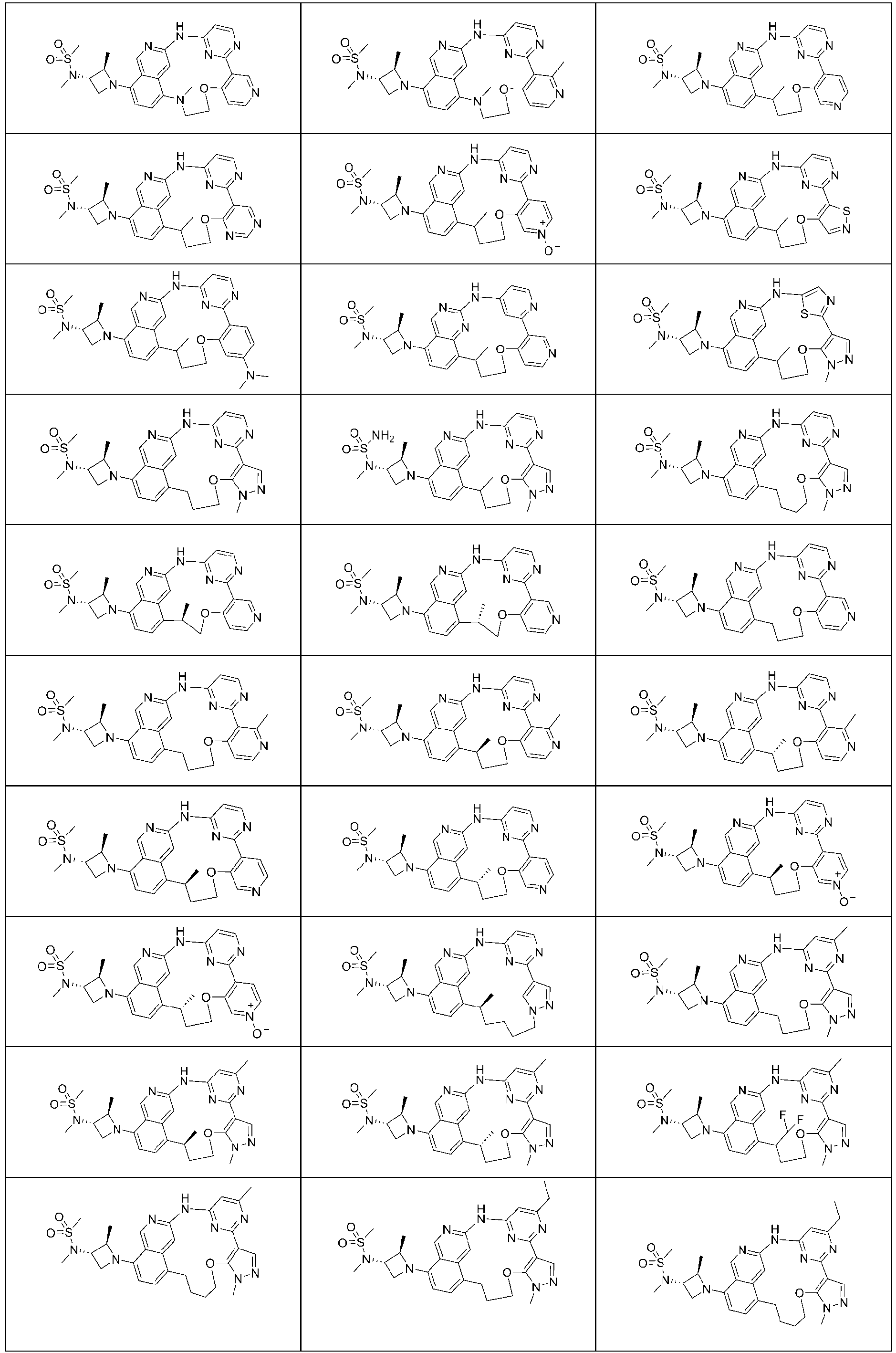

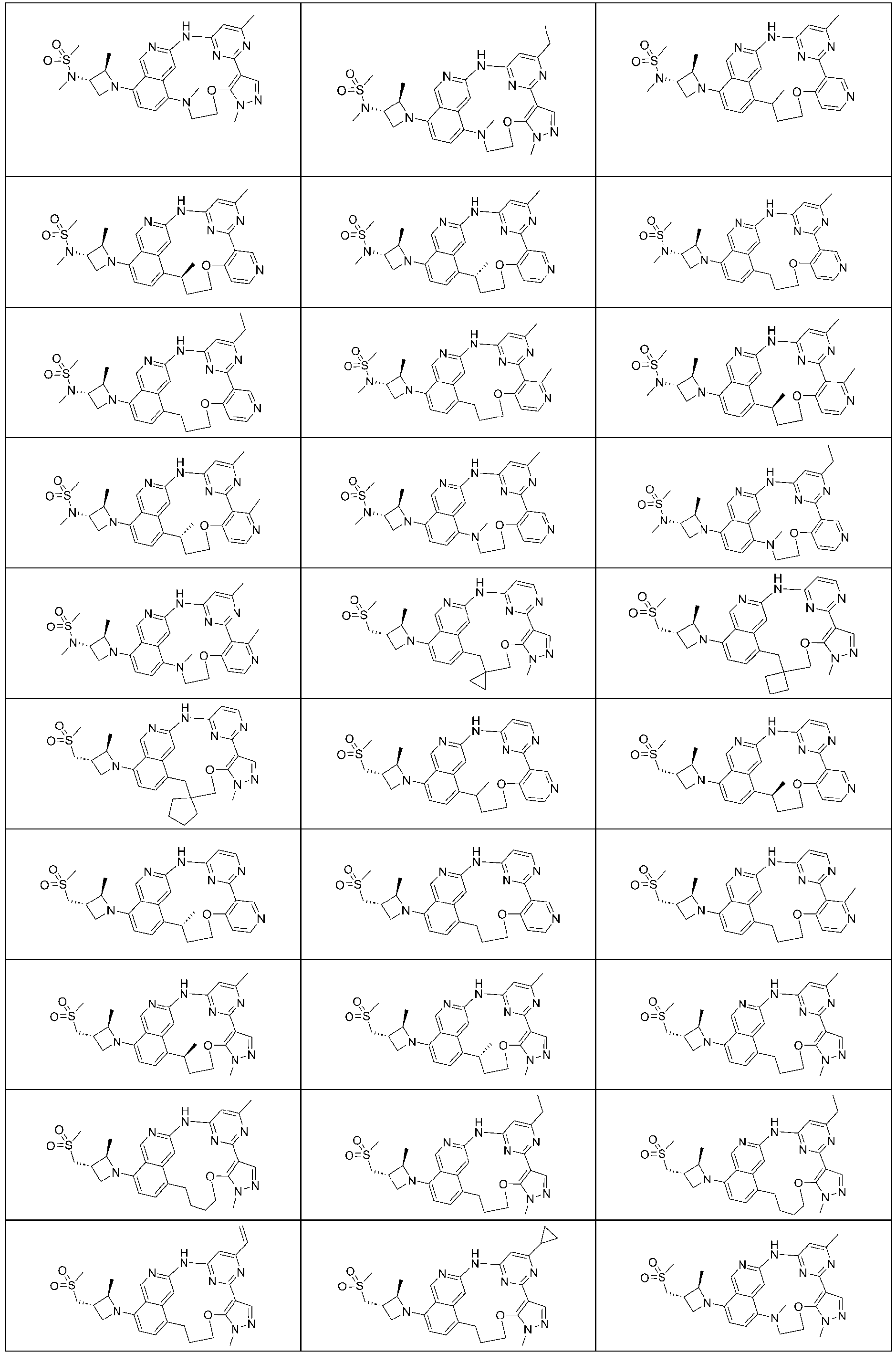

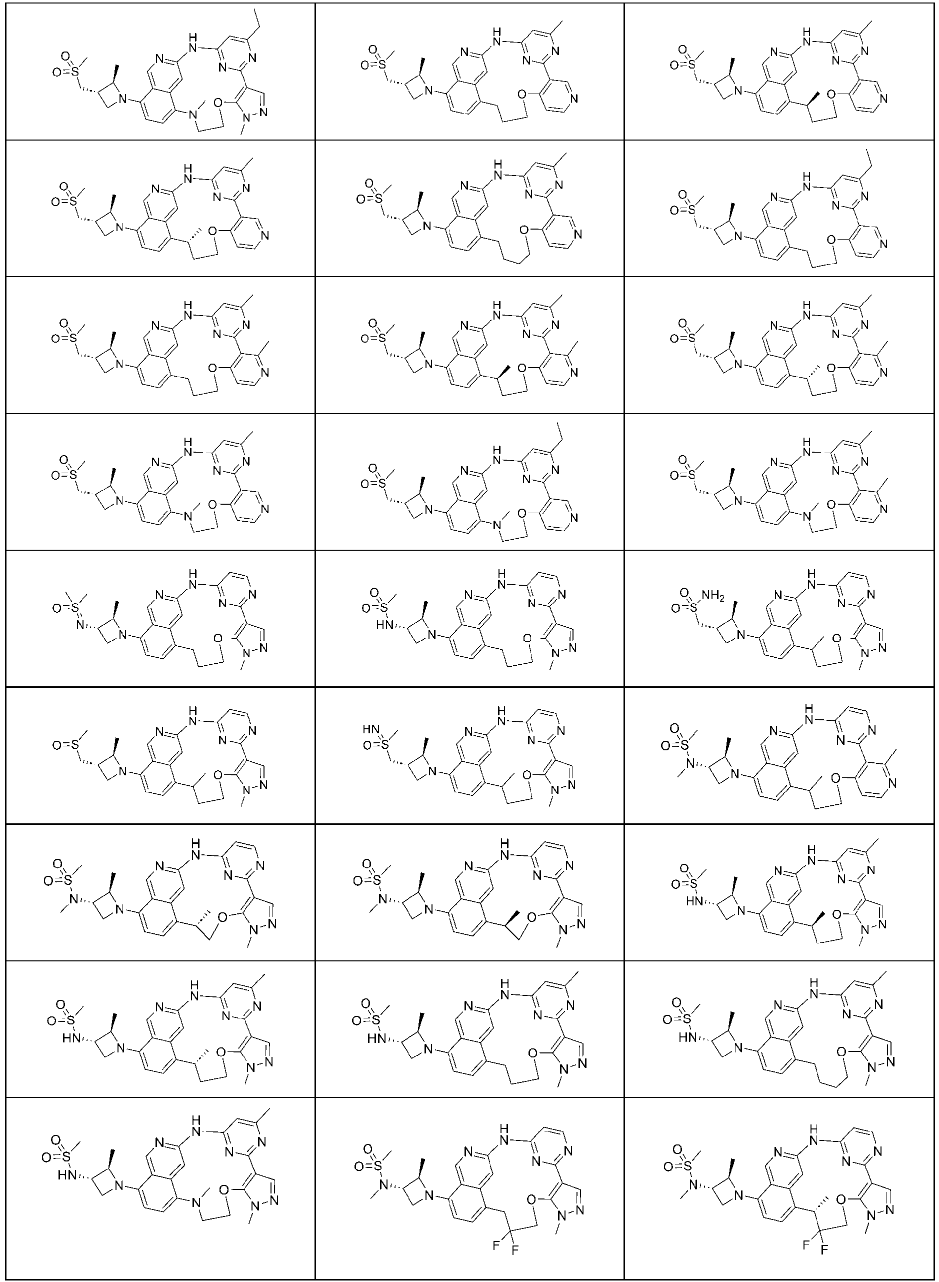

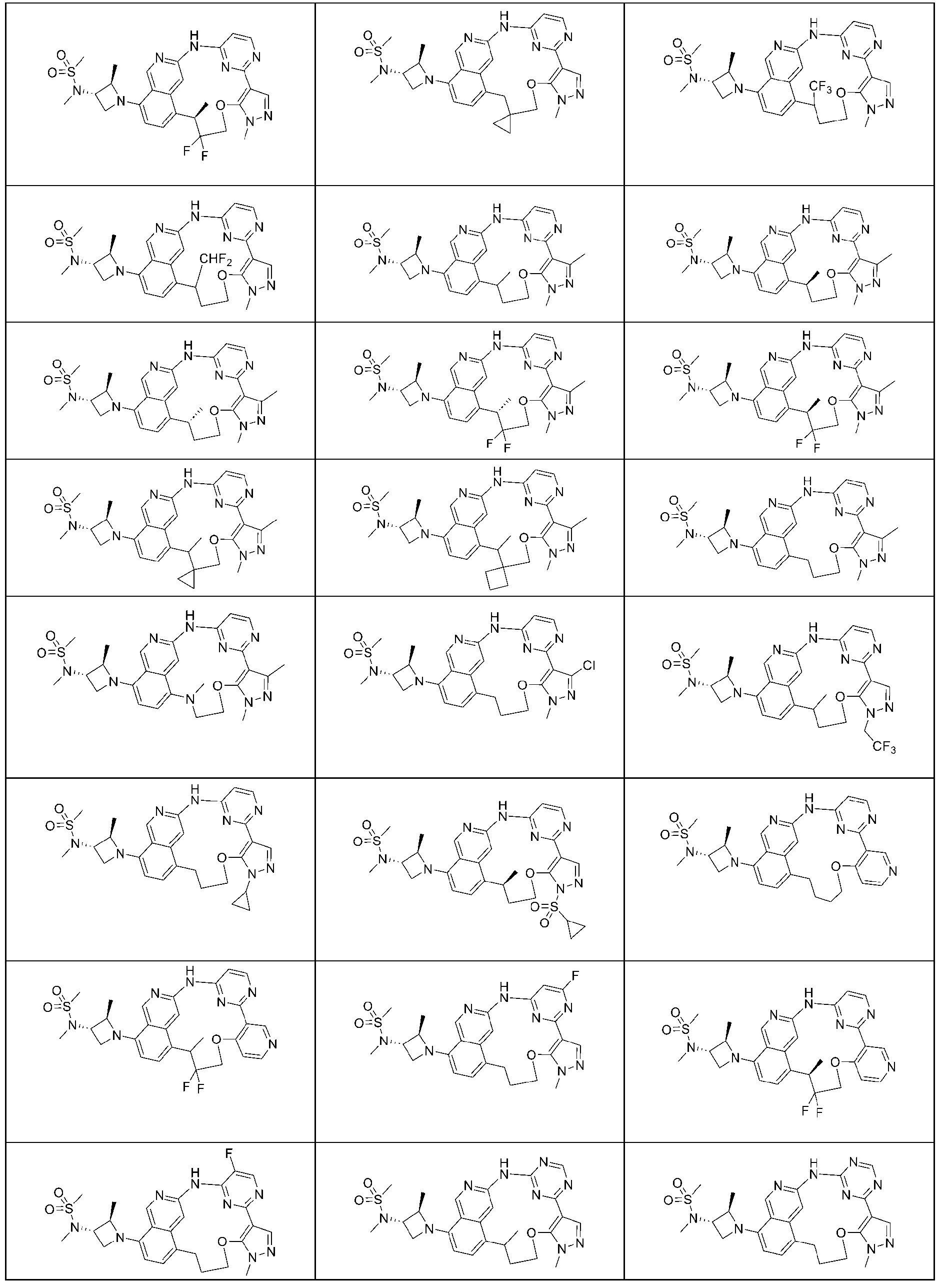

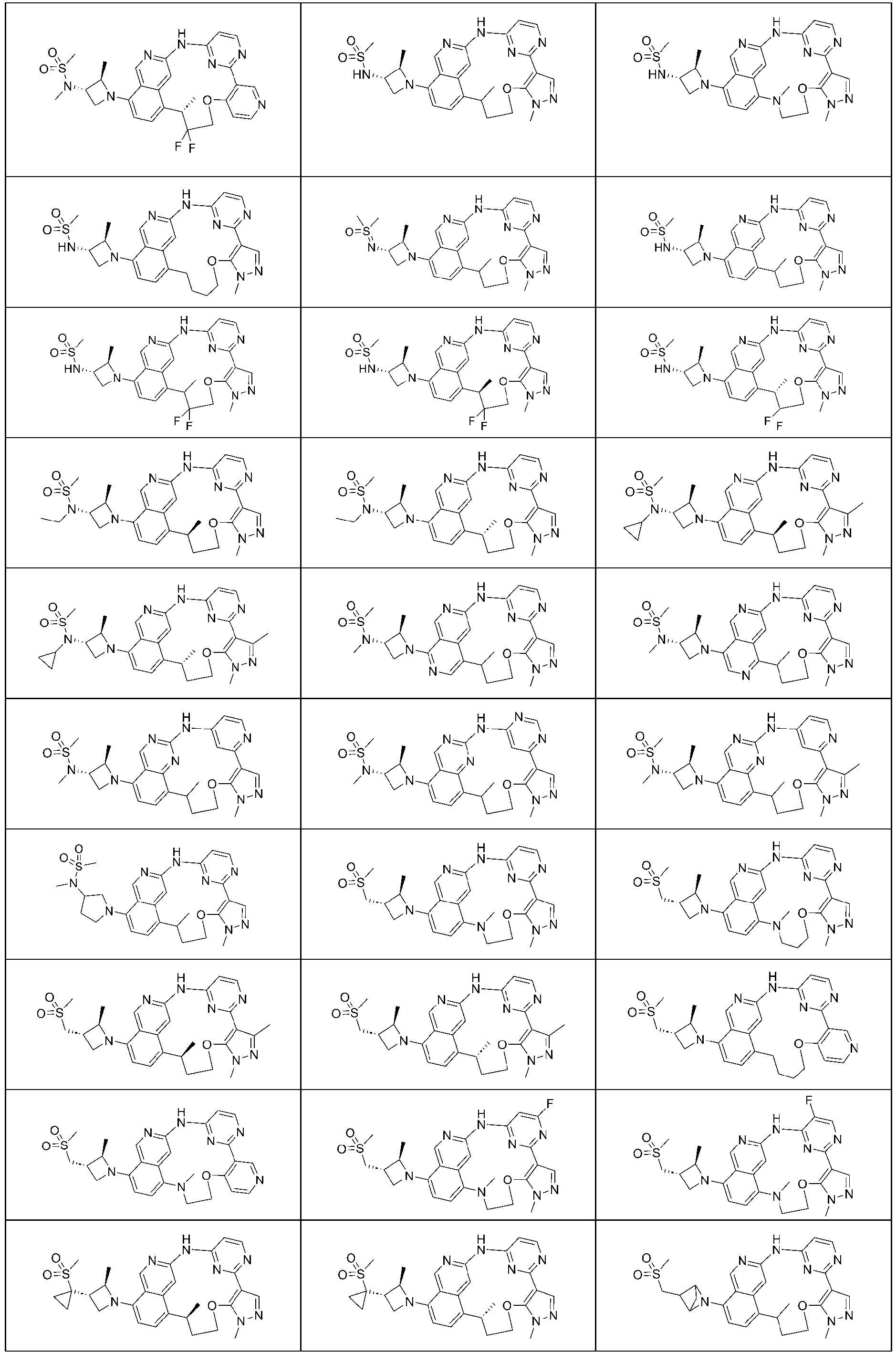

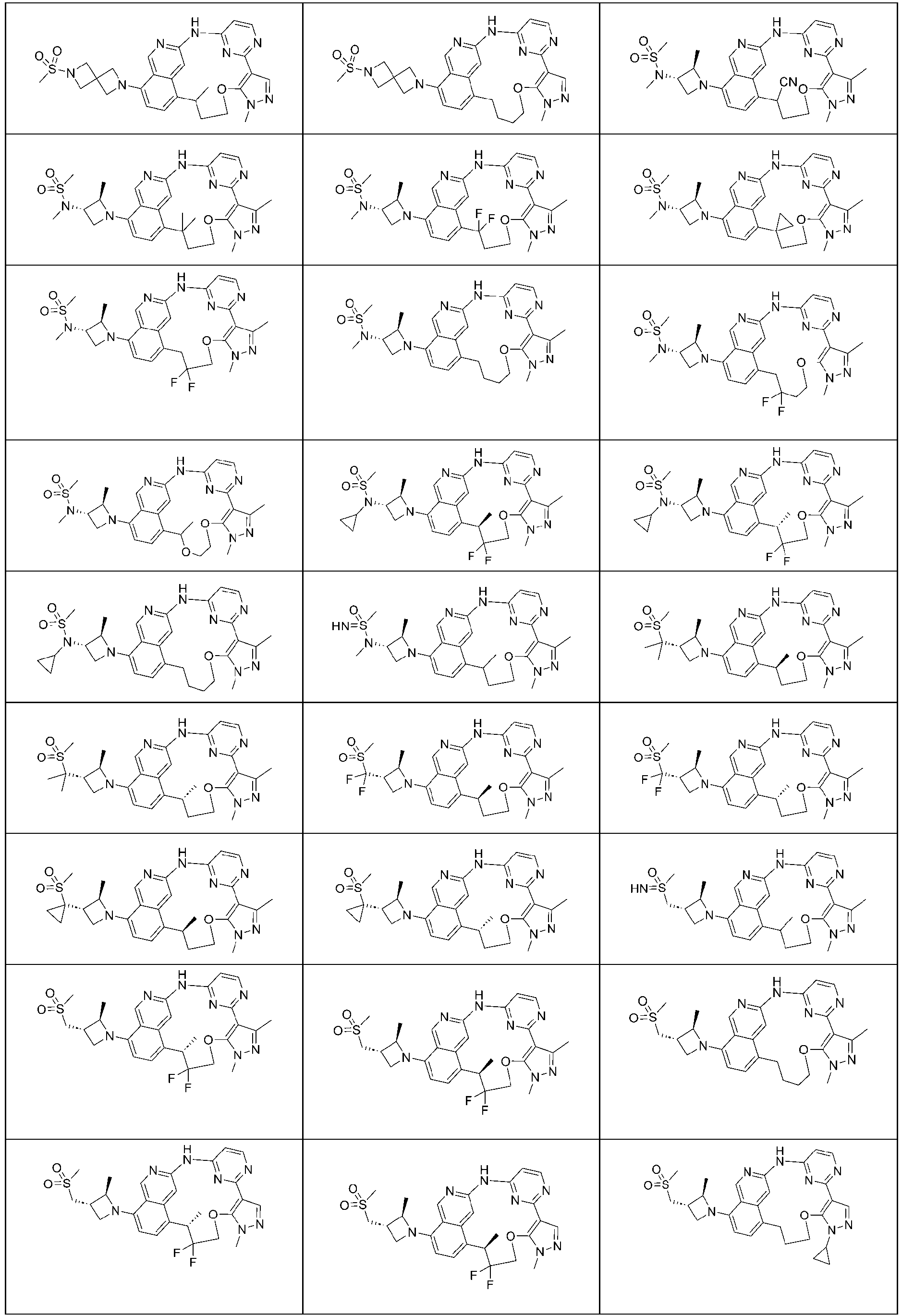

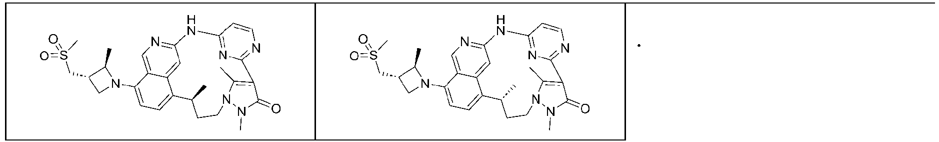

10. A pharmaceutical composition comprising the amino heteroaryl macrocyclic compound or a pharmaceutically acceptable salt, stereoisomer, racemate, tautomer, isotope-labeled derivative, deuterated derivative, N-oxide, prodrug, hydrate, or solvate thereof according to any one of claims 1 to 9, and a pharmaceutically acceptable carrier or excipient;
preferably, the pharmaceutical composition is a tablet, capsule, pill, granule, powder, suppository, injection, solution, suspension, ointment, patch, lotion, drop, liniment, or spray.

11. Use of the amino heteroaryl macrocyclic compound or a pharmaceutically acceptable salt, stereoisomer, racemate, tautomer, isotope-labeled derivative, deuterated derivative, N-oxide, prodrug, hydrate, or solvate thereof according to any one of claims 1 to 9, and/or the pharmaceutical composition according to claim 10, in the preparation of a medicament for the treatment of a disease mediated by EGFR abnormality;
preferably, the disease is a neoplastic disease;
more preferably, the neoplastic disease includes: head and neck cancer, nasopharyngeal carcinoma, melanoma, bladder cancer, esophageal cancer, renal cancer, breast cancer, colorectal cancer, ovarian cancer, cervical cancer, pancreatic cancer, glioma, prostate cancer, leukemia, lymphoma, gastric cancer, lung cancer, liver cancer, gastrointestinal stromal tumor, thyroid cancer, squamous cell carcinoma, cholangiocarcinoma, endometrial cancer, multiple myeloma, mesothelioma, atherosclerosis, or pulmonary fibrosis.

12. A method for treating a disease mediated by EGFR abnormality, the method comprising administering to a patient in need thereof the amino heteroaryl macrocyclic compound or a pharmaceutically acceptable salt, stereoisomer, racemate, tautomer, isotope-labeled derivative, deuterated derivative, N-oxide, prodrug, hydrate, or solvate thereof according to any one of claims 1 to 9, and/or the pharmaceutical composition according to claim 10;
preferably, the patient is preferably a mammal, and the mammal is preferably a human;
preferably, the disease is a neoplastic disease;
more preferably, the neoplastic disease includes: head and neck cancer, nasopharyngeal carcinoma, melanoma, bladder cancer, esophageal cancer, renal cancer, breast cancer, colorectal cancer, ovarian cancer, cervical cancer, pancreatic cancer, glioma, prostate cancer, leukemia, lymphoma, gastric cancer, lung cancer, liver cancer, gastrointestinal stromal tumor, thyroid cancer, squamous cell carcinoma, cholangiocarcinoma, endometrial cancer, multiple myeloma, mesothelioma, atherosclerosis, or pulmonary fibrosis.
